# EUROPEAN PATENT APPLICATION

(11) **EP 1 975 184 A2**
(43) Date of publication of application: **01.10.2008**
(21) Application number: 08102942.3
(22) Date of filing: 26.03.2008
(51) Int. Cl.: C07K 16/44, G01N 33/68

(54) **Serine or threonine phosphorylation sites**

(30) Priority: 26.03.2007 US 920026 P
(71) Applicant: Moritz, Albrecht, Salem, MA 01970 (US); Farnsworth, Charles, Concord, MA 01742 (US); Stokes, Matthew, Beverly, MA 01915 (US); Possemato, Anthony, Framingham, MA 01702 (US)
(72) Inventor: Moritz, Albrecht, Salem, MA 01970 (US); Farnsworth, Charles, Concord, MA 01742 (US); Stokes, Matthew, Beverly, MA 01915 (US); Possemato, Anthony, Framingham, MA 01702 (US)
(74) Representative: Hill, Justin John

(57) **Abstract**

The invention discloses 345 novel phosphorylation sites identified in carcinoma and leukemia, peptides (including AQUA peptides) comprising a phosphorylation site of the invention, antibodies specifically bind to a novel phosphorylation site of the invention, and diagnostic and therapeutic uses of the above.

## Description

### RELATED APPLICATIONS

Pursuant to 35 U.S.C. § 119(e) this application claims the benefit of, and priority to, provisional application U.S.S.N. 60/920,026, filed March 26, 2007, the disclosure of which is incorporated herein, in its entirety, by reference.

### FIELD OF THE INVENTION

The invention relates generally to novel serine and threonine phosphorylation sites, methods and compositions for detecting, quantitating and modulating same.

### BACKGROUND OF THE INVENTION

The activation of proteins by post-translational modification is an important cellular mechanism for regulating most aspects of biological organization and control, including growth, development, homeostasis, and cellular communication. Protein phosphorylation, for example, plays a critical role in the etiology of many pathological conditions and diseases, including to mention but a few: cancer, developmental disorders, autoimmune diseases, and diabetes. Yet, in spite of the importance of protein modification, it is not yet well understood at the molecular level, due to the extraordinary complexity of signaling pathways, and the slow development of technology necessary to unravel it.

Protein phosphorylation on a proteome-wide scale is extremely complex as a result of three factors: the large number of modifying proteins, e.g., kinases, encoded in the genome, the much larger number of sites on substrate proteins that are modified by these enzymes, and the dynamic nature of protein expression during growth, development, disease states, and aging. The human genome, for example, encodes over 520 different protein kinases, making them the most abundant class of enzymes known. (Hunter, Nature 411: 355-65 (2001)). Most kinases phosphorylate many different substrate proteins, at distinct tyrosine, serine, and/or threonine residues. Indeed, it is estimated that one-third of all proteins encoded by the human genome are phosphorylated, and many are phosphorylated at multiple sites by different kinases.

Many of these phosphorylation sites regulate critical biological processes and may prove to be important diagnostic or therapeutic targets for molecular medicine. For example, of the more than 100 dominant oncogenes identified to date, 46 are protein kinases. *See* Hunter, *supra.*

Protein kinases are often divided into two groups based on the amino acid residue they phosphorylate. The Ser/Thr kinases, which phosphorylate serine or threonine(Ser, S; Thr, T) residues, include cyclic AMP(cAMP-) and cGMP-dependent protein kinases, calcium- and phospholipid-dependent protein kinase C, calmodulin dependent protein kinases, casein kinases, cell division cycle (CDC) protein kinases, and others. These kinases are usually cytoplasmic or associated with the particulate fractions of cells, possibly by anchoring proteins. The second group of kinases, which phosphorylate Tyrosine (Tyr, T) residues, are present in much smaller quantities, but play an equally important role in cell regulation. These kinases include several receptors for molecules such as growth factors and hormones, including epidermal growth factor receptor, insulin receptor, platelet-derived growth factor receptor, and others. Some Ser/Thr kinases are known to be downstream to tyrosine kinases in cell signaling pathways.

Many of the protein kinases and their phosphorylated substrates regulate critical biological processes and may prove to be important diagnostic or therapeutic targets for molecular medicine. For example, of the more than 100 dominant oncogenes identified to date, 46 are protein kinases. *See* Hunter, *supra.* Understanding which proteins are modified by these kinases will greatly expand our understanding of the molecular mechanisms underlying oncogenic transformation. Therefore, the identification of, and ability to detect, phosphorylation sites on a wide variety of cellular proteins is crucially important to understanding the key signaling proteins and pathways implicated in the progression of diseases like cancer.

Carcinoma is one of the two main categories of cancer, and is generally characterized by the formation of malignant tumors or cells of epithelial tissue original, such as skin, digestive tract, glands, etc. Carcinomas are malignant by definition, and tend to metastasize to other areas of the body. The most common forms of carcinoma are skin cancer, lung cancer, breast cancer, and colon cancer, as well as other numerous but less prevalent carcinomas. Current estimates show that, collectively, various carcinomas will account for approximately 1.65 million cancer diagnoses in the United States alone, and more than 300,000 people will die from some type of carcinoma during 2005. (Source: American Cancer Society (2005)). The worldwide incidence of carcinoma is much higher.

It has been shown that a number of Ser/Thr kinase family members are involved in tumor growth or cellular transformation by either increasing cellular proliferation or decreasing the rate of apoptosis. For example, the mitogen-activated protein kinases (MAPKs) are Ser/Thr kinases which act as intermediates within the signaling cascades of both growth/survival factors, such as EGF, and death receptors, such as the TNF receptor. Expression of Ser/Thr kinases, such as protein kinase A, protein kinase B and protein kinase C, have been shown be elevated in some tumor cells. Further, cyclin dependent kinases (cdk) are Ser/Thr kinases that play an important role in cell cycle regulation. Increased expression or activation of these kinases may cause uncontrolled cell proliferation leading to tumor growth. (See Cross et al., Exp. Cell Res. 256: 34-41,2000).

Leukemia, another form of cancer in which a number of underlying signal transduction events have been elucidated, has become a disease model for phosphoproteomic research and development efforts. As such, it represent a paradigm leading the way for many other programs seeking to address many classes of diseases (See, Harrison's Principles of Internal Medicine, McGraw-Hill, New York, N.Y.).

Most varieties of leukemia are generally characterized by genetic alterations associated with the etiology of the disease, and it has recently become apparent that, in many instances, such alterations (chromosomal translocations, deletions or point mutations) result in the constitutive activation of protein kinase genes, and their products, particularly tyrosine kinases. The most well known alteration is the oncogenic role of the chimeric BCR-Ab1 gene, which is generated by translocation of chromosome 9 to chromosome 22, creating the so-called Philadelphia chromosome characteristic of CML *(see* Nowell, Science 132: 1497 (1960)). The resulting BCR-Abl kinase protein is constitutively active and elicits characteristic signaling pathways that have been shown to drive the proliferation and survival of CML cells *(see* Daley, Science 247: 824-830 (1990); Raitano et al., Biochim. Biophys. Acta. Dec 9;1333(3): F201-16 (1997)). The recent success of Imanitib (also known as STI571 or Gleevec®), the first molecularly targeted compound designed to specifically inhibit the tyrosine kinase activity of BCR-Abl, provided critical confirmation of the central role of BCR-Ab1 signaling in the progression of CML *(see* Schindler et al., Science 289: 1938-1942 (2000); Nardi et al., Curr. Opin. Hematol, 11: 35-43 (2003)).

The success of Gleevec® now serves as a paradigm for the development of targeted drugs designed to block the activity of other tyrosine kinases known to be involved in many diseased including leukemias and other malignancies *(see,* e.g., Sawyers, Curr. Opin. Genet. Dev. Feb; 12(1): I I 1-5 (2002); Druker, Adv. Cancer Res. 91: 1-30 (2004)). For example, recent studies have demonstrated that mutations in the FLT3 gene occur in one third of adult patients with AML. FLT3 (Fms-like tyrosine kinase 3) is a member of the class III receptor tyrosine kinase (RTK) family including FMS, platelet-derived growth factor receptor (PDGFR) and c-KIT (*see* Rosnet et al., Crit. Rev. Oncog. 4: 595-613 (1993). In 20-27% of patients with AML, internal tandem duplication in the juxta-membrane region of FLT3 can be detected (see Yokota et al., Leukemia 11: 1605-1609 (1997)). Another 7% of patients have mutations within the active loop of the second kinase domain, predominantly substitutions of aspartate residue 835 (D835), while additional mutations have been described (see Yamamoto et al., Blood 97: 2434-2439 (2001); Abu-Duhier et al., Br.J. Haematol. 113. 983-988 (2001)). Expression of mutated FLT3 receptors results in constitutive tyrosine phosphorylation of FLT3, and subsequent phosphorylation and activation of downstream molecules such as STATS, Akt and MAPK, resulting in factor-independent growth of hematopoietic cell lines.

Although most of the research effort regarding leukemia to date has been focused on tyrosine kinases, a small of group of serine/ threonine kinases, cyclin dependent kinase (Cdks), Erks, Raf, PI3K, PKB, and Akt, have been identified as major players in cell proliferation, cell division, and anti-apoptotic signaling. Akt/PKB (protein kinase B) kinases mediate signaling pathways downstream of activated tyrosine kinases and phosphatidylinositol 3-kinase. Akt kinases regulate diverse cellular processes including cell proliferation and survival, cell size and response to nutrient availability, tissue invasion and angiogenesis. Many oncoproteins and tumor suppressors implicated in cell signaling/metabolic regulation converge within the Akt signal transduction pathway in an equilibrium that is altered in many human cancers by activating and inactivating mechanisms, respectively, targeting these inter-related proteins.

Despite the identification of a few key signaling molecules involved in cancer and other disease progression are known, the vast majority of signaling protein changes and signaling pathways underlying these disease types remain unknown. Therefore, there is presently an incomplete and inaccurate understanding of how protein activation within signaling pathways drives various diseases including these complex cancers, such as leukemia for example. Accordingly, there is a continuing and pressing need to unravel the molecular mechanisms of disease progression by identifying the downstream signaling proteins mediating cellular transformation in these diseases.

Presently, diagnosis of many diseases including carcinoma and leukemia is made by tissue biopsy and detection of different cell surface markers. However, misdiagnosis can occur since some disease types can be negative for certain markers and because these markers may not indicate which genes or protein kinases may be deregulated. Although the genetic translocations and/or mutations characteristic of a particular form of a disease including cancer can be sometimes detected, it is clear that other downstream effectors of constitutively active signaling molecules having potential diagnostic, predictive, or therapeutic value, remain to be elucidated.

Accordingly, identification of downstream signaling molecules and phosphorylation sites involved in different types of diseases including for example, carcinoma or leukemia and development of new reagents to detect and quantify these sites and proteins may lead to improved diagnostic/prognostic markers, as well as novel drug targets, for the detection and treatment of many diseases.

### SUMMARY OF THE INVENTION

The present invention provides in one aspect novel serine or threoninephosphorylation sites (Table 1) identified in carcinoma and/or leukemia. The novel sites occur in proteins such as: Adaptor/Scaffold proteins, adhesion/extra cellular matrix proteins, cell cycle regulation, chaperone proteins, chromatin or DNA binding/repair/ proteins, cytoskeleton proteins, endoplasmic reticulum or golgi proteins, enzyme proteins, g proteins or regulator proteins, kinases, protein kinases receptor/channel/transporter/cell surface proteins, transcriptional regulators, ubiquitan conjugating proteins, RNA processing proteins, secreted proteins, motor or contractile proteins, apoptosis proteins proteins of unknown function and vesicle proteins.

In another aspect, the invention provides peptides comprising the novel phosphorylation sites of the invention, and proteins and peptides that are mutated to eliminate the novel phosphorylation sites.

In another aspect, the invention provides modulators that modulate serine or threoninephosphorylation at a novel phosphorylation sites of the invention, including small molecules, peptides comprising a novel phosphorylation site, and binding molecules that specifically bind at a novel phosphorylation site, including but not limited to antibodies or antigen-binding fragments thereof.

In another aspect, the invention provides compositions for detecting, quantitating or modulating a novel phosphorylation site of the invention, including peptides comprising a novel phosphorylation site and antibodies or antigen-binding fragments thereof that specifically bind at a novel phosphorylation site. In certain embodiments, the compositions for detecting, quantitating or modulating a novel phosphorylation site of the invention are Heavy-Isotype Labeled Peptides (AQUA peptides) comprising a novel phosphorylation site.

In another aspect, the invention discloses phosphorylation site specific antibodies or antigen-binding fragments thereof. In one embodiment, the antibodies specifically bind to an amino acid sequence comprising a phosphorylation site identified in Table 1 when the serine or threonine identified in Column D is phosphorylated, and do not significantly bind when the serine or threonineis not phosphorylated. In another embodiment, the antibodies specifically bind to an amino acid sequence comprising a phosphorylation site when the serine or threonine is not phosphorylated, and do not significantly bind when the serine or threonine is phosphorylated.

In another aspect, the invention provides a method for making phosphorylation site-specific antibodies.

In another aspect, the invention provides compositions comprising a peptide, protein, or antibody of the invention, including pharmaceutical compositions.

In a further aspect, the invention provides methods of treating or preventing carcinoma and/leukemia in a subject, wherein the carcinoma and/or leukemia is associated with the phosphorylation state of a novel phosphorylation site in Table 1, whether phosphorylated or dephosphorylated. In certain embodiments, the methods comprise administering to a subject a therapeutically effective amount of a peptide comprising a novel phosphorylation site of the invention. In certain embodiments, the methods comprise administering to a subject a therapeutically effective amount of an antibody or antigen-binding fragment thereof that specifically binds at a novel phosphorylation site of the invention.

In a further aspect, the invention provides methods for detecting and quantitating phosphorylation at a novel serine or threoninephosphorylation site of the invention.

In another aspect, the invention provides a method for identifying an agent that modulates a serine or threoninephosphorylation at a novel phosphorylation site of the invention, comprising: contacting a peptide or protein comprising a novel phosphorylation site of the invention with a candidate agent, and determining the phosphorylation state or level at the novel phosphorylation site. A change in the phosphorylation state or level at the specified serine or threoninein the presence of the test agent, as compared to a control, indicates that the candidate agent potentially modulates serine or threoninephosphorylation at a novel phosphorylation site of the invention.

In another aspect, the invention discloses immunoassays for binding, purifying, quantifying and otherwise generally detecting the phosphorylation of a protein or peptide at a novel phosphorylation site of the invention.

Also provided are pharmaceutical compositions and kits comprising one or more antibodies or peptides of the invention and methods of using them.

### BRIEF DESCRIPTION OF THE DRAWINGS

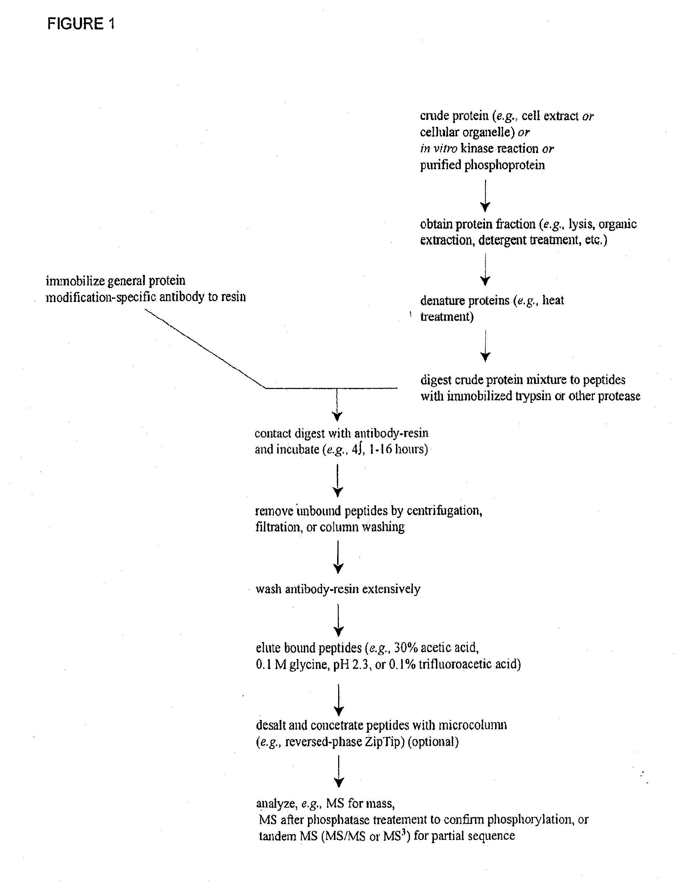

**FIGURE 1** is a diagram depicting the immuno-aftinity isolation and mass-spectrometric characterization methodology (IAP) used in the Examples to identify the novel phosphorylation sites disclosed herein.

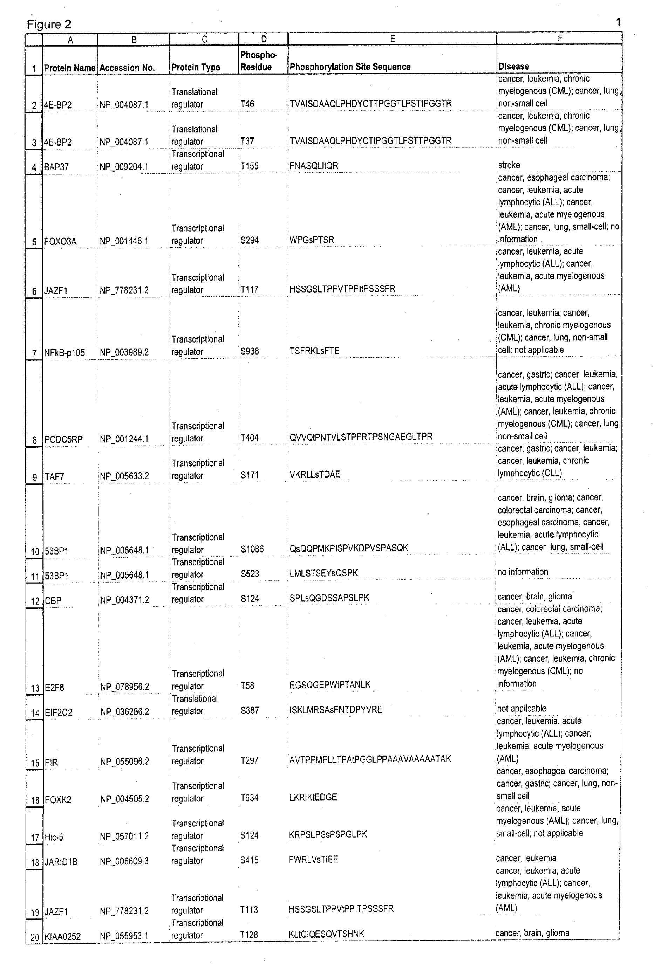

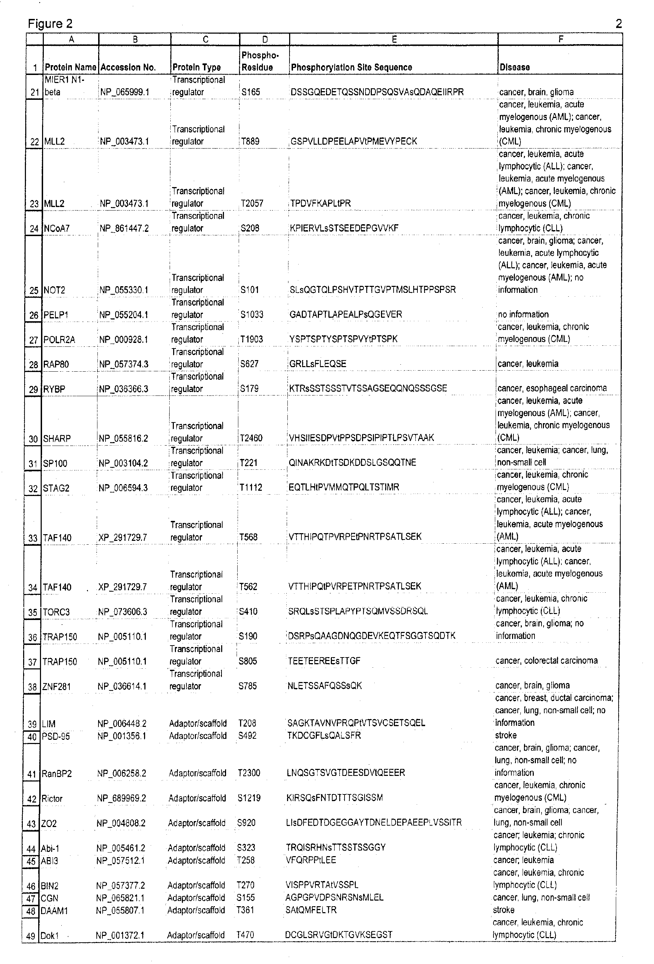

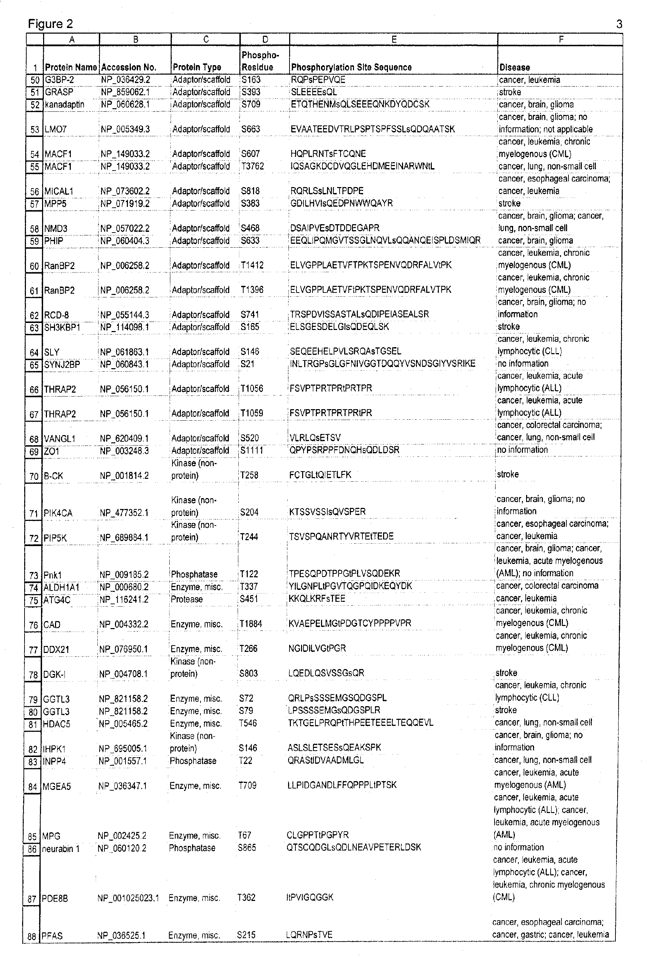

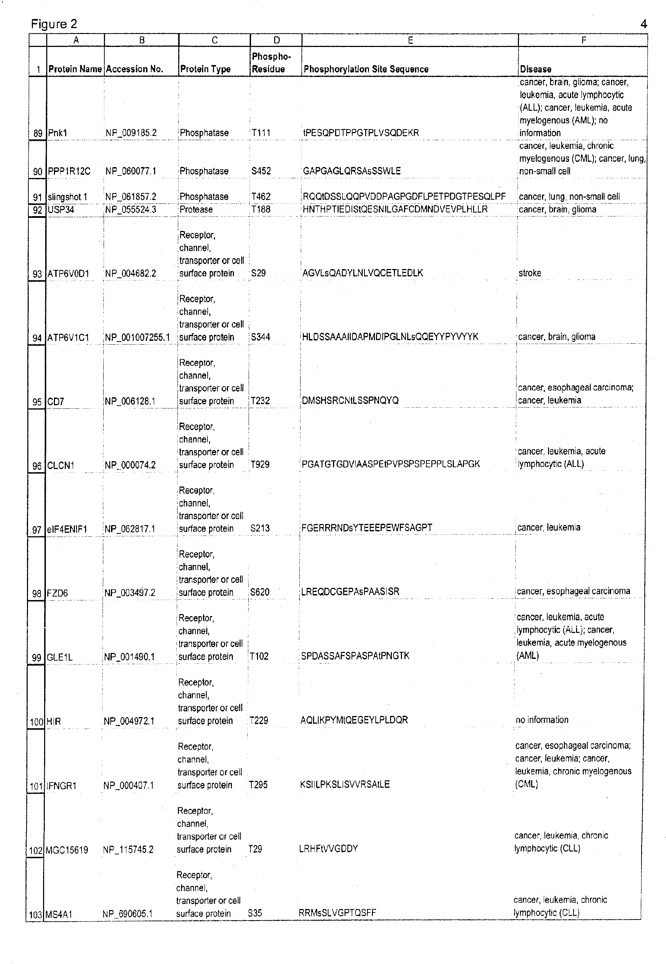

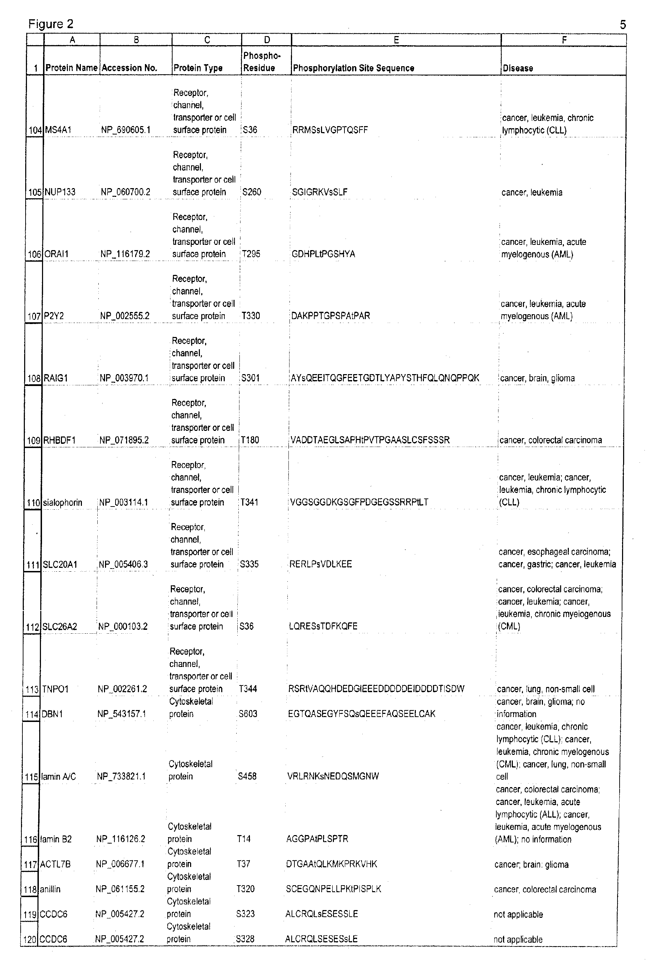

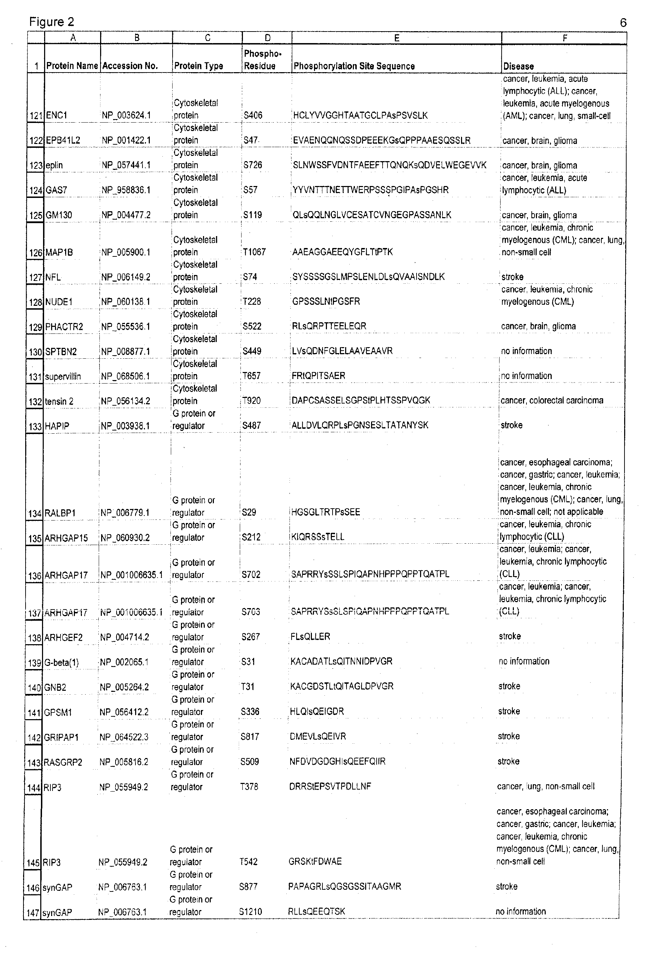

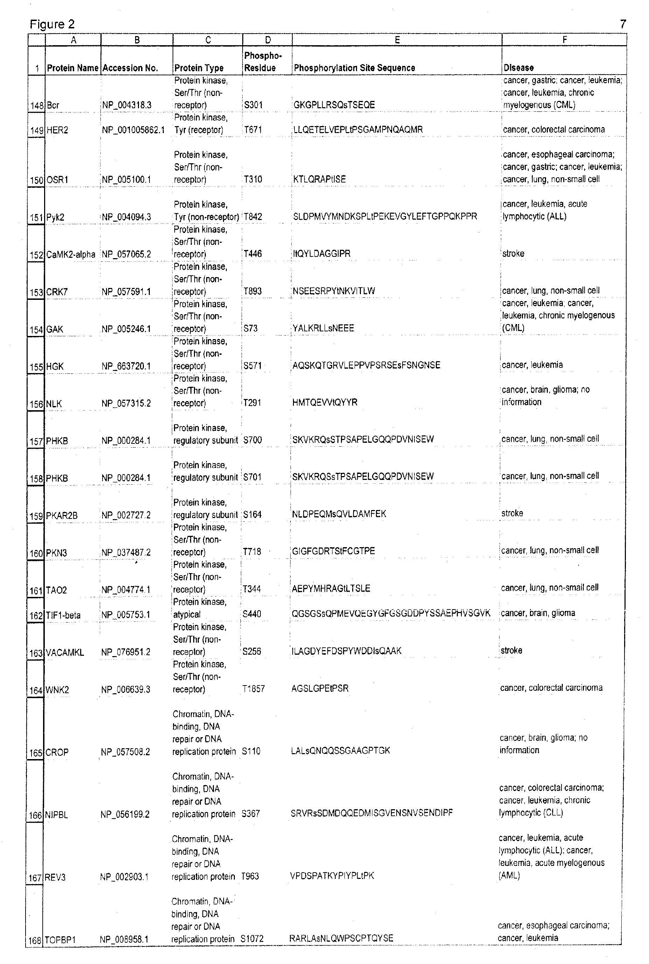

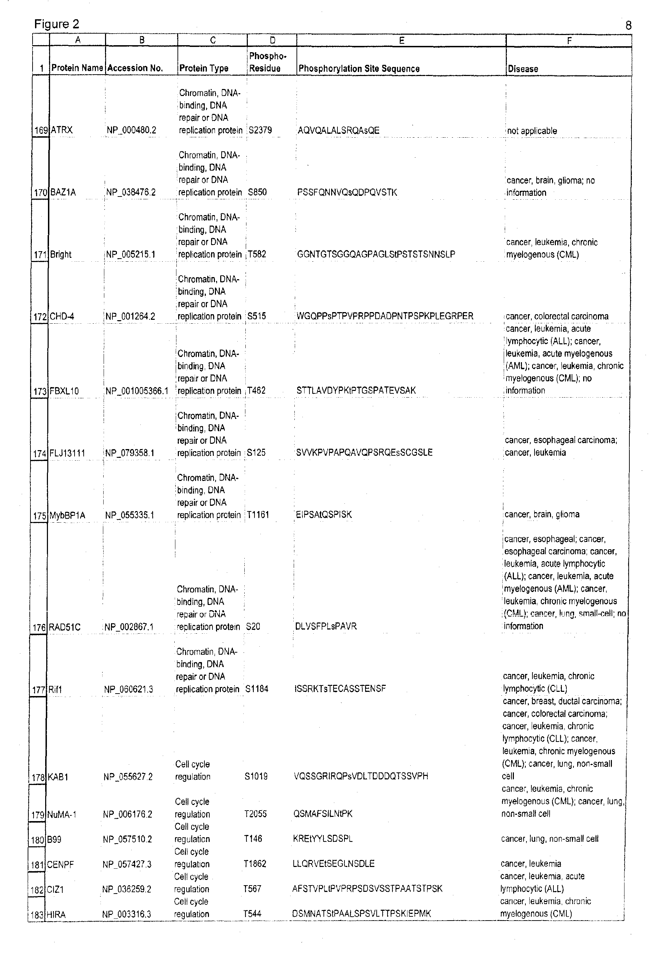

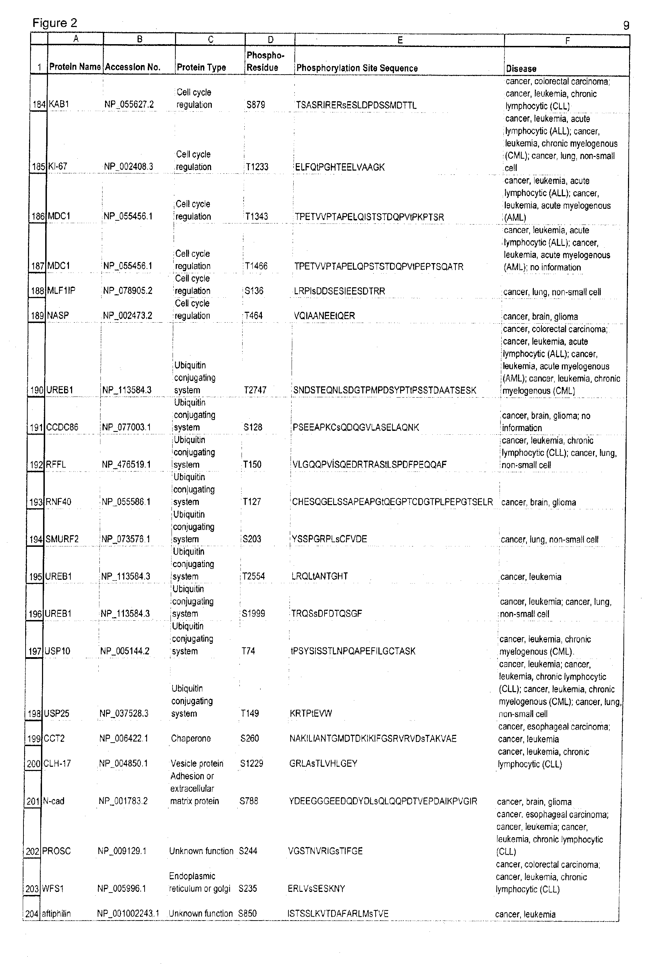

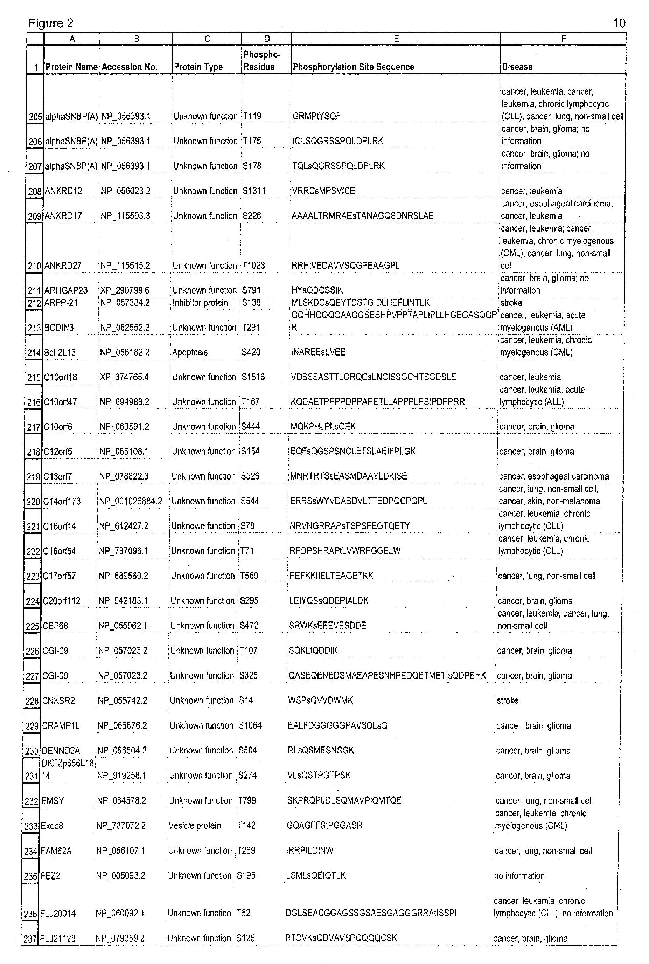

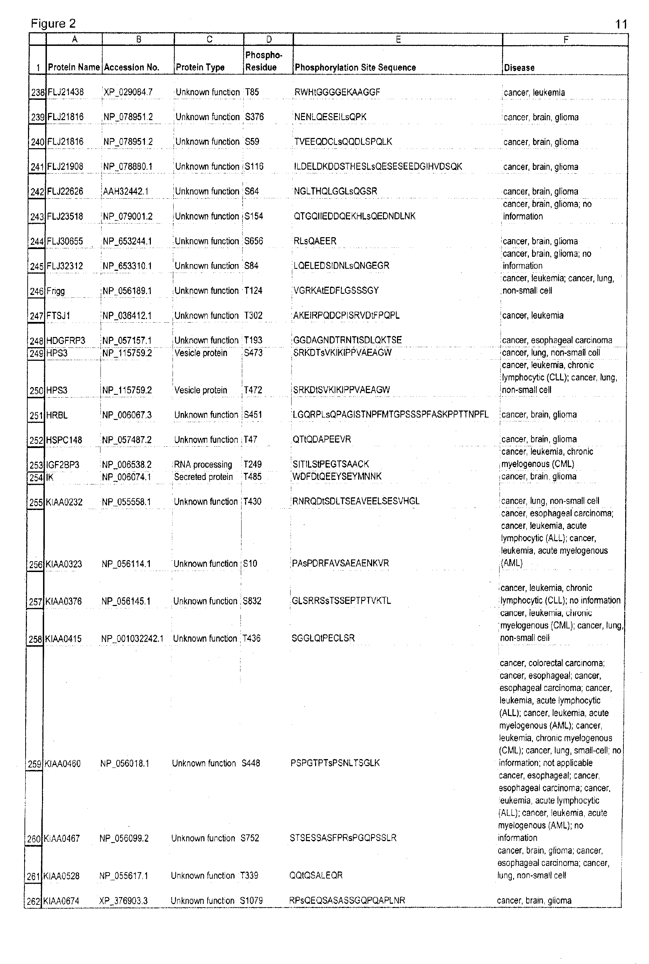

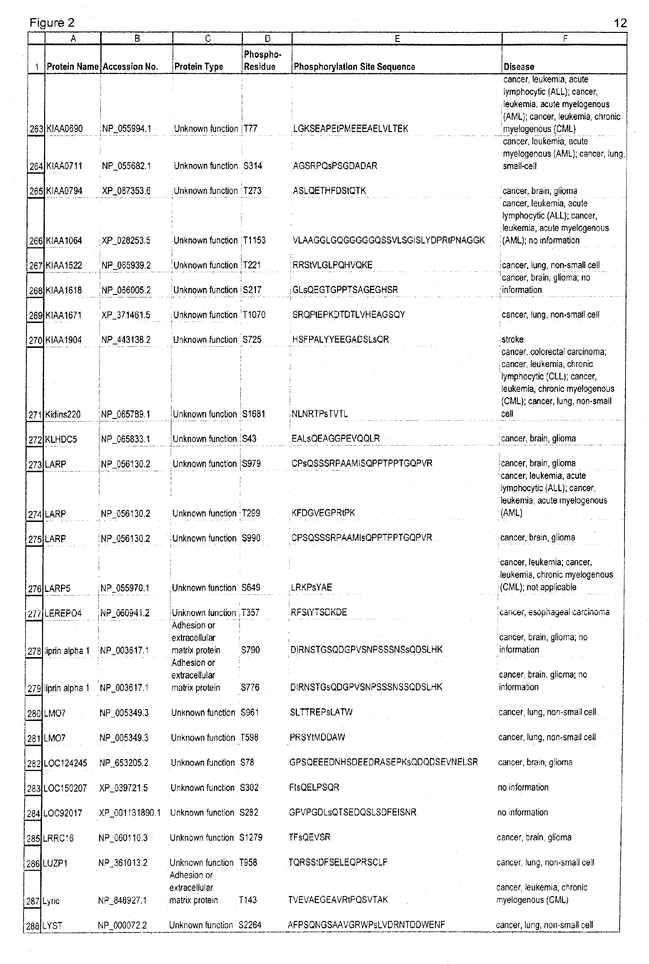

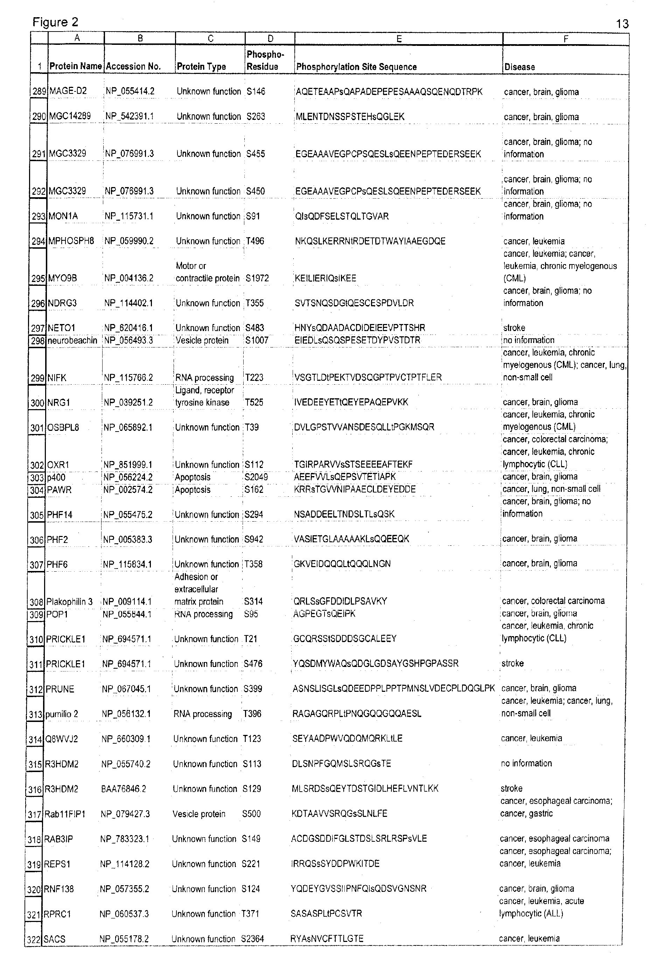

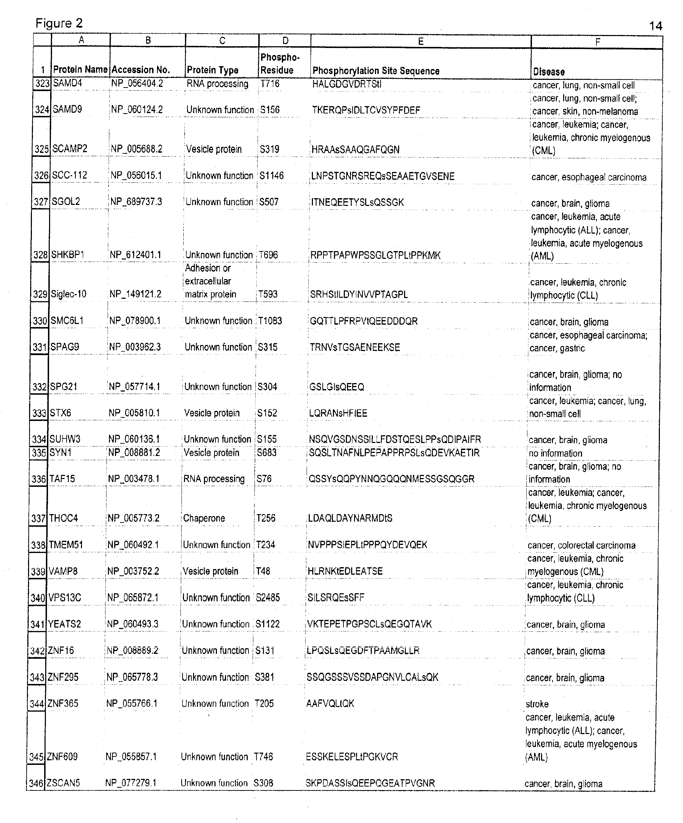

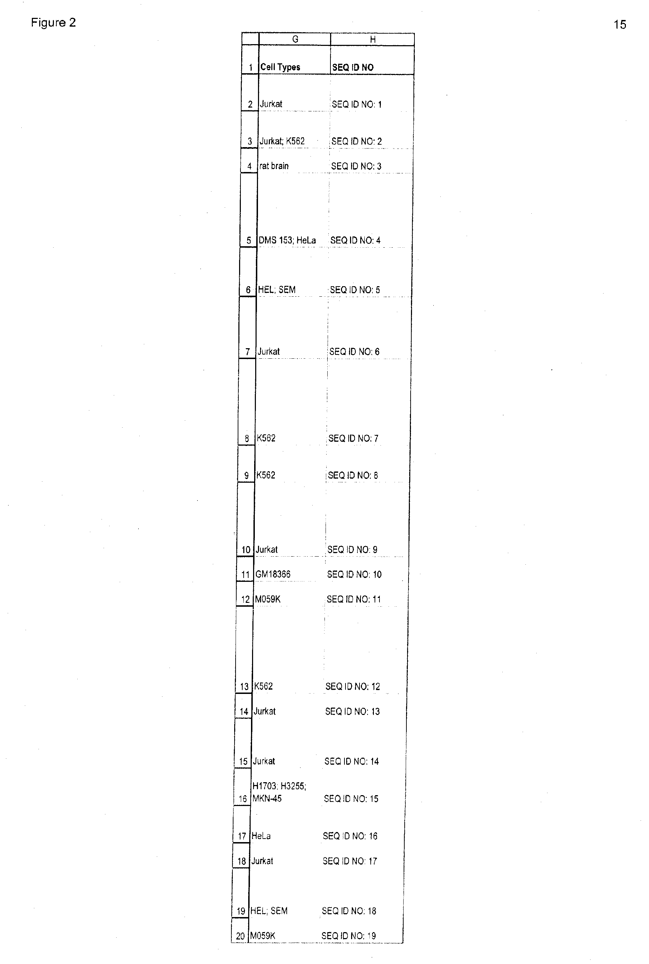

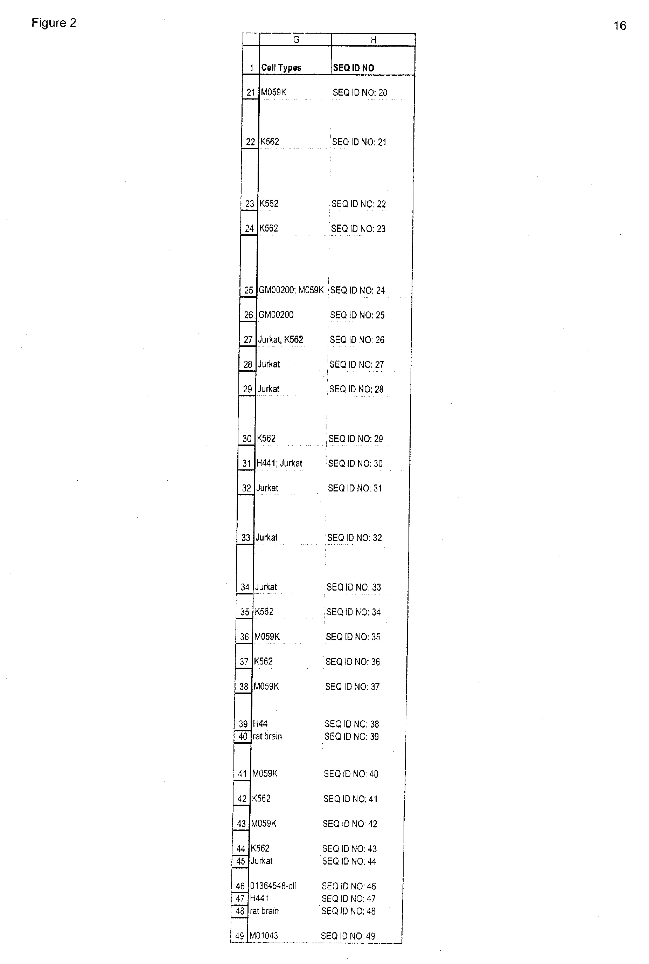

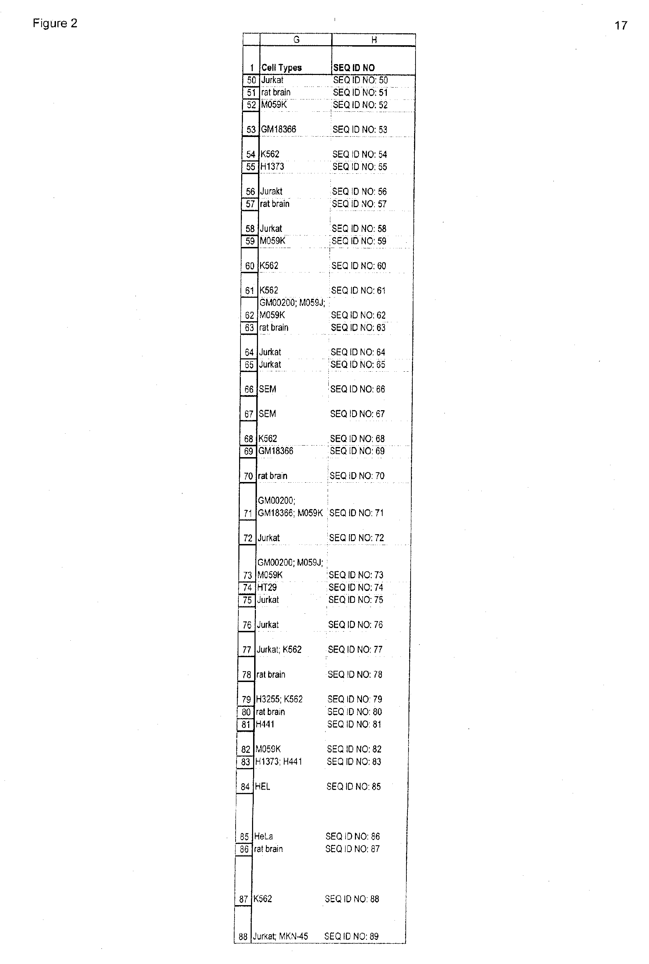

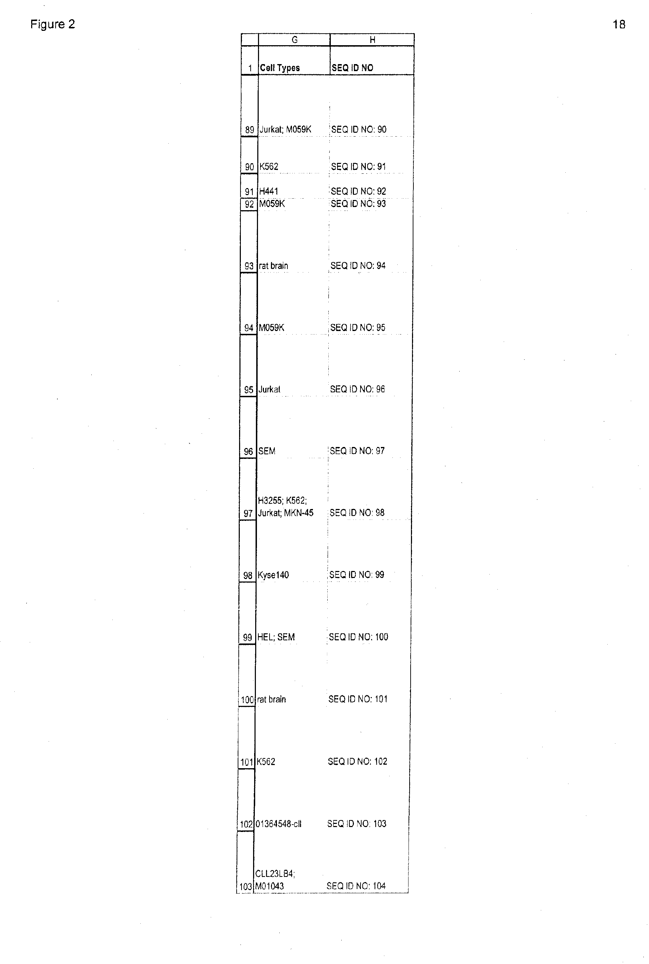

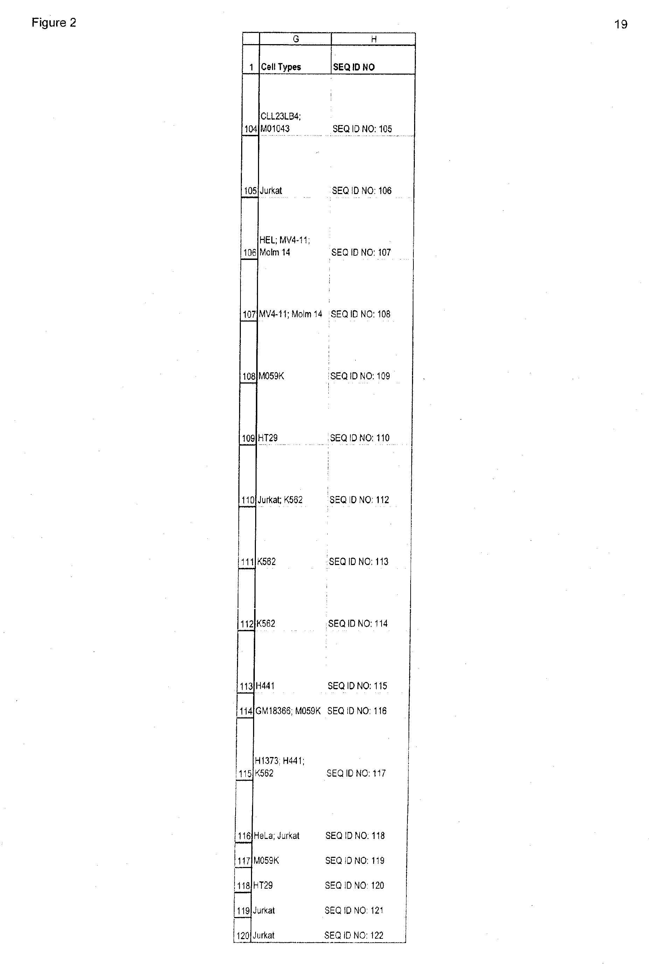

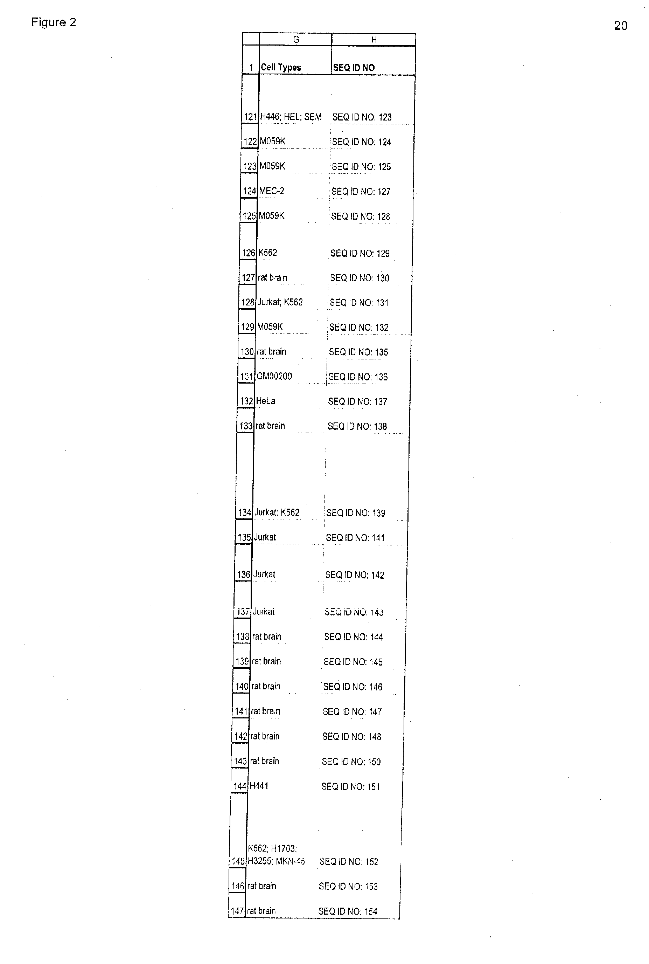

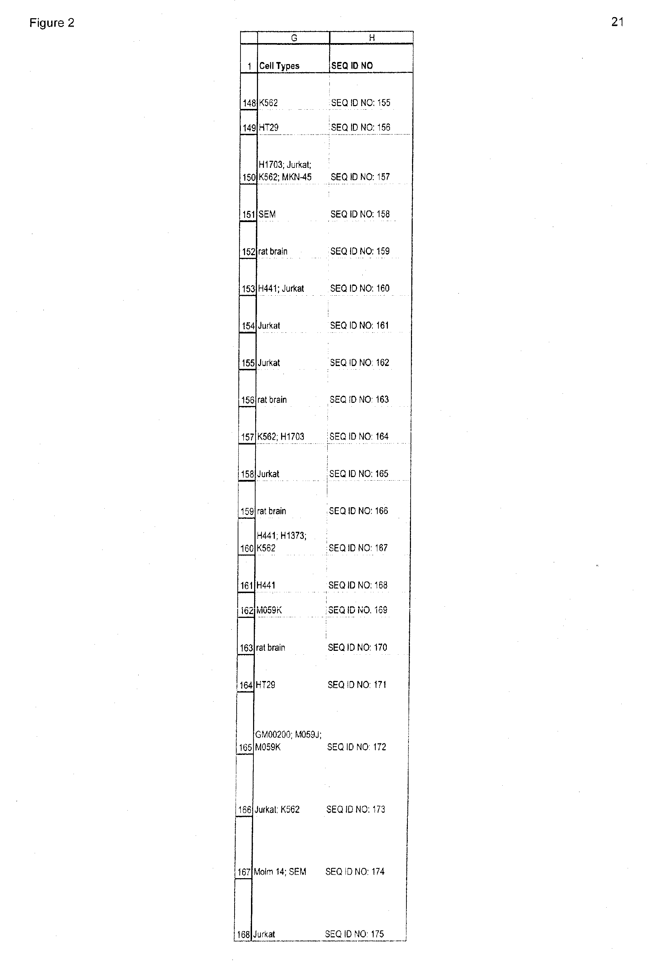

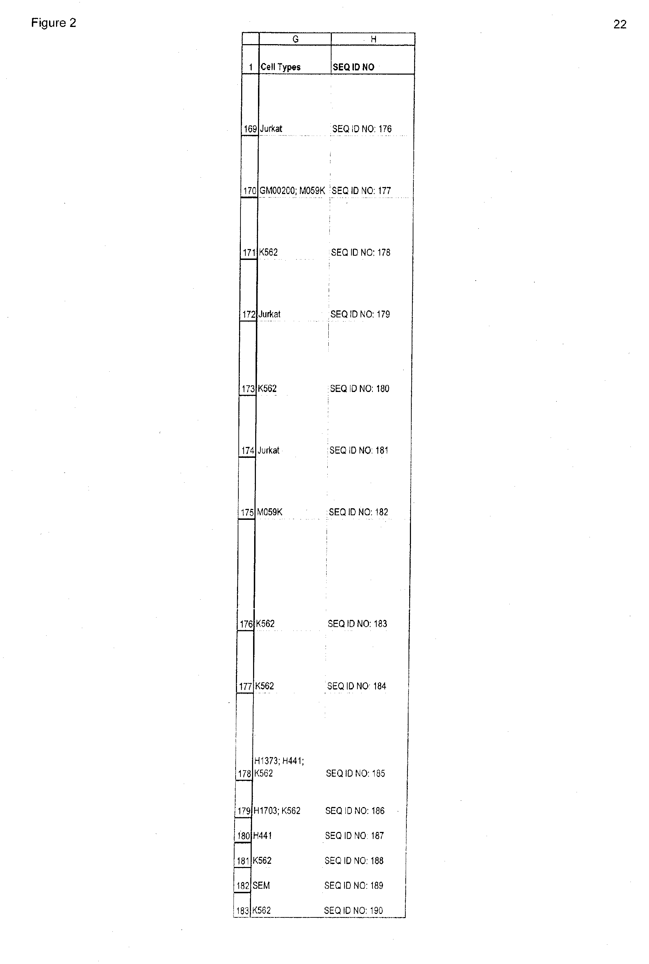

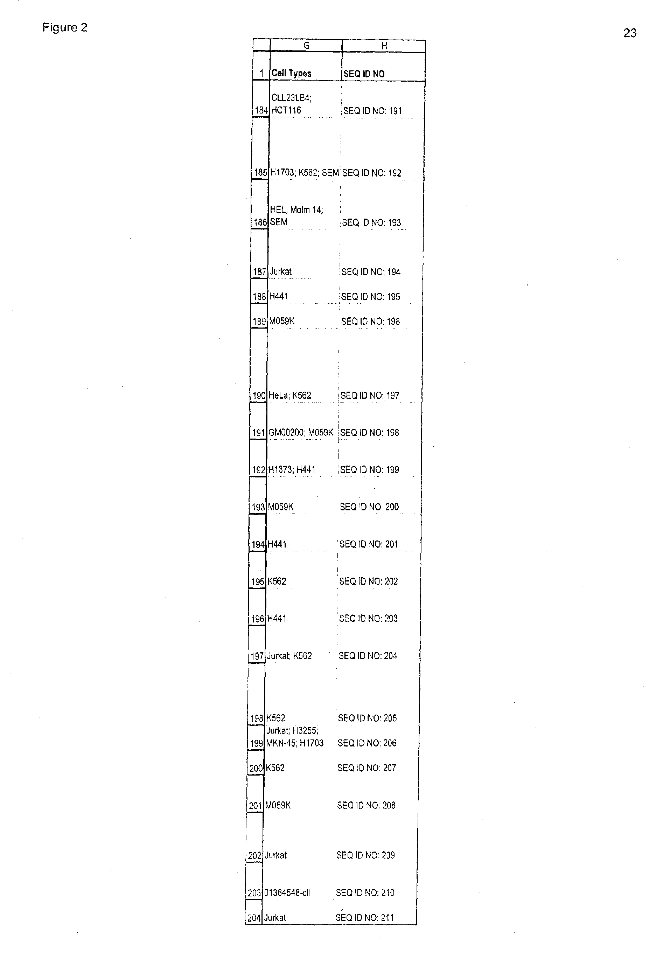

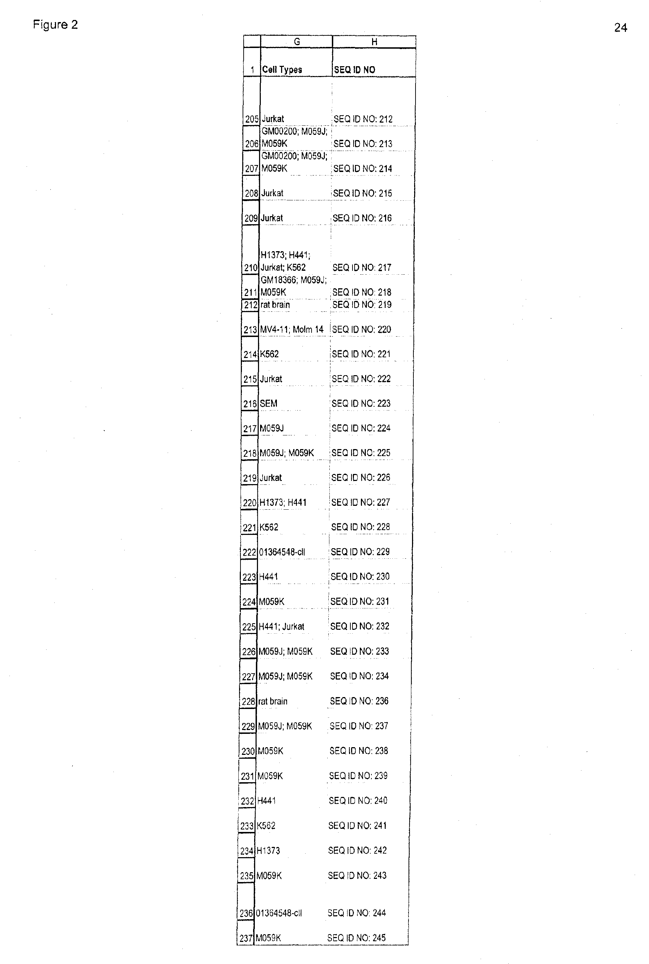

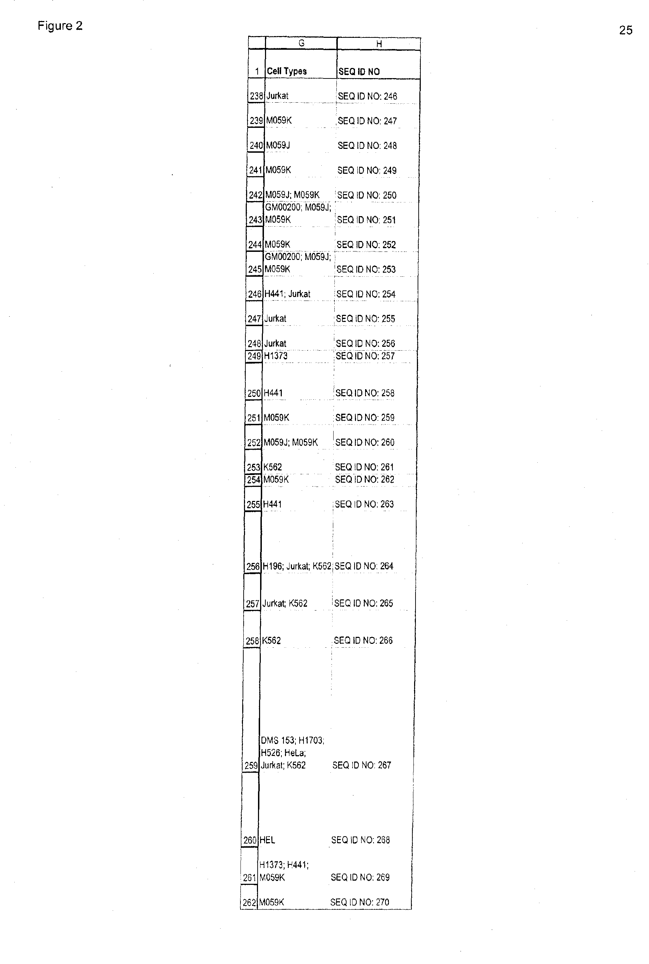

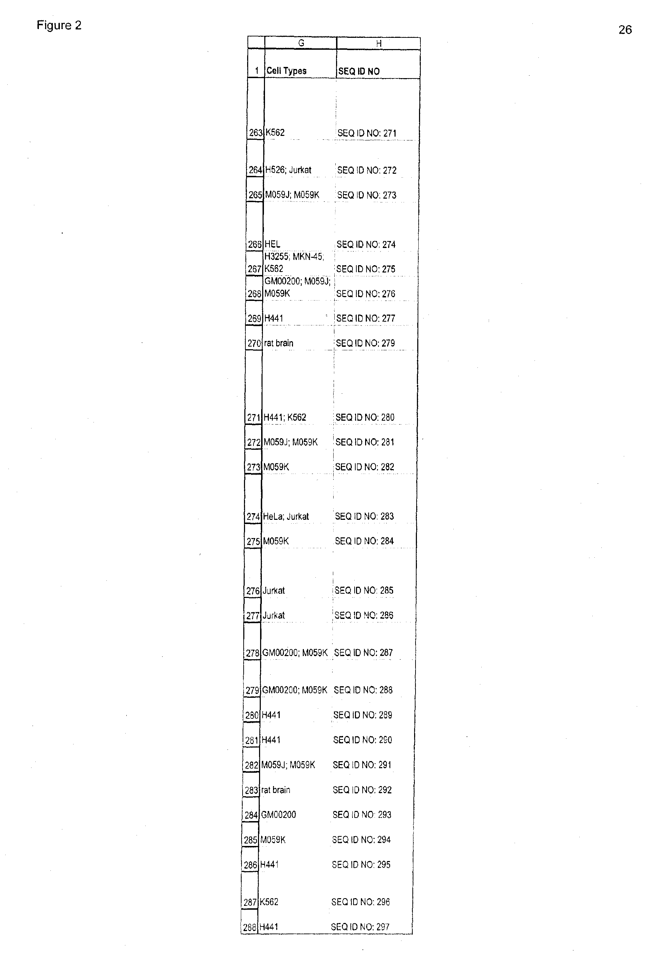

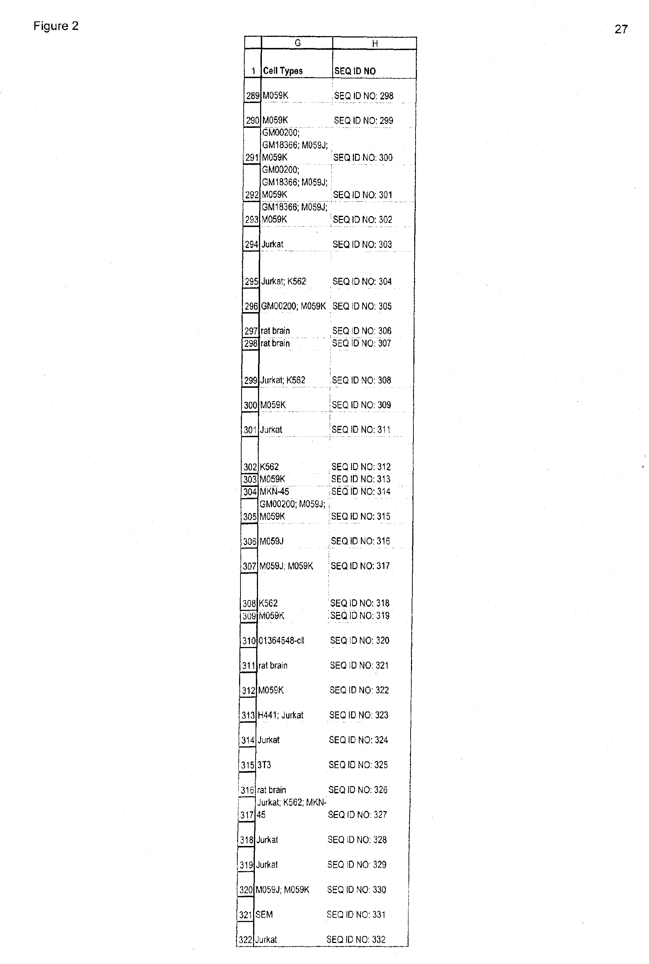

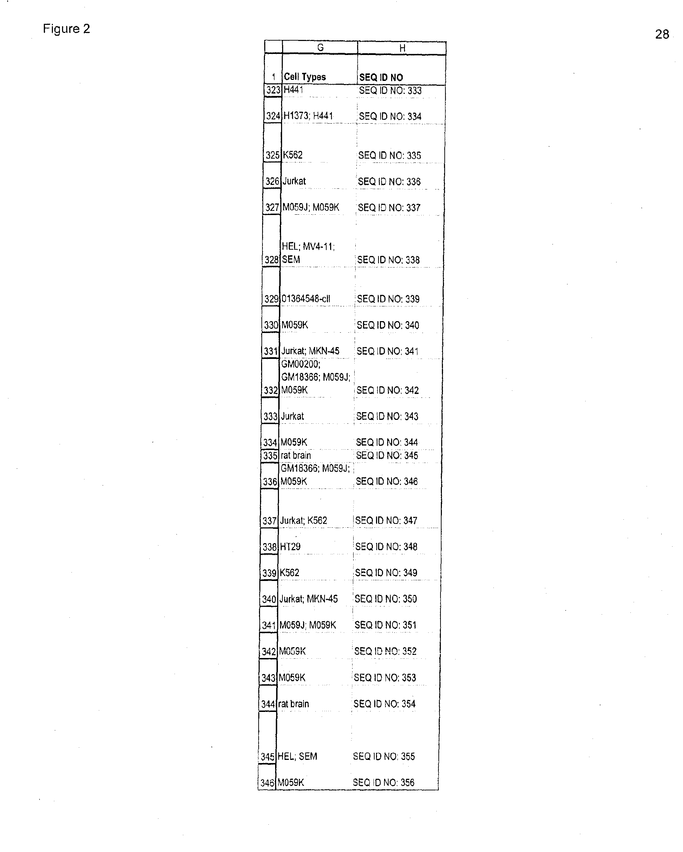

**FIGURE 2** is a table (corresponding to Table 1) summarizing the 345 novel phosphoryLation sites of the invention: Column A = the parent proteins from which the phosphorylation sites are derived; Column B = the SwissProt accession number for the human homologue of the identified parent proteins; Column C = the protein type/classification; Column D = the serine or threonine residues at which phosphorylation occurs (each number refers to the amino acid residue position of the serine or threonine in the parent human protein, according to the published sequence retrieved by the SwissProt accession number); Column E = flanking sequences of the phosphorylatable serine or threonineresidues; sequences (SEQ ID NOs: 1-44, 46-83, 85-110, 112-125, 127-132, 135-139, 141-148, 150-234, 236-277, 279-309, 311-356) were identified using Trypsin digestion of the parent proteins; in each sequence, the serine or threonine (see corresponding rows in Column D) appears in lowercase; Column F = the type of diseases with which the phosphorylation site is associated; Column G = the cell type(s)/ Tissue/ Patient Sample in which each of the phosphorylation site was discovered; and Column H= the SEQ ID NOs of the trypsin-digested peptides identified in Column E.

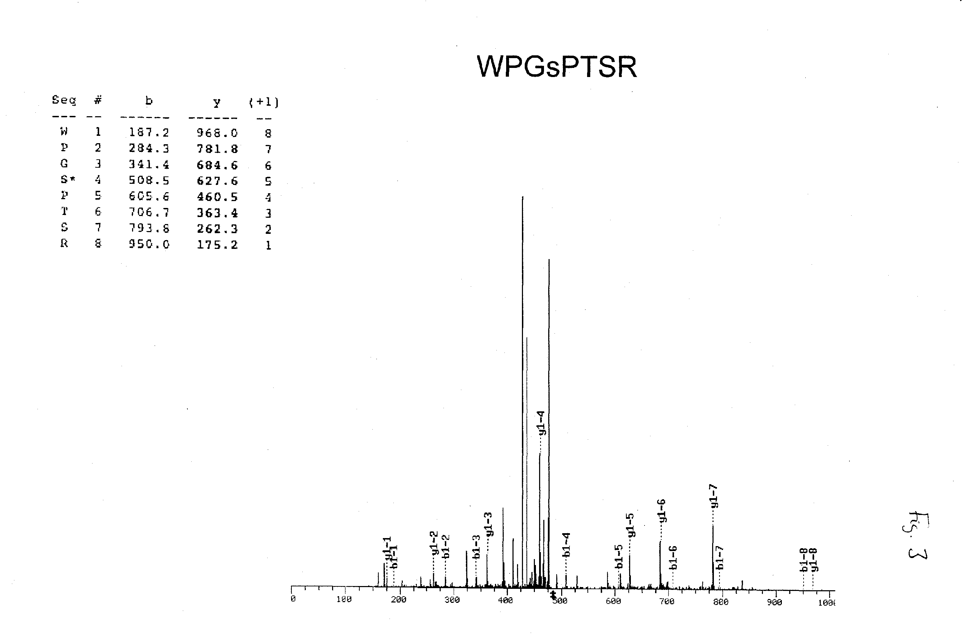

**FIGURE 3** is an exemplary mass spectrograph depicting the detection of the phosphorylation of serine 294 in FOXO3A, as further described in Example 1 (red and blue indicate ions detected in MS/MS spectrum); S* (and pS) indicates the phosphorylated serine (corresponds to lowercase "s" in Column E of Table 1; SEQ ID NO: 4).

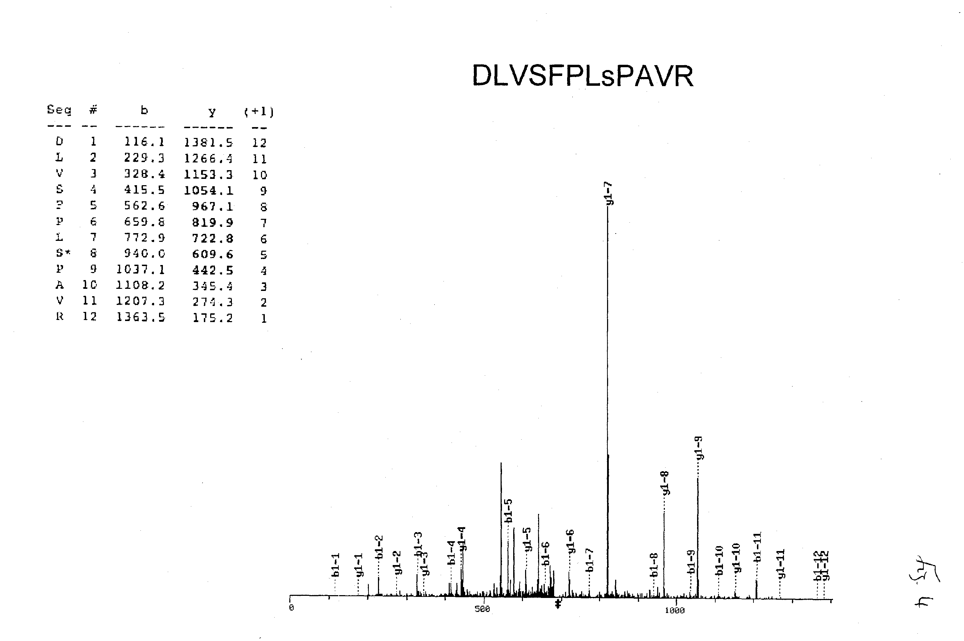

**FIGURE 4** is an exemplary mass spectrograph depicting the detection of the phosphorylation of serine 20 in RAD51C, as further described in Example 1 (red and blue indicate ions detected in MS/MS spectrum); S* (and pS) indicates the phosphorylated serine (corresponds to lowercase "s" in Column E of Table 1: SEQ ID NO: 183).

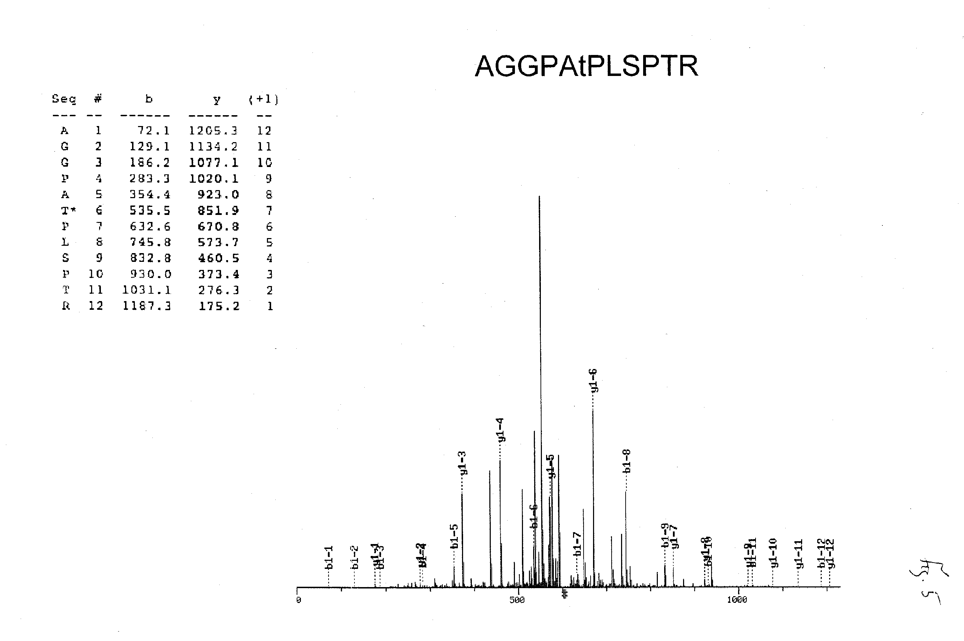

**FIGURE 5** is an exemplary mass spectrograph depicting the detection of the phosphorylation of threonine 14 in lamin B2, as further described in Example 1 (red and blue indicate ions detected in MS/MS spectrum); T* (and pT) indicates the phosphorylated threonine (corresponds to lowercase "t" in Column E of Table 1; SEQ ID NO: 118).

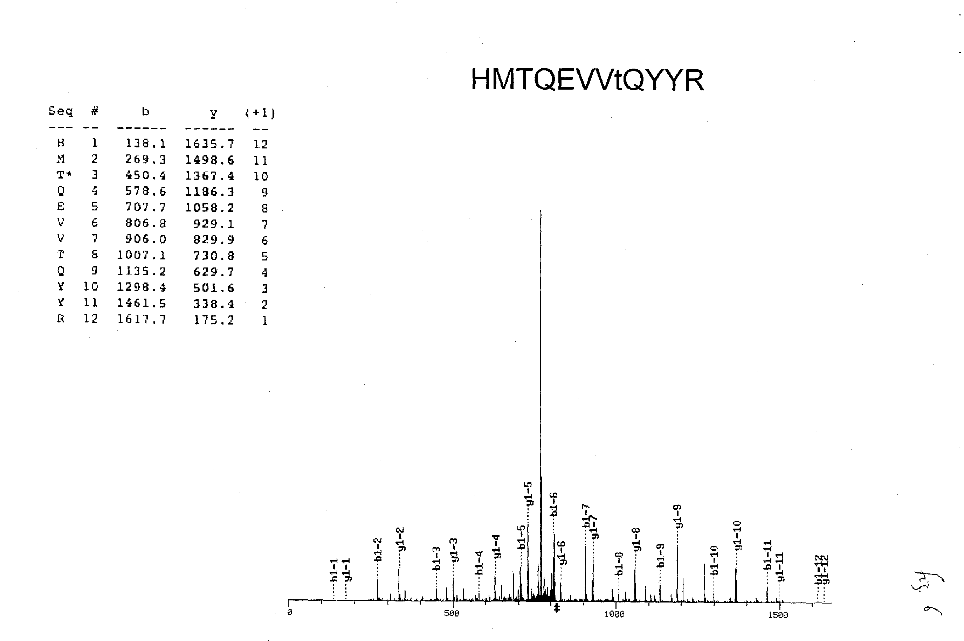

**FIGURE 6** is an exemplary mass spectrograph depicting the detection of the phosphorylation of threonine 29 in NLK, as further described in Example 1(red and blue indicate ions detected in MS/MS spectrum): T* (and pT) indicates the phosphorylated threonine (corresponds to lowercase "t" in Column E of Table 1; SEQ ID NO: 163).

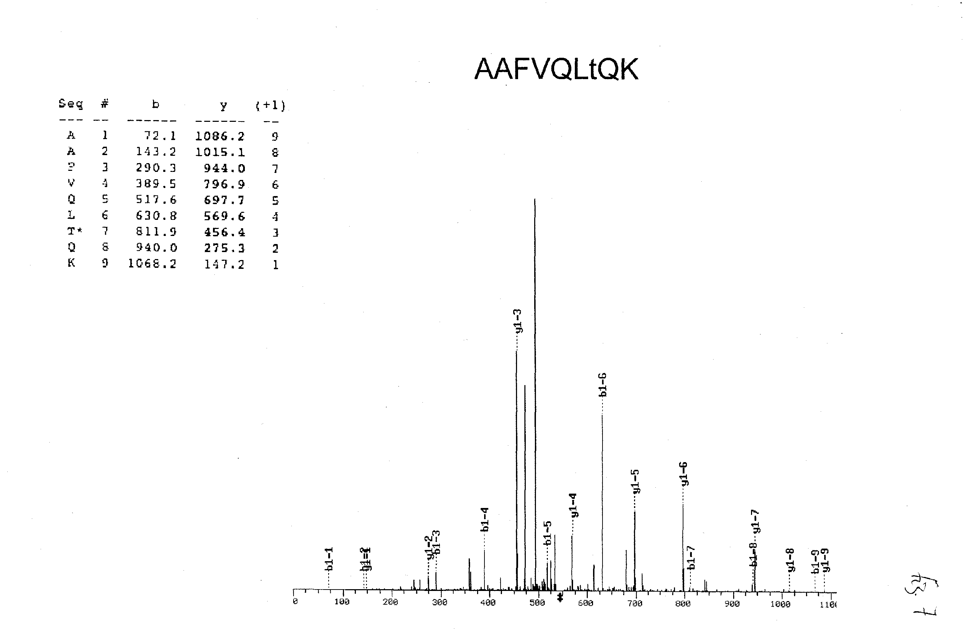

**FIGURE 7** is an exemplary mass spectrograph depicting the detection of the phosphorylation of threonine 205 in ZNF365, as further described in Example 1(red and blue indicate ions detected in MS/MS spectrum); T* (and pT) indicates the phosphorylated threonine (corresponds to lowercase "t" in Column E of Table 1; SEQ ID NO: 354).

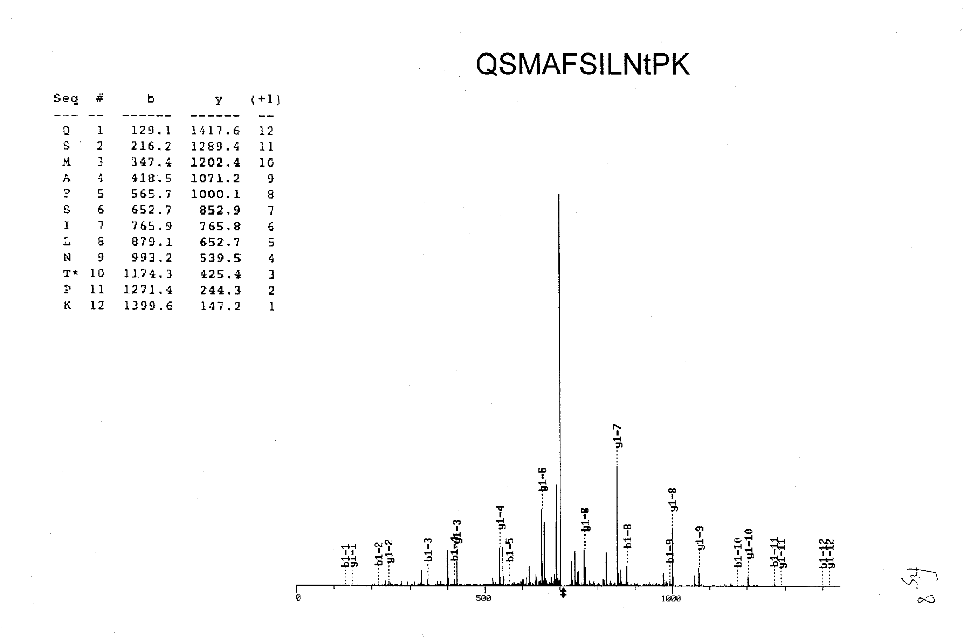

**FIGURE 8** is an exemplary mass spectrograph depicting the detection of the phosphorylation of threonine 2055 in NuMA-l, as further described in Example 1 (red and blue indicate ions detected in MS/MS spectrum); T* (and pT) indicates the phosphorylated threonine (corresponds to lowercase "t" in Column E of Table 1; SEQ ID NO: 186).

### DETAILED DESCRIPTION OF THE INVENTION

The inventors have discovered and disclosed herein novel serine or threonine phosphorylation sites in signaling proteins extracted from the cell line/tissue/patient sample listed in column G of Figure 2. The newly discovered phosphorylation sites significantly extend our knowledge of kinase substrates and of the proteins in which the novel sites occur. The disclosure herein of the novel phosphorylation sites and reagents including peptides and antibodies specific for the sites add important new tools for the elucidation of signaling pathways that are associate with a host of biological processes including cell division, growth, differentiation, develomental changes and disease. Their discovery in carcinoma and leukemia cells provides and focuses further elucidation of the disease process. And, the novel sites provide additional diagnostic and therapeutic targets.

### 1. Novel Phosphorylation Sites in Carcinoma and Leukemia

In one aspect, the invention provides 345 novel serine or threoninephosphorylation sites in signaling proteins from cellular extracts from a variety of human carcinoma and leukemia-derived cell lines and tissue samples (such as HeLa, K562 and Jurkat etc., as further described below in Examples), identified using the techniques described in "Immunoaffinity Isolation of Modified Peptides From Complex Mixtures," U.S. Patent Publication No. 20030044848, Rush et al.*,* using Table 1 summarizes the identified novel phosphorylation sites.

These phosphorylation sites thus occur in proteins found in carcinoma and leukemia. The sequences of the human homologues are publicly available in SwissProt database and their Accession numbers listed in Column B of Table 1. The novel sites occur in proteins such as: adaptor/scaffold proteins, kinase/protease/phosphatase/enzyme proteins, protein kinases, cytoskeletal proteins ubiquitan conjugating system proteins, chromatin or DNA binding/repair proteins, g proteins or regulator proteins, receptor/channeUtransporter/cell surface proteins, transcriptional regulators and cell cycle regulation proteins. *(see* Column C of Table 1).

The novel phosphorylation sites of the invention were identified according to the methods described by Rush et al., U.S. Patent Publication No. 20030044848, which are herein incorporated by reference in its entirety. Briefly, phosphorylation sites were isolated and characterized by immunoaffinity isolation and mass-spectrometric characterization (IAP) (Figure 1), using the following human carcinoma-derived cell lines and tissue samples: HeLa, Jurkat, K562, DMS 153, SEM, DMS153, GM18366, H1703, M059K, H3255, MKN-45, HEL, GM00200, H44, rat brain, H441, 001364548-cll, M059J, HT29, H1373, Kyse140, CLL23LB4, M01043, MV4-11, MOLM 14, H446, MEC-2, HCT116, and 3T3. In addition to the newly discovered phosphorylation sites (all having a phosphorylatable serine or threonine), many known phosphorylation sites were also identified.

The immunoaffinity/mass spectrometric technique described in Rush et al, *i.e.,* the "IAP" method, is described in detail in the Examples and briefly summarized below.

The IAP method generally comprises the following steps: (a) a proteinaceous preparation (e.g., a digested cell extract) comprising phosphopeptides from two or more different proteins is obtained from an organism; (b) the preparation is contacted with at least one immobilized motif-specific, context-independent antibody; (c) at least one phosphopeptide specifically bound by the immobilized antibody in step (b) is isolated; and (d) the modified peptide isolated in step (c) is characterized by mass spectrometry (MS) and/or tandem mass spectrometry (MS-MS). Subsequently, (e) a search program (e.g., Sequest) may be utilized to substantially match the spectra obtained for the isolated, modified peptide during the characterization of step (d) with the spectra for a known peptide sequence. A quantification step, e.g., using SILAC or AQUA, may also be used to quantify isolated peptides in order to compare peptide levels in a sample to a baseline.

In the IAP method as disclosed herein, Akt substrate, ATM/ATR substrate, ATM/R sub mono4F7, P-Chk2(T26/S28), PKA substrate, PKD Substrate, P-Thr-Pro-101, PXsP, PXtP, SsP, t(D/E)X(D/E), tP and tPP antibodies (*See* Cell Signaling Technology, Danvers MA Catalogue or Website). may be used in the immunoaffinity step to isolate the widest possible number of phospho-tyrosine, phospho-serine and/or phospho-threonine containing peptides from the cell extracts.

As described in more detail in the Examples, lysates may be prepared from various carcinoma cell lines or tissue samples and digested with trypsin after treatment with DTT and iodoacetamide to alkylate cysteine residues. Before the immunoaffinity step, peptides may be pre-fractionated (e.g., by reversed-phase solid phase extraction using Sep-Pak C₁₈ columns) to separate peptides from other cellular components. The solid phase extraction cartridges may then be eluted (e.g., with acetonitrile). Each lyophilized peptide fraction can be redissolved and treated with Akt substrate, ATM/ATR substrate, ATM/R sub mono4F7, P-Chk2(T26/S28), PKA substrate, PKD Substrate, P-Thr-Pro-101, PXsP, PXtP, SsP, t(D/E)X(D/E), tP and tPP antibodies (*See* Cell Signaling Technology, Danvers MA Catalogue or Website) immobilized on protein Agarose. Immunoaffinity-purified peptides can be eluted and a portion of this fraction may be concentrated (e.g., with Stage or Zip tips) and analyzed by LC-MS/MS (e.g., using a ThermoFinnigan LCQ Deca XP Plus ion trap mass spectrometer or LTQ). MS/MS spectra can be evaluated using, e.g., the program Sequest with the NCBI human protein database.

The novel phosphorylation sites identified are summarized in Table1/Figure 2. Column A lists the parent (signaling) protein in which the phosphorylation site occurs. Column D identifies the serine or threonineresidue at which phosphorylation occurs (each number refers to the amino acid residue position of the serine or threonine in the parent human protein, according to the published sequence retrieved by the SwissProt accession number). Column E shows flanking sequences of the identified serine or threonine residues (which are the sequences of trypsin-digested peptides). Figure 2 also shows the particular type of cancer (see Column G) and cell line(s) (see Column F) in which a particular phosphorylation site was discovered.

**Table 1. Novel Serine or threoninePhosphorylation Sites.**

| | **A** | **B** | **C** | **D** | **E** | **H** |
|---|---|---|---|---|---|---|
| 1 | **Protein Name** | **Accession No.** | **Protein Type** | **Phospho-Residue** | **Phosphorylation Site Sequence** | **SEQ ID NO** |
| 2 | 4E-BP2 | NP_004087.1 | Translational regulator | T46 | TVAISDAAQLPHDYCTTPGGTLFSTtPGGTR | SEQ ID NO: 1 |
| 3 | 4E-BP2 | NP_004087.1 | Translational regulator | T37 | TVAISDAAQLPHDYCTtPGGTLFSTTPGGTR | SEQ ID NO: 2 |
| 4 | BAP37 | NP_009204.1 | Transcriptional regulator | T155 | FNASQLItQR | SEQ ID NO: 3 |
| 5 | FOXO3A | NP_001446.1 | Transcriptional regulator | S294 | WPGsPTSR | SEQ ID NO: 4 |
| 6 | JAZF1 | NP_778231.2 | Transcriptional regulator | T117 | HSSGSLTPPVTPPltPSSSFR | SEO ID NO: 5 |
| 7 | NFkB-p105 | NP_003989.2 | Transcriptional regulator | S938 | TSFRKLsFTE | SEO ID NO: 6 |
| 8 | PCDC5RP | NP_001244.1 | Transcriptional regulator | T404 | QVVQtPNTVLSTPFRTPSNGAEGLTPR | SEQ ID NO: 7 |
| 9 | TAF7 | NP_005633.2 | Transcriptional regulator | S171 | VKRLLsTDAE | SEO ID NO: 8 |
| 10 | 536P1 | NP_005648.1 | Transcriptional regulator | S1086 | QsQQPMKPISPVKDPVSPASQK | SEQ ID NO: 9 |
| 11 | 53BP1 | NP_005648.1 | Transcriptional regulator | S523 | LMLSTSEYsQSPK | SEQ ID NO:10 |
| 12 | CBP | NP_004371.2 | Transcriptional regulator | S124 | SPLsQGDSSAPSLPK | SEQ ID NO: 11 |
| 13 | E2F8 | NP_0789562 | Transcriptional regulator | T58 | EGSQGEPWtPTANLK | SEQ ID NO: 12 |
| 14 | EIF2C2 | NP_036286.2 | Translational regulator | S387 | ISKLMRSAsFNTDPYVRE | SEQ ID NO: 13 |
| 15 | FIR | NP_055096.2 | Transcriptional regulator | | T297 AVTPPMPLLTPAtPGGLPPAAAVAAAAATAK | SEQ ID NO:14 |
| 16 | FOXK2 | NP_004505.2 | Transcriptional regulator | T634 | LKRIKtEDGE | SEQ ID NO:15 |
| 17 | Hic-5 | NP_057011.2 | Transcriptional regulator | S124 | KRPSLPSsPSPGLPK | SEQ ID NO:16 |
| 18 | JARIDLB | NP_006609.3 | Transcriptional regulator | S415 | FWRLVsTIEE | SEQ ID NO:17 |
| 19 | JAZF1 | NP_778231.2 | Transcriptional regulator | T113 | HSSGSLTPPVtPPITPSSSFR | SEQ ID NO: 18 |
| 20 | KIAA0252 | NP_055953.1 | Transcriptional regulator | T128 | KLtQtQESQVTSHNK | SEQ ID NO: 19 |
| 21 | MIER1 N1-beta | NP_065999.1 | Transcriptional regulator | S165 | DSSGQEDETQSSNDDPSQSVAsQDAQEIIR PR | SEO ID NO: 20 |
| 22 | MLL2 | NP_003473.1 | Transcriptional regulator | T889 | GSPVLLDPEELAPVTPMEVYPECK | SEQ ID NO: 21 |
| 23 | MLL2 | NP-003473.1 | Transcriptional regulator | T2057 | TPDVFKAPLtPR | SEQ ID NO: 22 |
| 24 | NCoA7 | NP_8614472 | Transcriptional regulator | S208 | KPIERVLsSTSEEDEPGVVKF | SEQ ID NO: 23 |
| 25 | NOT2 | NP_055330.1 | Transcriptional regulator | S101 | SLsQGTQLPSHVTPTTGVPTMSLHTPPSPS R | SEQ ID NO: 24 |
| 26 | PELP1 | NP 055204.1 | Transcriptional regulator | S1033 | GADTAPTLAPEALPsQGEVER | SEQ ID NO: 25 |
| 27 | POLR2A | NP-OG0928.1 | Transcriptional regulator | T1903 | YSPTSPTYSPTSPVYtPTSPK | SEQ ID NO: 26 |
| 28 | RAP80 | NP_057374.3 | Transcriptional regulator | S627 | GRLLsFLEQSE | SEQ ID NO:27 |
| 29 | RYBP | NP_036366.3 | Transcriptional regulator | S179 | KTRsSSTSSSTVTSSAGSEQQNQSSSGSE | SEQ ID NO:28 |
| 30 | SHARP | NP_0558162 | Transcriptional regulator | T2460 | VHSIIESDPVtPPSDPSIPIPTLPSVTAAK | SEQ ID NO:29 |
| 31 | SP100 | NP_003104.2 | Transcriptional regulator | T221 | QINAKRKDtTSDKDDSLGSQQTNE | SEQ ID NO:30 |
| 32 | STAG2 | NP_006594.3 | Transcriptional regulator | T1112 | EQTLHtPVMMQTPQLTSTIMR | SEOIDN0:31 |
| 33 | TAF140 | XP 291729.7 | Transcriptional regulator | T568 | VTTHIPQTPVRPEtPNRTPSATLSEK | SEO ID N0: 32 |
| 34 | TAF140 | XP_291729.7 | Transcriptional regulator | T562 | VTTHIPOTPVRPETPNRTPSATLSEK | SEQ ID NO: 33 |
| 35 | TORC3 | NP_073606.3 | Transcriptional regulator | S410 | SRQLsSTSPLAPYPTSQMVSSDRSQL | SEQ ID NO: 34 |
| 36 | TRAP150 | NP_005110.1 | Transcriptional regulator | S190 | DSRPsQAAGDNGGDEVKEQTFSGGTSQDT K | SEQ ID NO: 35 |
| 37 | TRAP150 | NP_005110.1 | Transcriptional regulator | S805 | TEETEEREEsTTGF | SEQ ID NO: 36 |
| 38 | ZNF281 | NP_036614.1 | Transcriptional regulator | S785 | NLETSSAFQSSsQK | SEO ID NO: 37 |
| 39 | LIM | NP 006448.2 | Adaptor/scaffold | T208 | SAGKTAVNVPRQPtVTSVCSETSQEL | SEQ ID NO: 38 |
| 40 | PSD-95 | NP-001356.1 | Adaptor/scaffold | S492 | TKDCGFLsQALSFR | SEQ ID NO: 39 |
| 41 | RanBP2 | NP_006258.2 | Adaptor/scaffold | T2300 | LNQSGTSVGTDEESDVTQEEER | SEO ID NO: 40 |
| 42 | Rictor | NP_689969.2 | Adaptor/scaffold | S1219 | KIRSQsFNTDTTTSGISSM | SEQ 10 NO: 41 |
| 43 | Z02 | NP_ 004808.2 | Adaptor/scafiold | S920 | LIsDFEDTDGEGGAYTDNELDEPAEEPLVSS ITR | SEQ ID NO: 42 |
| 44 | Abi-1 | NP_005461.2 | Adaptor/scaffold | S323 | TRQISRHNsTTSSTSSGGY | SEO ID NO: 43 |
| 45 | AB13 | NP_057512.1 | Adaptor/scaffold | T258 | VFQRPPtLEE | SEO ID NO:44 |
| 46 | BIN2 | NP 0573772 | Adaptor/scaffold | T270 | VISPPVRTAtVSSPL | SEO ID NO: 46 |
| 47 | CGN | NP_065821.1 | Adaptor/scaffold | S155 | AGPGPVDPSNRSNsMLEL | SEQ ID NO: 47 |
| 48 | DAAM1 | NP _055807.1 | Adaptor/scafMd | T361 | SAtQMFELTR | SEO 10 NO: 48 |
| 49 | Dok1 | NP_001372.1 | Adaptor/scatfold | T470 | DCGLSRVGtDKTGVKSEGST | SEO ID NO: 49 |
| 50 | G3BP-2 | NP_036429.2 | Adaptor/scaffold | S163 | RQPsPEPVQE | SEQ I D NO: 50 |
| 51 | GRASP | NP_859062.1 | Adaptor/scaffofd | S393 | SLEEEEsQL | SEO ID NO: 51 |
| 52 | kanoaptin | NP 060628.1 | Adaptor/scaffold | S709 | ETQTHENMsQLSEEEQNKDYQDCSK | SEO ID NO:52_ |
| 53 | LMO7 | NP_005349.3 | Adaptor/scaffold | S663 | EVAATEEDVTRLPSPTSPFSSLsQDQAATS K | SEQ ID NO:53 |
| 54 | MACF1 | NP_149033.2 | Adaptor/scaffold | S607 | HQPLRNTsFTCQNE | SEQ ID NO: 54 |
| 55 | MACF1 | NP_149033.2 | Adaptor/scaffold | T3762 | IQSAGKDCDVQGLEHOMEEINARWNtL | SEQ ID NO: 55 |
| 56 | MICAL1 | NP_073602.2 | Adaptor/scaffold | S818 | RQRLSSLNLTPDPE | SEQ ID NO: 56 |
| 57 | MPP5 | NP_071919.2 | Adaptor/scaffold | S383 | GDILHVIsQEDPNWWQAYR | SEQ ID NO: 57 |
| 58 | NMD3 | NP_057022.2 | Adapter/scaffold | S468 | DSAIPVEsDTDDEGAPR | SEQ ID NO: 58 |
| 59 | PHIP | NP_060404.3 | Adaptor/scaffold | S633 | EEQLIPQMGVTSSGLNQVLsQQANQEISPL DSMIQR | SEQ ID NO: 59 |
| 60 | RanBP2 | NP_0062582 | Adaptor/scaffold | T1412 | ELVGPPLAETVFTPKTSPENVQDRFALVtPK | SEQ ID NO: 60 |
| 61 | RanBP2 | NP 006258.2 | Adaptor/scaffold | T1396 | ELVGPPLAETVFtPKTSPENVQDRFALVTPK | SEQ ID NO: 61 |
| 62 | RCD-8 | NP_055144.3 | Adaptor/scaffold | S741 | TRSPDVISSASTALsQDIPEIASEALSR | SEQ ID NO: 62 |
| 63 | SH3KBP1 | MP_114098.1 | Adaptor/scaffold | S165 | ELSGESDELGlsQDEQLSK | SEQ ID NO: 63 |
| 64 | SLY | NP_061863.1 | Adaptor/scaffold | S146 | SEQEEHELPVLSRQAsTGSEL | SEQ ID NO: 64 |
| 65 | SYNJ2BP | NP_060843.1 | Adaptor/scaffold | S21 | INLTRGPsGLGFNIVGGTQQQYVSNDSGIYV SRIKE | SEQ ID NO: 65 |
| 66 | THRAP2 | NP_056150.1 | Adaptor/scaffold | T1056 | FSVPTPRTPRtPRTPR | SEQ ID NO: 66 |
| 67 | THRAP2 | NP_056150.1 | Adaptor/scaffold | T1059 | FSVPTPRTPRTPRtPR | SEQ ID NO: 67 |
| 68 | VANGL1 | NP_620409.1 | Adaptor/scaffold | S520 | VLRLQsETSV | SEQ 10 NO: 68 |
| 69 | Z01 | NP_003248.3 | Adaptor/scaffold | S1111 | QPYPSRPPFDNQHsQDLDSR | SEQ ID NO: 69 |
| 70 | B-CK | NP_001814.2 | Kinase (non-protein) | T258 | FCTGLtQIETLFK | SEQ ID NO: 70 |
| 71 | PIK4CA | NP_477352.1 | Kinase (non-protein) | S204 | KTSSVSSIsQVSPER | SEQ ID NO: 71 |
| 72 | PIP5K | NP_689884.1 | Kinase (non-protein) | T244 | TSVSPQANRTYVRTEtTEDE | SEQ ID NO: 72 |
| 73 | Pnk1 | NP_0091852 | Phosphatase | T122 | TPESQPDTPPGtPLVSQDEKR | SEQ ID NO: 73 |
| 74 | ALDH1A1 | NP-0006802 | Enzyme, misc. | T337 | YILGNPLtPGVTQGPQIDKEQYDK | SEQ ID NO: 74 |
| 75 | ATG4C | NP_1162412 | Protease | S451 | KKQLKRFsTEE | SEQ ID NO: 75 |
| 76 | CAD | NP_004332.2 | Enzyme, misc. | T1884 | KVAEPELMGTPDGTCYPPPPVPR | SEQ ID NO: 76 |
| 77 | DDX21 | NP-076950.1 | Enzyme, misc. | T266 | NGIDILVGtPGR | SEQ ID NO: 77 |
| 78 | DGK-1 | NP_004708.1 | Kinase (non-protein) | S803 | LQEDLQSVSSGsQR | SEQ ID NO: 78 |
| 79 | GGTL3 | NP_821158.2 | Enzyme, misc. | S72 | QRLPsSSSEMGSQDGSPL | SEQ ID NO: 79 |
| 80 | GGTL3 | NP_821158.2 | Enzyme, misc. | S79 | LPSSSSEMGsQDGSPLR | SEQ ID NO: 80 |
| 81 | HDAC5 | NP_005465.2 | Enzyme, misc. | T546 | TKTGELPRQPtTHPEETEEELTEQQEVL | SEQ ID NO: 81 |
| 82 | IHPK1 | NP_695005.1 | Kinase (non-protein) | S146 | ASLSLETSESsQEAKSPK | SEQ ID NO: 82 |
| 83 | INPP4 | NP_001557.1 | Phosphatase | T22 | QRAStlDVAADMLGL | SEQ ID NO: 83 |
| 84 | MGEA5 | NP_036347.1 | Enzyme, misc. | T709 | LLPIDGANDLFFQPPPLTPTSK | SEQ ID NO: 85 |
| 85 | MPG | NP_002425.2 | Enzyme, misc. | T67 | CLGPPTtPGPYR | SEQ ID NO: 86 |
| 86 | neurabin 1 | NP_060120.2 | Phosphatase | S865 | QTSCQDGLsQDLNEAVPETERLDSK | SEQ ID NO: 87 |
| 87 | PDE8B | NP_00102502 3.1 | Enzyme, misc. | T362 | ItPVIGQGGK | SEQ ID NO: 88 |
| 88 | PFAS | NP_036525.1 | Enzyme, misc. | S215 | LQRNPsTVE | SEQ ID NO: 89 |
| 89 | Pnk1 | NP_0091852 | Phosphatase | T111 | tPESQPDTPPGTPLVSQDEKR | SEQ ID NO: 90 |
| 90 | PPP1R12C | NP_060077.1 | Phosphatase | S452 | GAPGAGLQRSAsSSWLE | SEQ ID NO: 91 |
| 91 | slingshot 1 | NP_0618572 | Phosphatase | T462 | RQQtDSSLQQPVDDPAGPGDFLPETPDGT PESQLPF | SEQ ID NO: 92 |
| 92 | USP34 | NP_055524.3 | Protease | T188 | HNTHPTIEDISTOESNILGAFCDMNDVEVPL HLLR | SEQ ID NO: 93 |
| 93 | ATP6V001 | NP_004682.2 | Receptor, channel, transporter or cell surface protein | S29 | AGVLsQADYLNLVQCETLEDLK | SEQ ID NO: 94 |
| 94 | ATP6V1C1 | NP_00100725 5.1 | Receptor, channel, transporter or cell surface protein | S344 | HLDSSAAAIIDAPMDIPGLNLsQQEYYPYVY YK | SEQ ID NO: 95 |
| 95 | CD7 | NP_006128.1 | Receptor, channel, transporter or cell surface protein | T232 | DMSHSRCNTLSSPNQYQ | SEO ID N0: 96 |
| 96 | CLCN1 | NP-000074.2 | Receptor, channel, transporter or cell surface protein | T929 | PGATGTGDVIAASPEtPVPSPSPEPPLSLAP GK | SEQ ID NO: 97 |
| 97 | elF4ENIF1 | NP_062817.1 | Receptor, channel, transporter or cell surface protein | S213 | FGERRRNDsYTEEEPEWFSAGPT | SEQ ID NO: 98 |
| 98 | FZD6 | NP_003497.2 | Receptor, channel, transporter or cell surface protein | S620 | LREQDCGEPAsPAASISR | SEQ ID NO: 99 |
| 99 | GLE1L | NP_001490.1 | Receptor, channel, transporter or cell surface protein | T102 | SPDASSAFSPASPATPNGTK | SEQ ID NO: 100 |
| 100 | HIR | NP_004972.1 | Receptor, channel, transporter or cell surface protein | T229 | AC1LIKPYMtOEGEYLPLDOR | SEQ ID NO: 101 |
| 101 | IFNGR1 | NP_000407.1 | Receptor, channel, transporter or cell surface protein | T295 | KSIILPKSLISVVRSAtLE | SEO ID N0: 102 |
| 102 | MGC15619 | NP_115745.2 | Receptor, channel, transporter or cell surface protein | T29 | LRHFTVVGDDY | SEQ ID NO: 103 |
| 103 | MS4A1 | NP_690605.1 | Receptor, channel, transporter or cell surface protein | S35 | RRMsSLVGPTQSFF | SEQ ID NO: 104 |
| 104 | MS4A1 | NP_690605.1 | Receptor, channel, transporter or cell surface protein | S36 | RRMSsLVGPTQSFF | SEQ ID NO: 105 |
| 105 | NUP133 | NP_060700.2 | Receptor, channel, transporter or cell surface protein | S260 | SGIGRKVsSLF | SEQ ID NO: 106 |
| 106 | ORAI1 | NP_116179.2 | Receptor, channel, transporter or cell surface protein | T295 | GDHPLtPGSHYA | SEQ ID NO: 107 |
| 107 | P2Y2 | NP_002555.2 | Receptor, channel, transporter or cell surface protein | T330 | DAKPPTGPSPAtPAR | SEQ ID NO: 108 |
| 108 | RAIG1 | NP 003970.1 | Receptor, channel, transporter or cell surface protein | S301 | AYsQEEffQGFEETGDTLYAPYSTHFQLQN OPPOK | SEQ ID N0: 109 |
| 109 | RHBDF1 | NP_071895.2 | Receptor, channel, transporter or cell surface protein | T180 | VADDTAEGLSAPHtPVTPGAASLCSFSSSR | SEQ ID NO: 110 |
| 110 | sialophorin | NP 003114.1 | Receptor, channel, transporter or cell surface protein | T341 | VGGSGGDKGSGFPDGEGSSRRPtLT | SEQ ID NO: 112 |
| 111 | SLC20A1 | NP_005406.3 | Receptor, channel, transporter or cell surface protein | S335 | RERLPsVDLKEE | SEQ ID NO: 113 |
| 112 | SLC26A2 | NP_000103.2 | Receptor, channel, transporter or cell surface protein | S36 | LQRESsTDFKQFE | SEQ ID NO: 114 |
| 113 | TNPO1 | NP_002261.2 | Receptor, channel, transporter or cell surface protein | T344 | RSRtVAQQHDEDGIEEEDDDDDEIDDDDTIS DW | SEQ ID NO: 115 |
| 114 | DBN1 | NP_543157.1 | Cytoskeletal protein | S603 | EGTQASEGYFSQsQEEEFAQSEELCAK | SEQ ID NO: 116 |
| 115 | lamin A/C | NP ,733821.1 | Cytoskeletal protein | S458 | VRLRNKsNEDQSMGNW | SEO ID NO: 117 |
| 116 | lamin B2 | NP_116126.2 | Cytoskeletal protein | T14 | AGGPAtPLSPTR | SEQ ID NO: 118 |
| 117 | ACTL7B | NP_006677.1 | Cytoskeletal protein | T37 | DTGAAtQLKMKPRKVHK | SEQ ID NO: 119 |
| 118 | anilin N | P_061155.2 | Cytoskeletal protein | T320 | SCMQNPELLPKtPISPLK | SEQ ID NO: 120 |
| 119 | CCDC6 | NP_005427.2 | Cytoskeletal protein | S323 | ALCRQLsESESSLE | SEQ ID NO: 121 |
| 120 | CCDC6 | NP_0054272 | Cytoskeletal protein | S328 | ALCRQLSESESsLE | SEQ ID NO: 122 |
| 121 | ENC1 | NP-003624.1 | Cytoskeletal protein | S406 | HCLYWGGHTAATGCLPAsPSVSLK | SEQ ID NO: 123 |
| 122 | EPB41L2 | NP_001422,1 | Cytoskeletal protein | S47 | EVAENQQNQSSDPEEEKGsQPPPAAESQS SLR | SEQ ID NO: 124 |
| 123 | eplin | NP_057441.1 | Cytoskeletal protein | S726 | SLNWSSFVDNTFAEEFTTQNQKsQDVELW EGEWK | SEQ ID NO: 125 |
| 124 | GAS7 | NP_958836.1 | Cytoskeletal protein | S57 | YYVNTTTNETTWERPSSSPGIPAsPGSHR | SEQ ID NO: 127 |
| 125 | GM130 | NP 004477.2 | Cytoskeletal protein | S119 | QLsQQLNGLVCESATCVNGEGPASSANLK | SEQ ID NO: 128 |
| 126 | MAP18 | NP_005900,1 | Cytoskeletal protein | T1067 | AAEAGGAEEQYGFLTtPTK | SEQ ID NO: 129 |
| 127 | NFL | NP_006149.2 | Cytoskeletal protein | S74 | SYSSSSGSLMPSLENLDLsQVAAISNDLK | SEQ ID NO: 130 |
| 128 | NUDE1 | NP 060138.1 | Cytoskeletal protein | T228 | GPSSSLNtPGSFR | SEQ ID NO: 131 |
| 129 | PHACTR2 | NP_055536.1 | Cytoskeletal protein | S522 | RLsQRPTTEELEQR | SEQ ID NO: 132 |
| 130 | SPTBN2 | NP_008877.1 | Cytoskeletal protein | S449 | LVsQDNFGLELAAVEAAVR | SEQ ID NO: 135 |
| 131 | supervillin | NP_068506.1 | Cytoskeletal protein | T657 | FRTOPITSAER | SEQ ID NO: 136 |
| 132 | tensin 2 | NP_056134.2 | Cytoskeletal protein | T920 | DAPCSASSELSGPStPLHTSSPVQGK | SEQ ID NO: 137 |
| 133 | HAPIP | NP_003938,1 | G protein or regulator | S487 | ALLDVLQRPLsPGNSESLTATANYSK | SEQ ID NO: 138 |
| 134 | RALBP1 | NP_006779.1 | G protein or regulator | S29 | HGSGLTRTPsSEE | SEQ ID NO: 139 |
| 135 | ARHGAP15 | NP-060930.2 | G protein or regulator | 5212 | KIORSSsTELL | SEQ ID NO:141 |
| 136 | ARHGAPt7 | NP_00100663 5.1 | G protein or regulator | S702 | SAPRRYsSSLSPIQAPNHPPPQPPTQATPL | SEQ ID NO: 142 |
| 137 | ARHGAP17 | NP_00100663 5.1 | G protein or regulator | S703 | SAPRRYSsSLSPIQAPNHPPPOPPTQATPL | SEQ ID NO: 143 |
| 138 | ARHGEF2 | NP_0047142 | G protein or regulator | S267 | FLsQLLER | SEQ ID NO: 144 |
| 139 | G-beta(1) | NP_002065.1 | G protein or regulator | S31 | KACADATLsQITNNIDPVGR | SEQ ID NO:145 |
| 140 | GNB2 | NP_0052642 | G protein or regulator | T31 | KACGDSTLtQITAGLDPVGR | SEQ ID NO: 146 |
| 141 | GPSM1 | G NP 056412.2 | protein or regulator | S336 | HLQIsQEIGDR | SEQ ID NO: 147 |
| 142 | GRIPAPI | NP_064522.3 | G protein or regulator | 5817 | DMEVLsQEIVR | SEQ ID NO: 148 |
| 143 | RASGRP2 | NP_0058162 | G protein or regulator | S509 | NFDVDGDGHIsQEEFQIIR | SEQ ID NO: 150 |
| 144 | RIP3 | NP_0559492 | G protein or regulator | T378 | DRRStEPSVTPDLLNF | SEQ ID NO: 151 |
| 145 | RIP3 | NP_0559492 | G protein or regulator | T542 | GRSKTFDWAE | SEQ ID NO: 152 |
| 146 | synGAP | NP_ 006763.1 | G protein or regulator | S877 | PAPAGRLsQGSGSSiTAAGMR | SEQ ID NO: 153 |
| 147 | synGAP | NP_006763.1 | G protein or regulator | S1210 | RLLsQEEQTSK | SEQ ID NO: 154 |
| 148 | Bcr | NP_004318.3 | Protein kinase, Ser/Thr (non-receptor) | S301 | GKGPLLRSQsTSEQE | SEQ ID NO: 155 |
| 149 | HER2 | NP_00100586 2.1 | Protein kinase, Tyr (receptor) | T671 | LLQETELVEPUPSGAMPNQAQMR | SEQ ID NO: 156 |
| 150 | OSR1 | NP 005100.1 | Protein kinase, Ser/Thr (non-receptor) | T310 0 | KTLQRAPTISE | SEQ ID NO: 157 |
| 151 | Pyk2 | NP_004094.3 | Protein kinase, Tyr (non-receptor) | T842 | SLDPMVYMNDKSPLtPEKEVGYLEFTGPPQ KPPR | SEQ ID NO: 158 |
| 152 | CaMK2-alpha | NP_0570652 | Protein kinase, Ser/Thr (non-receptor) | T446 | ItQYLDAGGIPR | SEQ ID NO: 159 |
| 153 | CRK7 | NP_057591.1 | Protein kinase, Ser/Thr (non-receptor) | T893 | NSEESRPYtNKVITLW | SEQ ID NO: 160 |
| 154 | GAK | NP_005246.1 | Protein kinase, Ser/Thr (non-receptor) | S73 | YALKRLLsNEEE | SEQ ID NO: 161 |
| 155 | HGK | NP 663720.1 | Protein kinase, Ser/Thr (non-receptor) | S571 | AQSKQTGRVLEPPVPSRSEsFSNGNSE | SEQ ID NO: 162 |
| 156 | NLK | NP_057315.2 | Protein kinase, Ser/Thr (non-receptor) | T291 | HMTQEWtQYYR | SEQ ID NO:163 |
| 157 | PHKB | NP_000284.1 | Protein kinase, regulatory subunit | S700 | SKVKRQsSTPSAPELGQQPDVNISEW | SEQ ID NO: 164 |
| 158 | PHKB | NP_000284.1 | Protein kinase, regulatory subunit | S701 | SKVKROSsTPSAPELGC10POVNISEW | SEQ ID NO: 165 |
| 159 | PKAR2B | NP_002727.2 | Protein kinase, regulatory subunit | S164 | NLDPEQMsQVLDAMFEK | SEQ ID NO: 166 |
| 160 | PKN₃ | NP_037487.2 | Protein kinase, Ser/Thr (non-receptor) | T718 | GIGFGDRTStFCGTPE | SEQ ID NO: 167 |
| 161 | TA02 | NP_004774.1 | Protein kinase, Ser/Thr (non-receptor) | T344 | AEPYN1HRAGtLTSLE | SEQ ID NO: 1 68 |
| 162 | TIF1-beta | NP_005753.1 | Protein kinase, atypical | S440 | QGSGSsQPMEVQEGYGFGSGDDPYSSAE PHVSGVK | SEQ ID NO: 169 |
| 163 | VACAMKL | NP_076951.2 | Protein kinase, Ser/Thr (non-receptor) | S256 | ILAGDYEFDSPYWDDIsQAAK | SEQ ID NO: 170 |
| 164 | WNK₂ | NP_006639.3 | Protein kinase, Ser/Thr (non-receptor) | T1857 | AGSLGPEtPSR | SEQ ID NO: 171 |
| 165 | CROP | NP-057508.2 | Chromatin, DNA-binding, DNA repair or DNA replication protein | S110 | LALsQNQQSSGAAGPTGK | SEQ ID NO: 172 |
| 166 | NIPBL | NP_056199.2 | Chromatin, DNA-binding, DNA repair or DNA replication protein | S367 | SRVRsSDMDQQEDMISGVENSNVSENDIPF | SEQ ID NO: 173 |
| 167 | REV3 | NP_002903.1 | Chromatin, DNA-binding, DNA repair or DNA replication protein | T963 | VPDSPATKYPIYPLtPK | SEQ ID NO: 174 |
| 168 | TOPBP1 | NP_008958.1 | Chromatin, DNA-binding, DNA repair or DNA replication protein | S1072 | RARLAsNLQWPSCPTQYSE | SEQ ID NO: 175 |
| 169 | ATRX | NP_000480.2 | Chromatin, DNA-binding, DNA repair or DNA replication protein ; | S2379 | AQVQALALSRQAsQE | SEQ ID NO: 176 |
| 170 | BAZ1A | NP 038476.2 | Chromatin, DNA-binding, DNA repair or DNA replication protein | S850 | PSSFQNNVQsQDPQVSTK | SEQ ID NO:177 |
| 171 | Bright | NP_ 005215.1 | Chromatin, DNA-binding, DNA repair or DNA replication protein | T582 | GGNTGTSGGQAGPAGLStPSTSTSNNSLP | SEQ ID NO:178 |
| 172 | CHD-4 | NP 001264.2 | Chromatin, DNA- binding, DNA repair or DNA replication protein | S515 | WGQPPsPTPVPRPPDADPNTPSPKPLEGR PER | SEQ ID NO: 179 |
| 173 | FBXL10 | NP_00100536 6.1 | Chromatin, DNA-binding, DNA repair or DNA replication protein | T462 | STTLAVDYPKtPTGSPATEVSAK | SEQ ID NO: 180 |
| 174 | FL13111 | NP-079358.1 | Chromatin, DNA-binding, DNA repair or DNA replication protein | S125 | SVVKPVPAPQAVQPSRQEsSCGSLE | SEQ ID NO: 181 |
| 175 | MybBP1A | NP_055335.1 | Chromatin, DNA-binding, DNA repair or DNA replication protein | T1161 | EIPSATQSPISK | SEQ ID NO: 182 |
| 176 | RAD51C | NP_002867.1 | Chromatin, DNA-binding, DNA repair or DNA replication protein | S20 | DLVSFPLsPAVR | SEQ ID NO: 183 |
| 177 | Rif1 | NP 060621.3 .3 | Chromatin, DNA-binding, DNA repair or DNA replication protein | S1184 | ISSRKTsTECASSTENSF | SEQ ID NO: 184 |
| 178 | KAB1 | NP 055627.2 | Cell cycle regulation | S1019 9 | VQSSGRIRQPsVDLTDDDQTSSVPH | SEQ ID NO: 185 |
| 179 | NuMA-1 | NP_0061762 | Cell cycle regulation | T2055 | QSMAFSILNtPK | SEQ ID NO: 186 |
| 180 | B99 | NP 057510.2 | Cell cycle regulation | T146 | KRETYYLSDSPL | SEQ ID NO: 187 |
| 181 | CENPF | NP_057427.3 | Cell cycle regulation | T1862 | LLQRVEtSEGLNSDLE | SEQ ID NO: 188 |
| 182 | CIZ1 | NP_036259.2 | Cell cycle regulation | T567 | AFSTVPLtPVPRPSDSVSSTPAATSTPSK | SEQ ID NO: 189 |
| 183 | HIRA | NP_003316.3 | Cell cycle regulation | T544 | DSMNATStPAALSPSVLTTPSKIEPMK | SEQ ID NO: 190 |
| 184 | KAB1 | NP 055627.2 | Cell cycle regulation | S879 | TSASRIRERsESLDPDSSMDTTL | SEQ ID NO: 191 |
| 185 | KI-67 | NP_002408.3 | Cell cycle regulation | T1233 | ELFQtPGHTEELVAAGK | SEQ ID NO:192 |
| 186 | MDC1 | NP_055456.1 | Cell cycle regulation | T1343 | TPETVVPTAPELQISTSTDQPVtPKPTSR | SEQ ID NO:193 |
| 187 | MDC1 | NP_055456.1 | Cell cycle regulation | T1466 | TPETVVPTAPELQPSTSTDQPVtPEPTSQAT R | SEQ ID NO: 1 94 |
| 188 | MLF11P | NP_078905.2 | Cell cycle regulation | S136 | LRPIsDDSESIEESDTRR | SEQ ID NO: 195 |
| 189 | NASP | NP_002473.2 | cell cycle regulation | T464 | VQIAANEETQER | SEQ ID NO: 1 96 |
| 190 | UREB1 | NP_113584.3 | Ubiquitin conjugating system | T2747 | SNDSTEONLSDGTPMPDSYPTPSSTDAAT SESK | SEQ ID NO: 197 |
| 191 | CCDC86 | NP_077003.1 | Ubiquitin conjugating system | S128 | PSEEAPKCsQDQGVLASELAQNK | SEQ ID NO: 198 |
| 192 | RFFL | NP 476519.1 | Ubiquitin conjugating system | T150 | VLGOOPVISOEDRTRAStLSPDFPE00AF | SEQ ID NO: 199 |
| 193 | RNF40 | NP G55586.1 | Ubiquitin conjugating system | T127 | CHESQGELSSAPEAPGtQEGPTCDGTPLPE PGTSELR | SEQ ID NO: 200 |
| 194 | SMURF2 | NP_073576.1 | Ubiquitin conjugating system | S203 | YSSPGRPLsCFVDE | SEQ ID NO:201 |
| 195 | UREB1 | NP_113584.3 | Ubiquitin conjugating system | T2554 | LRQLtANTGHT | SEQ ID NO: 202 |
| 196 | UREB1 | NP 113584.3 | Ubiquitin conjugating system | S1999 | TRQSsDFDTQSGF | SEQ ID NO: 203 |
| 197 | USP10 | NP_0051442 | Ubiquitin conjugating system | T74 | tPSYSISSTLNPQAPEFILGCTASK | SEQ ID NO: 204 |
| 198 | USP25 | NP_037528.3 | Ubiquitin conjugating system | T149 | KRTPtEVW | SEQ ID NO:205 |
| 199 | CCT2 | NP_006422.1 | Chaperone | S260 | NAKILIANTGMDTDKIKIFGSRVRVDsTAKVA E | SEQ ID NO:206 |
| 200 | CLH-17 | NP_004850.1 | Vesicle protein | S1229 | GRLAsTLVHLGEY | SEQ ID NO:207 |
| 201 | N-cad | NP_001783.2 | Adhesion or extracellular matrix protein | S788 | YDEEGGGEEDODYDLsQLQQPDTVEPDAIK PVGIR | SEQ ID NO: 208 |
| 202 | PROSC | NP_009129.1 | Unknown function | S244 | VGSTNVRIGsTIFGE | SEQ ID NO: 209 |
| 203 | WFS1 | NP_005996.1 | Endoplasmic reticulum or golgi | S235 | ERLVsSESKNY | SEQ ID NO: 210 |
| 204 | aftiphilin | NP_00100224 3.1 | Unknown function | S850 | ISTSSLKVTDAFARLMsTVE | SEQ ID NO: 211 |
| 205 | alphaSNBP(A) | NP 056393.1 | Unknown function | T119 | GRMPtYSQF | SEQ ID NO:212 |
| 206 | alphaSNBP(A) | NP_056393.1 | Unknown function | T175 | tQLSQGRSSPQLDPLRK | SEQ ID NO:213 |
| 207 | alphaSNBP(A) | NP_056393.1 | Unknown function | S178 | TQLsQGRSSPQLDPLRK | SEQ ID NO: 214 |
| 208 | ANKRD12 | NP_056023.2 | Unknown function | S1311 | VRRCsMPSVICE | SEQ ID NO:215 |
| 209 | ANKRD17 | NP 115593.3 | Unknown function | S226 | AAAALTRMRAEsTANAGQSDNRSLAE | SEQ ID NO:216 |
| 210 | ANKRD27 | NP_115515.2 | Unknown function | T1023 | RRHtVEDAVVSQGPEAAGPL | SEQ ID NO: 217 |
| 211 | ARHGAP23 | XP_290799.6 | Unknown function | S791 | HYsQDCSSIK | SEQ ID NO:218 |
| 212 | ARPP-21 | NP_057384.2 | Inhibitor protein | S138 | MLSKDCsQEYTDSTGIDLHEFLINTLK | SEQ ID NO:219 |
| 213 | BCDIN3 | NP_062552.2 | Unknown function | T291 | GQHHQQQQAAGGSESHPVPPTAPLtPLLH GEGASQQPR | SEQ ID NO: 220 |
| 214 | Bcl-2L13 | NP_056182.2 | Apoptosis | S420 | INAREEsLVEE | SEQ ID NO: 221 |
| 215 | C10orf18 | XP_374765.4 | Unknownfunction | S1516 | VDSSSASTtLGRQCsLNCISSGCHTSGDSL E | SEQ ID NO:222 |
| 216 | C10orf47 | NP_694988.2 | Unknown function | T167 | KQDAETPPPPDPPAPETLLAPPPLPStPDPP RR | SEQ ID NO: 223 |
| 217 | C10orf6 | NP_060591.2 | Unknown function | S444 | MQKPHLPLsQEK | SEQ ID NO: 224 |
| 218 | C12orf5 | NP_065108.1 | Unknown function | S154 | EQFsQGSPSNCLETSLAEIFPLGK | SEQ ID NO: 225 |
| 219 | C13orf7 | NP_078822.3 | Unknown function | S526 | MNRTRTSsEASMDAAYLDKISE | SEQ ID NO: 226 |
| 220 | C14orf173 | NP_00102688 4.2 | Unknown function | S544 | ERRSsWYVDASDVLT7EDPQCPQPL | SEQ ID NO: 227 |
| 221 | C16orf14 | NP_6124272 | Unknown function | S78 | NRVNGRRAPsTSPSFEGTQETY | SEQ ID NO:228 |
| 222 | C16orf54 | NP_787096.1 | Unknown function | T71 | RPDPSHRAPtLVWRPGGELW | SEQ ID NO:229 |
| 223 | C17orf57 | NP_6895602 | Unknown function | T569 | PEFKKItELTEAGETKK | SEQ ID NO:230 |
| 224 | C20orf112 | NP-542183.1 | Unknown function | S295 | LEIYQSsQDEPIALDK | SEQ ID NO: 231 |
| 225 | CEP68 | NP_055962.1 | Unknown function | S472 | SRWKsEEEVESDDE | SEQ ID NO:232 |
| 226 | CGI-09 | NP_057023.2 | Unknown function | T107 | SQKLtQDDIK | SEQ ID NO: 233 |
| 227 | CGI-09 | NP_0570232 | Unknown function | S325 | QASEQENEDSMAEAPESNHPEDQETMETIs QDPEHK | SEQ ID NO:234 |
| 228 | CNKSR2 | NP_055742.2 | Unknown function | S14 | WSPsQVVDWMK | SEQ ID NO: 236 |
| 229 | CRAMP1L | NP_065876.2 | Unknown function | S1064 | EALFDGGGGGPAVSDLsQ | SEQ ID NO:237 |
| 230 | DENND2A | NP_056504.2 | Unknown function | S504 | RLsQSMESNSGK | SEQ ID NO: 238 |
| 231 | DKFZp686L18 14 | NP_919258.1 | Unknown function | S274 | VLsQSTPGTPSK | SEQ ID NO: 239 |
| 232 | EMSY | NP_0645782 | Unknown function | T799 | SKPRQPtIDLSQMAVPIQMTQE | SEQ ID NO: 240 |
| 233 | Exoc8 | NP_787072.2 | Vesicle protein | T142 | GQAGFFStPGGASR | SEQ ID NO: 241 |
| 234 | FAM62A | NP_056107.1 | Unknown function | T269 | IRRPtLDINW | SEQ ID NO:242 |
| 235 | FEZ2 | NP_0050932 | Unknown function | S195 | LSMLsQEICTLK | SEQ ID NO: 243 |
| 236 | FLJ20014 | NP_060092.1 | Unknown function | T62 | DGLSEACGGAGSSGSAESGAGGGRRAtIS SPL | SEQ ID NO: 244 |
| 237 | FLJ21128 | NP_079359.2 | Unknown function | S125 | RTDVKsQDVAVSPQQQQCSK | SEQ ID NO: 245 |
| 238 | FLJ21438 | XP_029084.7 | Unknown function | T85 | RWHtGGGGEKAAGGF | SEQ ID NO: 246 |
| 239 | FLJ21816 | NP_078951.2 | Unknown function | S376 | NENLQESEILsQPK | SEQ ID NO:247 |
| 240 | FLJ21816 | NP_0789512 | Unknown function | S59 | TVEEQDCLsQQDLSPQLK | SEQ ID NO:248 |
| 241 | FLJ21908 | NP_078880_1 | Unknown function | S116 | ILDELDKDDSTHESLsQESESEEDGIHVDSQ K | SEQ ID NO: 249 |
| 242 | FLJ12626 | AAH32442.1 | Unknown function | S64 | NGL THQLGGLsQGSR | SEQ ID NO: 250 |
| 243 | FLJ23518 | NP_079001.2 | Unknown function | S154 | QTGQIIEDDQEKHs-sQEDNDLNK | SEQ ID NO: 251 |
| 244 | FLJ30655 | NP_653244.1 | Unknown function , | S656 | RLsQAEER | SEQ ID NO: 252 |
| 245 | FLJ32312 | NP 653310.1 | Unknown function | S84 | LQELEDSiDNLsQNGEGR | SEQ ID NO:253 |
| 246 | Frigg | NP.0561891 | Unknown function | T124 | VGRKAIEDFLGSSSGY | SEQ ID NO: 254 |
| 247 | FTSJ1 | NP_036412.1 | Unknown function | T302 | AKEIRPQDCPISRVDTFPQPL | SEQ ID NO: 255 |
| 248 | HDGFRP3 | NP_057157.1 | Unknown function | T193 | GGDAGNDTRNTtSDLQKTSE | SEQ ID NO: 256 |
| 249 | HPS3 | NP_115759.2 | Vesicle protein | S473 | SRKDTsVKIKIPPVAEAGW | SEQ ID NO: 257 |
| 250 | HPS3 | NP_115759.2 | Vesicle protein | T472 | SRKDtSVKIKIPPVAEAGW | SEQ ID NO: 258 |
| 251 | HRBL | NP_006067.3 | Unknown function | S451 | LGQRPLsQPAGISTNPFMTGPSSSPFASKP PTTNPFL | SEQ ID NO: 259 |
| 252 | HSPC148 | NP_057487.2 | Unknown function | T47 | QTTQDAPEEVR | SEQ ID NO: 260 |
| 253 | IGF2BP3 | NP_0065362 | RNA processing | T249 | SITILSTPEGTSAACK | SEQ ID NO:261 |
| 254 | IK | NP_006074.1 | Secreted protein | T485 | WDFDTQEEYSEYMNNK | SEQ ID NO: 262 |
| 255 | KIAA0232 | NP_055558.1 | Unknown function | T430 | RNRQDtSDLTSEAVEELSESVHGL | SEQ ID NO: 263 |
| 256 | KIAA0323 | NP_056114.1 | Unknown function | S10 | PAsPDRFAVSAEAENKVR | SEQ ID NO: 264 |
| 257 | KIAA0376 | NP_056145.1 | Unknown function | S832 | GLSRRSsTSSEPTPTVKTL | SEQ ID NO: 265 |
| 258 | KIAA0415 | NP_00103224 2.1 | Unknown function | T436 | SGGLQtPECLSR | SEO ID NO: 266 |
| 259 | KIAA0460 | NP-056018.1 | Unknown function | S448 | PSPGTPTsPSNLTSGLK | SEQ ID NO: 267 |
| 260 | KIAA0467 | NP_056099.2 | Unknown function | S752 | STSESSASFPRsPGQPSSLR | SEQ ID NO: 268 |
| 261 | KIAA0528 | NP_055617.1 | Unknown function | T339 | QQtQSALEQR | SEQ ID NO: 269 |
| 262 | KIAA0674 | XP_376903.3 | Unknown function | S1079 | RPsQEQSASASSGQPQAPLNR | SEQ ID NO: 270 |
| 263 | KIAA0690 | NP_055994.1 | Unknown function | T77 | LGKSEAPEtPMEEEAELVLTEK | SEQ ID NO: 271 |
| 264 | KIAA0711 | NP_055682.1 | Unknown function | S314 | AGSRPQsPSGDADAR | SEQ ID NO: 272 |
| 265 | KIAA0794 | XP _087353.6 | Unknown function | T273 | ASLQETHFDStQTK | SEQ ID NO: 273 |
| 266 | KIAA1064 | XP_028253.5 | Unknown function | T1153 | VLAAGGLGQGGGGGQSSVLSGISLYDPRtP NAGGK | SEQ ID NO: 274 |
| 267 | KIAA1522 | NP_065939.2 | Unknown function | T221 | RRStVLGLPQHVQKE | SEQ ID NO: 275 |
| 268 | KAA1618 | NP_066005.2 | Unknown function | S217 | GLsQEGTGPPTSAGEGHSR | SEQ ID NO: 276 |
| 269 | KIAA1671 | XP_371461.5 | Unknown function | T1070 | SRQPtEPKDTDTLVHEAGSQY | SEQ ID NO: 277 |
| 270 | KIAA1904 | NP_443138.2 | Unknown function | S725 | HSFPALYYEEGADSLsQR | SEQ ID NO: 279 |
| 271 | Kidins220 | NP_065789.1 | Unknown function , | S1681 | NLNRTPsTVTL | SEQ ID NO: 280 |
| 272 | KLHDC5 | NP_065833.1 | Unknown function | S43 | EALsQEAGGPEVQQLR | SEQ ID NO: 281 |
| 273 | LARP | NP_0561302 | Unknown function | S979 | CPsQSSSRPAAMISQPPTPPTGQPVR | SEQ ID NO: 282 |
| 274 | LARP | NP_056130.2 | Unknown function | T299 | KFDGVEGPRtPK | SEQ ID NO: 283 |
| 275 | LARP | NP_056130.2 | Unknown function | S990 | CPSQSSSRPAAMIsQPPTPPTGQPVR | SEQ ID NO: 284 |
| 276 | LARP5 | NP_055970.1 | Unknown function | S649 | LRKPsYAE | SEQ ID NO: 285 |
| 277 | LEREPO4 | NP_0609412 | Unknown function | T357 | RFStYTSDKDE | SEQ ID NO: 286 |
| 278 | liprin alpha 1 | NP_003617.1 | Adhesion or extracellular matrix protein | S790 | DIRNSTGSQDGPVSNPSSSNSsQDSLHK | SEQ ID NO: 287 |
| 279 | liprin alpha 1 | NP- 003617.1 | Adhesion or extracellular matrix protein | S776 | DIRNSTGsQDGPVSNPSSSNSSQDSLHK | SEQ ID NO: 288 |
| 280 | LM07 | NP_005349.3 | Unknown function | S961 | SLTTREPsLATW | SEQ ID NO: 289 |
| 281 | LM07 | NP_005349.3 | Unknown function | T598 | PRSYtMDDAW | SEQ ID NO: 290 |
| 282 | LOC124245 | NP_6532052 | Unknown function | S78 | GPSOEEEDNHSDEEDRASEPKsQDQDSEV NELSR | SEQ ID NO: 291 |
| 283 | LOC150207 | XP_039721.5 | Unknown function | S302 | FIsQELPSQR | SEQ ID NO: 292 |
| 284 | LOC92017 | XP_00113189 0.1 | Unknown function | S282 | GPVPGDLsQTSEDQSLSDFEISNR | SEQ ID NO: 293 |
| 285 | LRRC16 | NP 060110.3 | Unknown function | S1279 | TFsQEVSR | SEQ ID NO: 294 |
| 286 | LUZP1 | NP_3610132 | Unknown function | T958 | TORSSTDFSELEOPRSCLF | SEQ ID NO: 295 |
| 287 | Lyric | NP 848927.1 | Adhesion or extracellular matrix protein | T143 | TVEVAEGEAVRtPQSVTAK | SEQ ID NO: 296 |
| 288 | LYST | NP 000072.2 | Unknown function | S2264 | AFPSQNGSAAVGRWPsLVDRNTDDWENF | SEQ ID NO: 297 |
| 289 | MAGE-D2 | NP-055414.2 | Unknown function | S146 | AQETEAAPsQAPADEPEPESAAAQSQENQ DTRPK | SEQ ID NO: 298 |
| 290 | MGC14289 | NP_542391.1 | Unknown function, | S263 | MLENTDNSSPSTEHsQGLEK | SEQ ID NO: 299 |
| 291 | MGC3329 | NP 076991.3 | Unknown function | S455 | EGEAAAVEGPCPSQESLsQEENPEPTEDE RSEEK | SEQ ID NO: 300 |
| 292 | MGC3329 | NP_076991.3 | Unknown function | S450 | EGEAAAVEGPCPsQESLSQEENPEPTEDE RSEEK | SEQ ID NO: 301 |
| 293 | MON1A | NP_115731.1 | Unknown function | S91 | QlsQDFSELSTQLTGVAR | SEQ ID NO: 302 |
| 294 | MPHOSPH8 | NP_059990.2 | Unknown function | T496 | NKQSLKERRNtRDETDTWAYIAAEGDQE | SEQ ID NO:303 |
| 295 | MYO98 | NP_004136.2 | Motor or contractile protein | S1972 | KEILIERIQsIKEE | SEQ ID NO: 304 |
| 296 | NDRG3 | NP_114402.1 | Unknown function , | T355 | SVTSNQSDGtQESCESPQVLDR | SEQ ID NO: 305 |
| 297 | NETO1 | NP_620416.1 | Unknown function | S483 | HNYsQDAADACDIDEIEEVPTTSHR | SEQ ID NO:306 |
| 298 | neurobeachin | NP_056493.3 | Vesicle protein | S1007 | EIEDLsQSQSPESETDYPVSTDTR | SEQ ID NO: 307 |
| 299 | NIFK | NP_115766.2 | RNA processing | T223 | VSGTLDtPEKTVDSQGPTPVCTPTFLER | SEQ ID NO: 308 |
| 300 | NRG1 | NP_039251.2 | Ligand, receptor tyrosine kinase | T525 | IVEDEEYETTQEYEPAQEPVKK | SEQ ID NO: 309 |
| 301 | OSBPL8 | NP_065892.1 | Unknown function | T39 | DVLGPSTWANSDESQLLtPGKMSQR | SEQ ID NO: 311 |
| 302 | OXR1 | NP_851999.1 | Unknown function | S112 | TGiRPARWsSTSEEEEAFTEKF | SEQ ID NO: 312 |
| 303 | p400 | NP_0562242 | Apoptosis | S2049 | AEEFWLsQEPSVTETIAPK | SEQ ID NO: 313 |
| 304 | PAWR | NP_002574.2 | Apoptosis | S162 | KRRsTGVVNIPAAECLDEYEDDE | SEQ ID NO: 314 |
| 305 | PHF14 | NP_055475.2 | Unknown function | S294 | NSADDEELTNDSLTLsQSK | SEQ 10 NO: 315 |
| 306 | PHF2 | NP_005383.3 | Unknown function | S942 | VASIETGLAAAAAKLsQQEEQK | SEQ ID NO: 316 |
| 307 | PHF6 | NP-115834.1 | Unknown function | T358 | GKVEiDQQQLtQQQLNGN | SEQ ID N0: 317 |
| 308 | Plakophilin 3 | NP_009114.1 | Adhesion or extracellular matrix protein | S314 | QRLSsGFDDiDLPSAVKY | SEQ ID NO: 318 |
| 309 | POP1 | NP_055844.1 | RNA processing | S95 | AGPEGTsQEIPK | SEQ ID NO: 319 |
| 310 | PRICKLE1 | NP_694571.1 | Unknown function | T21 | GCQRSStSDDDSGCALEEY | SEQ ID NO: 320 |
| 311 | PRICKLE1 | NP 694571.1 | Unknown function | S476 | YQSDMYWAQsQDGLGDSAYGSHPGPASS R | SEQ ID NO: 321 |
| 312 | PRUNE | NP_067045.1 | Unknown function | S399 | ASNSLISGLsQDEEDPPLPPTPMNSLVDEC PLDOGLPK | SEQ ID NO: 322 |
| 313 | pumilio 2 | NP_056132.1 | RNA processing | T396 | RAGAGQRPLtPNQGQQGQQAESL | SEO ID NO: 323 |
| 314 | Q8WVJ2 | NP 660309.1 | Unknown function | T123 | SEYAADPWVQDQMQRKLtLE | SEO ID NO: 324 |
| 315 | R3HDM2 | NP_055740.2 | Unknown function | S113 | DLSNPFGQMSLSRQGsTE | SEQ ID NO: 325 |
| 316 | R3HDM2 | BAA768462 | Unknown function | S129 | MLSRDSsQEYTDSTGIDLHEFLVNTLKK | SEQ ID NO:326 |
| 317 | Rab 11FIP1 | NP_079427.3 | Vesicle protein | S500 | KDTAAVVSRQGsSLNLFE | SEQ ID NO: 327 |
| 318 | RAB31P | NP_783323.1 | Unknown function | S149 | ACDGSDDIFGLSTDSLSRLRSPsVLE | SEQ ID NO: 328 |
| 319 | REPS1 | NP _1141282 | Unknown function | S221 | IRRQSsSYDDPWKITDE | SEQ ID NO:329 |
| 320 | RNF138 | NP_057355.2 | Unknown function | S124 | YQDEYGVSSIIPNFQlsQDSVGNSNR | SEQ ID NO: 330 |
| 321 | RPRC1 | NP_060537.3 | Unknown function | T371 | SASASPLtPCSVTR | SEQ ID NO: 331 |
| 322 | SACS | NP_055178.2 | Unknown function | S2364 | RYAsNVCFTTLGTE | SEQ ID NO: 332 |
| 323 | SAMD4 | NP_056404.2 | RNA processing | T716 | HALGDGVDRTSU | SEQ ID NO: 333 |
| 324 | SAMD9 | NP_ 060124.2 | Unknown function | S156 | TKERQPsIDLTCVSYPFDEF | SEQ ID NO: 334 |
| 325 | SCAMP2 | NP_005688.2 | Vesicle protein | S319 | HRAAsSAAQGAFQGN | SEQ ID NO: 335 |
| 326 | SCC-112 | NP_056015.1 | Unknown function | S1146 | LNPSTGNRSREQsSEAAETGVSENE | SEQ ID NO: 336 |
| 327 | SGOL2 | NP_689737.3 | Unknown function | S507 | ITNEQEETYSLsQSSGK | SEQ ID NO: 337 |
| 328 | SHKBP1 | NP_612401.1 | Unknown function | T696 | RPPTPAPWPSSGLGTPLtPPKMK | SEQ ID NO: 338 |
| 329 | Siglec-10 | NP 1491212 2 | Adhesion or extracellular matrix protein | T593 | SRHStILDYINWPTAGPL | SEQ ID NO: 339 |
| 330 | SMC6L1 | NP_078900.1 | Unknown function | T1083 | GQTTLPFRPVTQEEDDDQR | SEQ ID NO: 340 |
| 331 | SPAG9 | NP_003962.3 | Unknown function | S315 | TRNVsTGSAENEEKSE | SEQ ID NO: 341 |
| 332 | SPG21 | NP_057714.1 | Unknown function | S304 | GSLGIsQEEQ | SEQ ID NO: 342 |
| 333 | STX6 | NP_005810.1 | Vesicle protein | S152 | LQRANsHFIEE | SEQ ID NO: 343 |
| 334 | SUHW3 | NP.060136.1 | Unknown function | S155 | NSQVGSDNSSILLFDSTQESLPPsQEIPAIFR | SEQ ID NO: 344 |
| 335 | SYN1 | NP_008881.2 | Vesicle protein | S683 | SQSLTNAFNLPEPAPPRPSLsQDEVKAETIR | SEQ ID NO: 345 |
| 336 | TAF15 | NP_003478.1 | RNA processing | S76 | QSSYsQQPYNNQGQQQNMESSGSQGGR | SEQ ID NO: 346 |
| 337 | THOC4 | NP_0057732 | Chaperone | T256 | LDAQLDAYNARMDtS | SEQ ID NO: 347 |
| 338 | TMEM51 | NP _060492.1 | Unknown function | T234 | NVPPPSIEPLtPPPQYDEVQEK | SEQ ID NO: 348 |
| 339 | VAMP8 | NP_003752.2 | Vesicle protein | T48 | HLRNKLEDLEATSE | SEQ ID NO: 349 |
| 340 | VPS13C | NP_065872.1 | Unknown function | S2485 | SILSRQEsSFF | SEQ ID NO :350 |
| 341 | YEATS2 | NP_060493.3 | Unknown function | S1122 | VKTEPETPGPSCLsQEGQTAVK | SEQ ID NO: 351 |
| 342 | ZNF16 | NP_008889.2 | Unknown function | S131 | LPQSLsQEGDFTPAAMGLLR | SEQ ID NO: 352 |
| 343 | ZNF295 | NP_065778.3 | Unknown function | S381 | SSQGSSSVSSDAPGNVLCALsQK | SEQ ID NO: 353 |
| 344 | ZNF365 | NP_55766.1 | Unknown function | T205 | AAFVQLtQK | SEQ ID NO: 354 |
| 345 | ZNF609 | NP_055857.1 | Unknown function | T746 | ESSKELESPLtPGKVCR | SEQ ID NO: 355 |
| 346 | ZSCAN5 | NP_077279.1 | Unknown function | S308 | SKPDASSlsQEEPQGEATPVGNR | SEQ ID NO: 356 |

One of skill in the art will appreciate that, in many instances the utility of the instant invention is best understood in conjunction with an appreciation of the many biological roles and significance of the various target signaling proteins/polypeptides of the invention. The foregoing is illustrated in the following paragraphs summarizing the knowledge in the art relevant to a few non-limiting representative peptides containing selected phosphorylation sites according to the invention.

FOXO3A, phosphorylated at S294, is among the proteins listed in this patent. Forkhead box 03 is a member of the forkhead family of transcription factors and regulates the expression of genes that mediate apoptosis (Biochim Biophys Acta 2008 Jan;1785(l):63-84; Oncogene. 2005 Mar 17;24(12):2020-31), cell cycle progression (Cell. 2004 May 14;117(4):421-6), oxidative stress responses (Mol Cell. 2007 Dec 28;28(6):941-53), and tumorigenesis (J Biol Chem. 2007 Jan 26;282(4):2211-20). Recently, Yang and colleagues demonstrated that S294 is one of the phosphorylated sites that determines the subcellular localization of FOXO3A and, therefore, its ability to regulate these genes (Fig 1; Nat Cell Biol 2008 Feb;10(2):13848). Molecular probes, including antibodies, for pS294 would provide tools to elucidate the pathways involved in apoptosis, cell cycle progression, oxidative stress responses, and tumorigenesis. Consistent with its role in affecting apoptosis and tumorigenesis, FOXO3A has been connected with several diseases, specifically: increased phosphorylation may be associated with breast neoplasms and multiple myeloma; gene mutation causes premature ovarian failure, gene translocation is associated with acute myeloblastic leukemia (Science. 2003 Jul 11;301(5630):215-8; Biochem Biophys Res Commun 2002 Oct 4;297(4):760-4.; PhosphoSite(R), Cell Signaling Technology (Danvers, MA); Human PSD(TM), Biobase Corporation, (Beverly, MA)). It has also been proposed as a target for cancer therapy (Biochim Biophys Acta 2008 Jan;1785(1):63-84).

Lamin A/C, phosphorylated at S458, is among the proteins listed in this patent. Lamin A/C is a nuclear intermediate filament protein that forms the nuclear lamina lining the inside surface of the nuclear envelop, directly interacting with nuclear components. The nuclear envelop disassembles and reassembles during mitosis, and the proteins of the nuclear lamina, including lamin A/C, interact with DNA to mediate transcriptional repression (Reddy KL, et al (2008) Nature 452:243-7). We postulate that post-translational modifications of Lamin A/C may, among other things, regulate the ability of the inner nuclear membrane to bind DNA, thus regulation transcriptional activity. The inner nuclear envelop also sequesters transcription factors away from chromatin, providing another mechanism whereby nuclear lamins can repress transcription (Heessen S, Fomerod M (2007) EMBO Rep. 8:914-9). This provides another mechanism whereby post-translational modifications of Lamin A/C may regulate transcriptional activity, this time by sequestering transcription factors away from active chromatin. Antibodies or other molecular probes against lamin A/C and its post-translational modifications will help researchers elucidate the role of lamin A/C in nuclear dissolution during mitosis, transcriptional regulation, protein-protein interactions, and regulation of molecular events at the nuclear peripohery. Lamin A/C is cleaved during apoptosis. The amino acid sequence surrounding S458, having Arg at -5 and at -3 (RLRNKpS), has the hallmarks of being a substrate of a subset of critical growth-regulating kinases of the AGC family like Akt and p90-RSK. Antibodies against this site may be of especial value in investigating the effects of pro-growth and anti apoptotic kinases of the AGC family in regulating growth-associated and anti-apoptotic signals at the inner nuclear membrane, and the anti-apoptotic effects of Akt and similar kinases in promoting many forms of cancer. Mutations in the lamin A/C gene cause progeria (Proc Natl Acad Sci U S A. 2007 Mar 20;104(12):4949-54; Proc Natl Acad Sci USA. 2007 Mar 20;104(12):4955-60), Emery-Dreifuss muscular dystrophy(Am J Hum Genet 2000 Apr;66(4):1407-12.), familial partial lipodystrophy(Nat Genet. 2000 Feb;24(2): 153-6; Am J Hum Genet. 2000 Apr;66(4):1192-8); metabolic syndrome and dilated cardiomyopathy (Exp Mol Med. 2007 Feb 28:39(1):114-20). This protein has potential diagnostic and/or therapeutic value for these diseases as well as the many forms of cancer in which Akt and similar kinases play crucial roles. (PhosphoSite(R), Cell Signaling Technology (Danvers, MA), Human PSD(TM), Biobase Corporation, (Beverly, VIA)). In addition, molecular probes, including antibodies, for pS458 may help understand the role of kinases such as Akt in regulating mitosis in normal and diseased cells.

Bcr, phosphorylated at S301, is among the proteins listed in this patent. Bcr, also known as breakpoint cluster region protein, is a serine/threonine protein kinase and a GTPase-activating protein (GAP) for RAC1 and CDC42 (cell division cycle 42). Bcr participates in a t(9;22)(q34;q11) chromosomal translocation that produces the BCR-ABL oncogene responsible for chronic myeloid leukemia (CML), acute myeloid leukemia (AML) and acute lymphoblastic leukemia (ALL). The function of wild type Bcr in cells remains unclear, but its GTPase-activating activity implies critical regulatory functions. Rac and Cdc42 and their downstream effectors control many cellular processes, including morphogenesis, cell survival, motility, transcription, apoptosis and receptor signalling (Arias-Romero LE, Chernoff J.(2008) Biol Cell. 100:97-108). Antibodies or other molecular probes against Bcr and its post-translational modifications will help researchers elucidate the role of Bcr including morphogenesis, cell survival, motility, transcription, apoptosis and receptor signalling. The PDGF receptor may use Bcr as a downstream signaling mediator. Amino acid variants of the Bcr protein may be associated with bipolar disorder (Prog Neuropsychopharmacol Biol Psychiatry. 2008 Jan 1;32(1):204-8; Biol Psychiatry. 2005 May 15;57(10):1097-102). Bcr has potential diagnostic and/or therapeutic implications for multiple leukemias, multiple myeloma, proliferative disorders, and biopolar disorder (PhosphoSite(R), Cell Signaling Technology (Danvers, MA), Human PSD(TM), Biobase Corporation, (Beverly, MA)). In addition, antibodies against modifications such as pS301 on Bcr may provide insight into its roles in rac and ras signalling pathways (Mol Cell Biol. 2003 Jul;23(13):4663-72).

RALBP 1, phosphorylated at S29, is among the proteins listed in this patent. The protein, also known as RalA binding protein 1, is an ATPase that catalyzes the transport of xenobiotics (Biochemistry. 2000 Aug 8:39(3 1):9327-34). It confers doxorubicin and vinorelbine resistance to lung cancer cells (FEBS Lett. 2005 Aug 29;579(21):4635-41) and may play a role in drug resistance in epilepsy (BMC Neurosci. 2005 Sep 27;6:61). RALBP1 also functions as a GTPase activating protein for RACI and CDC42 (J Cell Biol. 2006 Sep 11;174(6):877-88; J Biol Chem. 1995 Sep 22;270(38):22473-7) and interacts with RaIGTP in response to heat shock (J Biol Chem. 2003 May 9;278(19):17299-306; PhosphoSite(R), Cell Signaling Technology (Danvers, MA); Human PSD(TM), Biobase Corporation, (Beverly, MA)). Molecular probes for modifications to RALBP1 such as pS29 may help elucidate both immediate and adaptive cellular responses to stress.

IFNGR1, phosphorylated at T295, is among the proteins listed in this patent. Interferon gamma receptor 1 is involved in defense response and STAT phosphorylation. Gene mutations and reduced expression are associated with mycobacterial infection (Lancet. 2004 Dec 11-17;364(9451):2113-21), viral infection (J Pediatr. 1999 Nov;135(5):640-3), liver cirrhosis (Hepatogastroenterology. 1988 Aug;35(4):158-61), chronic hepatitis C (Biochem J. 2004 Apr 1;379(Pt 1):199-208), tumor formation (Nature. 2001 Apr 26;410(6832):1107-11), immune-mediated fibrotic disorders (Am J Pathol. 2001 Jun; 158(6):2117-25), and systemic lupus erythematosus (Ann N Y Acad Sci. 2003 Apr;987:236-9). IFNGR1 has potential diagnostic and/or therapeutic implications for these diseases (EurJ Hum Genet. 2005 May;13(5):660-8; Am J Hum Genet 1999 Sep;65(3):709-21; PhosphoSite(R), Cell Signaling Technology (Danvers, MA); Human PSD(TM), Biobase Corporation, (Beverly, MA)). Molecular probes for INFGRI and modifications such as pT295 would aid in differentiating type I and type II responses to challenges to the immune system.

POLR2A, phosphorylated at T1903, is among the proteins listed in this patent. Polymerase (RNA) II (DNA directed) polypeptide A 220kDa is a subunit of the DNA-directed RNA polymerase that synthesizes mRNA in eukaryotes. T1903 resides in a variation of the highly repeated heptapeptide comprising the C-terminal domain (CTD) of POLR2A. Differential phosphorylation of the five phosphorylatable residues in the CTD regulates its functions in capping, splicing, cleaving, and polyadenylating mRNAs (Genes Dev. 2005 Jun 15;19(12):1401-15). An antibody against pT1903 would provide a valuable tool in breaking the code that "specifies the position of Pol II within the transcription cycle" (Genes Dev. 2005 Jun 15;19(12):1401-15). This protein also has potential diagnostic and/or therapeutic implications for Systemic Scleroderma (J Immunol 2001 Dec 15;167(12):7126-33; PhosphoSite(R), Cell Signaling Technology (Danvers, MA); Human PSD(TM), Biobase Corporation, (Beverly, MA)).

CHD-4, phosphorylated at S515, is among the proteins listed in this patent. CHD-4 (chromodomain helicase DNA binding protein 4) is an ATP-dependent helicase and a central component of the HDAC-containing nucleosome remodeling and histone deacetylase (NuRD) transcriptional complex. CHD-4 (Mi-2) interacts with MTA1/2, HDAC1, and RbAp46 in the NuRD complex. CHD-4 belongs to the SNF2/RAD54 helicase family of proteins. The NuRD complex, whose subunit composition varies by cell type and in response to physiologic signals, plays central roles in many processes including oncogenesis and metastasis (Denslow SA (2007) Oncogene 26:5433-8), B cell differentiation (Fujita N (2004) Cell. 2004 119:75-86), invasive growth of breast cancer (Fujita N et al (2003) Cell 113:207-19), and maintaining the balance between the undifferentiated state and the process of differentiation in embryonic stem cells (Crook JM (2006) Nature Cell Biology 8, 212-214). CHD-4 has potential research, diagnostic and/or therapeutic implications for stem cell research, and in the processes of development and differentiation including B cell development and leukemogenesis and lymphomagenesis, and the process of metastasis and aggressiveness in various cancers including breast cancer. (PhosphoSite(R), Cell Signaling Technology (Danvers, MA), Human PSD(TM); Biobase Corporation, (Beverly, MA)).

Pyk2, phosphorylated at T842, is among the proteins listed in this patent. Protein tyrosine kinase 2 beta or focal adhesion kinase activates the MAP kinase pathway, plays roles in T-cell activation, cell motility and apoptosis. Its expression is altered in breast and prostate cancer, and it plays roles in glioma and glioblastoma. This protein has potential diagnostic and/or therapeutic implications based on association with the following diseases: Glioma (Int J Cancer. 2005 Feb 20;113(5):689-98.). (PhosphoSite(R), Cell Signaling Technology (Danvers, MA), Human PSD(TM), Biobase Corporation, (Beverly, MA)).

NFkB-p105, phosphorylated at S938, is among the proteins listed in this patent. Nuclear factor NF-kappa-B p50 is a transcriptional activator. Its increased expression correlates with oral cancer. Increased transcription factor activity may be associated with B-cell leukemia, arteriosclerosis, prostatic, and pancreatic neoplasms. This protein has potential diagnostic and/or therapeutic implications based on association with the following diseases: Prostatic Neoplasms (Anticancer Res 2003 Sep-Oct;23(5A):3855-61.). (PhosphoSite(R), Cell Signaling Technology (Danvers, MA), Human PSD(TM), Biobase Corporation, (Beverly, MA)).

CCT2, phosphorylated at S260, is among the proteins listed in this patent. Chaperonin containing T-complex polypeptide-1 (TCP1) subunit 2 is the beta subunit of the chaperonin containing complex (CCT) and plays a role in the folding of cytosolic proteins. It may be required for the proper folding of cyclin E. (PhosphoSite(R), Cell Signaling Technology (Danvers, MA), Human PSD(TM), Biobase Corporation, (Beverly, MA)).

WFS1, phosphorylated at S235, is among the proteins listed in this patent and has potential diagnostic and/or therapeutic implications based on association with type II diabetes (Nat Genet. 2007 Aug;39(8):951-3) and Wolfram Syndrome (Am J Hum Genet 1999 Nov;65(5): 1279-90.). (PhosphoSite(R), Cell Signaling Technology (Danvers, MA), Human PSD(TM), Biobase Corporation, (Beverly, MA)).

OSR1, phosphorylated at T310, is among the proteins listed in this patent. Oxidative-stress responsive 1, a serine-threonine kinase that binds and threonine phosphorylates p21 activated kinase 1 (PAK1), is activated by osmotic stress. (PhosphoSite(R), Cell Signaling Technology (Danvers, MA), Human PSD(TM), Biobase Corporation, (Beverly, MA)).

PKN3, phosphorylated at T718, is among the proteins listed in this patent. Protein kinase N3 is regulated by phosphatidylinositol 3-kinase and binds GRAF, GRAF2 and phospholipase D1 (PLD1). It is upregulated in prostate cancer and involved in lymph node metastasis of prostate cancer cells in nude mice. (PhosphoSite(R), Cell Signaling Technology (Danvers, MA), Human PSD(TM), Biobase Corporation, (Beverly, MA)).

HER2, phosphorylated at T671, is among the proteins listed in this patent. HER2, also known as herstatin, is a receptor tyrosine kinase that is involved in angiogenesis, immune response, and protein kinase C activation. It regulates transcription, cell proliferation, differentiation, and migration. HER2's aberrant expression is associated with several neoplasms. This protein has potential diagnostic and/or therapeutic implications based on association with Pancreatic Neoplasms (Anticancer Res 1994 Sep-Oct; 14(5A): 1919-22.). (PhosphoSite(R), Cell Signaling Technology (Danvers, MA), Human PSD(TM), Biobase Corporation, (Beverly, MA)).

53BP1, phosphorylated at S523, is among the proteins listed in this patent. Tumor protein p53 binding protein 1, binds to TP53 and promotes transcriptional activation. It also acts in DNA damage checkpoint signaling and DNA repair. A TP53BP1 - PDGFRB fusion protein has been identified in a patient with in atypical chronic myeloid leukemia and thus has potential diagnostic and/or therapeutic implications for CML (Cancer Res. 2004 Oct 15;64(20):7216-9.). (PhosphoSitel,R), Cell Signaling Technology (Danvers, MA), Human PSD(TM), Biobase Corporation, (Beverly, MA)).

SLY, phosphorylated at S146, is among the proteins listed in this patent. SLY, an SH3 domain and a C-terminal sterile alpha motif (SAM) containing protein is phosphorylated in response to T cell receptor stimulation. (PhosphoSite(R), Cell Signaling Technology (Danvers, MA), Human PSD(TM), Biobase Corporation, (Beverly, MA)).

ORAII, phosphorylated at T295, is among the proteins listed in this patent. ORAI1 is a component or regulator of the Ca2+ release activated Ca2+ channel complex; missense mutation causes defective store operated Ca2+ entry and CRAC channel function in hereditary severe combined immune deficiency (SCID) syndrome. (Nature. 2006 May 11;441(7090):179-85; PhosphoSite(R), Cell Signaling Technology (Danvers, MA), Human PSD(TM), Biobase Corporation, (Beverly, MA)).

Rif1, phosphorylated at S 1184, is among the proteins listed in this patent. Rif1 localizes to dysfunctional, but not functional, telomeres. It functions downstream of TP53BP1 and ATM and plays a role in DNA damage response and S-phase checkpoint control. (PhosphoSite(R), Cell Signaling Technology (Danvers, MA), Human PSD(TM), Biobase Corporation, (Beverly, MA)).

MDCl, phosphorylated at T1466, is among the proteins listed in this patent. Mediator of DNA damage checkpoint protein 1, undergoes (ATM)-dependent phosphorylation in response to ionizing radiation, which enables binding to CHEK2 and localization to sites of DNA damage, stabilizes TP53 following DNA damage, and regulates BRCA1. (PhosphoSite(R), Cell Signaling Technology (Danvers, MA), Human PSD(TM), Biobase Corporation, (Beverly, MA)).

PHKB, phosphorylated at S700 and S701, is among the proteins listed in this patent. Phosphorylase kinase beta phosphorylates and thereby activates glycogen phosphorylase; mutations in the corresponding gene cause phosphorylase-kinase-deficient liver and muscle glycogenosis. This protein has potential diagnostic and/or therapeutic implications based on association with the following diseases: Glycogen Storage Disease (Hum Genet 1997 Dec 10 1 (2)): 170-4). (PhosphoSite(R), Cell Signaling Technology (Danvers, MA), Human PSD(TM), Biobase Corporation, (Beverly, MA)).

The invention also provides peptides comprising a novel phosphorylation site of the invention. In one particular embodiment, the peptides comprise any one of the amino acid sequences as set forth in SEQ ID NOs: 1-44, 46-83, 85-110, 112-125, 127-132, 135-139, 141-148, 150-234, 236-277, 279-309, 311-356, which are trypsin-digested peptide fragments of the parent proteins. Alternatively, a parent signaling protein listed in Table 1 may be digested with another protease, and the sequence of a peptide fragment comprising a phosphorylation site can be obtained in a similar way. Suitable proteases include, but are not limited to, serine and threonine proteases (e.g. hepsin), metallo proteases (e.g. PUMP1), chymotrypsin, cathepsin, pepsin, thermolysin, carboxypeptidases, etc.

The invention also provides proteins and peptides that are mutated to eliminate a novel phosphorylation site of the invention. Such proteins and peptides are particular useful as research tools to understand complex signaling transduction pathways of cancer cells, for example, to identify new upstream kinase(s) or phosphatase(s) or other proteins that regulates the activity of a signaling protein; to identify downstream effector molecules that interact with a signaling protein, etc.

Various methods that are well known in the art can be used to eliminate a phosphorylation site. For example, the phosphorylatable serine or threonine may be mutated into a non-phosphorylatable residue, such as phenylalanine. A "phosphorylatable" amino acid refers to an amino acid that is capable of being modified by addition of a phosphate group (any includes both phosphorylated form and unphosphorylated form). Alternatively, the serine or threonine may be deleted. Residues other than the serine or threonine may also be modified (e.g., delete or mutated) if such modification inhibits the phosphorylation of the serine or threonine residue. For example, residues flanking the serine or threonine may be deleted or mutated, so that a kinase cannot recognize/phosphorylate the mutated protein or the peptide. Standard mutagenesis and molecular cloning techniques can be used to create amino acid substitutions or deletions.

### 2. Modulators of the Phosphorylation Sites

In another aspect, the invention provides a modulator that modulates serine or threonine phosphorylation at a novel phosphorylation site of the invention, including small molecules, peptides comprising a novel phosphorylation site, and binding molecules that specifically bind at a novel phosphorylation site, including but not limited to antibodies or antigen-binding fragments thereof.

Modulators of a phosphorylation site include any molecules that directly or indirectly counteract, reduce, antagonize or inhibit serine or threonine phosphorylation of the site. The modulators may compete or block the binding of the phosphorylation site to its upstream kinase(s) or phosphatase(s), or to its downstream signaling transduction molecule(s).

The modulators may directly interact with a phosphorylation site. The modulator may also be a molecule that does not directly interact with a phosphorylation site. For example, the modulators can be dominant negative mutants, *i.e.,* proteins and peptides that are mutated to eliminate the phosphorylation site. Such mutated proteins or peptides could retain the binding ability to a downstream signaling molecule but lose the ability to trigger downstream signaling transduction of the wild type parent signaling protein.

The modulators include small molecules that modulate the serine or threonine phosphorylation at a novel phosphorylation site of the invention. Chemical agents, referred to in the art as "small molecule" compounds are typically organic, non-peptide molecules, having a molecular weight less than 10,000, less than 5,000, less than 1,000, or less than 500 daltons. This class of modulators includes chemically synthesized molecules, for instance, compounds from combinatorial chemical libraries. Synthetic compounds may be rationally designed or identified based on known or inferred properties of a phosphorylation site of the invention or may be identified by screening compound libraries. Alternative appropriate modulators of this class are natural products, particularly secondary metabolites from organisms such as plants or fungi, which can also be identified by screening compound libraries. Methods for generating and obtaining compounds are well known in the art (Schreiber SL, Science 151: 1964-1969(2000); Radmann J. and Gunther J., Science 151: 1947-1948 (2000)).

The modulators also include peptidomimetics, small protein-like chains designed to mimic peptides. Peptidomimetics may be analogues of a peptide comprising a phosphorylation site of the invention. Peptidomimetics may also be analogues of a modified peptide that are mutated to eliminate a phosphorylation site of the invention. Peptidomimetics (both peptide and non-peptidyl analogues) may have improved properties (e.g., decreased proteolysis, increased retention or increased bioavailability). Peptidomimetics generally have improved oral availability, which makes them especially suited to treatment of disorders in a human or animal.

In certain embodiments, the modulators are peptides comprising a novel phosphorylation site of the invention. In certain embodiments, the modulators are antibodies or antigen-binding fragments thereof that specifically bind at a novel phosphorylation site of the invention.

### 3. Heavy-Isotope Labeled Peptides (AQUA Peptides).

In another aspect, the invention provides peptides comprising a novel phosphorylation site of the invention. In a particular embodiment, the invention provides Heavy-Isotype Labeled Peptides (AQUA peptides) comprising a novel phosphorylation site. Such peptides are useful to generate phosphorylation site-specific antibodies for a novel phosphorylation site. Such peptides are also useful as potential diagnostic tools for screening for diseases such as carcinoma or leukemia, or as potential therapeutic agents for treating diseases such as carcinoma or leukemia.

The peptides may be of any length, typically six to fifteen amino acids. The novel serine or threoninephosphorylation site can occur at any position in the peptide; if the peptide will be used as an immnogen, it preferably is from seven to twenty amino acids in length. In some embodiments, the peptide is labeled with a detectable marker.

"Heavy-isotope labeled peptide" (used interchangeably with AQUA peptide) refers to a peptide comprising at least one heavy-isotope label, as described in WO/03016861, "Absolute Quantification of Proteins and Modified Forms Thereof by Multistage Mass Spectrometry" (Gygi *et al.)* (the teachings of which are hereby incorporated herein by reference, in their entirety). The amino acid sequence of an AQUA peptide is identical to the sequence of a proteolytic fragment of the parent protein in which the novel phosphorylation site occurs. AQUA peptides of the invention are highly useful for detecting, quantitating or modulating a phosphorylation site of the invention (both in phosphorylated and unphosphorylated forms) in a biological sample.

A peptide of the invention, including an AQUA peptides comprises any novel phosphorylation site. Preferably, the peptide or AQUA peptide comprises a novel phosphorylation site of a protein in Table 1 that is an adaptor/scaffold protein, kinase/protease/phosphatase/enzyme proteins, protein kinase, cytoskeletal protein, ubiquitan conjugating system protein, chromatin or DNA binding/repair protein, g protein or regulator protein, receptor/channel/transporter/cell surface protein, transcriptional regulator and cell cycle regulation protein.

Particularly preferred peptides and AQUA peptides are these comprising a novel serine or threonine phosphorylation site (shown as a lower case "s" or "t" (respectively) within the sequences listed in Table 1) selected from the group consisting of SEQ ID NOs: 1 (4E-BP2), 2 (4E-BP2), 3 (BAP37), 4 (FOXO3A), 5 (JAZF1). 6 (NFkB-p105), 7 (PCDCSRP) 8 (TAF7), 38 (LIM), 39 (SD-95), 40 (RanBP2), 41 (Rictor), =12 (ZO2), 70 (B-CK), 71 (PIK4CA), 72 (PIP5K), 73 (Pnk1). 94 (ATP6VOD1), 116 (DBN1), 117 (lamin A/C), 118 (lamin B2), 138 (H.APIP), 139 (RALBP1), 140 (RGS12), 155 (Bcr), 156 (HER2), 157 (OSR1), 158 (Pyk2), 185 (KAB 1) 186 (NuMa-1), 197 (UREB 1), 207 (CCT2), 208 (CLH-17), 209 (N-cad), 210 (PROSC) and WFS1 211 (S235).

In some embodiments, the peptide or AQUA peptide comprises the amino acid sequence shown in any one of the above listed SEQ ID NOs. In some embodiments, the peptide or AQUA peptide consists of the amino acid sequence in said SEQ ID NOs. In some embodiments, the peptide or AQUA peptide comprises a fragment of the amino acid sequence in said SEQ ID NOs., wherein the fragment is six to twenty amino acid long and includes the phosphorylatable serine and/or threonine. In some embodiments, the peptide or AQUA peptide consists of a fragment of the amino acid sequence in said SEQ ID NOs., wherein the fragment is six to twenty amino acid long and includes the phosphorylatable serine and/or threonine.

In certain embodiments, the peptide or AQUA peptide comprises any one of SEQ ID NOs: 1-44, 46-83, 85- 110, 112-125, 127-132, 135-139, 141-148, 150-234, 236-277, 279-309, 311-356, which are trypsin-digested peptide fragments of the parent proteins.

It is understood that parent protein listed in Table 1 may be digested with any suitable protease (e.g., serine proteases (e.g. trypsin, hepsin), metallo proteases (e.g. PUMP1), chymotrypsin, cathepsin, pepsin, thermolysin, carboxypeptidases, etc), and the resulting peptide sequence comprising a phosphorylated site of the invention may differ from that of trypsin-digested fragments (as set forth in Column E), depending the cleavage site of a particular enzyme. An AQUA peptide for a particular a parent protein sequence should be chosen based on the amino acid sequence of the parent protein and the particular protease for digestion; that is, the AQUA peptide should match the amino acid sequence of a proteolytic fragment of the parent protein in which the novel phosphorylation site occurs.

An AQUA peptide is preferably at least about 6 amino acids long. The preferred ranged is about 7 to 15 amino acids.

The AQUA method detects and quantifies a target protein in a sample by introducing a known quantity of at least one heavy-isotope labeled peptide standard (which has a unique signature detectable by LC-SftM chromatography) into a digested biological sample. By comparing to the peptide standard, one may readily determines the quantity of a peptide having the same sequence and protein modification(s) in the biological sample. Briefly, the AQUA methodology has two stages:(1) peptide internal standard selection and validation; method development; and (2) implementation using validated peptide internal standards to detect and quantify a target protein in a sample. The method is a powerful technique for detecting and quantifying a given peptide/protein within a complex biological mixture, such as a cell lysate, and may be used, e.g., to quantify change in protein phosphorylation as a result of drug treatment, or to quantify a protein in different biological states.

Generally, to develop a suitable internal standard, a particular peptide (or modified peptide) within a target protein sequence is chosen based on its amino acid sequence and a particular protease for digestion. The peptide is then generated by solid-phase peptide synthesis such that one residue is replaced with that same residue containing stable isotopes (¹³C, ¹⁵N). The result is a peptide that is chemically identical to its native counterpart formed by proteolysis, but is easily distinguishable by MS via a mass shift. A newly synthesized AQUA internal standard peptide is then evaluated by LC-MS/MS. This process provides qualitative information about peptide retention by reverse-phase chromatography, ionization efficiency, and fragmentation via collision-induced dissociation. Informative and abundant fragment ions for sets of native and internal standard peptides are chosen and then specifically monitored in rapid succession as a function of chromatographic retention to form a selected reaction monitoring (LC-SRM) method based on the unique profile of the peptide standard.

The second stage of the AQUA strategy is its implementation to measure the amount of a protein or the modified form of the protein from complex mixtures. Whole cell lysates are typically fractionated by SDS-PAGE gel electrophoresis, and regions of the gel consistent with protein migration are excised. This process is followed by in-gel proteolysis in the presence of the AQUA peptides and LC-SRM analysis. (*See Gerber et al. supra.)* AQUA peptides are spiked in to the complex peptide mixture obtained by digestion of the whole cell lysate with a proteolytic enzyme and subjected to immunoaffinity purification as described above. The retention time and fragmentation pattern of the native peptide formed by digestion (e.g., trypsinization) is identical to that of the AQUA internal standard peptide determined previously; thus, LC-MS/MS analysis using an SRM experiment results in the highly specific and sensitive measurement of both internal standard and analyte directly from extremely complex peptide mixtures. Because an absolute amount of the AQUA peptide is added (e.g. 250 fmol), the ratio of the areas under the curve can be used to determine the precise expression levels of a protein or phosphorylated form of a protein in the original cell lysate. In addition, the internal standard is present during in-gel digestion as native peptides are formed, such that peptide extraction efficiency from gel pieces, absolute losses during sample handling (including vacuum centrifugation), and variability during introduction into the LC-MS system do not affect the determined ratio of native and AQUA peptide abundances.

An AQUA peptide standard may be developed for a known phosphorylation site previously identified by the IAP-LC-MS/MS method within a target protein. One AQUA peptide incorporating the phosphorylated form of the site, and a second AQUA peptide incorporating the unphosphorylated form of site may be developed. In this way, the two standards may be used to detect and quantify both the phosphorylated and unphosphorylated forms of the site in a biological sample.

Peptide internal standards may also be generated by examining the primary amino acid sequence of a protein and determining the boundaries of peptides produced by protease cleavage. Alternatively, a protein may actually be digested with a protease and a particular peptide fragment produced can then sequenced. Suitable proteases include, but are not limited to, serine proteases (e.g. trypsin, hepsin), metallo proteases (*e.g.* PUMP1), chymotrypsin, cathepsin, pepsin, thermolysin, carboxypeptidases, etc.

A peptide sequence within a target protein is selected according to one or more criteria to optimize the use of the peptide as an internal standard. Preferably, the size of the peptide is selected to minimize the chances that the peptide sequence will be repeated elsewhere in other non-target proteins. Thus, a peptide is preferably at least about 6 amino acids. The size of the peptide is also optimized to maximize ionization frequency. Thus, peptides longer than about 20 amino acids are not preferred. The preferred ranged is about 7 to 15 amino acids. A peptide sequence is also selected that is not likely to be chemically reactive during mass spectrometry, thus sequences comprising cysteine, tryptophan, or methionine are avoided.

A peptide sequence that is outside a phosphorylation site may be selected as internal standard to determine the quantity of all forms of the target protein. Alternatively, a peptide encompassing a phosphorylated site may be selected as internal standard to detect and quantify only the phosphorylated form of the target protein. Peptide standards for both phosphorylated form and unphosphorylated form can be used together, to determine the extent of phosphorylation in a particular sample.

The peptide is labeled using one or more labeled amino acids *(i.e.* the label is an actual part of the peptide) or less preferably, labels may be attached after synthesis according to standard methods. Preferably, the label is a mass-altering label selected based on the following considerations: The mass should be unique to shift fragment masses produced by MS analysis to regions of the spectrum with low background; the ion mass signature component is the portion of the labeling moiety that preferably exhibits a unique ion mass signature in MS analysis; the sum of the masses of the constituent atoms of the label is preferably uniquely different than the fragments of all the possible amino acids. As a result, the labeled amino acids and peptides are readily distinguished from unlabeled ones by the ion/mass pattern in the resulting mass spectrum. Preferably, the ion mass signature component imparts a mass to a protein fragment that does not match the residue mass for any of the 20 natural amino acids.

The label should be robust under the fragmentation conditions of MS and not undergo unfavorable fragmentation. Labeling chemistry should be efficient under a range of conditions, particularly denaturing conditions, and the labeled tag preferably remains soluble in the MS buffer system of choice. The label preferably does not suppress the ionization efficiency of the protein and is not chemically reactive. The label may contain a mixture of two or more isotopically distinct species to generate a unique mass spectrometric pattern at each labeled fragment position. Stable isotopes, such as ¹³C, ¹⁵N, ¹⁷O, ¹⁸O, or ³⁴5, are among preferred labels. Pairs of peptide internal standards that incorporate a different isotope label may also be prepared. Preferred amino acid residues into which a heavy isotope label may be incorporated include leucine, proline, valine, and phenylalanine.

Peptide internal standards are characterized according to their mass-to-charge (m/z) ratio, and preferably, also according to their retention time on a chromatographic column (e.g. an HPLC column). Internal standards that coelute with unlabeled peptides of identical sequence are selected as optimal internal standards. The internal standard is then analyzed by fragmenting the peptide by any suitable means, for example by collision-induced dissociation (CID) using, e.g., argon or helium as a collision gas. The fragments are then analyzed, for example by multi-stage mass spectrometry (MSⁿ) to obtain a fragment ion spectrum, to obtain a peptide fragmentation signature. Preferably, peptide fragments have significant differences in m/z ratios to enable peaks corresponding to each fragment to be well separated, and a signature that is unique for the target peptide is obtained. If a suitable fragment signature is not obtained at the first stage, additional stages of MS are performed until a unique signature is obtained.

Fragment ions in the MS/MS and MS³ spectra are typically highly specific for the peptide of interest, and, in conjunction with LC methods, allow a highly selective means of detecting and quantifying a target peptide/protein in a complex protein mixture, such as a cell lysate, containing many thousands or tens of thousands of proteins. Any biological sample potentially containing a target protein/peptide of interest may be assayed. Crude or partially purified cell extracts are preferably used. Generally, the sample has at least 0.01 mg of protein, typically a concentration of 0.1-10 mg/mL, and may be adjusted to a desired buffer concentration and pH.

A known amount of a labeled peptide internal standard, preferably about 10 femtomoles, corresponding to a target protein to be detected/quantified is then added to a biological sample, such as a cell lysate. The spiked sample is then digested with one or more protease(s) for a suitable time period to allow digestion. A separation is then performed (e.g., by HPLC, reverse-phase HPLC, capillary electrophoresis, ion exchange chromatography, etc.) to isolate the labeled internal standard and its corresponding target peptide from other peptides in the sample. Microcapillary LC is a preferred method.

Each isolated peptide is then examined by monitoring of a selected reaction in the MS. This involves using the prior knowledge gained by the characterization of the peptide internal standard and then requiring the MS to continuously monitor a specific ion in the MS/MS or MSⁿ spectrum for both the peptide of interest and the internal standard. After elution, the area under the curve (AUC) for both peptide standard and target peptide peaks are calculated. The ratio of the two areas provides the absolute quantification that can be normalized for the number of cells used in the analysis and the protein's molecular weight, to provide the precise number of copies of the protein per cell. Further details of the AQUA methodology are described in Gygi *et al.,* and Gerber *et al. supra.*

Accordingly, AQUA internal peptide standards (heavy-isotope labeled peptides) may be produced, as described above, for any of the 345 novel phosphorylation sites of the invention (see Table 1/Figure 2). For example, peptide standards for a given phosphorylation site (e.g., an AQUA peptide having the sequence WPGsPTSr (SEQ ID NO: 4), wherein "s" corresponds to phosphorylatable serine 294 of FOXO3A) may be produced for both the phosphorylated and unphosphorylated forms of the sequence. Such standards may be used to detect and quantify both phosphorylated form and unphosphorylated form of the parent signaling protein (e.g., FOX03A) in a biological sample.

Heavy-isotope labeled equivalents of a phosphorylation site of the invention, both in phosphorylated and unphosphorylated form, can be readily synthesized and their unique MS and LC-SRM signature determined, so that the peptides are validated as AQUA peptides and ready for use in quantification.

The novel phosphorylation sites of the invention are particularly well suited for development of corresponding AQUA peptides, since the IAP method by which they were identified (see Part A above and Example 1) inherently confirmed that such peptides are in fact produced by enzymatic digestion (e.g., trypsinization) and are in fact suitably fractionated/ionized in MS/MS. Thus, heavy-isotope labeled equivalents of these peptides (both in phosphorylated and unphosphorylated form) can be readily synthesized and their unique MS and LC-SRM signature determined, so that the peptides are validated as AQUA peptides and ready for use in quantification experiments.

Accordingly, the invention provides heavy-isotope labeled peptides (AQUA peptides) that may be used for detecting, quantitating, or modulating any of the phosphorylation sites of the invention (Table 1). For example, an AQUA peptide having the sequence VKRLLsTDAE (SEQ ID NO: 8), wherein y (Ser 171) is phosphotyrosine, and wherein V = labeled valine (e.g., ¹⁴C)) is provided for the quantification of phosphorylated (or unphosphorylated) form of TAF7 (a transcriptional regulator) in a biological sample.

Example 4 is provided to further illustrate the construction and use, by standard methods described above, of exemplary AQUA peptides provided by the invention. For example, AQUA peptides corresponding to both the phosphorylated and unphosphorylated forms of SEQ ID NO: 8 (a trypsin-digested fragment of TAF7, with a Tyrosine 1827 phosphorylation site) may be used to quantify the amount of phosphorylated TAF7 in a biological sample, e.g., a tumor cell sample or a sample before or after treatment with a therapeutic agent.

Peptides and AQUA peptides provided by the invention will be highly useful in the further study of signal transduction anomalies underlying cancer, including carcinomas and leukemias. Peptides and AQUA peptides of the invention may also be used for identifying diagnostic/bio-markers of carcinomas, identifying new potential drug targets, and/or monitoring the effects of test therapeutic agents on signaling proteins and pathways.

### 4. Phosphorylation Site-Specific Antibodies

In another aspect, the invention discloses phosphorylation site-specific binding molecules that specifically bind at a novel serine or threoninephosphorylation site of the invention, and that distinguish between the phosphorylated and unphosphorylated forms. In one embodiment, the binding molecule is an antibody or an antigen-binding fragment thereof. The antibody may specifically bind to an amino acid sequence comprising a phosphorylation site identified in Table 1.

In some embodiments, the antibody or antigen-binding fragment thereof specifically binds the phosphorylated site. In other embodiments, the antibody or antigen-binding fragment thereof specially binds the unphosphorylated site. An antibody or antigen-binding fragment thereof specially binds an amino acid sequence comprising a novel serine or threoninephosphorylation site in Table 1 when it does not significantly bind any other site in the parent protein and does not significantly bind a protein other than the parent protein. An antibody of the invention is sometimes referred to herein as a" phospho-specific" antibody.

An antibody or antigen-binding fragment thereof specially binds an antigen when the dissociation constant is < 1 mM, preferably ≤100nM, and more preferably ≤ 10nM.

In some embodiments, the antibody or antigen-binding fragment of the invention binds an amino acid sequence that comprises a novel phosphorylation site of a protein in Table 1 that is adaptor/scaffold protein, kinase/protease/phosphatase/enzyme proteins, protein kinase, cytoskeletal protein, ubiquitan conjugating system protein, chromatin or DNA binding/repair protein, g protein or regulator protein, receptor/channel/transporter/cell surface protein, transcriptional regulator and cell cycle regulation protein.

In particularly preferred embodiments, an antibody or antigen-binding fragment thereof of the invention specially binds an amino acid sequence comprising a novel serine or threoninephosphorylation site shown as a lower case "y," "s," or "t" (respectively) in a sequence listed in Table 1 selected from the group consisting of SEQ ID NOS: 1 (4E-BP2), 2 (4E-BP2), 3 (BAP37), 4 (FOX03A), 5 (JAZF1), 6 (NFkB-p105), 7 (PCDC5RP) 8 (TAF7), 38 (LIM), 39 (SD-95), 40 (RanBP2), 41 (Rictor), 42 (Z02), 70 (B-CK), 71 (PIK4CA), 72 (PIP5K), 73 (Pnk1), 94 (ATP6VOD1), 116 (DBN1), 117 (lamin A/C), 118 (lamin B2), 138 (HAPIP), 139 (RALBP1), 140 (RGS12), 155 (Bcr), 156 (HER2), 157 (OSR1), 158 (Pyk2), 185 (KAB1) 186 (NuMa-1), 197 (UREB1), 207 (CCT2), 208 (CLH-17), 209 (N-cad), 210 (PROSC) and WFS 1211 (S235).

In some embodiments, an antibody or antigen-binding fragment thereof of the invention specifically binds an amino acid sequence comprising any one of the above listed SEQ ID NOs. In some embodiments, an antibody or antigen-binding fragment thereof of the invention especially binds an amino acid sequence comprises a fragment of one of said SEQ ID NOs., wherein the fragment is four to twenty amino acid long and includes the phosphorylatable serine and/or threonine.

In certain embodiments, an antibody or antigen-binding fragment thereof of the invention specially binds an amino acid sequence that comprises a peptide produced by proteolysis of the parent protein with a protease wherein said peptide comprises a novel serine or threoninephosphorylation site of the invention. In some embodiments, the peptides are produced from trypsin digestion of the parent protein. The parent protein comprising the novel serine or threoninephosphorylation site can be from any species, preferably from a mammal including but not limited to non-human primates, rabbits, mice, rats, goats, cows, sheep, and guinea pigs. In some embodiments, the parent protein is a human protein and the antibody binds an epitope comprising the novel serine or threoninephosphorylation site shown by a (ower case "y," "s" or "t" in Column E of Table 1. Such peptides include any one of SEQ ID NOs: 1-44, 46-83, 85-110, 112-125, 127-132,135-139, 141-148,150-234,236-277.279-309,3)1-356.

An antibody of the invention can be an intact, four immunoglobulin chain antibody comprising two heavy chains and two light chains. The heavy chain of the antibody can be of any isotype including IgM, IgG, IgE, IgG, IgA or IgD or sub-isotype including IgG1, IgG2, IgG3, IgG4, IgE1, IgE2, etc. The light chain can be a kappa light chain or a lambda light chain.

Also within the invention are antibody molecules with fewer than 4 chains, including single chain antibodies, Camelid antibodies and the like and components of the antibody, including a heavy chain or a light chain. The term "antibody" (or "antibodies") refers to all types of immunoglobulins. The term "an antigen-binding fragment of an antibody" refers to any portion of an antibody that retains specific binding of the intact antibody. An exemplary antigen-binding fragment of an antibody is the heavy chain and/or light chain CDR, or the heavy and/or light chain variable region. The term "does not bind," when appeared in context of an antibody's binding to one phospho-form (e.g., phosphorylated form) of a sequence, means that the antibody does not substantially react with the other phospho-form (e.g., non-phosphorylated form) of the same sequence. One of skill in the art will appreciate that the expression may be applicable in those instances when (1) a phospho-specific antibody either does not apparently bind to the non-phospho form of the antigen as ascertained in commonly used experimental detection systems (Western blotting, IHC, Immunofluorescence, etc.); (2) where there is some reactivity with the surrounding amino acid sequence, but that the phosphorylated residue is an immunodominant feature of the reaction. In cases such as these, there is an apparent difference in affinities for the two sequences. Dilutional analyses of such antibodies indicates that the antibodies apparent affinity for the phosphorylated form is at least 10-100 fold higher than for the non-phosphorylated form; or where (3) the phospho-specific antibody reacts no more than an appropriate control antibody would react under identical experimental conditions. A control antibody preparation might be, for instance, purified immunoglobulin from a pre-immune animal of the same species, an isotype- and species-matched monoclonal antibody. Tests using control antibodies to demonstrate specificity are recognized by one of skill in the art as appropriate and definitive.

In some embodiments an immunoglobulin chain may comprise in order from 5' to 3', a variable region and a constant region. The variable region may comprise three complementarity determining regions (CDRs), with interspersed framework (FR) regions for a structure FR1, CDR1, FR2, CDR2, FR3, CDR3 and FR4. Also within the invention are heavy or light chain variable regions, framework regions and CDRs. An antibody of the invention may comprise a heavy chain constant region that comprises some or all of a CH1 region, hinge, CH2 and CH3 region.

An antibody of the invention may have an binding affinity (K_{D}) of 1x 10⁻⁷ M or less. In other embodiments, the antibody binds with a K_{D} of 1 x10⁻⁸M, 1 × 10⁻⁹M, 1 x 10⁻¹⁰M, 1 x 10⁻¹¹ M, 1 x 10⁻¹² M or less. In certain embodiments, the K_{D} is 1 pM to 500 pM, between 500 pM to 1 M, between 1 M to 100 nM, or between 100 mM to 10 nM.

Antibodies of the invention can be derived from any species of animal, preferably a mammal. Non-limiting exemplary natural antibodies include antibodies derived from human, chicken, goats, and rodents (e.g., rats, mice, hamsters and rabbits), including transgenic rodents genetically engineered to produce human antibodies (see, e.g., Lonberg et al., WO93/12227; U.S. Pat. No. 5,545,806; and Kucherlapati, et al., WO91/1074 U.S. Pat. No. 6,150,584, which are herein incorporated by reference in their entirety). Natural antibodies are the antibodies produced by a host animal. "Genetically altered antibodies" refer to antibodies wherein the amino acid sequence has been varied from that of a native antibody. Because of the relevance of recombinant DNA techniques to this application, one need not be confined to the sequences of amino acids found in natural antibodies; antibodies can be redesigned to obtain desired characteristics. The possible variations are many and range from the changing of just one or a few amino acids to the complete redesign of, for example, the variable or constant region. Changes in the constant region will, in general, be made in order to improve or alter characteristics, such as complement fixation, interaction with membranes and other effector functions. Changes in the variable region will be made in order to improve the antigen binding characteristics.

The antibodies of the invention include antibodies of any isotype including IgM, IgG, IgD, IgA and IgE, and any sub-isotype, including IgG1, IgG2a, IgG2b, IgG3 and IgG4, IgE1, IgE2 etc.. The light chains of the antibodies can either be kappa light chains or lambda light chains.

Antibodies disclosed in the invention may be polyclonal or monoclonal. As used herein, the term "epitope" refers to the smallest portion of a protein capable of selectively binding to the antigen binding site of an antibody. It is well accepted by those skilled in the art that the minimal size of a protein epitope capable of selectively binding to the antigen binding site of an antibody is about five or six to seven amino acids.

Other antibodies specifically contemplated are oligoclonal antibodies. As used herein, the phrase "oligoclonal antibodies" refers to a predetermined mixture of distinct monoclonal antibodies. *See,* e.g., PCT publication WO 95/20401; U.S. Patent Nos. 5,789,208 and 6,335,163. In one embodiment, oligoclonal antibodies consisting of a predetermined mixture of antibodies against one or more epitopes are generated in a single cell. In other embodiments, oligoclonal antibodies comprise a plurality of heavy chains capable of pairing with a common light chain to generate antibodies with multiple specificities (e.g., PCT publication WO 04/009618). Oligoclonal antibodies are particularly useful when it is desired to target multiple epitopes on a single target molecule. In view of the assays and epitopes disclosed herein, those skilled in the art can generate or select antibodies or mixtures of antibodies that are applicable for an intended purpose and desired need.

Recombinant antibodies against the phosphorylation sites identified in the invention are also included in the present application. These recombinant antibodies have the same amino acid sequence as the natural antibodies or have altered amino acid sequences of the natural antibodies in the present application. They can be made in any expression systems including both prokaryotic and eukaryotic expression systems or using phage display methods (see, e.g., Dower et al., WO91 /17271 and McCafferty et al., WO92/01047; U.S. Pat. No. 5,969,108, which are herein incorporated by reference in their entirety).

Antibodies can be engineered in numerous ways. They can be made as single-chain antibodies (including small modular immunopharmaceuticals or SMIPs^{™}), Fab and F(ab')₂ fragments, etc. Antibodies can be humanized, chimerized, deimmunized, or fully human. Numerous publications set forth the many types of antibodies and the methods of engineering such antibodies. For example, see U.S. Patent Nos. 6,355,245; 6,180,370; 5,693,762; 6,407,213; 6,548,640; 5,565,332; 5,225,539; 6,103,889; and 5,260,203.

The genetically altered antibodies should be functionally equivalent to the above-mentioned natural antibodies. In certain embodiments, modified antibodies provide improved stability or/and therapeutic efficacy. Examples of modified antibodies include those with conservative substitutions of amino acid residues, and one or more deletions or additions of amino acids that do not significantly deleteriously alter the antigen binding utility. Substitutions can range from changing or modifying one or more amino acid residues to complete redesign of a region as long as the therapeutic utility is maintained. Antibodies of this application can be modified post-translationally (e.g., acetylation, and/or phosphorylation) or can be modified synthetically (e.g., the attachment of a labeling group).

Antibodies with engineered or variant constant or Fc regions can be useful in modulating effector functions, such as, for example, antigen-dependent cytotoxicity (ADCC) and complement-dependent cytotoxicity (CDC). Such antibodies with engineered or variant constant or Fc regions may be useful in instances where a parent singling protein (Table 1) is expressed in normal tissue; variant antibodies without effector function in these instances may elicit the desired therapeutic response while not damaging normal tissue. Accordingly, certain aspects and methods of the present disclosure relate to antibodies with altered effector functions that comprise one or more amino acid substitutions, insertions, and/or deletions.

In certain embodiments, genetically altered antibodies are chimeric antibodies and humanized antibodies.

The chimeric antibody is an antibody having portions derived from different antibodies. For example, a chimeric antibody may have a variable region and a constant region derived from two different antibodies. The donor antibodies may be from different species. In certain embodiments, the variable region of a chimeric antibody is non-human, e.g., murine, and the constant region is human.

The genetically altered antibodies used in the invention include CDR grafted humanized antibodies. In one embodiment, the humanized antibody comprises heavy and/or light chain CDRs of a non-human donor immunoglobulin and heavy chain and light chain frameworks and constant regions of a human acceptor immunoglobulin. The method of making humanized antibody is disclosed in U.S. Pat. Nos: 5,530,101; 5,585,089; 5,693,761; 5,693,762; and 6,180,370 each of which is incorporated herein by reference in its entirety.

Antigen-binding fragments of the antibodies of the invention, which retain the binding specificity of the intact antibody, are also included in the invention. Examples of these antigen-binding fragments include, but are not limited to, partial or full heavy chains or light chains, variable regions, or CDR regions of any phosphorylation site-specific antibodies described herein.

In one embodiment of the application, the antibody fragments are truncated chains (truncated at the carboxyl end). In certain embodiments, these truncated chains possess one or more immunoglobulin activities (e.g., complement fixation activity). Examples of truncated chains include, but are not limited to, Fab fragments (consisting of the VL, VH, CL and CH1 domains); Fd fragments (consisting of the VH and CH1 domains); Fv fragments (consisting of VL and VH domains of a single chain of an antibody); dAb fragments (consisting of a VH domain); isolated CDR regions; (Fab')₂ fragments, bivalent fragments (comprising two Fab fragments linked by a disulphide bridge at the hinge region). The truncated chains can be produced by conventional biochemical techniques, such as enzyme cleavage, or recombinant DNA techniques, each of which is known in the art. These polypeptide fragments may be produced by proteolytic cleavage of intact antibodies by methods well known in the art, or by inserting stop codons at the desired locations in the vectors using site-directed mutagenesis, such as after CH1 to produce Fab fragments or after the hinge region to produce (Fab')₂ fragments. Single chain antibodies may be produced by joining VL- and VH-coding regions with a DNA that encodes a peptide linker connecting the VL and VH protein fragments

Papain digestion of antibodies produces two identical antigen-binding fragments, called "Fab" fragments, each with a single antigen-binding site, and a residual "Fc" fragment, whose name reflects its ability to crystallize readily. Pepsin treatment of an antibody yields an F(ab')₂ fragment that has two antigen-combining sites and is still capable of cross-linking antigen.

"Fv" usually refers to the minimum antibody fragment that contains a complete antigen-recognition and -binding site. This region consists of a dimer of one heavy- and one light-chain variable domain in tight, noncovalent association. It is in this configuration that the three CDRs of each variable domain interact to define an antigen-binding site on the surface of the V_{H}-V_{L} dimer. Collectively, the CDRs confer antigen-binding specificity to the antibody. However, even a single variable domain (or half of an Fv comprising three CDRs specific for an antigen) has the ability to recognize and bind antigen, although likely at a lower affinity than the entire binding site.

Thus, in certain embodiments, the antibodies of the application may comprise 1, 2, 3, 4, 5, 6, or more CDRs that recognize the phosphorylation sites identified in Column E of Table 1.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments that have hinge cysteines between them. Other chemical couplings of antibody fragments are also known.

"Single-chain Fv" or "scFv" antibody fragments comprise the V_{H} and V_{L} domains of an antibody, wherein these domains are present in a single polypeptide chain. In certain embodiments, the Fv polypeptide further comprises a polypeptide linker between the V_{H} and V_{L} domains that enables the scFv to form the desired structure for antigen binding. For a review of scFv see Pluckthun in The Pharmacology of Monoclonal Antibodies, vol. 113, Rosenburg and Moore, eds. (Springer-Verlag: New York, 1994), pp. 269-315.

SMIPs are a class of single-chain peptides engineered to include a target binding region and effector domain (CH2 and CH3 domains). *See,* e.g., U.S. Patent Application Publication No. 20050238646. The target binding region may be derived from the variable region or CDRs of an antibody, e.g., a phosphorylation site-specific antibody of the application. Alternatively, the target binding region is derived from a protein that binds a phosphorylation site.

Bispecific antibodies may be monoclonal, human or humanized antibodies that have binding specificities for at least two different antigens. In the present case, one of the binding specificities is for the phosphorylation site, the other one is for any other antigen, such as for example, a cell-surface protein or receptor or receptor subunit. Alternatively, a therapeutic agent may be placed on one arm. The therapeutic agent can be a drug, toxin, enzyme, DNA, radionuclide, etc.

In some embodiments, the antigen-binding fragment can be a diabody. The term "diabody" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90: 6444-6448 (1993).

Camelid antibodies refer to a unique type of antibodies that are devoid of light chain, initially discovered from animals of the camelid family. The heavy chains of these so-called heavy-chain antibodies bind their antigen by one single domain, the variable domain of the heavy immunoglobulin chain, referred to as VHH. VHHs show homology with the variable domain of heavy chains of the human VHIII family. The VHHs obtained from an immunized camel, dromedary, or llama have a number of advantages, such as effective production in microorganisms such as Saccharomyces cerevisiae.

In certain embodiments, single chain antibodies, and chimeric, humanized or primatized (CDR-grafted) antibodies, as well as chimeric or CDR-grafted single chain antibodies, comprising portions derived from different species, are also encompassed by the present disclosure as antigen-binding fragments of an antibody. The various portions of these antibodies can be joined together chemically by conventional techniques, or can be prepared as a contiguous protein using genetic engineering techniques. For example, nucleic acids encoding a chimeric or humanized chain can be expressed to produce a contiguous protein. See, e.g., U.S. Pat. Nos. 4,816,567 and 6,331,415; U.S. Pat. No. 4,816,397; European Patent No. 0,120,694; WO 86/01533; European Patent No. 0,194,276 B1; U.S. Pat. No. 5,225,539; and European Patent No. 0,239,400 B1. See also, Newman et al., BioTechnology, 10: 1455-1460 (1992), regarding primatized antibody. See, e.g., Ladner et al., U.S. Pat. No. 4,946,778; and Bird et al., Science, 242: 423-426 (1988)), regarding single chain antibodies.

In addition, functional fragments of antibodies, including fragments of chimeric, humanized, primatized or single chain antibodies, can also be produced. Functional fragments of the subject antibodies retain at least one binding function and/or modulation function of the full-length antibody from which they are derived.

Since the immunoglobulin-related genes contain separate functional regions, each having one or more distinct biological activities, the genes of the antibody fragments may be fused to functional regions from other genes (e.g., enzymes, U.S. Pat. No. 5,004,692, which is incorporated by reference in its entirety) to produce fusion proteins or conjugates having novel properties.

Non-immunoglobulin binding polypeptides are also contemplated. For example, CDRs from an antibody disclosed herein may be inserted into a suitable non-immunoglobulin scaffold to create a non-immunoglobulin binding polypeptide. Suitable candidate scaffold structures may be derived from, for example, members of fibronectin type III and cadherin superfamilies.

Also contemplated are other equivalent non-antibody molecules, such as protein binding domains or aptamers, which bind, in a phospho-specific manner, to an amino acid sequence comprising a novel phosphorylation site of the invention. *See,* e.g., Neuberger *et al., Nature 312:* 604 (1984). Aptamers are oligonucleic acid or peptide molecules that bind a specific target molecule. DNA or RNA aptamers are typically short oligonucleotides, engineered through repeated rounds of selection to bind to a molecular target. Peptide aptamers typically consist of a variable peptide loop attached at both ends to a protein scaffold. This double structural constraint generally increases the binding affinity of the peptide aptamer to levels comparable to an antibody (nanomolar range).

The invention also discloses the use of the phosphorylation site-specific antibodies with immunotoxins. Conjugates that are immunotoxins including antibodies have been widely described in the art. The toxins may be coupled to the antibodies by conventional coupling techniques or immunotoxins containing protein toxin portions can be produced as fusion proteins. In certain embodiments, antibody conjugates may comprise stable linkers and may release cytotoxic agents inside cells (see U.S. Patent Nos. 6,867,007 and 6,884,869). The conjugates of the present application can be used in a corresponding way to obtain such immunotoxins. Illustrative of such immunotoxins are those described by Byers et al., Seminars Cell Biol 2:59-70 (1991) and by Fanger et al., Immunol Today 12:51-54 (1991). Exemplary immunotoxins include radiotherapeutic agents, ribosome-inactivating proteins (RIPs), chemotherapeutic agents, toxic peptides, or toxic proteins.

The phosphorylation site-specific antibodies disclosed in the invention may be used singly or in combination. The antibodies may also be used in an array format for high throughput uses. An antibody microarray is a collection of immobolized antibodies, typically spotted and fixed on a solid surface (such as glass, plastic and silicon chip).

In another aspect, the antibodies of the invention modulate at least one, or all, biological activities of a parent protein identified in Column A of Table 1. The biological activities of a parent protein identified in Column A of Table 1 include: 1) ligand binding activities (for instance, these neutralizing antibodies may be capable of competing with or completely blocking the binding of a parent signaling protein to at least one, or all, of its ligands; 2) signaling transduction activities, such as receptor dimerization, or serine or threoninephosphorylation; and 3) cellular responses induced by a parent signaling protein, such as oncogenic activities (e.g., cancer cell proliferation mediated by a parent signaling protein), and/or angiogenic activities.

In certain embodiments, the antibodies of the invention may have at least one activity selected from the group consisting of: 1) inhibiting cancer cell growth or proliferation; 2) inhibiting cancer cell survival; 3) inhibiting angiogenesis; 4) inhibiting cancer cell metastasis, adhesion, migration or invasion; 5) inducing apoptosis of cancer cells; 6) incorporating a toxic conjugate; and 7) acting as a diagnostic marker.

In certain embodiments, the phosphorylation site specific antibodies disclosed in the invention are especially indicated for diagnostic and therapeutic applications as described herein. Accordingly, the antibodies may be used in therapies, including combination therapies, in the diagnosis and prognosis of disease, as well as in the monitoring of disease progression. The invention, thus, further includes compositions comprising one or more embodiments of an antibody or an antigen binding portion of the invention as described herein. The composition may further comprise a pharmaceutically acceptable carrier. The composition may comprise two or more antibodies or antigen-binding portions, each with specificity for a different novel serine or threoninephosphorylation site of the invention or two or more different antibodies or antigen-binding portions all of which are specific for the same novel serine or threoninephosphorylation site of the invention. A composition of the invention may comprise one or more antibodies or antigen-binding portions of the invention and one or more additional reagents, diagnostic agents or therapeutic agents.

The present application provides for the polynucleotide molecules encoding the antibodies and antibody fragments and their analogs described herein. Because of the degeneracy of the genetic code, a variety of nucleic acid sequences encode each antibody amino acid sequence. The desired nucleic acid sequences can be produced by de novo solid-phase DNA synthesis or by PCR mutagenesis of an earlier prepared variant of the desired polynucleotide. In one embodiment, the codons that are used comprise those that are typical for human or mouse (see, e.g., Nakamura, Y., Nucleic Acids Res. 28: 292 (2000)).

The invention also provides immortalized cell lines that produce an antibody of the invention. For example, hybridoma clones, constructed as described above, that produce monoclonal antibodies to the targeted signaling protein phosphorylation sties disclosed herein are also provided. Similarly, the invention includes recombinant cells producing an antibody of the invention, which cells may be constructed by well known techniques; for example the antigen combining site of the monoclonal antibody can be cloned by PCR and single-chain antibodies produced as phage-displayed recombinant antibodies or soluble antibodies in *E*. *coli (see,* e.g., ANTIBODY ENGINEERING PROTOCOLS, 1995, Humana Press, Sudhir Paul editor.)

### 5. Methods of Making Phosphorylation site-Specific Antibodies

In another aspect, the invention provides a method for making phosphorylation site-specific antibodies.

Polyclonal antibodies of the invention may be produced according to standard techniques by immunizing a suitable animal (e.g., rabbit, goat, etc.) with an antigen comprising a novel serine or threoninephosphorylation site of the invention. (i.e. a phosphorylation site shown in Table 1) in either the phosphorylated or unphosphorylated state, depending upon the desired specificity of the antibody, collecting immune serum from the animal, and separating the polyclonal antibodies from the immune serum, in accordance with known procedures and screening and isolating a polyclonal antibody specific for the novel serine or threoninephosphorylation site of interest as further described below. Methods for immunizing non-human animals such as mice, rats, sheep, goats, pigs, cattle and horses are well known in the art. See, e.g., Harlow and Lane, Antibodies: A Laboratory Manual, New York: Cold Spring Harbor Press, 1990.

The immunogen may be the full length protein or a peptide comprising the novel serine or threoninephosphorylation site of interest. In some embodiments the immunogen is a peptide of from 7 to 20 amino acids in length, preferably about 8 to 17 amino acids in length. In some embodiments, the peptide antigen desirably will comprise about 3 to 8 amino acids on each side of the phosphorylatable serine and/or threonine. In yet other embodiments, the peptide antigen desirably will comprise four or more amino acids flanking each side of the phosphorylatable amino acid and encompassing it. Peptide antigens suitable for producing antibodies of the invention may be designed, constructed and employed in accordance with well-known techniques. *See,* e.g., Antibodies: A Laboratory Manual, Chapter 5, p. 75-76, Harlow & Lane Eds., Cold Spring Harbor Laboratory (1988); Czernik, Methods In Enzymology, 201: 264-283 (1991); Merrifield, J. Am. Chem. Soc. 85: 21-49 (1962)).

Suitable peptide antigens may comprise all or partial sequence of a trypsin-digested fragment as set forth in Column E of Table 1/Figure 2. Suitable peptide antigens may also comprise all or partial sequence of a peptide fragment produced by another protease digestion.

Preferred immunogens are those that comprise a novel phosphorylation site of a protein in Table I that is an adaptor/scaffold protein, kinase/protease/phosphatase/enzyme proteins, protein kinase, cytoskeletal protein, ubiquitan conjugating system protein, chromatin or DNA binding/repair protein, g protein or regulator protein, receptor/channel/transporter/cell surface protein, transcriptional regulator and cell cycle regulation protein. In some embodiments, the peptide immunogen is an AQUA peptide, for example, any one of SEQ ID NOS: 1-44, 46-83, 85-110, 112-125, 127-132, 135-139, 141-148, 150-234, 236-277, 279-309, 311-356.

Particularly preferred immunogens are peptides comprising any one of the novel serine or threoninephosphorylation site shown as a lower case "y," "s" or "t" the sequences listed in Table 1 selected from the group consisting of SEQ ID NOS: 1 (4E-BP2), 2 (4E-BP2), (BAP37), 4 (FOX03A), 5 (JAZF1), 6 (NFkB-p105), 7 (PCDCSRP) 8 (TAF7), 38 (LIM), 39 (SD-95), 40 (RanBP2), 41 (Rictor), 42 (Z02), 70 (B-CK), 71 (PTK4CA), 72 (PIP5K), 73 (Pnk1), 94 (ATP6VOD1), 116 (DBN1), 117 (lamin A/C), 118 (lamin B2), 138(HAPIP), 139 (RALBP1), 140 (RGS12), 155 (Bcr), 156 (HER2), 157 (OSR1), 158 (Pyk2), 185 (KAB1) 186 (NuMa-1), 197 (UREB1), 207 (CCT2), 208 (CLH-17), 209 (N-cad), 210 (PROSC) and WFS 211 (S235).

In some embodiments the immunogen is administered with an adjuvant. Suitable adjuvants will be well known to those of skill in the art. Exemplary adjuvants include complete or incomplete Freund's adjuvant, RIBI (muramyl dipeptides) or ISCOM (immunostimulating complexes).

For example, a peptide antigen comprising the novel adaptor/scaffold protein phosphorylation site in SEQ ID NO: 36 shown by the lower case "s" in Table 1 may be used to produce antibodies that specifically bind the novel tyrosine phosphorylation site.

When the above-described methods are used for producing polyclonal antibodies, following immunization, the polyclonal antibodies which secreted into the bloodstream can be recovered using known techniques. Purified forms of these antibodies can, of course, be readily prepared by standard purification techniques, such as for example, affinity chromatography with Protein A, anti-immunoglobulin, or the antigen itself. In any case, in order to monitor the success of immunization, the antibody levels with respect to the antigen in serum will be monitored using standard techniques such as ELISA, RIA and the like.

Monoclonal antibodies of the invention may be produced by any of a number of means that are well-known in the art. In some embodiments, antibody-producing B cells are isolated from an animal immunized with a peptide antigen as described above. The B cells may be from the spleen, lymph nodes or peripheral blood. Individual B cells are isolated and screened as described below to identify cells producing an antibody specific for the novel serine or threoninephosphorylation site of interest. Identified cells are then cultured to produce a monoclonal antibody of the invention.

Alternatively, a monoclonal phosphorylation site-specific antibody of the invention may be produced using standard hybridoma technology, in a hybridoma cell line according to the well-known technique of Kohler and Milstein. See Nature 265: 495-97 (1975); Kohler and Milstein, Eur. J Immunol. 6: 511 (1976); *see also,* Current Protocols in Molecular Biology, Ausubel et al. Eds. (1989). Monoclonal antibodies so produced are highly specific, and improve the selectivity and specificity of diagnostic assay methods provided by the invention. For example, a solution containing the appropriate antigen may be injected into a mouse or other species and, after a sufficient time (in keeping with conventional techniques), the animal is sacrificed and spleen cells obtained. The spleen cells are then immortalized by any of a number of standard means. Methods of immortalizing cells include, but are not limited to, transfecting them with oncogenes, infecting them with an oncogenic virus and cultivating them under conditions that select for immortalized cells, subjecting them to carcinogenie or mutating compounds, fusing them with an immortalized cell, e.g., a myeloma cell, and inactivating a tumor suppressor gene. See, e.g., Harlow and Lane, supra. If fusion with myeloma cells is used, the myeloma cells preferably do not secrete immunoglobulin polypeptides (a non-secretory cell line). Typically the antibody producing cell and the immortalized cell (such as but not limited to myeloma cells) with which it is fused are from the same species. Rabbit fusion hybridomas, for example, may be produced as described in U.S Patent No. 5,675,063, C. Knight, Issued October 7, 1997. The immortalized antibody producing cells, such as hybridoma cells, are then grown in a suitable selection media, such as hypoxanthine-aminopterin-thymidine (HAT), and the supernatant screened for monoclonal antibodies having the desired specificity, as described below. The secreted antibody may be recovered from tissue culture supernatant by conventional methods such as precipitation, ion exchange or affinity chromatography, or the like.

The invention also encompasses antibody-producing cells and cell lines, such as hybridomas, as described above.

Polyclonal or monoclonal antibodies may also be obtained through in vitro immunization. For example, phage display techniques can be used to provide libraries containing a repertoire of antibodies with varying affinities for a particular antigen. Techniques for the identification of high affinity human antibodies from such libraries are described by Griffiths et al., (1994) EMBO J., 13:3245-3260 ; Nissim et al., ibid, pp. 692-698 and by Griffiths et al., ibid, 12:725-734, which are incorporated by reference.

The antibodies may be produced recombinantly using methods well known in the art for example, according to the methods disclosed in U.S. Pat. No. 4,349,893 (Reading) or U.S. Pat. No. 4,816,567 (Cabilly et al.*)* The antibodies may also be chemically constructed by specific antibodies made according to the method disclosed in U.S. Pat. No. 4,676,980 (Segel et al.*)*

Once a desired phosphorylation site-specific antibody is identified, polynucleotides encoding the antibody, such as heavy, light chains or both (or single chains in the case of a single chain antibody) or portions thereof such as those encoding the variable region, may be cloned and isolated from antibody-producing cells using means that are well known in the art. For example, the antigen combining site of the monoclonal antibody can be cloned by PCR and single-chain antibodies produced as phage-displayed recombinant antibodies or soluble antibodies in *E. coli (see,* e.g., Antibody Engineering Protocols, 1995, Humana Press, Sudhir Paul editor.)

Accordingly, in a further aspect, the invention provides such nucleic acids encoding the heavy chain, the light chain, a variable region, a framework region or a CDR of an antibody of the invention. In some embodiments, the nucleic acids are operably linked to expression control sequences. The invention, thus, also provides vectors and expression control sequences useful for the recombinant expression of an antibody or antigen-binding portion thereof of the invention. Those of skill in the art will be able to choose vectors and expression systems that are suitable for the host cell in which the antibody or antigen-binding portion is to be expressed.

Monoclonal antibodies of the invention may be produced recombinantly by expressing the encoding nucleic acids in a suitable host cell under suitable conditions. Accordingly, the invention further provides host cells comprising the nucleic acids and vectors described above.

Monoclonal Fab fragments may also be produced in *Escherichia coli* by recombinant techniques known to those skilled in the art. *See,* e.g., W. Huse, Science 246: 1275-81 (1989); Mullinax et al., Proc. Nat Acad Sci. 87: 8095 (1990).

If monoclonal antibodies of a single desired isotype are preferred for a particular application, particular isotypes can be prepared directly, by selecting from the initial fusion, or prepared secondarily, from a parental hybridoma secreting a monoclonal antibody of different isotype by using the sib selection technique to isolate class-switch variants (Steplewski, et al., Proc. Nat'l.-Acad. Sci., 82: 8653 (1985); Spira et al.,J.Immunol. Methods, 74: 307 (1984)). Alternatively, the isotype of a monoclonal antibody with desirable propertied can be changed using antibody engineering techniques that are well-known in the art.

Phosphorylation site-specific antibodies of the invention, whether polyclonal or monoclonal, may be screened for epitope and phospho-specificity according to standard techniques. *See,* e.g., Czernik et al., Methods in Enzymology, 201: 264-283 (1991). For example, the antibodies may be screened against the phosphorylated and/or unphosphosphorylated peptide library by ELISA to ensure specificity for both the desired antigen (i.e. that epitope including a phosphorylation site of the invention and for reactivity only with the phosphorylated (or unphosphorylated) form of the antigen. Peptide competition assays may be carried out to confirm lack of reactivity with other phospho-epitopes on the parent protein. The antibodies may also be tested by Western blotting against cell preparations containing the parent signaling protein, e.g., cell lines over-expressing the parent protein, to confirm reactivity with the desired phosphorylated epitope/target.

Specificity against the desired phosphorylated epitope may also be examined by constructing mutants lacking phosphorylatable residues at positions outside the desired epitope that are known to be phosphorylated, or by mutating the desired phospho-epitope and confirming lack of reactivity. Phosphorylation site-specific antibodies of the invention may exhibit some limited cross-reactivity to related epitopes in non-target proteins. This is not unexpected as most antibodies exhibit some degree of cross-reactivity, and antipeptide antibodies will often cross-react with epitopes having high homology to the immunizing peptide. *See,* e.g., *Czernik, supra.* Cross-reactivity with non-target proteins is readily characterized by Western blotting alongside markers of known molecular weight. Amino acid sequences of cross-reacting proteins may be examined to identify phosphorylation sites with flanking sequences that are highly homologous to that of phosphorylation site of the invention.

In certain cases, polyclonal antisera may exhibit some undesirable general cross-reactivity to phosphoserine or threonineitself, which may be removed by further purification of antisera, e.g., over a phosphotyramine column. Antibodies of the invention specifically bind their target protein *(i.e.* a protein listed in Column A of Table 1) only when phosphorylated (or only when not phosphorylated, as the case may be) at the site disclosed in corresponding Columns D/E, and do not (substantially) bind to the other form (as compared to the form for which the antibody is specific).

Antibodies may be further characterized via immunohistochemical (IHC) staining using normal and diseased tissues to examine phosphorylation and activation state and level of a phosphorylation site in diseased tissue. IHC may be carried out according to well-known techniques. *See,* e.g., Antibodies: A Laboratory Manual, Chapter 10, Harlow & Lane Eds., Cold Spring Harbor Laboratory (1988). Briefly, paraffin-embedded tissue (e.g., tumor tissue) is prepared for immunohistochemical staining by deparaffinizing tissue sections with xylene followed by ethanol; hydrating in water then PBS; unmasking antigen by heating slide in sodium citrate buffer; incubating sections in hydrogen peroxide; blocking in blocking solution; incubating slide in primary antibody and secondary antibody; and finally detecting using ABC avidin/biotin method according to manufacturer's instructions.

Antibodies may be further characterized by flow cytometry carried out according to standard methods. *See* Chow et al., Cytometry (Communications in Clinical Cytometry) 46: 72-78 (2001). Briefly and by way of example, the following protocol for cytometric analysis may be employed: samples may be centrifuged on Ficoll gradients to remove lysed erythrocytes and cell debris. Adherring cells may be scrapped off plates and washed with PBS. Cells may then be fixed with 2% paraformaldehyde for 10 minutes at 37 °C followed by permeabilization in 90% methanol for 30 minutes on ice. Cells may then be stained with the primary phosphorylation site-specific antibody of the invention (which detects a parent signaling protein enumerated in Table 1), washed and labeled with a fluorescent-labeled secondary antibody. Additional fluorochrome-conjugated marker antibodies (e.g., CD45, CD34) may also be added at this time to aid in the subsequent identification of specific hematopoietic cell types. The cells would then be analyzed on a flow cytometer (e.g. a Beckman Coulter FC500) according to the specific protocols of the instrument used.

Antibodies of the invention may also be advantageously conjugated to fluorescent dyes *(e.g.* Alexa488, PE) for use in multi-parametric analyses along with other signal transduction (phospho-CrkL, phospho-Erk 1/2) and/or cell marker (CD34) antibodies.

Phosphorylation site-specific antibodies of the invention may specifically bind to a signaling protein or polypeptide listed in Table 1 only when phosphorylated at the specified serine or threonineresidue, but are not limited only to binding to the listed signaling proteins of human species, *per se.* The invention includes antibodies that also bind conserved and highly homologous or identical phosphorylation sites in respective signaling proteins from other species (e.g., mouse, rat, monkey, yeast), in addition to binding the phosphorylation site of the human homologue. The term "homologous" refers to two or more sequences or subsequences that have at least about 85%, at least 90%, at least 95%, or higher nucleotide or amino acid residue identity, when compared and aligned for maximum correspondence, as measured using sequence comparison method (e.g., BLAST) and/or by visual inspection. Highly homologous or identical sites conserved in other species can readily be identified by standard sequence comparisons (such as BLAST).

Methods for making bispecific antibodies are within the purview of those skilled in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Antibody variable domains with the desired binding specificities (antibody-antigen combining sites) can be fused to immunoglobulin constant domain sequences. In certain embodiments, the fusion is with an immunoglobulin heavy-chain constant domain, including at least part of the hinge, CH2, and CH3 regions. DNAs encoding the immunoglobulin heavy-chain fusions and, if desired, the immunoglobulin light chain, are inserted into separate expression vectors, and are co-transfected into a suitable host organism. For further details of illustrative currently known methods for generating bispecific antibodies see, for example, Suresh et al., Methods in Enzymology, 121:210 (1986); WO 96/27011; Brennan et al., Science 229:81 (1985); Shalaby et al., J. Exp. Med. 175:217-225 (1992); Kostelny et al., J. Immunol. 148(5):1547-1553 (1992); Hollinger et al., Proc. Natl. Acad. Sci. USA 90:6444-6448 (1993); Gruber et al., J. Immunol. 152:5368 (1994); and Tutt et al., J. Immunol. 147:60 (1991). Bispecific antibodies also include cross-linked or heteroconjugate antibodies. Heteroconjugate antibodies may be made using any convenient cross-linking methods. Suitable cross-linking agents are well known in the art, and are disclosed in U.S. Pat. No. 4,676,980, along with a number of cross-linking techniques.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins may be linked to the Fab' portions of two different antibodies by gene fusion. The antibody homodimers may be reduced at the hinge region to form monomers and then re-oxidized to form the antibody heterodimers. This method can also be utilized for the production of antibody homodimers. A strategy for making bispecific antibody fragments by the use of single-chain Fv (scFv) dimers has also been reported. See Gruber et al., J. Immunol., 152:5368 (1994). Alternatively, the antibodies can be "linear antibodies" as described in Zapata et al. Protein Eng. 8(10):1057-1062 (1995). Briefly, these antibodies comprise a pair of tandem Fd segments (V_{H} -C_{H}1-V_{H} - C_{H}l) which form a pair of antigen binding regions. Linear antibodies can be bispecific or monospecific.

To produce the chimeric antibodies, the portions derived from two different species (e.g., human constant region and murine variable or binding region) can be joined together chemically by conventional techniques or can be prepared as single contiguous proteins using genetic engineering techniques. The DNA molecules encoding the proteins of both the light chain and heavy chain portions of the chimeric antibody can be expressed as contiguous proteins. The method of making chimeric antibodies is disclosed in U.S. Pat. No. 5,677.427; U.S. Pat. No. 6,120,767; and U.S. Pat. No. 6,329,508, each of which is incorporated by reference in its entirety.

Fully human antibodies may be produced by a variety of techniques. One example is trioma methodology. The basic approach and an exemplary cell fusion partner, SPAZ-4, for use in this approach have been described by Oestberg et al., Hybridoma 2:361-367 (1983); Oestberg, U.S. Pat. No. 4,634,664; and Engleman et al., U.S. Pat. No. 4,634,666 (each of which is incorporated by reference in its entirety).

Human antibodies can also be produced from non-human transgenic animals having transgenes encoding at least a segment of the human immunoglobulin locus. The production and properties of animals having these properties are described in detail by, see, e.g., Lonberg et al., W093/12227; U.S. Pat. No. 5,545,806; and Kucherlapati, et al., WO91/10741; U.S. Pat. No. 6,150,584, which are herein incorporated by reference in their entirety.

Various recombinant antibody library technologies may also be utilized to produce fully human antibodies. For example, one approach is to screen a DNA library from human B cells according to the general protocol outlined by Huse et al., Science 246:1275-1281 (1989). The protocol described by Huse is rendered more efficient in combination with phage-display technology. See, e.g., Dower et al., WO 91/17271 and McCafferty et al., WO 92/01047; U.S. Pat. No. 5,969,108, (each of which is incorporated by reference in its entirety).

Eukaryotic ribosome can also be used as means to display a library of antibodies and isolate the binding human antibodies by screening against the target antigen, as described in Coia G, et al., J. Immunol. Methods 1: 254 (1-2):191-7 (2001);Hanes J. et al., Nat. Biotechnol. 18(12):1287-92 (2000); Proc. Natl. Acad. Sci. U. S. A. 95(24):14130-5 (1998); Proc. Natl. Acad. Sci. U. S. A. 94(10):4937-42 (1997), each which is incorporated by reference in its entirety.

The yeast system is also suitable for screening mammalian cell-surface or secreted proteins, such as antibodies. Antibody libraries may be displayed on the surface of yeast cells for the purpose of obtaining the human antibodies against a target antigen. This approach is described by Yeung. et al., Biotechnol. Prog. 18(2):212-20 (2002); Boeder, E. T., et al., Nat. Biotechnol. 15(6):553-7 (1997), each of which is herein incorporated by reference in its entirety. Alternatively, human antibody libraries may be expressed intracellularly and screened via the yeast two-hybrid system (WO0200729A2, which is incorporated by reference in its entirety).

Recombinant DNA techniques can be used to produce the recombinant phosphorylation site-specific antibodies described herein, as well as the chimeric or humanized phosphorylation site-specific antibodies, or any other genetically-altered antibodies and the fragments or conjugate thereof in any expression systems including both prokaryotic and eukaryotic expression systems, such as bacteria, yeast, insect cells, plant cells, mammalian cells (for example, NS0 cells).

Once produced, the whole antibodies, their dimers, individual light and heavy chains, or other immunoglobulin forms of the present application can be purified according to standard procedures of the art, including ammonium sulfate precipitation, affinity columns, column chromatography, gel electrophoresis and the like (see, generally, Scopes, R., Protein Purification (Springer-Verlag, N.Y., 1982)). Once purified, partially or to homogeneity as desired, the polypeptides may then be used therapeutically (including extracorporeally) or in developing and performing assay procedures, immunofluorescent staining, and the like. (See, generally, Immunological Methods, Vols. I and II (Lefkovits and Pemis, eds., Academic Press, NY, 1979 and 1981).

### 6. Therapeutic Uses

In a further aspect, the invention provides methods and compositions for therapeutic uses of the peptides or proteins comprising a phosphorylation site of the invention, and phosphorylation site-specific antibodies of the invention.

In one embodiment, the invention provides for a method of treating or preventing carcinoma in a subject, wherein the carcinoma is associated with the phosphorylation state of a novel phosphorylation site in Table 1, whether phosphorylated or dephosphorylated, comprising: administering to a subject in need thereof a therapeutically effective amount of a peptide comprising a novel phosphorylation site (Table 1) and/or an antibody or antigen-binding fragment thereof that specifically bind a novel phosphorylation site of the invention (Table 1). The antibodies maybe full-length antibodies, genetically engineered antibodies, antibody fragments, and antibody conjugates of the invention.

The term "subject" refers to a vertebrate, such as for example, a mammal, or a human. Although present application are primarily concerned with the treatment of human subjects, the disclosed methods may also be used for the treatment of other mammalian subjects such as dogs and cats for veterinary purposes.

In one aspect, the disclosure provides a method of treating carcinoma in which a peptide or an antibody that reduces at least one biological activity of a targeted signaling protein is administered to a subject. For example, the peptide or the antibody administered may disrupt or modulate the interaction of the target signaling protein with its ligand. Alternatively, the peptide or the antibody may interfere with, thereby reducing, the down-stream signal transduction of the parent signaling protein. An antibody that specifically binds the novel serine or threoninephosphorylation site only when the serine or threonineis phosphorylated, and that does not substantially bind to the same sequence when the serine or threonineis not phosphorylated, thereby prevents downstream signal transduction triggered by a phospho-serine and/or threonine. Alternatively, an antibody that specifically binds the unphosphorylated target phosphorylation site reduces the phosphorylation at that site and thus reduces activation of the protein mediated by phosphorylation of that site. Similarly, an unphosphorylated peptide may compete with an endogenous phosphorylation site for the same target (e.g., kinases), thereby preventing or reducing the phosphorylation of the endogenous target protein. Alternatively, a peptide comprising a phosphorylated novel serine or threoninesite of the invention but lacking the ability to trigger signal transduction may competitively inhibit interaction of the endogenous protein with the same down-stream ligand(s).

The antibodies of the invention may also be used to target cancer cells for effector-mediated cell death. The antibody disclosed herein may be administered as a fusion molecule that includes a phosphorylation site-targeting portion joined to a cytotoxic moiety to directly kill cancer cells. Alternatively, the antibody may directly kill the cancer cells through complement-mediated or antibody-dependent cellular cytotoxicity.

Accordingly in one embodiment, the antibodies of the present disclosure may be used to deliver a variety of cytotoxic compounds. Any cytotoxic compound can be fused to the present antibodies. The fusion can be achieved chemically or genetically (e.g., via expression as a single, fused molecule). The cytotoxic compound can be a biological, such as a polypeptide, or a small molecule. As those skilled in the art will appreciate, for small molecules, chemical fusion is used, while for biological compounds, either chemical or genetic fusion can be used.

Non-limiting examples of cytotoxic compounds include therapeutic drugs, radiotherapeutic agents, ribosome-inactivating proteins (RIPs), chemotherapeutic agents, toxic peptides, toxic proteins, and mixtures thereof. The cytotoxic drugs can be intracellularly acting cytotoxic drugs, such as short-range radiation emitters, including, for example, short-range, high-energy - emitters. Enzymatically active toxins and fragments thereof, including ribosome-inactivating proteins, are exemplified by saporin, luffin, momordins, ricin, trichosanthin, gelonin, abrin, etc. Procedures for preparing enzymatically active polypeptides of the immunotoxins are described in WO84/03508 and W085/03508, which are hereby incorporated by reference. Certain cytotoxic moieties are derived from adriamycin, chlorambucil, daunomycin, methotrexate, neocarzinostatin, and platinum, for example.

Exemplary chemotherapeutic agents that may be attached to an antibody or antigen-binding fragment thereof include taxol, doxorubicin, verapamil, podophyllotoxin, procarbazine, mechlorethamine, cyclophosphamide, camptothecin, ifosfamide, melphalan, chlorambucil, bisulfan, nitrosurea, dactinomycin, daunorubicin, doxorubicin, bleomycin, plicomycin, mitomycin, etoposide (VP16), tamoxifen, transplatinum, 5-fluorouracil, vincristin, vinblastin, or methotrexate.

Procedures for conjugating the antibodies with the cytotoxic agents have been previously described and are within the purview of one skilled in the art.

Alternatively, the antibody can be coupled to high energy radiation emitters, for example, a radioisotope, such as ¹³¹I, a -emitter, which, when localized at the tumor site, results in a killing of several cell diameters. See, e.g., S. E. Order, "Analysis, Results, and Future Prospective of the Therapeutic Use of Radiolabeled Antibody in Cancer Therapy", Monoclonal Antibodies for Cancer Detection and Therapy, Baldwin et al. (eds.), pp. 303-316 (Academic Press 1985), which is hereby incorporated by reference. Other suitable radioisotopes include -emitters, such as ²¹²Bi, ²¹³Bi, and ²¹¹At, and -emitters, such as ¹⁸⁶Re and ⁹⁰Y.

Because many of the signaling proteins in which novel serine or threoninephosphorylation sites of the invention occur also are expressed in normal cells and tissues, it may also be advantageous to administer a phosphorylation site-specific antibody with a constant region modified to reduce or eliminate ADCC or CDC to limit damage to normal cells. For example, effector function of an antibodies may be reduced or eliminated by utilizing an IgG1 constant domain instead of an IgG2/4 fusion domain. Other ways of eliminating effector function can be envisioned such as, e.g., mutation of the sites known to interact with FcR or insertion of a peptide in the hinge region, thereby eliminating critical sites required for FcR interaction. Variant antibodies with reduced or no effector function also include variants as described previously herein.

The peptides and antibodies of the invention may be used in combination with other therapies or with other agents. Other agents include but are not limited to polypeptides, small molecules, chemicals, metals, organometallic compounds, inorganic compounds, nucleic acid molecules, oligonucleotides, aptamers, spiegelmers, antisense nucleic acids, locked nucleic acid (LNA) inhibitors, peptide nucleic acid (PNA) inhibitors, immunomodulatory agents, antigen-binding fragments, prodrugs, and peptidomimetic compounds. In certain embodiments, the antibodies and peptides of the invention may be used in combination with cancer therapies known to one of skill in the art.

In certain aspects, the present disclosure relates to combination treatments comprising a phosphorylation site-specific antibody described herein and immunomodulatory compounds, vaccines or chemotherapy. Illustrative examples of suitable immunomodulatory agents that may be used in such combination therapies include agents that block negative regulation of T cells or antigen presenting cells (e.g., anti-CTLA4 antibodies, anti-PD-L1 antibodies, anti-PDL-2 antibodies, anti-PD-1 antibodies and the like) or agents that enhance positive co-stimulation of T cells (e.g., anti-CD40 antibodies or anti 4-1BB antibodies) or agents that increase NK cell number or T-cell activity (e.g., inhibitors such as IMiDs, thalidomide, or thalidomide analogs). Furthermore, immunomodulatory therapy could include cancer vaccines such as dendritic cells loaded with tumor cells, proteins, peptides, RNA, or DNA derived from such cells, patient derived heat-shock proteins (hsp's) or general adjuvants stimulating the immune system at various levels such as CpG, Luivac®, Biostim®, Ribomunyl®, Imudon®, Bronchovaxom® or any other compound or other adjuvant activating receptors of the innate immune system (e.g., toll like receptor agonist, anti-CTLA-4 antibodies, etc.). Also, immunomodulatory therapy could include treatment with cytokines such as IL-2, GM-CSF and IFN-gamma.

Furthermore, combination of antibody therapy with chemotherapeutics could be particularly useful to reduce overall tumor burden, to limit angiogenesis, to enhance tumor accessibility, to enhance susceptibility to ADCC, to result in increased immune function by providing more tumor antigen, or to increase the expression of the T cell attractant LIGHT.

Pharmaceutical compounds that may be used for combinatory anti-tumor therapy include, merely to illustrate: aminoglutethimide, amsacrine, anastrozale, asparaginase, bcg, bicalutamide, bleomycin, buserelin, busulfan, camptothecin, capecitabine, carboplatin, carmustine, chlorambucil, cisplatin, cladribine, clodronate, colchicine, cyclophosphamide, cyproterone, cytarabine, dacarbazine, dactinomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, letrozole, leucovorin, leuprolide, levamisole, lomustine, mechlorethamine, medroxyprogesterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.

These chemotherapeutic anti-tumor compounds may be categorized by their mechanism of action into groups, including, for example, the following classes of agents: anti-metaboGtes/anti-caneer agents, such as pyrimidine analogs (5-fluorouracil, floxuridine, capecitabine, gemcitabine and cytarabine) and purine analogs, folate inhibitors and related inhibitors (mercaptopurine, thioguanine, pentostatin and 2-chlorodeoxyadenosine (cladribine)); antiproliferative/antimitotic agents including natural products such as vinca alkaloids (vinblastine, vincristine, and vinorelbine), microtubule disruptors such as taxane (paclitaxel, docetaxel), vincristine, vinblastine, nocodazole, epothilones and navelbine, epidipodophyllotoxins (etoposide, teniposide), DNA damaging agents (actinomycin, amsacrine, anthracyclines, bleomycin, busulfan, camptothecin, carboplatin, chlorambucil, cisplatin, cyclophosphamide, cytoxan, dactinomycin, daunorubicin, doxorubicin, epirubicin, hexamethylmelamineoxaliplatin, iphosphamide, melphalan, mechlorethamine, mitomycin, mitoxantrone, nitrosourea, plicamycin, procarbazine, taxol, taxotere, teniposide, triethylenethiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (L-asparaginase which systemically metabolizes L-asparagine and deprives cells which do not have the capacity to synthesize their own asparagine); antiplatelet agents; antiproliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), trazenes - dacarbazinine (DTIC); antiproliferative/antimitotic antimetabolites such as folic acid analogs (methotrexate); platinum coordination complexes (cisplatin, carboplatin), procarbazine, hydroxyurea, mitotane, aminoglutethimide; hormones, hormone analogs (estrogen, tamoxifen, goserelin, bicalutamide, nilutamide) and aromatase inhibitors (letrozole, anastrozole); anticoagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory agents; antisecretory agents (breveldin); immunosuppressives (cyclosporine, tacrolimus (FK-506), sirolimus (rapamycin), azathioprine, mycophenolate mofetil); immunomodulatory agents (thalidomide and analogs thereof such as lenalidomide (Revlimid, CC-5013) and CC-4047 (Actimid)), cyclophosphamide; anti-angiogenic compounds (TNP-470, genistein) and growth factor inhibitors (vascular endothelial growth factor (VEGF) inhibitors, fibroblast growth factor (FGF) inhibitors); angiotensin receptor blocker; nitric oxide donors; anti-sense oligonucleotides; antibodies (trastuzumab); cell cycle inhibitors and differentiation inducers (tretinoin); mTOR inhibitors, topoisomerase inhibitors (doxorubicin (adriamycin), amsacrine, camptothecin, daunorubicin, dactinomycin, eniposide, epirubicin, etoposide, idarubicin and mitoxantrone, topotecan, irinotecan), corticosteroids (cortisone, dexamethasone, hydrocortisone, methylprednisolone, prednisone, and prenisolone); growth factor signal transduction kinase inhibitors; mitochondrial dysfunction inducers and caspase activators; and chromatin disruptors.

In certain embodiments, pharmaceutical compounds that may be used for combinatory anti-angiogenesis therapy include: (1) inhibitors of release of "angiogenic molecules," such as bFGF (basic fibroblast growth factor); (2) neutralizers of angiogenic molecules, such as anti- bFGF antibodies; and (3) inhibitors of endothelial cell response to angiogenic stimuli, including collagenase inhibitor, basement membrane turnover inhibitors, angiostatic steroids, fungal-derived angiogenesis inhibitors, platelet factor 4, thrombospondin, arthritis drugs such as D-penicillamine and gold thiomalate, vitamin D₃ analogs, alpha-interferon, and the like. For additional proposed inhibitors of angiogenesis, see Blood et al., Biochim. Biophys. Acta, 1032:89-118 (1990), Moses et al., Science, 248:1408-1410 (1990), Ingber et al., Lab. Invest., 59:44-51 (1988), and U.S. Pat. Nos. 5,092,885, 5,112,946, 5,192,744, 5,202,352, and 6,573,256. In addition, there are a wide variety of compounds that can be used to inhibit angiogenesis, for example, peptides or agents that block the VEGF-mediated angiogenesis pathway, endostatin protein or derivatives, lysine binding fragments of angiostatin, melanin or melanin-promoting compounds, plasminogen fragments (e.g., Kringle; 1-3 of plasminogen), troponin subunits, inhibitors of vitronectin 3, peptides derived from Saposin B, antibiotics or analogs (e.g., tetracycline or neomycin), dienogest-containing compositions, compounds comprising a MetAP-2 inhibitory core coupled to a peptide, the compound EM-138, chalcone and its analogs, and naaladase inhibitors. See, for example, U.S. Pat. Nos. 6,395,718, 6,462,075, 6,465,431, 6,475,784, 6,482,802, 6,482,810, 6,500,431, 6,500,924, 6,518,298, 6,521,439, 6,525,019, 6,538,103, 6,544,758, 6,544,947, 6,548,477, 6,559,126, and 6,569,845.

### 7. Diagnostic Uses

In a further aspect, the invention provides methods for detecting and quantitating phosphoyrlation at a novel serine or threoninephospliorylation site of the invention. For example, peptides, including AQUA peptides of the invention, and antibodies of the invention are useful in diagnostic and prognostic evaluation of carcinomas, wherein the carcinoma is associated with the phosphorylation state of a novel phosphorylation site in Table 1, whether phosphorylated or dephosphorylated.

Methods of diagnosis can be performed *in vitro* using a biological sample (e.g., blood sample, lymph node biopsy or tissue) from a subject, or in vivo. The phosphorylation state or level at the serine or threonineresidue identified in the corresponding row in Column D of Table 1 may be assessed. A change in the phosphorylation state or level at the phosphorylation site, as compared to a control, indicates that the subject is suffering from, or susceptible to, carcinoma.

In one embodiment, the phosphorylation state or level at a novel phosphorylation site is determined by an AQUA peptide comprising the phosphorylation site. The AQUA peptide may be phosphorylated or unphosphorylated at the specified serine or threonineposition.

In another embodiment, the phosphorylation state or level at a phosphorylation site is determined by an antibody or antigen-binding fragment thereof, wherein the antibody specifically binds the phosphorylation site. The antibody may be one that only binds to the phosphorylation site when the serine or threonineresidue is phosphorylated, but does not bind to the same sequence when the serine or threonineis not phosphorylated; or vice versa.

In particular embodiments, the antibodies of the present application are attached to labeling moieties, such as a detectable marker. One or more detectable labels can be attached to the antibodies. Exemplary labeling moieties include radiopaque dyes, radiocontrast agents, fluorescent molecules, spin-labeled molecules, enzymes, or other labeling moieties of diagnostic value, particularly in radiologic or magnetic resonance imaging techniques.

A radiolabeled antibody in accordance with this disclosure can be used for in vitro diagnostic tests. The specific activity of an antibody, binding portion thereof, probe, or ligand, depends upon the half-life, the isotopic purity of the radioactive label, and how the label is incorporated into the biological agent. In immunoassay tests, the higher the specific activity, in general, the better the sensitivity. Radioisotopes useful as labels, e.g., for use in diagnostics, include iodine (¹³¹I or ¹²⁵I), indium (¹¹¹In), technetium (⁹⁹Tc), phosphorus ³²P), carbon (¹⁴C), and tritium (³H), or one of the therapeutic isotopes listed above.

Fluorophore and chromophore labeled biological agents can be prepared from standard moieties known in the art. Since antibodies and other proteins absorb light having wavelengths up to about 310 nm, the fluorescent moieties may be selected to have substantial absorption at wavelengths above 310 nm, such as for example, above 400 nm. A variety of suitable fluorescers and chromophores are described by Stryer, Science, 162:526 (1968) and Brand et al., Annual Review of Biochemistry, 41:843-868 (1972), which are hereby incorporated by reference. The antibodies can be labeled with fluorescent chromophore groups by conventional procedures such as those disclosed in U.S. Patent Nos. 3,940,475, 4,289,747, and 4,376,110, which are hereby incorporated by reference.

The control may be parallel samples providing a basis for comparison, for example, biological samples drawn from a healthy subject, or biological samples drawn from healthy tissues of the same subject. Alternatively, the control may be a pre-determined reference or threshold amount. If the subject is being treated with a therapeutic agent, and the progress of the treatment is monitored by detecting the serine or threonine phosphorylation state level at a phosphorylation site of the invention, a control may be derived from biological samples drawn from the subject prior to, or during the course of the treatment.

In certain embodiments, antibody conjugates for diagnostic use in the present application are intended for use in vitro, where the antibody is linked to a secondary binding ligand or to an enzyme (an enzyme tag) that will generate a colored product upon contact with a chromogenic substrate. Examples of suitable enzymes include urease, alkaline phosphatase, (horseradish) hydrogen peroxidase and glucose oxidase. In certain embodiments, secondary binding ligands are biotin and avidin or streptavidin compounds.

Antibodies of the invention may also be optimized for use in a flow cytometry (FC) assay to determine the activation/phosphorylation status of a target signaling protein in subjects before, during, and after treatment with a therapeutic agent targeted at inhibiting serine or threoninephosphorylation at the phosphorylation site disclosed herein. For example, bone marrow cells or peripheral blood cells from patients may be analyzed by flow cytometry for target signaling protein phosphorylation, as well as for markers identifying various hematopoietic cell types. In this manner, activation status of the malignant cells may be specifically characterized. Flow cytometry may be carried out according to standard methods. *See,* e.g., Chow et al., Cytometry (Communications in Clinical Cytometry) 46: 72-78 (2001).

Alternatively, antibodies of the invention may be used in immunohistochemical (IHC) staining to detect differences in signal transduction or protein activity using normal and diseased tissues. IHC may be carried out according to well-known techniques. *See,* e.g., Antibodies: A Laboratory Manual, supra.

Peptides and antibodies of the invention may be also be optimized for use in other clinically-suitable applications, for example bead-based multiplex-type assays, such as IGEN, Luminex^{™} and/or Bioplex^{™} assay formats, or otherwise optimized for antibody arrays formats, such as reversed-phase array applications *(see,* e.g. Paweletz et al., Oncogene 20(16): 1981-89 (2001)). Accordingly, in another embodiment, the invention provides a method for the multiplex detection of the phosphorylation state or level at two or more phosphorylation sites of the invention (Table 1) in a biological sample, the method comprising utilizing two or more antibodies or AQUA peptides of the invention. In one preferred embodiment, two to five antibodies or AQUA peptides of the invention are used. In another preferred embodiment, six to ten antibodies or AQUA peptides of the invention are used, while in another preferred embodiment eleven to twenty antibodies or AQUA peptides of the invention are used.

In certain embodiments the diagnostic methods of the application may be used in combination with other cancer diagnostic tests.

The biological sample analyzed may be any sample that is suspected of having abnormal serine or threoninephosphorylation at a novel phosphorylation site of the invention, such as a homogenized neoplastic tissue sample.

### 8. Screening assays

In another aspect, the invention provides a method for identifying an agent that modulates serine or threoninephosphorylation at a novel phosphorylation site of the invention, comprising: a) contacting a candidate agent with a peptide or protein comprising a novel phosphorylation site of the invention; and b) determining the phosphorylation state or level at the novel phosphorylation site. A change in the phosphorylation level of the specified serine or threoninein the presence of the test agent, as compared to a control, indicates that the candidate agent potentially modulates serine or threonine phosphorylation at a novel phosphorylation site of the invention.

In one embodiment, the phosphorylation state or level at a novel phosphorylation site is determined by an AQUA peptide comprising the phosphorylation site. The AQUA peptide may be phosphorylated or unphosphorylated at the specified serine or threonineposition.

In another embodiment, the phosphorylation state or level at a phosphorylation site is determined by an antibody or antigen-binding fragment thereof, wherein the antibody specifically binds the phosphorylation site. The antibody may be one that only binds to the phosphorylation site when the serine or threonineresidue is phosphorylated, but does not bind to the same sequence when the serine or threonineis not phosphorylated; or vice versa.

In particular embodiments, the antibodies of the present application are attached to labeling moieties, such as a detectable marker.

The control may be parallel samples providing a basis for comparison, for example, the phosphorylation level of the target protein or peptide in absence of the testing agent. Alternatively, the control may be a pre-determined reference or threshold amount.

### 9. Immunoassays

In another aspect, the present application concerns immunoassays for binding, purifying, quantifying and otherwise generally detecting the phosphorylation state or level at a novel phosphorylation site of the invention.

Assays may be homogeneous assays or heterogeneous assays. In a homogeneous assay the immunological reaction usually involves a phosphorylation site-specific antibody of the invention, a labeled analyte, and the sample of interest. The signal arising from the label is modified, directly or indirectly, upon the binding of the antibody to the labeled analyte. Both the immunological reaction and detection of the extent thereof are carried out in a homogeneous solution. Immunochemical labels that may be used include free radicals, radioisotopes, fluorescent dyes, enzymes, bacteriophages, coenzymes, and so forth.

In a heterogeneous assay approach, the reagents are usually the specimen, a phosphorylation site-specific antibody of the invention, and suitable means for producing a detectable signal. Similar specimens as described above may be used. The antibody is generally immobilized on a support, such as a bead, plate or slide, and contacted with the specimen suspected of containing the antigen in a liquid phase. The support is then separated from the liquid phase and either the support phase or the liquid phase is examined for a detectable signal using means for producing such signal. The signal is related to the presence of the analyte in the specimen. Means for producing a detectable signal include the use of radioactive labels, fluorescent labels, enzyme labels, and so forth.

Phosphorylation site-specific antibodies disclosed herein may be conjugated to a solid support suitable for a diagnostic assay (e.g., beads, plates, slides or wells formed from materials such as latex or polystyrene) in accordance with known techniques, such as precipitation.

In certain embodiments, immunoassays are the various types of enzyme linked immunoadsorbent assays (ELISAs) and radioimmunoassays (RIA) known in the art. Immunohistochemical detection using tissue sections is also particularly useful. However, it will be readily appreciated that detection is not limited to such techniques, and Western blotting, dot and slot blotting, FACS analyses, and the like may also be used. The steps of various useful immunoassays have been described in the scientific literature, such as, e.g., Nakamura et al., in Enzyme Immunoassays: Heterogeneous and Homogeneous Systems, Chapter 27 (1987), incorporated herein by reference.

In general, the detection of immunocomplex formation is well known in the art and may be achieved through the application of numerous approaches. These methods are based upon the detection of radioactive, fluorescent, biological or enzymatic tags. Of course, one may find additional advantages through the use of a secondary binding ligand such as a second antibody or a biotin/avidin ligand binding arrangement, as is known in the art.

The antibody used in the detection may itself be conjugated to a detectable label, wherein one would then simply detect this label. The amount of the primary immune complexes in the composition would, thereby, be determined.

Alternatively, the first antibody that becomes bound within the primary immune complexes may be detected by means of a second binding ligand that has binding affinity for the antibody. In these cases, the second binding ligand may be linked to a detectable label. The second binding ligand is itself often an antibody, which may thus be termed a "secondary" antibody. The primary immune complexes are contacted with the labeled, secondary binding ligand, or antibody, under conditions effective and for a period of time sufficient to allow the formation of secondary immune complexes. The secondary immune complexes are washed extensively to remove any non-specifically bound labeled secondary antibodies or ligands, and the remaining label in the secondary immune complex is detected.

An enzyme linked immunoadsorbent assay (ELISA) is a type of binding assay. In one type of ELISA, phosphorylation site-specific antibodies disclosed herein are immobilized onto a selected surface exhibiting protein affinity, such as a well in a polystyrene microtiter plate. Then, a suspected neoplastic tissue sample is added to the wells. After binding and washing to remove non-specifically bound immune complexes, the bound target signaling protein may be detected.

In another type of ELISA, the neoplastic tissue samples are immobilized onto the well surface and then contacted with the phosphorylation site-specific antibodies disclosed herein. After binding and washing to remove non-specifically bound immune complexes, the bound phosphorylation site-specific antibodies are detected.

Irrespective of the format used, ELISAs have certain features in common, such as coating, incubating or binding, washing to remove non-specifically bound species, and detecting the bound immune complexes.

The radioimmunoassay (RIA) is an analytical technique which depends on the competition (affinity) of an antigen for antigen-binding sites on antibody molecules. Standard curves are constructed from data gathered from a series of samples each containing the same known concentration of labeled antigen, and various, but known, concentrations of unlabeled antigen. Antigens are labeled with a radioactive isotope tracer. The mixture is incubated in contact with an antibody. Then the free antigen is separated from the antibody and the antigen bound thereto. Then, by use of a suitable detector, such as a gamma or beta radiation detector, the percent of either the bound or free labeled antigen or both is determined. This procedure is repeated for a number of samples containing various known concentrations of unlabeled antigens and the results are plotted as a standard graph. The percent of bound tracer antigens is plotted as a function of the antigen concentration. Typically, as the total antigen concentration increases the relative amount of the tracer antigen bound to the antibody decreases. After the standard graph is prepared, it is thereafter used to determine the concentration of antigen in samples undergoing analysis.

In an analysis, the sample in which the concentration of antigen is to be determined is mixed with a known amount of tracer antigen. Tracer antigen is the same antigen known to be in the sample but which has been labeled with a suitable radioactive isotope. The sample with tracer is then incubated in contact with the antibody. Then it can be counted in a suitable detector which counts the free antigen remaining in the sample. The antigen bound to the antibody or immunoadsorbent may also be similarly counted. Then, from the standard curve, the concentration of antigen in the original sample is determined.

### 10. Pharmaceutical Formulations and Methods of Administration

Methods of administration of therapeutic agents, particularly peptide and antibody therapeutics, are well-known to those of skill in the art.

Peptides of the invention can be administered in the same manner as conventional peptide type pharmaceuticals. Preferably, peptides are administered parenterally, for example, intravenously, intramuscularly, intraperitoneally, or subcutaneously. When administered orally, peptides may be proteolytically hydrolyzed. Therefore, oral application may not be usually effective. However, peptides can be administered orally as a formulation wherein peptides are not easily hydrolyzed in a digestive tract, such as liposome-microcapsules. Peptides may be also administered in suppositories, sublingual tablets, or intranasal spray.

If administered parenterally, a preferred pharmaceutical composition is an aqueous solution that, in addition to a peptide of the invention as an active ingredient, may contain for example, buffers such as phosphate, acetate, etc., osmotic pressure-adjusting agents such as sodium chloride, sucrose, and sorbitol, etc., antioxidative or antioxygenic agents, such as ascorbic acid or tocopherol and preservatives, such as antibiotics. The parenterally administered composition also may be a solution readily usable or in a lyophilized form which is dissolved in sterile water before administration.

The pharmaceutical formulations, dosage forms, and uses described below generally apply to antibody-based therapeutic agents, but are also useful and can be modified, where necessary, for making and using therapeutic agents of the disclosure that are not antibodies.

To achieve the desired therapeutic effect, the phosphorylation site-specific antibodies or antigen-binding fragments thereof can be administered in a variety of unit dosage forms. The dose will vary according to the particular antibody. For example, different antibodies may have different masses and/or affinities, and thus require different dosage levels. Antibodies prepared as Fab or other fragments will also require differing dosages than the equivalent intact immunoglobulins, as they are of considerably smaller mass than intact immunoglobulins, and thus require lower dosages to reach the same molar levels in the patient's blood. The dose will also vary depending on the manner of administration, the particular symptoms of the patient being treated, the overall health, condition, size, and age of the patient, and the judgment of the prescribing physician. Dosage levels of the antibodies for human subjects are generally between about 1 mg per kg and about 100 mg per kg per patient per treatment, such as for example, between about 5 mg per kg and about 50 mg per kg per patient per treatment. In terms of plasma concentrations, the antibody concentrations may be in the range from about 25 µg/mL to about 500 µg/mL. However, greater amounts may be required for extreme cases and smaller amounts may be sufficient for milder cases.

Administration of an antibody will generally be performed by a parenteral route, typically via injection such as intra-articular or intravascular injection (e.g., intravenous infusion) or intramuscular injection. Other routes of administration, e.g., oral (p.o.); may be used if desired and practicable for the particular antibody to be administered. An antibody can also be administered in a variety of unit dosage forms and their dosages will also vary with the size, potency, and in vivo half-life of the particular antibody being administered. Doses of a phosphorylation site-specific antibody will also vary depending on the manner of administration, the particular symptoms of the patient being treated, the overall health, condition, size, and age of the patient, and the judgment of the prescribing physician.

The frequency of administration may also be adjusted according to various parameters. These include the clinical response, the plasma half-life of the antibody, and the levels of the antibody in a body fluid, such as, blood, plasma, serum, or synovial fluid. To guide adjustment of the frequency of administration, levels of the antibody in the body fluid may be monitored during the course of treatment.

Formulations particularly useful for antibody-based therapeutic agents are also described in U.S. Patent App. Publication Nos. 20030202972, 20040091490 and 20050158316. In certain embodiments, the liquid formulations of the application are substantially free of surfactant and/or inorganic salts. In another specific embodiment, the liquid formulations have a pH ranging from about 5.0 to about 7.0. In yet another specific embodiment, the liquid formulations comprise histidine at a concentration ranging from about 1 mM to about 100 mM. In still another specific embodiment, the liquid formulations comprise histidine at a concentration ranging from I mM to 100 mM. It is also contemplated that the liquid formulations may further comprise one or more excipients such as a saccharide, an amino acid (e.g., arginine, lysine, and methionine) and a polyol. Additional descriptions and methods of preparing and analyzing liquid formulations can be found, for example, in PCT publications WO 03/106644, WO 04/066957, and WO 04/091658.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the pharmaceutical compositions of the application.

In certain embodiments, formulations of the subject antibodies are pyrogen-free formulations which are substantially free of endotoxins and/or related pyrogenic substances. Endotoxins include toxins that are confined inside microorganisms and are released when the microorganisms are broken down or die. Pyrogenic substances also include fever-inducing, thermostable substances (glycoproteins) from the outer membrane of bacteria and other microorganisms. Both of these substances can cause fever, hypotension and shock if administered to humans. Due to the potential harmful effects, it is advantageous to remove even low amounts of endotoxins from intravenously administered pharmaceutical drug solutions. The Food & Drug Administration ("FDA") has set an upper limit of 5 endotoxin units (EU) per dose per kilogram body weight in a single one hour period for intravenous drug applications (The United States Pharmacopeial Convention, Pharmacopeial Forum 26 (1):223 (2000)). When therapeutic proteins are administered in amounts of several hundred or thousand milligrams per kilogram body weight, as can be the case with monoclonal antibodies, it is advantageous to remove even trace amounts of endotoxin.

The amount of the formulation which will be therapeutically effective can be determined by standard clinical techniques. In addition, *in vitro* assays may optionally be used to help identify optimal dosage ranges. The precise dose to be used in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. The dosage of the compositions to be administered can be determined by the skilled artisan without undue experimentation in conjunction with standard dose-response studies. Relevant circumstances to be considered in making those determinations include the condition or conditions to be treated, the choice of composition to be administered, the age, weight, and response of the individual patient, and the severity of the patient's symptoms. For example, the actual patient body weight may be used to calculate the dose of the formulations in milliliters (mL) to be administered. There may be no downward adjustment to "ideal" weight. In such a situation, an appropriate dose may be calculated by the following formula:

Dose (mL) = [patient weight (kg) x dose level (mg/kg)/ drug concentration (mg/mL)]

For the purpose of treatment of disease, the appropriate dosage of the compounds (for example, antibodies) will depend on the severity and course of disease, the patient's clinical history and response, the toxicity of the antibodies, and the discretion of the attending physician. The initial candidate dosage may be administered to a patient. The proper dosage and treatment regimen can be established by monitoring the progress of therapy using conventional techniques known to those of skill in the art.

The formulations of the application can be distributed as articles of manufacture comprising packaging material and a pharmaceutical agent which comprises, e.g., the antibody and a pharmaceutically acceptable carrier as appropriate to the mode of administration. The packaging material will include a label which indicates that the formulation is for use in the treatment of prostate cancer.

### 11. Kits

Antibodies and peptides (including AQUA peptides) of the invention may also be used within a kit for detecting the phosphorylation state or level at a novel phosphorylation site of the invention, comprising at least one of the following: an AQUA peptide comprising the phosphorylation site, or an antibody or an antigen-binding fragment thereof that binds to an amino acid sequence comprising the phosphorylation site. Such a kit may further comprise a packaged combination of reagents in predetermined amounts with instructions for performing the diagnostic assay. Where the antibody is labeled with an enzyme, the kit will include substrates and co-factors required by the enzyme. In addition, other additives may be included such as stabilizers, buffers and the like. The relative amounts of the various reagents may be varied widely to provide for concentrations in solution of the reagents that substantially optimize the sensitivity of the assay. Particularly, the reagents may be provided as dry powders, usually lyophilized, including excipients that, on dissolution, will provide a reagent solution having the appropriate concentration.

The following Examples are provided only to further illustrate the invention, and are not intended to limit its scope, except as provided in the claims appended hereto. The invention encompasses modifications and variations of the methods taught herein which would be obvious to one of ordinary skill in the art.

### EXAMPLE 1

### Isolation of Phospho-tyrosine, Phospho-serine and Phospho-threonine Containing Peptides from Extracts of Carcinoma and Leukemia Cell Lines and Tissues and Identification of Novel Phosphorylation Sites.

In order to discover novel serine or threoninephosphorylation sites in carcinoma, IAP isolation techniques were used to identify phosphoserine and/or threonine-containing peptides in cell extracts from human carcinoma cell lines and patient cell lines identified in Column G of Table 1 including HeLa, Jurkat, K562, DMS 153, SEM, DMS 153, GM18366, H1703, M059K, H3255, MKN-45, HEL, GM00200, H44, rat brain, H441, 001364548-cll, M059J, HT29, H1373, Kyse140, CLL23LB4, M01043, MV4-1 l, MOLM 14, H446, MEC-2, HCTI 16, and 3T3. Tryptic phosphoserine and/or threonine-containing peptides were purified and analyzed from extracts of each of the cell lines mentioned above, as follows. Cells were cultured in DMEM medium or RPMI 1640 medium supplemented with 10% fetal bovine serum and penicillin/streptomycin.

Suspension cells were harvested by low speed centrifugation. After complete aspiration of medium, cells were resuspended in 1 mL lysis buffer per 1.25 x 10⁸ cells (20 mM HEPES pH 8.0, 9 M urea, 1 mM sodium vanadate, supplemented or not with 2.5 mM sodium pyro-phosphate, 1 mM β-glycerol-phosphate) and sonicated.

Adherent cells at about 80% confluency were starved in medium without serum overnight and stimulated, with ligand depending on the cell type or not stimulated. After complete aspiration of medium from the plates, cells were scraped off the plate in 10 ml lysis buffer per 2 x 10⁸ cells (20 mM HEPES pH 8.0, 9M urea, 1 mM sodium vanadate, supplemented with 2.5 mM sodium pyrophosphate, 1 mM ß-glycerol-phosphate) and sonicated.

Frozen tissue samples were cut to small pieces, homogenize in lysis buffer (20 mM HEPES pH 8.0, 9 M Urea, 1 mN sodium vanadate, supplemented with 2.5 mM sodium pyrophosphate, 1mM.b-glycerol-phosphate, 1 ml lysis buffer for 100 mg of frozen tissue) using a polytron for 2 times of 20 sec. each time. Homogenate is then briefly sonicated.

Sonicated cell lysates were cleared by centrifugation at 20,000 x g, and proteins were reduced with DTT at a final concentration of 4.1 mM and alkylated with iodoacetamide at 8.3 mM. For digestion with trypsin, protein extracts were diluted in 20 mM HEPES pH 8.0 to a final concentration of 2 M urea and soluble TLCK-trypsin (Worthington) was added at 10-20 µg/mL. Digestion was performed for 1-2 days at room temperature.

Trifluoroacetic acid (TFA) was added to protein digests to a final concentration of 1 %, precipitate was removed by centrifugation, and digests were loaded onto Sep-Pak C₁₈ columns (Waters) equilibrated with 0.1% TFA. A column volume of 0.7-1.0 ml was used per 2 x 10⁸ cells. Columns were washed with 15 volumes of 0.1 % TFA, followed by 4 volumes of 5% acetonitrile (MeCN) in 0.1 % TFA. Peptide fraction I was obtained by eluting columns with 2 volumes each of 8, 12, and 15% MeCN in 0.1 % TFA and combining the eluates. Fractions II and III were a combination of eluates after eluting columns with 18, 22, 25% MeCN in 0.1% TFA and with 30, 35, 40% MeCN in 0.1 % TFA, respectively. All peptide fractions were lyophilized.

Peptides from each fraction corresponding to 2 x 10⁸ cells were dissolved in 1 ml of IAP buffer (20 mM Tris/HCl or 50 mM MOPS pH 7.2, 10 mM sodium phosphate, 50 mM NaCl) and insoluble matter (mainly in peptide fractions III) was removed by centrifugation. IAP was performed on each peptide fraction separately. The phosphoserine or threoninemonoclonal antibody P-Tyr-100 (Cell Signaling Technology, Inc., catalog number 9411) was coupled at 4 mg/ml beads to protein G (Roche), respectively. Immobilized antibody (15 µl, 60 µg) was added as 1:1 slurry in IAP buffer to 1 ml of each peptide fraction, and the mixture was incubated overnight at 4° C with gentle rotation. The immobilized antibody beads were washed three times with 1 ml IAP buffer and twice with 1 ml water, all at 4° C. Peptides were eluted from beads by incubation with 75 µl of 0.1% TFA at room temperature for 10 minutes.

Alternatively, one single peptide fraction was obtained from Sep-Pak C18 columns by elution with 2 volumes each of 10%, 15%, 20 %, 25 %, 30 %, 35 % and 40 % acetonitirile in 0.1% TFA and combination of all eluates. IAP on this peptide fraction was performed as follows: After

lyophilization, peptide was dissolved in 1.4 ml IAP buffer (MOPS pH 7.2,

10 mM sodium phosphate, 50 mM NaCl) and insoluble matter was removed by centrifugation. Immobilized antibody (40 µl 160 µg) was added as 1:1 slurry in IAP buffer, and the mixture was incubated overnight at 4° C with gentle shaking. The immobilized antibody beads were washed three times with 1 ml IAP buffer and twice with 1 ml water, all at 4° C. Peptides were eluted from beads by incubation with 55 µl of 0.15% TFA at room temperature for 10 min (eluate 1), followed by a wash of the beads (eluate 2) with 45 µl of 0.15% TFA. Both eluates were combined.

### Analysis by LC-MS/MS Mass Spectrometry.

40 µl or more of IAP eluate were purified by 0.2 µl StageTips or ZipTips. Peptides were eluted from the microcolumns with 1 µl of 40% MeCN, 0.1 % TFA (fractions I and II) or 1 µl of 60% MeCN, 0.1% TFA (fraction III) into 7.6-9.0 µl of 0.4% acetic acid/0.005% heptafluorobutyric acid. For single fraction analysis, 1 µl of 60% MeCN, 0.1% TFA, was used for elution from the microcolumns. This sample was loaded onto a 10 cm x 75 µm PicoFrit capillary column (New Objective) packed with Magic C18 AQ reversed-phase resin (Michrom Bioresources) using a Famos autosampler with an inert sample injection valve (Dionex). The column was then developed with a 45-min linear r gradient of acetonitrile delivered at 200 nl/min (Ultimate, Dionex), and tandem mass spectra were collected in a data-dependent manner with an LTQ ion trap mass spectrometer essentially as described by Gygi *et al., supra.*

### Database Analysis & Assignments.

MS/MS spectra were evaluated using TurboSequest in the Sequest Browser package (v. 27, rev. 12) supplied as part of BioWorks 3.0 (ThermoFinnigan). Individual MS/MS spectra were extracted from the raw data file using the Sequest Browser program CreateDta, with the following settings: bottom MW, 700; top MW, 4,500; minimum number of ions, 20 (40 for L TQ); minimum TIC, 4 x 10⁵ (2 x 10³ for LTQ); and precursor charge state, unspecified. Spectra were extracted from the beginning of the raw data file before sample injection to the end of the eluting gradient. The IonQuest and VuDta programs were not used to further select MS/MS spectra for Sequest analysis. MS/MS spectra were evaluated with the following TurboSequest parameters: peptide mass tolerance, 2.5; fragment ion tolerance, 0.0 (1,0 for LTQ); maximum number of differential amino acids per modification, 4; mass type parent, average; mass type fragment, average; maximum number of internal cleavage sites, 10; neutral losses of water and ammonia from b and y ions were considered in the correlation analysis. Proteolytic enzyme was specified except for spectra collected from elastase digests.

Searches were performed against the NCBI human protein database (NCBI RefSeq protein release #11; 8 May 2005; 1,826,611 proteins, including 47,859 human proteins. Peptides that did not match RefSeq were compared to NCBI GenPept release #148; 15 June 2005 release date; 2,479,172 proteins, including 196,054 human proteins.). Cysteine carboxamidomethylation was specified as a static modification, and phosphorylation was allowed as a variable modification on serine or threonineresidues. It was determined that restricting phosphorylation to serine or threonine residues had little effect on the number of phosphorylation sites assigned.

In proteomics research, it is desirable to validate protein identifications based solely on the observation of a single peptide in one experimental result, in order to indicate that the protein is, in fact, present in a sample. This has led to the development of statistical methods for validating peptide assignments, which are not yet universally accepted, and guidelines for the publication of protein and peptide identification results *(see* Carr et al., Mol. Cell Proteomics 3: 531-533 (2004)), which were followed in this Example. However, because the immunoaffinity strategy separates phosphorylated peptides from unphosphorylated peptides, observing just one phosphopeptide from a protein is a common result, since many phosphorylated proteins have only one serine and/or threonine-phosphorylated site. For this reason, it is appropriate to use additional criteria to validate phosphopeptide assignments. Assignments are likely to be correct if any of these additional criteria are met: (i) the same phosphopeptide sequence is assigned to co-eluting ions with different charge states, since the MS/MS spectrum changes markedly with charge state; (ii) the phosphorylation site is found in more than one peptide sequence context due to sequence overlaps from incomplete proteolysis or use of proteases other than trypsin; (iii) the phosphorylation site is found in more than one peptide sequence context due to homologous but not identical protein isoforms; (iv) the phosphorylation site is found in more than one peptide sequence context due to homologous but not identical proteins among species; and (v) phosphorylation sites validated by MS/MS analysis of synthetic phosphopeptides corresponding to assigned sequences, since the ion trap mass spectrometer produces highly reproducible MS/MS spectra. The last criterion is routinely used to confirm novel site assignments of particular interest.

All spectra and all sequence assignments made by Sequest were imported into a relational database. The following Sequest scoring thresholds were used to select phosphopeptide assignments that are likely to be correct: RSp < 6, XCorr > 2.2, and DeltaCN > 0.099. Further, the sequence assignments could be accepted or rejected with respect to accuracy by using the following conservative, two-step process.

In the first step, a subset of high-scoring sequence assignments should be selected by filtering for XCorr values of at least 1.5 for a charge state of +1, 2.2 for +2, and 3.3 for +3, allowing a maximum RSp value of 10. Assignments in this subset should be rejected if any of the following criteria are satisfied: (i) the spectrum contains at least one major peak (at least 10% as intense as the most intense ion in the spectrum) that can not be mapped to the assigned sequence as an *a, b,* or y ion, as an ion arising from neutral-loss of water or ammonia from a b or y ion, or as a multiply protonated ion; (ii) the spectrum does not contain a series of *b or y* ions equivalent to at least six uninterrupted residues; or (iii) the sequence is not observed at least five times in all the studies conducted (except for overlapping sequences due to incomplete proteolysis or use of proteases other than trypsin).

In the second step, assignments with below-threshold scores should be accepted if the low-scoring spectrum shows a high degree of similarity to a high-scoring spectrum collected in another study, which simulates a true reference library-searching strategy.

### EXAMPLE 2

### Production of Phosphorylation site-Specific Polyclonal Antibodies

Polyclonal antibodies that specifically bind a novel phosphorylation site of the invention (Table 1/Figure 2) only when the serine or threonineresidue is phosphorylated (and does not bind to the same sequence when the serine or threonineis not phosphorylated), and vice versa, are produced according to standard methods hy first constructing a synthetic peptide antigen comprising the phosphorylation site and then immunizing an animal to raise antibodies against the antigen, as further described below. Production of exemplary polyclonal antibodies is provided below.

### A. FOXO3A (serine 294).

An 8 amino acid phospho-peptide antigen, WPGs*PTSR (where s*= phosphoserine) that corresponds to the sequence encompassing the serine 294 phosphorylation site in human FOXO3A transcriptional regulator protein (see Row 5 of Table 1; SEQ ID NO: 4), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals to produce (and subsequently screen) phospho-specific FOXO3A (ser 294) polyclonal antibodies as described in Immunization/Screening below.

### B. NFkB-p105 (serine 938).

A 10 amino acid phospho-peptide antigen, TSFRKLs*FTE (where s*= phasphoserine) that corresponds to the sequence encompassing the serine 938 phosphorylation site in human NFkB-p105 transcriptional regulator protein *(see* Row 7 of Table 1 (SEQ ID NO: 6)), plus cysteine on the C-terminal for coupling, is constructed according to standard synthesis techniques using, *e.g.,* a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals to produce (and subsequently screen) phospho-specific NFkB-p105 (ser 938) polyclonal antibodies as described in Immunization/Screening below.

### C. TAF7 (serine 171).

A 10 amino acid phospho-peptide antigen, VKRLLs*TDAE (where s*= phosphoserine) that corresponds to the sequence encompassing the serine 171 phosphorylation site in human TAF7 transcriptional regulator protein (*see* Row 9 of Table 1 (SEQ ID NO: 8), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.:* Merrifield, *supra.* This peptide is then coupled to KLH and used to immunize animals to produce (and subsequently screen) phospho-specific TAF7 (ser 171) antibodies as described in Immunization/Screening below.

### Immunization/Screening.

A synthetic phospho-peptide antigen as described in A-C above is coupled to KLH, and rabbits are injected intradermally (ID) on the back with antigen in complete Freunds adjuvant (500 µg antigen per rabbit). The rabbits are boosted with same antigen in incomplete Freund adjuvant (250 µg antigen per rabbit) every three weeks. After the fifth boost, bleeds are collected. The sera are purified by Protein A-affinity chromatography by standard methods *(see* ANTIBODIES: A LABORATORY MANUAL, Cold Spring Harbor, *supra.).* The eluted immunoglobulins are further loaded onto an unphosphorylated synthetic peptide antigen-resin Knotes column to pull out antibodies that bind the unphosphorylated form of the phosphorylation sites. The flow through fraction is collected and applied onto a phospho-synthetic peptide antigen-resin column to isolate antibodies that bind the phosphorylated form of the phosphorylation sites. After washing the column extensively, the bound antibodies (i.e. antibodies that bind the phosphorylated peptides described in A-C above, but do not bind the unphosphorylated form of the peptides) are eluted and kept in antibody storage buffer.

The isolated antibody is then tested for phospho-specificity using Western blot assay using an appropriate cell line that expresses (or overexpresses) target phospho-protein (i.e. phosphorylated FOXO3A, NFkB-p 105 or TAF7), found in for example, Jurkat cells. Cells are cultured in DMEM or RPMI supplemented with 10% FCS. Cell are collected, washed with PBS and directly lysed in cell lysis buffer. The protein concentration of cell lysates is then measured. The loading buffer is added into cell lysate and the mixture is boiled at 100 °C for 5 minutes. 20 µl (10 µg protein) of sample is then added onto 7.5% SDS-PAGE gel.

A standard Western blot may be performed according to the Immunoblotting Protocol set out in the CELL SIGNALING TECHNOLOGY, INC, 2003-04 Catalogue, p. 390. The isolated phosphorylation site-specific antibody is used at dilution 1:1000. Phospho-specificity of the antibody will be shown by binding of only the phosphorylated form of the target amino acid sequence. Isolated phosphorylation site-specific polyclonal antibody does not (substantially) recognize the same target sequence when not phosphorylated at the specified serine or threonineposition (e.g., the antibody does not bind to FOXO3A in the non-stimulated cells, when serine 294 is not phosphorylated).

In order to confirm the specificity of the isolated antibody, different cell lysates containing various phosphorylated signaling proteins other than the target protein are prepared. The Western blot assay is performed again using these cell lysates. The phosphorylation site-specific polyclonal antibody isolated as described above is used (1:1000 dilution) to test reactivity with the different phosphorylated non-target proteins. The phosphorylation site-specific antibody does not significantly cross-react with other phosphorylated signaling proteins that do not have the described phosphorylation site, although occasionally slight binding to a highly homologous sequence on another protein may be observed. In such case the antibody may be further purified using affinity chromatography, or the specific immunoreactivity cloned by rabbit hybridoma technology.

### EXAMPLE 3

### Production of Phosphorylation site-specific Monoclonal Antibodies

Monoclonal antibodies that specifically bind a novel phosphorylation site of the invention (Table 1) only when the serine or threonineresidue is phosphorylated (and does not bind to the same sequence when the serine or threonineis not phosphorylated) are produced according to standard methods by first constructing a synthetic peptide antigen comprising the phosphorylation site and then immunizing an animal to raise antibodies against the antigen, and harvesting spleen cells from such animals to produce fusion hybridomas, as further described below. Production of exemplary monoclonal antibodies is provided below.

### A. PSD-95 (serine 492).

A 14 amino acid phospho-peptide antigen, TKDCGFLs*QALSFR (where s*= phosphoserine) that corresponds to the sequence encompassing the serine 492 phosphorylation site in human PSD-95 adaptor/scaffold protein (see Row 40 of Table 1 (SEQ ID NO:39)), plus cysteine on the C-terminal for coupling, is constructed according to standard synthesis techniques using, *e.g.,* a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phospho-specific monoclonal PSD-95 (ser 492) antibodies as described in Immunization/Fusion/Screening below.

### B. Rictor (serine 1219).

A 19 amino acid phospho-peptide antigen, KIRSQs*FNTDTTTSGISSM (where s*= phosphoserine) that corresponds to the sequence encompassing the serine 1219 phosphorylation site in human Rictor adaptor/scaffold protein (see Row 42 of Table 1 (SEQ ID NO: 41)), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phospho-specific monoclonal Rictor (ser 1219) antibodies as described in Immunization/Fusion/Screening below.

### C. B-CK (threonine 258).

A 13 amino acid phospho-peptide antigen, FCTGLt*QIETLFK (where t*= phosphothreonine) that corresponds to the sequence encompassing the threonine 258 phosphorylation site in human B-CK Kinase (see Row 71 of Table 1 (SEQ ID NO: 70)), plus cysteine on the C-terminal for coupling, is constructed according to standard synthesis techniques using, e.g., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phospho-specific monoclonal B-CK (thr 258) antibodies as described in Immunization/Fusion/Screening below.

### Immunization/Fusion/Screening.

A synthetic phospho-peptide antigen as described in A-C above is coupled to KLH, and BALB/C mice are injected intradermally (ID) on the back with antigen in complete Freunds adjuvant (e.g., 50 µg antigen per mouse). The mice are boosted with same antigen in incomplete Freund adjuvant (e.g. 25 µg antigen per mouse) every three weeks. After the fifth boost, the animals are sacrificed and spleens are harvested.

Harvested spleen cells are fused to SP2/0 mouse myeloma fusion partner cells according to the standard protocol of Kohler and Milstein (1975). Colonies originating from the fusion are screened by ELISA for reactivity to the phospho-peptide and non-phospho-peptide forms of the antigen and by Western blot analysis (as described in Example 1 above). Colonies found to be positive by ELISA to the phospho-peptide while negative to the non-phospho-peptide are further characterized by Western blot analysis. Colonies found to be positive by Western blot analysis are subcloned by limited dilution. Mouse ascites are produced from a single clone obtained from subcloning, and tested for phospho-specificity (against the PSD-95, Rictor or B-CK) phospho-peptide antigen, as the case may be) on ELISA. Clones identified as positive on Western blot analysis using cell culture supernatant as having phospho-specificity, as indicated by a strong band in the induced lane and a weak band in the uninduced lane of the blot, are isolated and subcloned as clones producing monoclonal antibodies with the desired specificity.

Ascites fluid from isolated clones may be further tested by Western blot analysis. The ascites fluid should produce similar results on Western blot analysis as observed previously with the cell culture supernatant, indicating phospho-specificity against the phosphorylated target.

### EXAMPLE 4

### Production and Use of AQUA Peptides for Detecting and Quantitating Phosphorylation at a Novel Phosphorylation Site

Heavy-isotope labeled peptides (AQUA peptides (internal standards)) for the detecting and quantitating a novel phosphorylation site of the invention (Table 1) only when the serine or threonineresidue is phosphorylated are produced according to the standard AQUA methodology *(see* Gygi *et al.,* Gerber *et al., supra.)* methods by first constructing a synthetic peptide standard corresponding to the phosphorylation site sequence and incorporating a heavy-isotope label. Subsequently, the MSⁿ and LC-SRM signature of the peptide standard is validated, and the AQUA peptide is used to quantify native peptide in a biological sample, such as a digested cell extract. Production and use of exemplary AQUA peptides is provided below.

### A. PIK4Cf1 (serine 204).

An AQUA peptide comprising the sequence, KTSSVSSIs*QVSPER (y*=phosphoserine or threonine; sequence incorporating ¹⁴C/¹⁵N-labcled valine (indicated by bold V), which corresponds to the serine 204 phosphorylation site in human INPP4A phosphatase (see Row 72 in Table 1 (SEQ ID NO: 71)), is constructed according to standard synthesis techniques using, e.g., a Rainin/Protein Technologies, Inc., Symphony peptide synthesizer (*see* Merrifield, *supra.*) as further described below in Synthesis & MS/MS Signature. The PIK4CA (ser 204) AQUA peptide is then spiked into a biological sample to quantify the amount of phosphorylated PIK4CA (ser 204) in the sample, as further described below in Analysis & Quantification.

### B. PIP5K (threonine 244).

An AQUA peptide comprising the sequence TSVSPQANRTYVRTEt*TEDE (t*= phosphothreonine; sequence incorporating ¹⁴C/¹⁵N-labeled proline (indicated by bold P), which corresponds to the serine 334 phosphorylation site in human PIP5K kinase (*see* Row 73 in Table 1 (SEQ ID NO: 72)), is constructed according to standard synthesis techniques using, e.g., a Rainin/Protein Technologies, Inc., Symphony peptide synthesizer (see Merrifield, *supra.*) as further described below in Synthesis & MS/MS Signature. The PIP5K (thr 244) AQUA peptide is then spiked into a biological sample to quantify the amount of phosphorylated PIP5K (thr 244) in the sample, as further described below in Analysis & Quantification.

### C. Pnk1 (threonine 122).

An AQUA peptide comprising the sequence TPESQPDTPPGt*PLVSQDEKR (t*= phosphothreonine; sequence incorporating ¹⁴C/¹⁵N-Iabeled Leucine (indicated by bold L), which corresponds to the serine 388 phosphorylation site in human Pnk1 phosphatase *(see* Row 74 in Table 1 (SEQ ID NO: 73)), is constructed according to standard synthesis techniques using, e.g., a Rainin/Protein Technologies, Inc., Symphony peptide synthesizer (see Merrifield, *supra.*) as further described below in Synthesis & MS/MS Signature. The Pnk1 (thr 122) AQUA peptide is then spiked into a biological sample to quantify the amount of phosphorylated Pnk1 (thr 122) in the sample, as further described below in Analysis & Quantification.

### D. Iamin B2 (threonine 14).

An AQUA peptide comprising the sequence AGG**P**At*PLSPTR (t*= phosphothreonine; sequence incorporating ¹⁴C/¹⁵N-labeled proline (indicated by bold **P**), which corresponds to the threonine 14 phosphorylation site in human lamin B2 cytoskeletal protein *(see* Row 119 in Table 1 (SEQ ID NO: 118)), is constructed according to standard synthesis techniques using, e.g., a Rainin/Protein Technologies, Inc., Symphony peptide synthesizer (see Merrifield, *supra.)* as further described below in Synthesis & MS/MS Signature. The lamin B2 (thr 14) AQUA peptide is then spiked into a biological sample to quantify the amount of phosphorylated lamin B2 (thr 14) in the sample, as further described below in Analysis & Quantification.

### Synthesis & MS/MS Spectra.

Fluorenylmethoxycarbonyl (Fmoc)-derivatized amino acid monomers may be obtained from AnaSpec (San Jose, CA). Fmoc-derivatized stable-isotope monomers containing one ¹⁵N and five to nine ¹³C atoms may be obtained from Cambridge Isotope Laboratories (Andover, MA). Preloaded Wang resins may be obtained from Applied Biosystems. Synthesis scales may vary from 5 to 25 µmol. Amino acids are activated *in situ* with 1-H-benzotriazolium, 1-bis(dimethylamino) methylene]-hexafluorophosphate (1-),3-oxide:1-hydroxybenzotriazole hydrate and coupled at a 5-fold molar excess over peptide. Each coupling cycle is followed by capping with acetic anhydride to avoid accumulation of one-residue deletion peptide by-products. After synthesis peptide-resins are treated with a standard scavenger-containing trifluoroacetic acid (TFA)-water cleavage solution, and the peptides are precipitated by addition to cold ether. Peptides *(i.e.* a desired AQUA peptide described in A-D above) are purified by reversed-phase C18 HPLC using standard TFA/acetonitrile gradients and characterized by matrix-assisted laser desorption ionization-time of flight (Biflex III, Bruker Daltonics, Billerica, MA) and ion-trap (ThermoFinnigan, LCQ DecaXP or LTQ) MS.

MS/MS spectra for each AQUA peptide should exhibit a strong y-type ion peak as the most intense fragment ion that is suitable for use in an SRM monitoring/analysis. Reverse-phase microcapillary columns (0.1 Å∼ 150-220 mm) are prepared according to standard methods. An Agilent 1100 liquid chromatograph may be used to develop and deliver a solvent gradient [0.4% acetic acid/0.005% heptafluorobutyric acid (HFBA)/7% methanol and 0.4% acetic acid/0.005% HFBA/65% methanol/35% acetonitrile] to the microcapillary column by means of a flow splitter. Samples are then directly loaded onto the microcapillary column by using a FAMOS inert capillary autosampler (LC Packings, San Francisco) after the flow split. Peptides are reconstituted in 6% acetic acid/0.01 % TFA before injection.

### Analysis & Quantification.

Target protein (e.g. a phosphorylated proteins of A-D above) in a biological sample is quantified using a validated AQUA peptide (as described above). The IAP method is then applied to the complex mixture of peptides derived from proteolytic cleavage of crude cell extracts to which the AQUA peptides have been spiked in.

LC-SRM of the entire sample is then carried out. MS/MS may be performed by using a ThermoFinnigan (San Jose, CA) mass spectrometer (LCQ DecaXP ion trap or TSQ Quantum triple quadrupole or LTQ). On the DecaXP, parent ions are isolated at 1.6 m/z width, the ion injection time being limited to 150 ms per microscan, with two microscans per peptide averaged, and with an AGC setting of 1 x 10⁸; on the Quantum, Q1 is kept at 0.4 and Q3 at 0.8 m/z with a scan time of 200 ms per peptide. On both instruments, analyte and internal standard are analyzed in alternation within a previously known reverse-phase retention window; well-resolved pairs of internal standard and analyte are analyzed in separate retention segments to improve duty cycle. Data are processed by integrating the appropriate peaks in an extracted ion chromatogram (60.15 m/z from the fragment monitored) for the native and internal standard, followed by calculation of the ratio of peak areas multiplied by the absolute amount of internal standard (e.g., 500 fmol).

## Claims

1. An isolated phosphorylation site-specific antibody that specifically binds a human signaling protein selected from Column A of Table 1 only when phosphorylated at the threonine or serine listed in corresponding Column D of Table 1, comprised within the phosphorylatable peptide sequence listed in corresponding Column E of Table 1 (SEQ ID NOs: 1-44, 46-83, 85-110, 112-125, 127-132, 135-139, 141-148, 150-234, 236-277, 279-309, 311-356), wherein said antibody does not bind said signaling protein when not phosphorylated at said threonine or serine.

2. An isolated phosphorylation site-specific antibody that specifically binds a human signaling protein selected from Column A of Table 1 only when not phosphorylated at the threonine or serine listed in corresponding Column D of Table l, comprised within the phosphorylatable peptide sequence listed in corresponding Column E of Table 1 (SEQ ID NOs: 1-44, 46-83, 85-110, 112-125, 127-132, 135-139, 141-148, 150-234, 236-277, 279-309, 311-356), wherein said antibody does not bind said signaling protein when phosphorylated at said threonine or serine.

3. (New) A method selected from the group consisting of:
(a) a method for detecting a human signaling protein selected from Column A of Table l, wherein said human signaling protein is phosphorylated at the threonine or serine listed in corresponding Column D of Table l, comprised within the phosphorylatable peptide sequence listed in corresponding Column E of Table 1 (SEQ ID NOs: l-44, 46-83, 85-1 It), l 12-125, 127-132, 135-139, 141-148, 150-234, 236-277, 279-309, 311-356), comprising the step of adding an isolated phosphorylation-specific antibody according to claim 1, to a sample comprising said human signaling protein under conditions that permit the binding of said antibody to said human carcinoma-related signaling protein, and detecting bound antibody:
(b) a method for quantifying the amount of a human signaling protein listed in Column A of Table 1 that is phosphorylated at the corresponding serine or threonine listed in Column D of Table 1, comprised within the phosphorylatable peptide sequence listed in corresponding Column E of Table 1 (SEQ ID NOs: 1-44, 46-83, 85-110, 112-125, 127-132, 135-139, 141-148, 150-234, 236-277, 279-309, 311-356), in a sample using a heavy-isotope labeled peptide (AQUA^{™} peptide), said labeled peptide comprising the phosphorylated serine or threonine listed in corresponding Column D of Table 1, comprised within the phosphorylatable peptide sequence listed in corresponding Column E of Table 1 as an internal standard; and
(c) a method comprising step (a) followed by step (b).

4. The method of claim 3, wherein said isolated phosphorylation-specific antibody is capable of specifically binding Bcr only when phosphorylated at 5301, comprised within the phosphorylatable peptide sequence listed in Column E, Row 156, of Table 1 (SEQ ID NO: 155), wherein said antibody does not bind said protein when not phosphorylated at said serine.

5. The method of claim 3, wherein said isolated phosphorylation-specific antibody is capable of specifically binding RALBP1 only when phosphorylated at S29, comprised within the phosphorylatable peptide sequence listed in Column E, Row 138, of Table 1 (SEQ ID NO: 139), wherein said antibody does not bind said protein when not phosphorylated at said serine.

6. The method of claim 3, wherein said isolated phosphorylation-specific antibody is capable of specifically binding IFNGR1 only when phosphorylated at T295, comprised within the phosphorylatable peptide sequence listed in Column E, Row 103, of Table 1 (SEQ ID NO: 102), wherein said antibody does not bind said protein when not phosphorylated at said threonine.

7. An isolated phosphorylation site-specific antibody capable of specifically binding FOXO3A only when phosphorylated at 5294, comprised within the phosphorylatable peptide sequence listed in Column E, Row 5, of Table 1 (SEQ ID NO: 4), wherein said antibody does not bind said protein when not phosphorylated at said serine.

8. An isolated phosphorylation site-specific antibody capable of specifically binding FOXO3A only when not phosphorylated at S294, comprised within the phosphorylatable peptide sequence listed in Column E, Row 5, of Table 1 (SEQ ID NO: 4), wherein said antibody does not bind said protein when phosphorylated at said serine.

9. An isolated phosphorylation site-specific antibody capable of specifically binding lamin A/C only when phosphorylated at S458, comprised within the phosphorylatable peptide sequence listed in Column E, Row 118, of Table 1 (SEQ ID NO: 117), wherein said antibody does not bind said protein when not phosphorylated at said serine.

10. An isolated phosphorylation site-specific antibody capable of specifically binding lamin A/C only when not phosphorylated at S45R, comprised within the phosphorylatable peptide sequence listed in Column E, Row 118, of Table 1 (SEQ ID NO: 117), wherein said antibody does not bind said protein when phosphorylated at said serine.


SEQUENCE LISTING

<110> CELL SIGNALING TECHNOLOGY, INC.

<120> SERINE AND THREONINE PHOSPHORYLATION SITES

<130> 602197PEP

<140> EP 08102942.3
<141> 2008-03-26

<150> 60/920,026
<151> 2007-03-26

<160> 356

<170> PatentIn version 3.3

<210> 1
<211> 31
<212> PRT
<213> Homo sapiens


<220>
<221> MOD_RES
<222> (26)..(26)
<223> Phosphorylated Thr

<400> 1
Thr Val Ala Ile Ser Asp Ala Ala Gln Leu Pro His Asp Tyr Cys Thr
1               5                   10                  15


Thr Pro Gly Gly Thr Leu Phe Ser Thr Thr Pro Gly Gly Thr Arg
            20                  25                  30


<210> 2
<211> 31
<212> PRT
<213> Homo sapiens


<220>
<221> MOD_RES
<222> (17)..(17)
<223> Phosphorylated Thr

<400> 2
Thr Val Ala Ile Ser Asp Ala Ala Gln Leu Pro His Asp Tyr Cys Thr
1               5                   10                  15


Thr Pro Gly Gly Thr Leu Phe Ser Thr Thr Pro Gly Gly Thr Arg
            20                  25                  30


<210> 3
<211> 10
<212> PRT
<213> Homo sapiens


<220>
<221> MOD_RES
<222> (8)..(8)
<223> Phosphorylated Thr

<400> 3
Phe Asn Ala Ser Gln Leu Ile Thr Gln Arg
1               5                   10


<210> 4
<211> 8
<212> PRT

<213> Homo sapiens


<220>
<221> MOD_RES
<222> (4)..(4)
<223> Phosphorylated Ser

<400> 4
Trp Pro Gly Ser Pro Thr Ser Arg
1               5


<210> 5
<211> 21
<212> PRT
<213> Homo sapiens


<220>
<221> MOD_RES
<222> (15)..(15)
<223> Phosphorylated Thr

<400> 5
His Ser Ser Gly Ser Leu Thr Pro Pro Val Thr Pro Pro Ile Thr Pro
1               5                   10                  15


Ser Ser Ser Phe Arg
            20


<210> 6
<211> 10
<212> PRT
<213> Homo sapiens


<220>
<221> MOD_RES
<222> (7)..(7)
<223> Phosphorylated Ser

<400> 6
Thr Ser Phe Arg Lys Leu Ser Phe Thr Glu
1               5                   10


<210> 7
<211> 27
<212> PRT
<213> Homo sapiens


<220>
<221> MOD_RES
<222> (5)..(5)
<223> Phosphorylated Thr

<400> 7
Gln Val Val Gln Thr Pro Asn Thr Val Leu Ser Thr Pro Phe Arg Thr
1               5                   10                  15


Pro Ser Asn Gly Ala Glu Gly Leu Thr Pro Arg
            20                  25


<210> 8
<211> 10
<212> PRT
<213> Homo sapiens


<220>
<221> MOD_RES

<222> (6)..(6)
<223> Phosphorylated Ser

<400> 8
Val Lys Arg Leu Leu Ser Thr Asp Ala Glu
1               5                   10


<210> 9
<211> 22
<212> PRT
<213> Homo sapiens


<220>
<221> MOD_RES
<222> (2)..(2)
<223> Phosphorylated Ser

<400> 9
Gln Ser Gln Gln Pro Met Lys Pro Ile Ser Pro Val Lys Asp Pro Val
1               5                   10                  15


Ser Pro Ala Ser Gln Lys
            20


<210> 10
<211> 13
<212> PRT
<213> Homo sapiens


<220>
<221> MOD_RES
<222> (9)..(9)
<223> Phosphorylated Ser

<400> 10
Leu Met Leu Ser Thr Ser Glu Tyr Ser Gln Ser Pro Lys
1               5                   10


<210> 11
<211> 15
<212> PRT
<213> Homo sapiens


<220>
<221> MOD_RES
<222> (4)..(4)
<223> Phosphorylated Ser

<400> 11
Ser Pro Leu Ser Gln Gly Asp Ser Ser Ala Pro Ser Leu Pro Lys
1               5                   10                  15


<210> 12
<211> 15
<212> PRT
<213> Homo sapiens


<220>
<221> MOD_RES
<222> (9)..(9)
<223> Phosphorylated Thr

<400> 12
Glu Gly Ser Gln Gly Glu Pro Trp Thr Pro Thr Ala Asn Leu Lys
1               5                   10                  15


<210> 13

<211> 18
<212> PRT
<213> Homo sapiens

<220>
<221> MOD_RES
<222> (9)..(9)
<223> Phosphorylated Ser

<400> 13
Ile Ser Lys Leu Met Arg Ser Ala Ser Phe Asn Thr Asp Pro Tyr Val
1               5                   10                  15

Arg Glu

<210> 14
<211> 31
<212> PRT
<213> Homo sapiens

<220>
<221> MOD_RES
<222> (13)..(13)
<223> Phosphorylated Thr

<400> 14
Ala Val Thr Pro Pro Met Pro Leu Leu Thr Pro Ala Thr Pro Gly Gly
1               5                   10                  15

Leu Pro Pro Ala Ala Ala Val Ala Ala Ala Ala Ala Thr Ala Lys
            20                  25                  30

<210> 15
<211> 10
<212> PRT
<213> Homo sapiens

<220>
<221> MOD_RES
<222> (6)..(6)
<223> Phosphorylated Thr

<400> 15
Leu Lys Arg Ile Lys Thr Glu Asp Gly Glu
1               5                   10

<210> 16
<211> 15
<212> PRT
<213> Homo sapiens

<220>
<221> MOD_RES
<222> (8)..(8)
<223> Phosphorylated Ser

<400> 16
Lys Arg Pro Ser Leu Pro Ser Ser Pro Ser Pro Gly Leu Pro Lys
1               5                   10                  15

<210> 17
<211> 10
<212> PRT
<213> Homo sapiens

<220>
<221> MOD_RES
<222> (6)..(6)
<223> Phosphorylated Ser

<400> 17
Phe Trp Arg Leu Val Ser Thr Ile Glu Glu
1               5                   10


<210> 18
<211> 21
<212> PRT
<213> Homo sapiens


<220>
<221> MOD_RES
<222> (11)..(11)
<223> Phosphorylated Thr

<400> 18
His Ser Ser Gly Ser Leu Thr Pro Pro Val Thr Pro Pro Ile Thr Pro
1               5                   10                  15


Ser Ser Ser Phe Arg
            20


<210> 19
<211> 15
<212> PRT
<213> Homo sapiens


<220>
<221> MOD_RES
<222> (3)..(3)
<223> Phosphorylated Thr

<400> 19
Lys Leu Thr Gln Ile Gln Glu Ser Gln Val Thr Ser His Asn Lys
1               5                   10                  15


<210> 20
<211> 32
<212> PRT
<213> Homo sapiens


<220>
<221> MOD_RES
<222> (22)..(22)
<223> Phosphorylated Ser

<400> 20
Asp Ser Ser Gly Gln Glu Asp Glu Thr Gln Ser Ser Asn Asp Asp Pro
1               5                   10                  15


Ser Gln Ser Val Ala Ser Gln Asp Ala Gln Glu Ile Ile Arg Pro Arg
            20                  25                  30


<210> 21
<211> 24
<212> PRT
<213> Homo sapiens


<220>
<221> MOD_RES
<222> (15)..(15)
<223> Phosphorylated Thr

<400> 21
Gly Ser Pro Val Leu Leu Asp Pro Glu Glu Leu Ala Pro Val Thr Pro
1               5                   10                  15

Met Glu Val Tyr Pro Glu Cys Lys
            20

<210> 22
<211> 12
<212> PRT
<213> Homo sapiens

<220>
<221> MOD_RES
<222> (10)..(10)
<223> Phosphorylated Thr

<400> 22
Thr Pro Asp Val Phe Lys Ala Pro Leu Thr Pro Arg
1               5                   10

<210> 23
<211> 21
<212> PRT
<213> Homo sapiens

<220>
<221> MOD_RES
<222> (8)..(8)
<223> Phosphorylated Ser

<400> 23
Lys Pro Ile Glu Arg Val Leu Ser Ser Thr Ser Glu Glu Asp Glu Pro
1               5                   10                  15

Gly Val Val Lys Phe
            20

<210> 24
<211> 31
<212> PRT
<213> Homo sapiens

<220>
<221> MOD_RES
<222> (3)..(3)
<223> Phosphorylated Ser

<400> 24
Ser Leu Ser Gln Gly Thr Gln Leu Pro Ser His Val Thr Pro Thr Thr
1               5                   10                  15

Gly Val Pro Thr Met Ser Leu His Thr Pro Pro Ser Pro Ser Arg
            20                  25                  30

<210> 25
<211> 21
<212> PRT
<213> Homo sapiens

<220>
<221> MOD_RES
<222> (15)..(15)
<223> Phosphorylated Ser

<400> 25

Gly Ala Asp Thr Ala Pro Thr Leu Ala Pro Glu Ala Leu Pro Ser Gln
1               5                   10                  15

Gly Glu Val Glu Arg
            20

<210> 26
<211> 21
<212> PRT
<213> Homo sapiens

<220>
<221> MOD_RES
<222> (16)..(16)
<223> Phosphorylated Thr

<400> 26
Tyr Ser Pro Thr Ser Pro Thr Tyr Ser Pro Thr Ser Pro Val Tyr Thr
1               5                   10                  15

Pro Thr Ser Pro Lys
            20

<210> 27
<211> 11
<212> PRT
<213> Homo sapiens

<220>
<221> MOD_RES
<222> (5)..(5)
<223> Phosphorylated Ser

<400> 27
Gly Arg Leu Leu Ser Phe Leu Glu Gln Ser Glu
1               5                   10

<210> 28
<211> 29
<212> PRT
<213> Homo sapiens

<220>
<221> MOD_RES
<222> (4)..(4)
<223> Phosphorylated Ser

<400> 28
Lys Thr Arg Ser Ser Ser Thr Ser Ser Ser Thr Val Thr Ser Ser Ala
1               5                   10                  15

Gly Ser Glu Gln Gln Asn Gln Ser Ser Ser Gly Ser Glu
            20                  25

<210> 29
<211> 30
<212> PRT
<213> Homo sapiens

<220>
<221> MOD_RES
<222> (11)..(11)
<223> Phosphorylated Thr

<400> 29
Val His Ser Ile Ile Glu Ser Asp Pro Val Thr Pro Pro Ser Asp Pro

1               5                   10                  15

Ser Ile Pro Ile Pro Thr Leu Pro Ser Val Thr Ala Ala Lys
            20                  25                  30

<210> 30
<211> 24
<212> PRT
<213> Homo sapiens

<220>
<221> MOD_RES
<222> (9)..(9)
<223> Phosphorylated Thr

<400> 30
Gln Ile Asn Ala Lys Arg Lys Asp Thr Thr Ser Asp Lys Asp Asp Ser
1               5                   10                  15

Leu Gly Ser Gln Gln Thr Asn Glu
            20

<210> 31
<211> 21
<212> PRT
<213> Homo sapiens

<220>
<221> MOD_RES
<222> (6)..(6)
<223> Phosphorylated Thr

<400> 31
Glu Gln Thr Leu His Thr Pro Val Met Met Gln Thr Pro Gln Leu Thr
1               5                   10                  15

Ser Thr Ile Met Arg
            20

<210> 32
<211> 26
<212> PRT
<213> Homo sapiens

<220>
<221> MOD_RES
<222> (14)..(14)
<223> Phosphorylated Thr

<400> 32
Val Thr Thr His Ile Pro Gln Thr Pro Val Arg Pro Glu Thr Pro Asn
1               5                   10                  15

Arg Thr Pro Ser Ala Thr Leu Ser Glu Lys
            20                  25

<210> 33
<211> 26
<212> PRT
<213> Homo sapiens

<220>
<221> MOD_RES
<222> (8)..(8)
<223> Phosphorylated Thr

<400> 33
Val Thr Thr His Ile Pro Gln Thr Pro Val Arg Pro Glu Thr Pro Asn
1               5                   10                  15

Arg Thr Pro Ser Ala Thr Leu Ser Glu Lys
            20                  25

<210> 34
<211> 26
<212> PRT
<213> Homo sapiens

<220>
<221> MOD_RES
<222> (5)..(5)
<223> Phosphorylated Ser

<400> 34
Ser Arg Gln Leu Ser Ser Thr Ser Pro Leu Ala Pro Tyr Pro Thr Ser
1               5                   10                  15

Gln Met Val Ser Ser Asp Arg Ser Gln Leu
            20                  25

<210> 35
<211> 30
<212> PRT
<213> Homo sapiens

<220>
<221> MOD_RES
<222> (5)..(5)
<223> Phosphorylated Ser

<400> 35
Asp Ser Arg Pro Ser Gln Ala Ala Gly Asp Asn Gln Gly Asp Glu Val
1               5                   10                  15

Lys Glu Gln Thr Phe Ser Gly Gly Thr Ser Gln Asp Thr Lys
            20                  25                  30

<210> 36
<211> 14
<212> PRT
<213> Homo sapiens

<220>
<221> MOD_RES
<222> (10)..(10)
<223> Phosphorylated Ser

<400> 36
Thr Glu Glu Thr Glu Glu Arg Glu Glu Ser Thr Thr Gly Phe
1               5                   10

<210> 37
<211> 14
<212> PRT
<213> Homo sapiens

<220>
<221> MOD_RES
<222> (12)..(12)
<223> Phosphorylated Ser

<400> 37
Asn Leu Glu Thr Ser Ser Ala Phe Gln Ser Ser Ser Gln Lys
1               5                   10

<210> 38
<211> 26
<212> PRT
<213> Homo sapiens

<220>
<221> MOD_RES
<222> (14)..(14)
<223> Phosphorylated Thr

<400> 38
Ser Ala Gly Lys Thr Ala Val Asn Val Pro Arg Gln Pro Thr Val Thr
1               5                   10                  15

Ser Val Cys Ser Glu Thr Ser Gln Glu Leu
            20                  25

<210> 39
<211> 14
<212> PRT
<213> Homo sapiens

<220>
<221> MOD_RES
<222> (8)..(8)
<223> Phosphorylated Ser

<400> 39
Thr Lys Asp Cys Gly Phe Leu Ser Gln Ala Leu Ser Phe Arg
1               5                   10

<210> 40
<211> 22
<212> PRT
<213> Homo sapiens

<220>
<221> MOD_RES
<222> (17)..(17)
<223> Phosphorylated Thr

<400> 40
Leu Asn Gln Ser Gly Thr Ser Val Gly Thr Asp Glu Glu Ser Asp Val
1               5                   10                  15

Thr Gln Glu Glu Glu Arg
            20

<210> 41
<211> 19
<212> PRT
<213> Homo sapiens

<220>
<221> MOD_RES
<222> (6)..(6)
<223> Phosphorylated Ser

<400> 41
Lys Ile Arg Ser Gln Ser Phe Asn Thr Asp Thr Thr Thr Ser Gly Ile
1               5                   10                  15

Ser Ser Met

<210> 42
<211> 34
<212> PRT
<213> Homo sapiens

<220>
<221> MOD_RES
<222> (3)..(3)
<223> Phosphorylated Ser

<400> 42
Leu Ile Ser Asp Phe Glu Asp Thr Asp Gly Glu Gly Gly Ala Tyr Thr
1               5                   10                  15

Asp Asn Glu Leu Asp Glu Pro Ala Glu Glu Pro Leu Val Ser Ser Ile
            20                  25                  30

Thr Arg

<210> 43
<211> 19
<212> PRT
<213> Homo sapiens

<220>
<221> MOD_RES
<222> (9)..(9)
<223> Phosphorylated Ser

<400> 43
Thr Arg Gln Ile Ser Arg His Asn Ser Thr Thr Ser Ser Thr Ser Ser
1               5                   10                  15

Gly Gly Tyr

<210> 44
<211> 10
<212> PRT
<213> Homo sapiens

<220>
<221> MOD_RES
<222> (7)..(7)
<223> Phosphorylated Thr

<400> 44
Val Phe Gln Arg Pro Pro Thr Leu Glu Glu
1               5                   10

<210> 45

<400> 45
000

<210> 46
<211> 15
<212> PRT
<213> Homo sapiens

<220>

<221> MOD_RES
<222> (10)..(10)
<223> Phosphorylated Thr

<400> 46
Val Ile Ser Pro Pro Val Arg Thr Ala Thr Val Ser Ser Pro Leu
1               5                   10                  15


<210> 47
<211> 18
<212> PRT
<213> Homo sapiens


<220>
<221> MOD_RES
<222> (14)..(14)
<223> Phosphorylated Ser

<400> 47
Ala Gly Pro Gly Pro Val Asp Pro Ser Asn Arg Ser Asn Ser Met Leu
1               5                   10                  15


Glu Leu



<210> 48
<211> 10
<212> PRT
<213> Homo sapiens


<220>
<221> MOD_RES
<222> (3)..(3)
<223> Phosphorylated Thr

<400> 48
Ser Ala Thr Gln Met Phe Glu Leu Thr Arg
1               5                   10


<210> 49
<211> 20
<212> PRT
<213> Homo sapiens


<220>
<221> MOD_RES
<222> (9)..(9)
<223> Phosphorylated Thr

<400> 49
Asp Cys Gly Leu Ser Arg Val Gly Thr Asp Lys Thr Gly Val Lys Ser
1               5                   10                  15


Glu Gly Ser Thr
            20


<210> 50
<211> 10
<212> PRT
<213> Homo sapiens


<220>
<221> MOD_RES
<222> (4)..(4)
<223> Phosphorylated Ser

<400> 50

Arg Gln Pro Ser Pro Glu Pro Val Gln Glu
1               5                   10

<210> 51
<211> 9
<212> PRT
<213> Homo sapiens

<220>
<221> MOD_RES
<222> (7)..(7)
<223> Phosphorylated Ser

<400> 51
Ser Leu Glu Glu Glu Glu Ser Gln Leu
1               5

<210> 52
<211> 25
<212> PRT
<213> Homo sapiens

<220>
<221> MOD_RES
<222> (9)..(9)
<223> Phosphorylated Ser

<400> 52
Glu Thr Gln Thr His Glu Asn Met Ser Gln Leu Ser Glu Glu Glu Gln
1               5                   10                  15

Asn Lys Asp Tyr Gln Asp Cys Ser Lys
            20                  25

<210> 53
<211> 31
<212> PRT
<213> Homo sapiens

<220>
<221> MOD_RES
<222> (23)..(23)
<223> Phosphorylated Ser

<400> 53
Glu Val Ala Ala Thr Glu Glu Asp Val Thr Arg Leu Pro Ser Pro Thr
1               5                   10                  15

Ser Pro Phe Ser Ser Leu Ser Gln Asp Gln Ala Ala Thr Ser Lys
            20                  25                  30

<210> 54
<211> 14
<212> PRT
<213> Homo sapiens

<220>
<221> MOD_RES
<222> (8)..(8)
<223> Phosphorylated Ser

<400> 54
His Gln Pro Leu Arg Asn Thr Ser Phe Thr Cys Gln Asn Glu
1               5                   10

<210> 55

<211> 27
<212> PRT
<213> Homo sapiens

<220>
<221> MOD_RES
<222> (26)..(26)
<223> Phosphorylated Thr

<400> 55
Ile Gln Ser Ala Gly Lys Asp Cys Asp Val Gln Gly Leu Glu His Asp
1               5                   10                  15

Met Glu Glu Ile Asn Ala Arg Trp Asn Thr Leu
            20                  25

<210> 56
<211> 14
<212> PRT
<213> Homo sapiens

<220>
<221> MOD_RES
<222> (6)..(6)
<223> Phosphorylated Ser

<400> 56
Arg Gln Arg Leu Ser Ser Leu Asn Leu Thr Pro Asp Pro Glu
1               5                   10

<210> 57
<211> 19
<212> PRT
<213> Homo sapiens

<220>
<221> MOD_RES
<222> (8)..(8)
<223> Phosphorylated Ser

<400> 57
Gly Asp Ile Leu His Val Ile Ser Gln Glu Asp Pro Asn Trp Trp Gln
1               5                   10                  15

Ala Tyr Arg

<210> 58
<211> 17
<212> PRT
<213> Homo sapiens

<220>
<221> MOD_RES
<222> (8)..(8)
<223> Phosphorylated Ser

<400> 58
Asp Ser Ala Ile Pro Val Glu Ser Asp Thr Asp Asp Glu Gly Ala Pro
1               5                   10                  15

Arg

<210> 59
<211> 36

<212> PRT
<213> Homo sapiens

<220>
<221> MOD_RES
<222> (20)..(20)
<223> Phosphorylated Ser

<400> 59
Glu Glu Gln Leu Ile Pro Gln Met Gly Val Thr Ser Ser Gly Leu Asn
1               5                   10                  15

Gln Val Leu Ser Gln Gln Ala Asn Gln Glu Ile Ser Pro Leu Asp Ser
            20                  25                  30

Met Ile Gln Arg
        35

<210> 60
<211> 31
<212> PRT
<213> Homo sapiens

<220>
<221> MOD_RES
<222> (29)..(29)
<223> Phosphorylated Thr

<400> 60
Glu Leu Val Gly Pro Pro Leu Ala Glu Thr Val Phe Thr Pro Lys Thr
1               5                   10                  15

Ser Pro Glu Asn Val Gln Asp Arg Phe Ala Leu Val Thr Pro Lys
            20                  25                  30

<210> 61
<211> 31
<212> PRT
<213> Homo sapiens

<220>
<221> MOD_RES
<222> (13)..(13)
<223> Phosphorylated Thr

<400> 61
Glu Leu Val Gly Pro Pro Leu Ala Glu Thr Val Phe Thr Pro Lys Thr
1               5                   10                  15

Ser Pro Glu Asn Val Gln Asp Arg Phe Ala Leu Val Thr Pro Lys
            20                  25                  30

<210> 62
<211> 28
<212> PRT
<213> Homo sapiens

<220>
<221> MOD_RES
<222> (15)..(15)
<223> Phosphorylated Ser

<400> 62
Thr Arg Ser Pro Asp Val Ile Ser Ser Ala Ser Thr Ala Leu Ser Gln
1               5                   10                  15

Asp Ile Pro Glu Ile Ala Ser Glu Ala Leu Ser Arg
            20                  25

<210> 63
<211> 19
<212> PRT
<213> Homo sapiens

<220>
<221> MOD_RES
<222> (12)..(12)
<223> Phosphorylated Ser

<400> 63
Glu Leu Ser Gly Glu Ser Asp Glu Leu Gly Ile Ser Gln Asp Glu Gln
1               5                   10                  15

Leu Ser Lys

<210> 64
<211> 21
<212> PRT
<213> Homo sapiens

<220>
<221> MOD_RES
<222> (16)..(16)
<223> Phosphorylated Ser

<400> 64
Ser Glu Gln Glu Glu His Glu Leu Pro Val Leu Ser Arg Gln Ala Ser
1               5                   10                  15

Thr Gly Ser Glu Leu
            20

<210> 65
<211> 36
<212> PRT
<213> Homo sapiens

<220>
<221> MOD_RES
<222> (8)..(8)
<223> Phosphorylated Ser

<400> 65
Ile Asn Leu Thr Arg Gly Pro Ser Gly Leu Gly Phe Asn Ile Val Gly
1               5                   10                  15

Gly Thr Asp Gln Gln Tyr Val Ser Asn Asp Ser Gly Ile Tyr Val Ser
            20                  25                  30

Arg Ile Lys Glu
        35

<210> 66
<211> 16
<212> PRT
<213> Homo sapiens

<220>
<221> MOD_RES

<222> (11)..(11)
<223> Phosphorylated Thr

<400> 66
Phe Ser Val Pro Thr Pro Arg Thr Pro Arg Thr Pro Arg Thr Pro Arg
1               5                   10                  15


<210> 67
<211> 16
<212> PRT
<213> Homo sapiens


<220>
<221> MOD_RES
<222> (14)..(14)
<223> Phosphorylated Thr

<400> 67
Phe Ser Val Pro Thr Pro Arg Thr Pro Arg Thr Pro Arg Thr Pro Arg
1               5                   10                  15


<210> 68
<211> 10
<212> PRT
<213> Homo sapiens


<220>
<221> MOD_RES
<222> (6)..(6)
<223> Phosphorylated Ser

<400> 68
Val Leu Arg Leu Gln Ser Glu Thr Ser Val
1               5                   10


<210> 69
<211> 20
<212> PRT
<213> Homo sapiens


<220>
<221> MOD_RES
<222> (14)..(14)
<223> Phosphorylated Ser

<400> 69
Gln Pro Tyr Pro Ser Arg Pro Pro Phe Asp Asn Gln His Ser Gln Asp
1               5                   10                  15


Leu Asp Ser Arg
            20


<210> 70
<211> 13
<212> PRT
<213> Homo sapiens


<220>
<221> MOD_RES
<222> (6)..(6)
<223> Phosphorylated Thr

<400> 70
Phe Cys Thr Gly Leu Thr Gln Ile Glu Thr Leu Phe Lys
1               5                   10


<210> 71

<211> 15
<212> PRT
<213> Homo sapiens

<220>
<221> MOD_RES
<222> (9)..(9)
<223> Phosphorylated Ser

<400> 71
Lys Thr Ser Ser Val Ser Ser Ile Ser Gln Val Ser Pro Glu Arg
1               5                   10                  15

<210> 72
<211> 20
<212> PRT
<213> Homo sapiens

<220>
<221> MOD_RES
<222> (16)..(16)
<223> Phosphorylated Thr

<400> 72
Thr Ser Val Ser Pro Gln Ala Asn Arg Thr Tyr Val Arg Thr Glu Thr
1               5                   10                  15

Thr Glu Asp Glu
            20

<210> 73
<211> 21
<212> PRT
<213> Homo sapiens

<220>
<221> MOD_RES
<222> (12)..(12)
<223> Phosphorylated Thr

<400> 73
Thr Pro Glu Ser Gln Pro Asp Thr Pro Pro Gly Thr Pro Leu Val Ser
1               5                   10                  15

Gln Asp Glu Lys Arg
            20

<210> 74
<211> 24
<212> PRT
<213> Homo sapiens

<220>
<221> MOD_RES
<222> (8)..(8)
<223> Phosphorylated Thr

<400> 74
Tyr Ile Leu Gly Asn Pro Leu Thr Pro Gly Val Thr Gln Gly Pro Gln
1               5                   10                  15

Ile Asp Lys Glu Gln Tyr Asp Lys
            20

<210> 75
<211> 11

<212> PRT
<213> Homo sapiens


<220>
<221> MOD_RES
<222> (8)..(8)
<223> Phosphorylated Ser

<400> 75
Lys Lys Gln Leu Lys Arg Phe Ser Thr Glu Glu
1               5                   10


<210> 76
<211> 23
<212> PRT
<213> Homo sapiens


<220>
<221> MOD_RES
<222> (10)..(10)
<223> Phosphorylated Thr

<400> 76
Lys Val Ala Glu Pro Glu Leu Met Gly Thr Pro Asp Gly Thr Cys Tyr
1               5                   10                  15


Pro Pro Pro Pro Val Pro Arg
            20


<210> 77
<211> 12
<212> PRT
<213> Homo sapiens


<220>
<221> MOD_RES
<222> (9)..(9)
<223> Phosphorylated Thr

<400> 77
Asn Gly Ile Asp Ile Leu Val Gly Thr Pro Gly Arg
1               5                   10


<210> 78
<211> 14
<212> PRT
<213> Homo sapiens


<220>
<221> MOD_RES
<222> (12)..(12)
<223> Phosphorylated Ser

<400> 78
Leu Gln Glu Asp Leu Gln Ser Val Ser Ser Gly Ser Gln Arg
1               5                   10


<210> 79
<211> 18
<212> PRT
<213> Homo sapiens


<220>
<221> MOD_RES
<222> (5)..(5)
<223> Phosphorylated Ser

<400> 79
Gln Arg Leu Pro Ser Ser Ser Ser Glu Met Gly Ser Gln Asp Gly Ser
1               5                   10                  15

Pro Leu

<210> 80
<211> 17
<212> PRT
<213> Homo sapiens

<220>
<221> MOD_RES
<222> (10)..(10)
<223> Phosphorylated Ser

<400> 80
Leu Pro Ser Ser Ser Ser Glu Met Gly Ser Gln Asp Gly Ser Pro Leu
1               5                   10                  15

Arg

<210> 81
<211> 28
<212> PRT
<213> Homo sapiens

<220>
<221> MOD_RES
<222> (11)..(11)
<223> Phosphorylated Thr

<400> 81
Thr Lys Thr Gly Glu Leu Pro Arg Gln Pro Thr Thr His Pro Glu Glu
1               5                   10                  15

Thr Glu Glu Glu Leu Thr Glu Gln Gln Glu Val Leu
            20                  25

<210> 82
<211> 18
<212> PRT
<213> Homo sapiens

<220>
<221> MOD_RES
<222> (11)..(11)
<223> Phosphorylated Ser

<400> 82
Ala Ser Leu Ser Leu Glu Thr Ser Glu Ser Ser Gln Glu Ala Lys Ser
1               5                   10                  15

Pro Lys

<210> 83
<211> 15
<212> PRT
<213> Homo sapiens

<220>
<221> MOD_RES

<222> (5)..(5)
<223> Phosphorylated Thr

<400> 83
Gln Arg Ala Ser Thr Ile Asp Val Ala Ala Asp Met Leu Gly Leu
1               5                   10                  15


<210> 84

<400> 84
000


<210> 85
<211> 22
<212> PRT
<213> Homo sapiens


<220>
<221> MOD_RES
<222> (18)..(18)
<223> Phosphorylated Thr

<400> 85
Leu Leu Pro Ile Asp Gly Ala Asn Asp Leu Phe Phe Gln Pro Pro Pro
1               5                   10                  15


Leu Thr Pro Thr Ser Lys
            20


<210> 86
<211> 12
<212> PRT
<213> Homo sapiens


<220>
<221> MOD_RES
<222> (7)..(7)
<223> Phosphorylated Thr

<400> 86
Cys Leu Gly Pro Pro Thr Thr Pro Gly Pro Tyr Arg
1               5                   10


<210> 87
<211> 25
<212> PRT
<213> Homo sapiens


<220>
<221> MOD_RES
<222> (9)..(9)
<223> Phosphorylated Ser

<400> 87
Gln Thr Ser Cys Gln Asp Gly Leu Ser Gln Asp Leu Asn Glu Ala Val
1               5                   10                  15


Pro Glu Thr Glu Arg Leu Asp Ser Lys
            20                  25


<210> 88
<211> 10
<212> PRT
<213> Homo sapiens


<220>

<221> MOD_RES
<222> (2)..(2)
<223> Phosphorylated Thr

<400> 88
Ile Thr Pro Val Ile Gly Gln Gly Gly Lys
1               5                   10


<210> 89
<211> 9
<212> PRT
<213> Homo sapiens


<220>
<221> MOD_RES
<222> (6)..(6)
<223> Phosphorylated Ser

<400> 89
Leu Gln Arg Asn Pro Ser Thr Val Glu
1               5


<210> 90
<211> 21
<212> PRT
<213> Homo sapiens


<220>
<221> MOD_RES
<222> (1)..(1)
<223> Phosphorylated Thr

<400> 90
Thr Pro Glu Ser Gln Pro Asp Thr Pro Pro Gly Thr Pro Leu Val Ser
1               5                   10                  15


Gln Asp Glu Lys Arg
            20


<210> 91
<211> 17
<212> PRT
<213> Homo sapiens


<220>
<221> MOD_RES
<222> (12)..(12)
<223> Phosphorylated Ser

<400> 91
Gly Ala Pro Gly Ala Gly Leu Gln Arg Ser Ala Ser Ser Ser Trp Leu
1               5                   10                  15


Glu



<210> 92
<211> 36
<212> PRT
<213> Homo sapiens


<220>
<221> MOD_RES
<222> (4)..(4)
<223> Phosphorylated Thr

<400> 92

Arg Gln Gln Thr Asp Ser Ser Leu Gln Gln Pro Val Asp Asp Pro Ala
1               5                   10                  15

Gly Pro Gly Asp Phe Leu Pro Glu Thr Pro Asp Gly Thr Pro Glu Ser
            20                  25                  30

Gln Leu Pro Phe
        35

<210> 93
<211> 35
<212> PRT
<213> Homo sapiens

<220>
<221> MOD_RES
<222> (12)..(12)
<223> Phosphorylated Thr

<400> 93
His Asn Thr His Pro Thr Ile Glu Asp Ile Ser Thr Gln Glu Ser Asn
1               5                   10                  15

Ile Leu Gly Ala Phe Cys Asp Met Asn Asp Val Glu Val Pro Leu His
            20                  25                  30

Leu Leu Arg
        35

<210> 94
<211> 22
<212> PRT
<213> Homo sapiens

<220>
<221> MOD_RES
<222> (5)..(5)
<223> Phosphorylated Ser

<400> 94
Ala Gly Val Leu Ser Gln Ala Asp Tyr Leu Asn Leu Val Gln Cys Glu
1               5                   10                  15

Thr Leu Glu Asp Leu Lys
            20

<210> 95
<211> 33
<212> PRT
<213> Homo sapiens

<220>
<221> MOD_RES
<222> (22)..(22)
<223> Phosphorylated Ser

<400> 95
His Leu Asp Ser Ser Ala Ala Ala Ile Ile Asp Ala Pro Met Asp Ile
1               5                   10                  15

Pro Gly Leu Asn Leu Ser Gln Gln Glu Tyr Tyr Pro Tyr Val Tyr Tyr
            20                  25                  30

Lys

<210> 96
<211> 17
<212> PRT
<213> Homo sapiens

<220>
<221> MOD_RES
<222> (9)..(9)
<223> Phosphorylated Thr

<400> 96
Asp Met Ser His Ser Arg Cys Asn Thr Leu Ser Ser Pro Asn Gln Tyr
1               5                   10                  15

Gln

<210> 97
<211> 33
<212> PRT
<213> Homo sapiens

<220>
<221> MOD_RES
<222> (16)..(16)
<223> Phosphorylated Thr

<400> 97
Pro Gly Ala Thr Gly Thr Gly Asp Val Ile Ala Ala Ser Pro Glu Thr
1               5                   10                  15

Pro Val Pro Ser Pro Ser Pro Glu Pro Pro Leu Ser Leu Ala Pro Gly
            20                  25                  30

Lys

<210> 98
<211> 23
<212> PRT
<213> Homo sapiens

<220>
<221> MOD_RES
<222> (9)..(9)
<223> Phosphorylated Ser

<400> 98
Phe Gly Glu Arg Arg Arg Asn Asp Ser Tyr Thr Glu Glu Glu Pro Glu
1               5                   10                  15

Trp Phe Ser Ala Gly Pro Thr
            20

<210> 99
<211> 18
<212> PRT
<213> Homo sapiens

<220>
<221> MOD_RES
<222> (11)..(11)
<223> Phosphorylated Ser

<400> 99
Leu Arg Glu Gln Asp Cys Gly Glu Pro Ala Ser Pro Ala Ala Ser Ile
1               5                   10                  15

Ser Arg

<210> 100
<211> 20
<212> PRT
<213> Homo sapiens

<220>
<221> MOD_RES
<222> (15)..(15)
<223> Phosphorylated Thr

<400> 100
Ser Pro Asp Ala Ser Ser Ala Phe Ser Pro Ala Ser Pro Ala Thr Pro
1               5                   10                  15

Asn Gly Thr Lys
            20

<210> 101
<211> 20
<212> PRT
<213> Homo sapiens

<220>
<221> MOD_RES
<222> (9)..(9)
<223> Phosphorylated Thr

<400> 101
Ala Gln Leu Ile Lys Pro Tyr Met Thr Gln Glu Gly Glu Tyr Leu Pro
1               5                   10                  15

Leu Asp Gln Arg
            20

<210> 102
<211> 19
<212> PRT
<213> Homo sapiens

<220>
<221> MOD_RES
<222> (17)..(17)
<223> Phosphorylated Thr

<400> 102
Lys Ser Ile Ile Leu Pro Lys Ser Leu Ile Ser Val Val Arg Ser Ala
1               5                   10                  15

Thr Leu Glu

<210> 103
<211> 11
<212> PRT
<213> Homo sapiens

<220>

<221> MOD_RES
<222> (5)..(5)
<223> Phosphorylated Thr

<400> 103
Leu Arg His Phe Thr Val Val Gly Asp Asp Tyr
1               5                   10


<210> 104
<211> 14
<212> PRT
<213> Homo sapiens


<220>
<221> MOD_RES
<222> (4)..(4)
<223> Phosphorylated Ser

<400> 104
Arg Arg Met Ser Ser Leu Val Gly Pro Thr Gln Ser Phe Phe
1               5                   10


<210> 105
<211> 14
<212> PRT
<213> Homo sapiens


<220>
<221> MOD_RES
<222> (5)..(5)
<223> Phosphorylated Ser

<400> 105
Arg Arg Met Ser Ser Leu Val Gly Pro Thr Gln Ser Phe Phe
1               5                   10


<210> 106
<211> 11
<212> PRT
<213> Homo sapiens


<220>
<221> MOD_RES
<222> (8)..(8)
<223> Phosphorylated Ser

<400> 106
Ser Gly Ile Gly Arg Lys Val Ser Ser Leu Phe
1               5                   10


<210> 107
<211> 12
<212> PRT
<213> Homo sapiens


<220>
<221> MOD_RES
<222> (6)..(6)
<223> Phosphorylated Thr

<400> 107
Gly Asp His Pro Leu Thr Pro Gly Ser His Tyr Ala
1               5                   10


<210> 108
<211> 15
<212> PRT
<213> Homo sapiens

<220>
<221> MOD_RES
<222> (12)..(12)
<223> Phosphorylated Thr

<400> 108
Asp Ala Lys Pro Pro Thr Gly Pro Ser Pro Ala Thr Pro Ala Arg
1               5                   10                  15


<210> 109
<211> 35
<212> PRT
<213> Homo sapiens


<220>
<221> MOD_RES
<222> (3)..(3)
<223> Phosphorylated Ser

<400> 109
Ala Tyr Ser Gln Glu Glu Ile Thr Gln Gly Phe Glu Glu Thr Gly Asp
1               5                   10                  15


Thr Leu Tyr Ala Pro Tyr Ser Thr His Phe Gln Leu Gln Asn Gln Pro
            20                  25                  30


Pro Gln Lys
        35


<210> 110
<211> 30
<212> PRT
<213> Homo sapiens


<220>
<221> MOD_RES
<222> (14)..(14)
<223> Phosphorylated Thr

<400> 110
Val Ala Asp Asp Thr Ala Glu Gly Leu Ser Ala Pro His Thr Pro Val
1               5                   10                  15


Thr Pro Gly Ala Ala Ser Leu Cys Ser Phe Ser Ser Ser Arg
            20                  25                  30


<210> 111

<400> 111
000


<210> 112
<211> 25
<212> PRT
<213> Homo sapiens


<220>
<221> MOD_RES
<222> (23)..(23)
<223> Phosphorylated Thr

<400> 112
Val Gly Gly Ser Gly Gly Asp Lys Gly Ser Gly Phe Pro Asp Gly Glu
1               5                   10                  15

Gly Ser Ser Arg Arg Pro Thr Leu Thr
            20                  25

<210> 113
<211> 12
<212> PRT
<213> Homo sapiens

<220>
<221> MOD_RES
<222> (6)..(6)
<223> Phosphorylated Ser

<400> 113
Arg Glu Arg Leu Pro Ser Val Asp Leu Lys Glu Glu
1               5                   10

<210> 114
<211> 13
<212> PRT
<213> Homo sapiens

<220>
<221> MOD_RES
<222> (6)..(6)
<223> Phosphorylated Ser

<400> 114
Leu Gln Arg Glu Ser Ser Thr Asp Phe Lys Gln Phe Glu
1               5                   10

<210> 115
<211> 33
<212> PRT
<213> Homo sapiens

<220>
<221> MOD_RES
<222> (4)..(4)
<223> Phosphorylated Thr

<400> 115
Arg Ser Arg Thr Val Ala Gln Gln His Asp Glu Asp Gly Ile Glu Glu
1               5                   10                  15

Glu Asp Asp Asp Asp Asp Glu Ile Asp Asp Asp Asp Thr Ile Ser Asp
            20                  25                  30

Trp

<210> 116
<211> 27
<212> PRT
<213> Homo sapiens

<220>
<221> MOD_RES
<222> (13)..(13)
<223> Phosphorylated Ser

<400> 116
Glu Gly Thr Gln Ala Ser Glu Gly Tyr Phe Ser Gln Ser Gln Glu Glu
1               5                   10                  15

Glu Phe Ala Gln Ser Glu Glu Leu Cys Ala Lys
            20                  25

<210> 117
<211> 16
<212> PRT
<213> Homo sapiens

<220>
<221> MOD_RES
<222> (7)..(7)
<223> Phosphorylated Ser

<400> 117
Val Arg Leu Arg Asn Lys Ser Asn Glu Asp Gln Ser Met Gly Asn Trp
1               5                   10                  15

<210> 118
<211> 12
<212> PRT
<213> Homo sapiens

<220>
<221> MOD_RES
<222> (6)..(6)
<223> Phosphorylated Thr

<400> 118
Ala Gly Gly Pro Ala Thr Pro Leu Ser Pro Thr Arg
1               5                   10

<210> 119
<211> 17
<212> PRT
<213> Homo sapiens

<220>
<221> MOD_RES
<222> (6)..(6)
<223> Phosphorylated Thr

<400> 119
Asp Thr Gly Ala Ala Thr Gln Leu Lys Met Lys Pro Arg Lys Val His
1               5                   10                  15

Lys

<210> 120
<211> 19
<212> PRT
<213> Homo sapiens

<220>
<221> MOD_RES
<222> (13)..(13)
<223> Phosphorylated Thr

<400> 120
Ser Cys Glu Gly Gln Asn Pro Glu Leu Leu Pro Lys Thr Pro Ile Ser
1               5                   10                  15

Pro Leu Lys

<210> 121

<211> 14
<212> PRT
<213> Homo sapiens

<220>
<221> MOD_RES
<222> (7)..(7)
<223> Phosphorylated Ser

<400> 121
Ala Leu Cys Arg Gln Leu Ser Glu Ser Glu Ser Ser Leu Glu
1               5                   10

<210> 122
<211> 14
<212> PRT
<213> Homo sapiens

<220>
<221> MOD_RES
<222> (12)..(12)
<223> Phosphorylated Ser

<400> 122
Ala Leu Cys Arg Gln Leu Ser Glu Ser Glu Ser Ser Leu Glu
1               5                   10

<210> 123
<211> 25
<212> PRT
<213> Homo sapiens

<220>
<221> MOD_RES
<222> (19)..(19)
<223> Phosphorylated Ser

<400> 123
His Cys Leu Tyr Val Val Gly Gly His Thr Ala Ala Thr Gly Cys Leu
1               5                   10                  15

Pro Ala Ser Pro Ser Val Ser Leu Lys
            20                  25

<210> 124
<211> 32
<212> PRT
<213> Homo sapiens

<220>
<221> MOD_RES
<222> (19)..(19)
<223> Phosphorylated Ser

<400> 124
Glu Val Ala Glu Asn Gln Gln Asn Gln Ser Ser Asp Pro Glu Glu Glu
1               5                   10                  15

Lys Gly Ser Gln Pro Pro Pro Ala Ala Glu Ser Gln Ser Ser Leu Arg
            20                  25                  30

<210> 125
<211> 35
<212> PRT
<213> Homo sapiens

<220>
<221> MOD_RES
<222> (23)..(23)
<223> Phosphorylated Ser

<400> 125
Ser Leu Asn Trp Ser Ser Phe Val Asp Asn Thr Phe Ala Glu Glu Phe
1               5                   10                  15

Thr Thr Gln Asn Gln Lys Ser Gln Asp Val Glu Leu Trp Glu Gly Glu
            20                  25                  30

Val Val Lys
        35

<210> 126

<400> 126
000

<210> 127
<211> 29
<212> PRT
<213> Homo sapiens

<220>
<221> MOD_RES
<222> (24)..(24)
<223> Phosphorylated Ser

<400> 127
Tyr Tyr Val Asn Thr Thr Thr Asn Glu Thr Thr Trp Glu Arg Pro Ser
1               5                   10                  15

Ser Ser Pro Gly Ile Pro Ala Ser Pro Gly Ser His Arg
            20                  25

<210> 128
<211> 29
<212> PRT
<213> Homo sapiens

<220>
<221> MOD_RES
<222> (3)..(3)
<223> Phosphorylated Ser

<400> 128
Gln Leu Ser Gln Gln Leu Asn Gly Leu Val Cys Glu Ser Ala Thr Cys
1               5                   10                  15

Val Asn Gly Glu Gly Pro Ala Ser Ser Ala Asn Leu Lys
            20                  25

<210> 129
<211> 19
<212> PRT
<213> Homo sapiens

<220>
<221> MOD_RES
<222> (16)..(16)
<223> Phosphorylated Thr

<400> 129

Ala Ala Glu Ala Gly Gly Ala Glu Glu Gln Tyr Gly Phe Leu Thr Thr
1               5                   10                  15

Pro Thr Lys

<210> 130
<211> 29
<212> PRT
<213> Homo sapiens

<220>
<221> MOD_RES
<222> (19)..(19)
<223> Phosphorylated Ser

<400> 130
Ser Tyr Ser Ser Ser Ser Gly Ser Leu Met Pro Ser Leu Glu Asn Leu
1               5                   10                  15

Asp Leu Ser Gln Val Ala Ala Ile Ser Asn Asp Leu Lys
            20                  25

<210> 131
<211> 13
<212> PRT
<213> Homo sapiens

<220>
<221> MOD_RES
<222> (8)..(8)
<223> Phosphorylated Thr

<400> 131
Gly Pro Ser Ser Ser Leu Asn Thr Pro Gly Ser Phe Arg
1               5                   10

<210> 132
<211> 14
<212> PRT
<213> Homo sapiens

<220>
<221> MOD_RES
<222> (3)..(3)
<223> Phosphorylated Ser

<400> 132
Arg Leu Ser Gln Arg Pro Thr Thr Glu Glu Leu Glu Gln Arg
1               5                   10

<210> 133

<400> 133
000

<210> 134

<400> 134
000

<210> 135
<211> 19
<212> PRT
<213> Homo sapiens

<220>
<221> MOD_RES
<222> (3)..(3)
<223> Phosphorylated Ser

<400> 135
Leu Val Ser Gln Asp Asn Phe Gly Leu Glu Leu Ala Ala Val Glu Ala
1               5                   10                  15

Ala Val Arg

<210> 136
<211> 11
<212> PRT
<213> Homo sapiens

<220>
<221> MOD_RES
<222> (3)..(3)
<223> Phosphorylated Thr

<400> 136
Phe Arg Thr Gln Pro Ile Thr Ser Ala Glu Arg
1               5                   10

<210> 137
<211> 26
<212> PRT
<213> Homo sapiens

<220>
<221> MOD_RES
<222> (15)..(15)
<223> Phosphorylated Thr

<400> 137
Asp Ala Pro Cys Ser Ala Ser Ser Glu Leu Ser Gly Pro Ser Thr Pro
1               5                   10                  15

Leu His Thr Ser Ser Pro Val Gln Gly Lys
            20                  25

<210> 138
<211> 26
<212> PRT
<213> Homo sapiens

<220>
<221> MOD_RES
<222> (11)..(11)
<223> Phosphorylated Ser

<400> 138
Ala Leu Leu Asp Val Leu Gln Arg Pro Leu Ser Pro Gly Asn Ser Glu
1               5                   10                  15

Ser Leu Thr Ala Thr Ala Asn Tyr Ser Lys
            20                  25

<210> 139
<211> 13
<212> PRT
<213> Homo sapiens

<220>
<221> MOD_RES
<222> (10)..(10)
<223> Phosphorylated Ser

<400> 139
His Gly Ser Gly Leu Thr Arg Thr Pro Ser Ser Glu Glu
1               5                   10


<210> 140

<400> 140
000


<210> 141
<211> 11
<212> PRT
<213> Homo sapiens


<220>
<221> MOD_RES
<222> (7)..(7)
<223> Phosphorylated Ser

<400> 141
Lys Ile Gln Arg Ser Ser Ser Thr Glu Leu Leu
1               5                   10


<210> 142
<211> 30
<212> PRT
<213> Homo sapiens


<220>
<221> MOD_RES
<222> (7)..(7)
<223> Phosphorylated Ser

<400> 142
Ser Ala Pro Arg Arg Tyr Ser Ser Ser Leu Ser Pro Ile Gln Ala Pro
1               5                   10                  15


Asn His Pro Pro Pro Gln Pro Pro Thr Gln Ala Thr Pro Leu
            20                  25                  30


<210> 143
<211> 30
<212> PRT
<213> Homo sapiens


<220>
<221> MOD_RES
<222> (8)..(8)
<223> Phosphorylated Ser

<400> 143
Ser Ala Pro Arg Arg Tyr Ser Ser Ser Leu Ser Pro Ile Gln Ala Pro
1               5                   10                  15


Asn His Pro Pro Pro Gln Pro Pro Thr Gln Ala Thr Pro Leu
            20                  25                  30


<210> 144
<211> 8
<212> PRT
<213> Homo sapiens

<220>
<221> MOD_RES
<222> (3)..(3)
<223> Phosphorylated Ser

<400> 144
Phe Leu Ser Gln Leu Leu Glu Arg
1               5


<210> 145
<211> 20
<212> PRT
<213> Homo sapiens


<220>
<221> MOD_RES
<222> (9)..(9)
<223> Phosphorylated Ser

<400> 145
Lys Ala Cys Ala Asp Ala Thr Leu Ser Gln Ile Thr Asn Asn Ile Asp
1               5                   10                  15


Pro Val Gly Arg
            20


<210> 146
<211> 20
<212> PRT
<213> Homo sapiens


<220>
<221> MOD_RES
<222> (9)..(9)
<223> Phosphorylated Thr

<400> 146
Lys Ala Cys Gly Asp Ser Thr Leu Thr Gln Ile Thr Ala Gly Leu Asp
1               5                   10                  15


Pro Val Gly Arg
            20


<210> 147
<211> 11
<212> PRT
<213> Homo sapiens


<220>
<221> MOD_RES
<222> (5)..(5)
<223> Phosphorylated Ser

<400> 147
His Leu Gln Ile Ser Gln Glu Ile Gly Asp Arg
1               5                   10


<210> 148
<211> 11
<212> PRT
<213> Homo sapiens


<220>
<221> MOD_RES
<222> (6)..(6)
<223> Phosphorylated Ser

<400> 148
Asp Met Glu Val Leu Ser Gln Glu Ile Val Arg
1               5                   10


<210> 149

<400> 149
000


<210> 150
<211> 19
<212> PRT
<213> Homo sapiens


<220>
<221> MOD_RES
<222> (11)..(11)
<223> Phosphorylated Ser

<400> 150
Asn Phe Asp Val Asp Gly Asp Gly His Ile Ser Gln Glu Glu Phe Gln
1               5                   10                  15


Ile Ile Arg



<210> 151
<211> 16
<212> PRT
<213> Homo sapiens


<220>
<221> MOD_RES
<222> (5)..(5)
<223> Phosphorylated Thr

<400> 151
Asp Arg Arg Ser Thr Glu Pro Ser Val Thr Pro Asp Leu Leu Asn Phe
1               5                   10                  15


<210> 152
<211> 10
<212> PRT
<213> Homo sapiens


<220>
<221> MOD_RES
<222> (5)..(5)
<223> Phosphorylated Thr

<400> 152
Gly Arg Ser Lys Thr Phe Asp Trp Ala Glu
1               5                   10


<210> 153
<211> 21
<212> PRT
<213> Homo sapiens


<220>
<221> MOD_RES
<222> (8)..(8)
<223> Phosphorylated Ser

<400> 153
Pro Ala Pro Ala Gly Arg Leu Ser Gln Gly Ser Gly Ser Ser Ile Thr

1                   5                   10                  15

Ala Ala Gly Met Arg
            20

<210> 154
<211> 11
<212> PRT
<213> Homo sapiens

<220>
<221> MOD_RES
<222> (4)..(4)
<223> Phosphorylated Ser

<400> 154
Arg Leu Leu Ser Gln Glu Glu Gln Thr Ser Lys
1               5                   10

<210> 155
<211> 15
<212> PRT
<213> Homo sapiens

<220>
<221> MOD_RES
<222> (10)..(10)
<223> Phosphorylated Ser

<400> 155
Gly Lys Gly Pro Leu Leu Arg Ser Gln Ser Thr Ser Glu Gln Glu
1               5                   10                  15

<210> 156
<211> 24
<212> PRT
<213> Homo sapiens

<220>
<221> MOD_RES
<222> (12)..(12)
<223> Phosphorylated Thr

<400> 156
Leu Leu Gln Glu Thr Glu Leu Val Glu Pro Leu Thr Pro Ser Gly Ala
1               5                   10                  15

Met Pro Asn Gln Ala Gln Met Arg
            20

<210> 157
<211> 11
<212> PRT
<213> Homo sapiens

<220>
<221> MOD_RES
<222> (8)..(8)
<223> Phosphorylated Thr

<400> 157
Lys Thr Leu Gln Arg Ala Pro Thr Ile Ser Glu
1               5                   10

<210> 158
<211> 34

<212> PRT
<213> Homo sapiens


<220>
<221> MOD_RES
<222> (15)..(15)
<223> Phosphorylated Thr

<400> 158
Ser Leu Asp Pro Met Val Tyr Met Asn Asp Lys Ser Pro Leu Thr Pro
1               5                   10                  15


Glu Lys Glu Val Gly Tyr Leu Glu Phe Thr Gly Pro Pro Gln Lys Pro
            20                  25                  30


Pro Arg



<210> 159
<211> 12
<212> PRT
<213> Homo sapiens


<220>
<221> MOD_RES
<222> (2)..(2)
<223> Phosphorylated Thr

<400> 159
Ile Thr Gln Tyr Leu Asp Ala Gly Gly Ile Pro Arg
1               5                   10


<210> 160
<211> 16
<212> PRT
<213> Homo sapiens


<220>
<221> MOD_RES
<222> (9)..(9)
<223> Phosphorylated Thr

<400> 160
Asn Ser Glu Glu Ser Arg Pro Tyr Thr Asn Lys Val Ile Thr Leu Trp
1               5                   10                  15


<210> 161
<211> 12
<212> PRT
<213> Homo sapiens


<220>
<221> MOD_RES
<222> (8)..(8)
<223> Phosphorylated Ser

<400> 161
Tyr Ala Leu Lys Arg Leu Leu Ser Asn Glu Glu Glu
1               5                   10


<210> 162
<211> 27
<212> PRT
<213> Homo sapiens


<220>

<221> MOD_RES
<222> (20)..(20)
<223> Phosphorylated Ser

<400> 162
Ala Gln Ser Lys Gln Thr Gly Arg Val Leu Glu Pro Pro Val Pro Ser
1               5                   10                  15

Arg Ser Glu Ser Phe Ser Asn Gly Asn Ser Glu
            20                  25

<210> 163
<211> 12
<212> PRT
<213> Homo sapiens

<220>
<221> MOD_RES
<222> (8)..(8)
<223> Phosphorylated Thr

<400> 163
His Met Thr Gln Glu Val Val Thr Gln Tyr Tyr Arg
1               5                   10

<210> 164
<211> 26
<212> PRT
<213> Homo sapiens

<220>
<221> MOD_RES
<222> (7)..(7)
<223> Phosphorylated Ser

<400> 164
Ser Lys Val Lys Arg Gln Ser Ser Thr Pro Ser Ala Pro Glu Leu Gly
1               5                   10                  15

Gln Gln Pro Asp Val Asn Ile Ser Glu Trp
            20                  25

<210> 165
<211> 26
<212> PRT
<213> Homo sapiens

<220>
<221> MOD_RES
<222> (8)..(8)
<223> Phosphorylated Ser

<400> 165
Ser Lys Val Lys Arg Gln Ser Ser Thr Pro Ser Ala Pro Glu Leu Gly
1               5                   10                  15

Gln Gln Pro Asp Val Asn Ile Ser Glu Trp
            20                  25

<210> 166
<211> 17
<212> PRT
<213> Homo sapiens

<220>
<221> MOD_RES

<222> (8)..(8)
<223> Phosphorylated Ser

<400> 166
Asn Leu Asp Pro Glu Gln Met Ser Gln Val Leu Asp Ala Met Phe Glu
1               5                   10                  15

Lys

<210> 167
<211> 16
<212> PRT
<213> Homo sapiens

<220>
<221> MOD_RES
<222> (10)..(10)
<223> Phosphorylated Thr

<400> 167
Gly Ile Gly Phe Gly Asp Arg Thr Ser Thr Phe Cys Gly Thr Pro Glu
1               5                   10                  15

<210> 168
<211> 15
<212> PRT
<213> Homo sapiens

<220>
<221> MOD_RES
<222> (10)..(10)
<223> Phosphorylated Thr

<400> 168
Ala Glu Pro Tyr Met His Arg Ala Gly Thr Leu Thr Ser Leu Glu
1               5                   10                  15

<210> 169
<211> 35
<212> PRT
<213> Homo sapiens

<220>
<221> MOD_RES
<222> (6)..(6)
<223> Phosphorylated Ser

<400> 169
Gln Gly Ser Gly Ser Ser Gln Pro Met Glu Val Gln Glu Gly Tyr Gly
1               5                   10                  15

Phe Gly Ser Gly Asp Asp Pro Tyr Ser Ser Ala Glu Pro His Val Ser
            20                  25                  30

Gly Val Lys
        35

<210> 170
<211> 21
<212> PRT
<213> Homo sapiens

<220>
<221> MOD_RES
<222> (17)..(17)

<223> Phosphorylated Ser

<400> 170
Ile Leu Ala Gly Asp Tyr Glu Phe Asp Ser Pro Tyr Trp Asp Asp Ile
1               5                   10                  15

Ser Gln Ala Ala Lys
            20

<210> 171
<211> 11
<212> PRT
<213> Homo sapiens

<220>
<221> MOD_RES
<222> (8)..(8)
<223> Phosphorylated Thr

<400> 171
Ala Gly Ser Leu Gly Pro Glu Thr Pro Ser Arg
1               5                   10

<210> 172
<211> 18
<212> PRT
<213> Homo sapiens

<220>
<221> MOD_RES
<222> (4)..(4)
<223> Phosphorylated Ser

<400> 172
Leu Ala Leu Ser Gln Asn Gln Gln Ser Ser Gly Ala Ala Gly Pro Thr
1               5                   10                  15

Gly Lys

<210> 173
<211> 30
<212> PRT
<213> Homo sapiens

<220>
<221> MOD_RES
<222> (5)..(5)
<223> Phosphorylated Ser

<400> 173
Ser Arg Val Arg Ser Ser Asp Met Asp Gln Gln Glu Asp Met Ile Ser
1               5                   10                  15

Gly Val Glu Asn Ser Asn Val Ser Glu Asn Asp Ile Pro Phe
            20                  25                  30

<210> 174
<211> 17
<212> PRT
<213> Homo sapiens

<220>
<221> MOD_RES
<222> (15)..(15)
<223> Phosphorylated Thr

<400> 174
Val Pro Asp Ser Pro Ala Thr Lys Tyr Pro Ile Tyr Pro Leu Thr Pro
1               5                   10                  15

Lys

<210> 175
<211> 19
<212> PRT
<213> Homo sapiens

<220>
<221> MOD_RES
<222> (6)..(6)
<223> Phosphorylated Ser

<400> 175
Arg Ala Arg Leu Ala Ser Asn Leu Gln Trp Pro Ser Cys Pro Thr Gln
1               5                   10                  15

Tyr Ser Glu

<210> 176
<211> 15
<212> PRT
<213> Homo sapiens

<220>
<221> MOD_RES
<222> (13)..(13)
<223> Phosphorylated Ser

<400> 176
Ala Gln Val Gln Ala Leu Ala Leu Ser Arg Gln Ala Ser Gln Glu
1               5                   10                  15

<210> 177
<211> 18
<212> PRT
<213> Homo sapiens

<220>
<221> MOD_RES
<222> (10)..(10)
<223> Phosphorylated Ser

<400> 177
Pro Ser Ser Phe Gln Asn Asn Val Gln Ser Gln Asp Pro Gln Val Ser
1               5                   10                  15

Thr Lys

<210> 178
<211> 29
<212> PRT
<213> Homo sapiens

<220>
<221> MOD_RES
<222> (18)..(18)
<223> Phosphorylated Thr

<400> 178
Gly Gly Asn Thr Gly Thr Ser Gly Gly Gln Ala Gly Pro Ala Gly Leu
1               5                   10                  15

Ser Thr Pro Ser Thr Ser Thr Ser Asn Asn Ser Leu Pro
            20                  25

<210> 179
<211> 32
<212> PRT
<213> Homo sapiens

<220>
<221> MOD_RES
<222> (6)..(6)
<223> Phosphorylated Ser

<400> 179
Trp Gly Gln Pro Pro Ser Pro Thr Pro Val Pro Arg Pro Pro Asp Ala
1               5                   10                  15

Asp Pro Asn Thr Pro Ser Pro Lys Pro Leu Glu Gly Arg Pro Glu Arg
            20                  25                  30

<210> 180
<211> 23
<212> PRT
<213> Homo sapiens

<220>
<221> MOD_RES
<222> (11)..(11)
<223> Phosphorylated Thr

<400> 180
Ser Thr Thr Leu Ala Val Asp Tyr Pro Lys Thr Pro Thr Gly Ser Pro
1               5                   10                  15

Ala Thr Glu Val Ser Ala Lys
            20

<210> 181
<211> 25
<212> PRT
<213> Homo sapiens

<220>
<221> MOD_RES
<222> (19)..(19)
<223> Phosphorylated Ser

<400> 181
Ser Val Val Lys Pro Val Pro Ala Pro Gln Ala Val Gln Pro Ser Arg
1               5                   10                  15

Gln Glu Ser Ser Cys Gly Ser Leu Glu
            20                  25

<210> 182
<211> 12
<212> PRT
<213> Homo sapiens

<220>
<221> MOD_RES

<222> (6)..(6)
<223> Phosphorylated Thr

<400> 182
Glu Ile Pro Ser Ala Thr Gln Ser Pro Ile Ser Lys
1               5                   10


<210> 183
<211> 12
<212> PRT
<213> Homo sapiens


<220>
<221> MOD_RES
<222> (8)..(8)
<223> Phosphorylated Ser

<400> 183
Asp Leu Val Ser Phe Pro Leu Ser Pro Ala Val Arg
1               5                   10


<210> 184
<211> 18
<212> PRT
<213> Homo sapiens


<220>
<221> MOD_RES
<222> (7)..(7)
<223> Phosphorylated Ser

<400> 184
Ile Ser Ser Arg Lys Thr Ser Thr Glu Cys Ala Ser Ser Thr Glu Asn
1               5                   10                  15


Ser Phe



<210> 185
<211> 25
<212> PRT
<213> Homo sapiens


<220>
<221> MOD_RES
<222> (11)..(11)
<223> Phosphorylated Ser

<400> 185
Val Gln Ser Ser Gly Arg Ile Arg Gln Pro Ser Val Asp Leu Thr Asp
1               5                   10                  15


Asp Asp Gln Thr Ser Ser Val Pro His
            20                  25


<210> 186
<211> 12
<212> PRT
<213> Homo sapiens


<220>
<221> MOD_RES
<222> (10)..(10)
<223> Phosphorylated Thr

<400> 186
Gln Ser Met Ala Phe Ser Ile Leu Asn Thr Pro Lys

1                   5                   10


<210> 187
<211> 12
<212> PRT
<213> Homo sapiens


<220>
<221> MOD_RES
<222> (4)..(4)
<223> Phosphorylated Thr

<400> 187
Lys Arg Glu Thr Tyr Tyr Leu Ser Asp Ser Pro Leu
1               5                   10


<210> 188
<211> 16
<212> PRT
<213> Homo sapiens


<220>
<221> MOD_RES
<222> (7)..(7)
<223> Phosphorylated Thr

<400> 188
Leu Leu Gln Arg Val Glu Thr Ser Glu Gly Leu Asn Ser Asp Leu Glu
1               5                   10                  15


<210> 189
<211> 29
<212> PRT
<213> Homo sapiens


<220>
<221> MOD_RES
<222> (8)..(8)
<223> Phosphorylated Thr

<400> 189
Ala Phe Ser Thr Val Pro Leu Thr Pro Val Pro Arg Pro Ser Asp Ser
1               5                   10                  15


Val Ser Ser Thr Pro Ala Ala Thr Ser Thr Pro Ser Lys
            20                  25


<210> 190
<211> 27
<212> PRT
<213> Homo sapiens


<220>
<221> MOD_RES
<222> (8)..(8)
<223> Phosphorylated Thr

<400> 190
Asp Ser Met Asn Ala Thr Ser Thr Pro Ala Ala Leu Ser Pro Ser Val
1               5                   10                  15


Leu Thr Thr Pro Ser Lys Ile Glu Pro Met Lys
            20                  25


<210> 191
<211> 23

<212> PRT
<213> Homo sapiens


<220>
<221> MOD_RES
<222> (10)..(10)
<223> Phosphorylated Ser

<400> 191
Thr Ser Ala Ser Arg Ile Arg Glu Arg Ser Glu Ser Leu Asp Pro Asp
1               5                   10                  15


Ser Ser Met Asp Thr Thr Leu
            20


<210> 192
<211> 17
<212> PRT
<213> Homo sapiens


<220>
<221> MOD_RES
<222> (5)..(5)
<223> Phosphorylated Thr

<400> 192
Glu Leu Phe Gln Thr Pro Gly His Thr Glu Glu Leu Val Ala Ala Gly
1               5                   10                  15


Lys



<210> 193
<211> 29
<212> PRT
<213> Homo sapiens


<220>
<221> MOD_RES
<222> (23)..(23)
<223> Phosphorylated Thr

<400> 193
Thr Pro Glu Thr Val Val Pro Thr Ala Pro Glu Leu Gln Ile Ser Thr
1               5                   10                  15


Ser Thr Asp Gln Pro Val Thr Pro Lys Pro Thr Ser Arg
            20                  25


<210> 194
<211> 32
<212> PRT
<213> Homo sapiens


<220>
<221> MOD_RES
<222> (23)..(23)
<223> Phosphorylated Thr

<400> 194
Thr Pro Glu Thr Val Val Pro Thr Ala Pro Glu Leu Gln Pro Ser Thr
1               5                   10                  15


Ser Thr Asp Gln Pro Val Thr Pro Glu Pro Thr Ser Gln Ala Thr Arg
            20                  25                  30

<210> 195
<211> 18
<212> PRT
<213> Homo sapiens

<220>
<221> MOD_RES
<222> (5)..(5)
<223> Phosphorylated Ser

<400> 195
Leu Arg Pro Ile Ser Asp Asp Ser Glu Ser Ile Glu Glu Ser Asp Thr
1               5                   10                  15

Arg Arg

<210> 196
<211> 12
<212> PRT
<213> Homo sapiens

<220>
<221> MOD_RES
<222> (9)..(9)
<223> Phosphorylated Thr

<400> 196
Val Gln Ile Ala Ala Asn Glu Glu Thr Gln Glu Arg
1               5                   10

<210> 197
<211> 34
<212> PRT
<213> Homo sapiens

<220>
<221> MOD_RES
<222> (22)..(22)
<223> Phosphorylated Thr

<400> 197
Ser Asn Asp Ser Thr Glu Gln Asn Leu Ser Asp Gly Thr Pro Met Pro
1               5                   10                  15

Asp Ser Tyr Pro Thr Thr Pro Ser Ser Thr Asp Ala Ala Thr Ser Glu
            20                  25                  30

Ser Lys

<210> 198
<211> 23
<212> PRT
<213> Homo sapiens

<220>
<221> MOD_RES
<222> (9)..(9)
<223> Phosphorylated Ser

<400> 198
Pro Ser Glu Glu Ala Pro Lys Cys Ser Gln Asp Gln Gly Val Leu Ala
1               5                   10                  15

Ser Glu Leu Ala Gln Asn Lys
            20

<210> 199
<211> 29
<212> PRT
<213> Homo sapiens

<220>
<221> MOD_RES
<222> (18)..(18)
<223> Phosphorylated Thr

<400> 199
Val Leu Gly Gln Gln Pro Val Ile Ser Gln Glu Asp Arg Thr Arg Ala
1               5                   10                  15

Ser Thr Leu Ser Pro Asp Phe Pro Glu Gln Gln Ala Phe
            20                  25

<210> 200
<211> 37
<212> PRT
<213> Homo sapiens

<220>
<221> MOD_RES
<222> (17)..(17)
<223> Phosphorylated Thr

<400> 200
Cys His Glu Ser Gln Gly Glu Leu Ser Ser Ala Pro Glu Ala Pro Gly
1               5                   10                  15

Thr Gln Glu Gly Pro Thr Cys Asp Gly Thr Pro Leu Pro Glu Pro Gly
            20                  25                  30

Thr Ser Glu Leu Arg
        35

<210> 201
<211> 14
<212> PRT
<213> Homo sapiens

<220>
<221> MOD_RES
<222> (9)..(9)
<223> Phosphorylated Ser

<400> 201
Tyr Ser Ser Pro Gly Arg Pro Leu Ser Cys Phe Val Asp Glu
1               5                   10

<210> 202
<211> 11
<212> PRT
<213> Homo sapiens

<220>
<221> MOD_RES
<222> (5)..(5)
<223> Phosphorylated Thr

<400> 202
Leu Arg Gln Leu Thr Ala Asn Thr Gly His Thr

1               5                   10


<210> 203
<211> 13
<212> PRT
<213> Homo sapiens


<220>
<221> MOD_RES
<222> (5)..(5)
<223> Phosphorylated Ser

<400> 203
Thr Arg Gln Ser Ser Asp Phe Asp Thr Gln Ser Gly Phe
1               5                   10


<210> 204
<211> 25
<212> PRT
<213> Homo sapiens


<220>
<221> MOD_RES
<222> (1)..(1)
<223> Phosphorylated Thr

<400> 204
Thr Pro Ser Tyr Ser Ile Ser Ser Thr Leu Asn Pro Gln Ala Pro Glu
1               5                   10                  15


Phe Ile Leu Gly Cys Thr Ala Ser Lys
            20                  25


<210> 205
<211> 8
<212> PRT
<213> Homo sapiens


<220>
<221> MOD_RES
<222> (5)..(5)
<223> Phosphorylated Thr

<400> 205
Lys Arg Thr Pro Thr Glu Val Trp
1               5


<210> 206
<211> 33
<212> PRT
<213> Homo sapiens


<220>
<221> MOD_RES
<222> (27)..(27)
<223> Phosphorylated Ser

<400> 206
Asn Ala Lys Ile Leu Ile Ala Asn Thr Gly Met Asp Thr Asp Lys Ile
1               5                   10                  15


Lys Ile Phe Gly Ser Arg Val Arg Val Asp Ser Thr Ala Lys Val Ala
            20                  25                  30


Glu

<210> 207
<211> 13
<212> PRT
<213> Homo sapiens

<220>
<221> MOD_RES
<222> (5)..(5)
<223> Phosphorylated Ser

<400> 207
Gly Arg Leu Ala Ser Thr Leu Val His Leu Gly Glu Tyr
1               5                   10

<210> 208
<211> 35
<212> PRT
<213> Homo sapiens

<220>
<221> MOD_RES
<222> (16)..(16)
<223> Phosphorylated Ser

<400> 208
Tyr Asp Glu Glu Gly Gly Gly Glu Glu Asp Gln Asp Tyr Asp Leu Ser
1               5                   10                  15

Gln Leu Gln Gln Pro Asp Thr Val Glu Pro Asp Ala Ile Lys Pro Val
            20                  25                  30

Gly Ile Arg
        35

<210> 209
<211> 15
<212> PRT
<213> Homo sapiens

<220>
<221> MOD_RES
<222> (10)..(10)
<223> Phosphorylated Ser

<400> 209
Val Gly Ser Thr Asn Val Arg Ile Gly Ser Thr Ile Phe Gly Glu
1               5                   10                  15

<210> 210
<211> 11
<212> PRT
<213> Homo sapiens

<220>
<221> MOD_RES
<222> (5)..(5)
<223> Phosphorylated Ser

<400> 210
Glu Arg Leu Val Ser Ser Glu Ser Lys Asn Tyr
1               5                   10

<210> 211
<211> 20
<212> PRT

<213> Homo sapiens


<220>
<221> MOD_RES
<222> (17)..(17)
<223> Phosphorylated Ser

<400> 211
Ile Ser Thr Ser Ser Leu Lys Val Thr Asp Ala Phe Ala Arg Leu Met
1               5                   10                  15


Ser Thr Val Glu
            20


<210> 212
<211> 9
<212> PRT
<213> Homo sapiens


<220>
<221> MOD_RES
<222> (5)..(5)
<223> Phosphorylated Thr

<400> 212
Gly Arg Met Pro Thr Tyr Ser Gln Phe
1               5


<210> 213
<211> 17
<212> PRT
<213> Homo sapiens


<220>
<221> MOD_RES
<222> (1)..(1)
<223> Phosphorylated Thr

<400> 213
Thr Gln Leu Ser Gln Gly Arg Ser Ser Pro Gln Leu Asp Pro Leu Arg
1               5                   10                  15


Lys



<210> 214
<211> 17
<212> PRT
<213> Homo sapiens


<220>
<221> MOD_RES
<222> (4)..(4)
<223> Phosphorylated Ser

<400> 214
Thr Gln Leu Ser Gln Gly Arg Ser Ser Pro Gln Leu Asp Pro Leu Arg
1               5                   10                  15


Lys



<210> 215
<211> 12
<212> PRT
<213> Homo sapiens

<220>
<221> MOD_RES
<222> (5)..(5)
<223> Phosphorylated Ser

<400> 215
Val Arg Arg Cys Ser Met Pro Ser Val Ile Cys Glu
1               5                   10


<210> 216
<211> 26
<212> PRT
<213> Homo sapiens


<220>
<221> MOD_RES
<222> (12)..(12)
<223> Phosphorylated Ser

<400> 216
Ala Ala Ala Ala Leu Thr Arg Met Arg Ala Glu Ser Thr Ala Asn Ala
1               5                   10                  15


Gly Gln Ser Asp Asn Arg Ser Leu Ala Glu
            20                  25


<210> 217
<211> 20
<212> PRT
<213> Homo sapiens


<220>
<221> MOD_RES
<222> (4)..(4)
<223> Phosphorylated Thr

<400> 217
Arg Arg His Thr Val Glu Asp Ala Val Val Ser Gln Gly Pro Glu Ala
1               5                   10                  15


Ala Gly Pro Leu
            20


<210> 218
<211> 10
<212> PRT
<213> Homo sapiens


<220>
<221> MOD_RES
<222> (3)..(3)
<223> Phosphorylated Ser

<400> 218
His Tyr Ser Gln Asp Cys Ser Ser Ile Lys
1               5                   10


<210> 219
<211> 27
<212> PRT
<213> Homo sapiens


<220>
<221> MOD_RES
<222> (7)..(7)

<223> Phosphorylated Ser

<400> 219
Met Leu Ser Lys Asp Cys Ser Gln Glu Tyr Thr Asp Ser Thr Gly Ile
1               5                   10                  15

Asp Leu His Glu Phe Leu Ile Asn Thr Leu Lys
            20                  25

<210> 220
<211> 38
<212> PRT
<213> Homo sapiens

<220>
<221> MOD_RES
<222> (25)..(25)
<223> Phosphorylated Thr

<400> 220
Gly Gln His His Gln Gln Gln Gln Ala Ala Gly Gly Ser Glu Ser His
1               5                   10                  15

Pro Val Pro Pro Thr Ala Pro Leu Thr Pro Leu Leu His Gly Glu Gly
            20                  25                  30

Ala Ser Gln Gln Pro Arg
        35

<210> 221
<211> 11
<212> PRT
<213> Homo sapiens

<220>
<221> MOD_RES
<222> (7)..(7)
<223> Phosphorylated Ser

<400> 221
Ile Asn Ala Arg Glu Glu Ser Leu Val Glu Glu
1               5                   10

<210> 222
<211> 31
<212> PRT
<213> Homo sapiens

<220>
<221> MOD_RES
<222> (15)..(15)
<223> Phosphorylated Ser

<400> 222
Val Asp Ser Ser Ser Ala Ser Thr Thr Leu Gly Arg Gln Cys Ser Leu
1               5                   10                  15

Asn Cys Ile Ser Ser Gly Cys His Thr Ser Gly Asp Ser Leu Glu
            20                  25                  30

<210> 223
<211> 33
<212> PRT
<213> Homo sapiens

<220>
<221> MOD_RES
<222> (27)..(27)
<223> Phosphorylated Thr

<400> 223
Lys Gln Asp Ala Glu Thr Pro Pro Pro Pro Asp Pro Pro Ala Pro Glu
1               5                   10                  15

Thr Leu Leu Ala Pro Pro Pro Leu Pro Ser Thr Pro Asp Pro Pro Arg
            20                  25                  30

Arg

<210> 224
<211> 12
<212> PRT
<213> Homo sapiens

<220>
<221> MOD_RES
<222> (9)..(9)
<223> Phosphorylated Ser

<400> 224
Met Gln Lys Pro His Leu Pro Leu Ser Gln Glu Lys
1               5                   10

<210> 225
<211> 24
<212> PRT
<213> Homo sapiens

<220>
<221> MOD_RES
<222> (4)..(4)
<223> Phosphorylated Ser

<400> 225
Glu Gln Phe Ser Gln Gly Ser Pro Ser Asn Cys Leu Glu Thr Ser Leu
1               5                   10                  15

Ala Glu Ile Phe Pro Leu Gly Lys
            20

<210> 226
<211> 22
<212> PRT
<213> Homo sapiens

<220>
<221> MOD_RES
<222> (8)..(8)
<223> Phosphorylated Ser

<400> 226
Met Asn Arg Thr Arg Thr Ser Ser Glu Ala Ser Met Asp Ala Ala Tyr
1               5                   10                  15

Leu Asp Lys Ile Ser Glu
            20

<210> 227
<211> 25
<212> PRT

<213> Homo sapiens

<220>
<221> MOD_RES
<222> (5)..(5)
<223> Phosphorylated Ser

<400> 227
Glu Arg Arg Ser Ser Trp Tyr Val Asp Ala Ser Asp Val Leu Thr Thr
1               5                   10                  15

Glu Asp Pro Gln Cys Pro Gln Pro Leu
            20                  25

<210> 228
<211> 22
<212> PRT
<213> Homo sapiens

<220>
<221> MOD_RES
<222> (10)..(10)
<223> Phosphorylated Ser

<400> 228
Asn Arg Val Asn Gly Arg Arg Ala Pro Ser Thr Ser Pro Ser Phe Glu
1               5                   10                  15

Gly Thr Gln Glu Thr Tyr
            20

<210> 229
<211> 20
<212> PRT
<213> Homo sapiens

<220>
<221> MOD_RES
<222> (10)..(10)
<223> Phosphorylated Thr

<400> 229
Arg Pro Asp Pro Ser His Arg Ala Pro Thr Leu Val Trp Arg Pro Gly
1               5                   10                  15

Gly Glu Leu Trp
            20

<210> 230
<211> 17
<212> PRT
<213> Homo sapiens

<220>
<221> MOD_RES
<222> (7)..(7)
<223> Phosphorylated Thr

<400> 230
Pro Glu Phe Lys Lys Ile Thr Glu Leu Thr Glu Ala Gly Glu Thr Lys
1               5                   10                  15

Lys

<210> 231
<211> 16
<212> PRT
<213> Homo sapiens

<220>
<221> MOD_RES
<222> (7)..(7)
<223> Phosphorylated Ser

<400> 231
Leu Glu Ile Tyr Gln Ser Ser Gln Asp Glu Pro Ile Ala Leu Asp Lys
1               5                   10                  15

<210> 232
<211> 14
<212> PRT
<213> Homo sapiens

<220>
<221> MOD_RES
<222> (5)..(5)
<223> Phosphorylated Ser

<400> 232
Ser Arg Trp Lys Ser Glu Glu Glu Val Glu Ser Asp Asp Glu
1               5                   10

<210> 233
<211> 10
<212> PRT
<213> Homo sapiens

<220>
<221> MOD_RES
<222> (5)..(5)
<223> Phosphorylated Thr

<400> 233
Ser Gln Lys Leu Thr Gln Asp Asp Ile Lys
1               5                   10

<210> 234
<211> 36
<212> PRT
<213> Homo sapiens

<220>
<221> MOD_RES
<222> (30)..(30)
<223> Phosphorylated Ser

<400> 234
Gln Ala Ser Glu Gln Glu Asn Glu Asp Ser Met Ala Glu Ala Pro Glu
1               5                   10                  15

Ser Asn His Pro Glu Asp Gln Glu Thr Met Glu Thr Ile Ser Gln Asp
            20                  25                  30

Pro Glu His Lys
        35

<210> 235

<400> 235
000

<210> 236
<211> 11
<212> PRT
<213> Homo sapiens

<220>
<221> MOD_RES
<222> (4)..(4)
<223> Phosphorylated Ser

<400> 236
Trp Ser Pro Ser Gln Val Val Asp Trp Met Lys
1               5                   10

<210> 237
<211> 18
<212> PRT
<213> Homo sapiens

<220>
<221> MOD_RES
<222> (17)..(17)
<223> Phosphorylated Ser

<400> 237
Glu Ala Leu Phe Asp Gly Gly Gly Gly Gly Pro Ala Val Ser Asp Leu
1               5                   10                  15

Ser Gln

<210> 238
<211> 12
<212> PRT
<213> Homo sapiens

<220>
<221> MOD_RES
<222> (3)..(3)
<223> Phosphorylated Ser

<400> 238
Arg Leu Ser Gln Ser Met Glu Ser Asn Ser Gly Lys
1               5                   10

<210> 239
<211> 12
<212> PRT
<213> Homo sapiens

<220>
<221> MOD_RES
<222> (3)..(3)
<223> Phosphorylated Ser

<400> 239
Val Leu Ser Gln Ser Thr Pro Gly Thr Pro Ser Lys
1               5                   10

<210> 240
<211> 22
<212> PRT
<213> Homo sapiens

<220>
<221> MOD_RES

<222> (7)..(7)
<223> Phosphorylated Thr

<400> 240
Ser Lys Pro Arg Gln Pro Thr Ile Asp Leu Ser Gln Met Ala Val Pro
1               5                   10                  15

Ile Gln Met Thr Gln Glu
            20

<210> 241
<211> 14
<212> PRT
<213> Homo sapiens

<220>
<221> MOD_RES
<222> (8)..(8)
<223> Phosphorylated Thr

<400> 241
Gly Gln Ala Gly Phe Phe Ser Thr Pro Gly Gly Ala Ser Arg
1               5                   10

<210> 242
<211> 10
<212> PRT
<213> Homo sapiens

<220>
<221> MOD_RES
<222> (5)..(5)
<223> Phosphorylated Thr

<400> 242
Ile Arg Arg Pro Thr Leu Asp Ile Asn Trp
1               5                   10

<210> 243
<211> 12
<212> PRT
<213> Homo sapiens

<220>
<221> MOD_RES
<222> (5)..(5)
<223> Phosphorylated Ser

<400> 243
Leu Ser Met Leu Ser Gln Glu Ile Gln Thr Leu Lys
1               5                   10

<210> 244
<211> 32
<212> PRT
<213> Homo sapiens

<220>
<221> MOD_RES
<222> (27)..(27)
<223> Phosphorylated Thr

<400> 244
Asp Gly Leu Ser Glu Ala Cys Gly Gly Ala Gly Ser Ser Gly Ser Ala
1               5                   10                  15

Glu Ser Gly Ala Gly Gly Gly Arg Arg Ala Thr Ile Ser Ser Pro Leu

20                  25                  30

<210> 245
<211> 20
<212> PRT
<213> Homo sapiens

<220>
<221> MOD_RES
<222> (6)..(6)
<223> Phosphorylated Ser

<400> 245
Arg Thr Asp Val Lys Ser Gln Asp Val Ala Val Ser Pro Gln Gln Gln
1               5                   10                  15

Gln Cys Ser Lys
            20

<210> 246
<211> 15
<212> PRT
<213> Homo sapiens

<220>
<221> MOD_RES
<222> (4)..(4)
<223> Phosphorylated Thr

<400> 246
Arg Trp His Thr Gly Gly Gly Gly Glu Lys Ala Ala Gly Gly Phe
1               5                   10                  15

<210> 247
<211> 14
<212> PRT
<213> Homo sapiens

<220>
<221> MOD_RES
<222> (11)..(11)
<223> Phosphorylated Ser

<400> 247
Asn Glu Asn Leu Gln Glu Ser Glu Ile Leu Ser Gln Pro Lys
1               5                   10

<210> 248
<211> 18
<212> PRT
<213> Homo sapiens

<220>
<221> MOD_RES
<222> (9)..(9)
<223> Phosphorylated Ser

<400> 248
Thr Val Glu Glu Gln Asp Cys Leu Ser Gln Gln Asp Leu Ser Pro Gln
1               5                   10                  15

Leu Lys

<210> 249
<211> 32

<212> PRT
<213> Homo sapiens


<220>
<221> MOD_RES
<222> (16)..(16)
<223> Phosphorylated Ser

<400> 249
Ile Leu Asp Glu Leu Asp Lys Asp Asp Ser Thr His Glu Ser Leu Ser
1               5                   10                  15


Gln Glu Ser Glu Ser Glu Glu Asp Gly Ile His Val Asp Ser Gln Lys
            20                  25                  30


<210> 250
<211> 15
<212> PRT
<213> Homo sapiens


<220>
<221> MOD_RES
<222> (11)..(11)
<223> Phosphorylated Ser

<400> 250
Asn Gly Leu Thr His Gln Leu Gly Gly Leu Ser Gln Gly Ser Arg
1               5                   10                  15


<210> 251
<211> 23
<212> PRT
<213> Homo sapiens


<220>
<221> MOD_RES
<222> (15)..(15)
<223> Phosphorylated Ser

<400> 251
Gln Thr Gly Gln Ile Ile Glu Asp Asp Gln Glu Lys His Leu Ser Gln
1               5                   10                  15


Glu Asp Asn Asp Leu Asn Lys
            20


<210> 252
<211> 8
<212> PRT
<213> Homo sapiens


<220>
<221> MOD_RES
<222> (3)..(3)
<223> Phosphorylated Ser

<400> 252
Arg Leu Ser Gln Ala Glu Glu Arg
1               5


<210> 253
<211> 18
<212> PRT
<213> Homo sapiens


<220>

<221> MOD_RES
<222> (12)..(12)
<223> Phosphorylated Ser

<400> 253
Leu Gln Glu Leu Glu Asp Ser Ile Asp Asn Leu Ser Gln Asn Gly Glu
1               5                   10                  15

Gly Arg

<210> 254
<211> 16
<212> PRT
<213> Homo sapiens

<220>
<221> MOD_RES
<222> (6)..(6)
<223> Phosphorylated Thr

<400> 254
Val Gly Arg Lys Ala Thr Glu Asp Phe Leu Gly Ser Ser Ser Gly Tyr
1               5                   10                  15

<210> 255
<211> 21
<212> PRT
<213> Homo sapiens

<220>
<221> MOD_RES
<222> (16)..(16)
<223> Phosphorylated Thr

<400> 255
Ala Lys Glu Ile Arg Pro Gln Asp Cys Pro Ile Ser Arg Val Asp Thr
1               5                   10                  15

Phe Pro Gln Pro Leu
            20

<210> 256
<211> 20
<212> PRT
<213> Homo sapiens

<220>
<221> MOD_RES
<222> (12)..(12)
<223> Phosphorylated Thr

<400> 256
Gly Gly Asp Ala Gly Asn Asp Thr Arg Asn Thr Thr Ser Asp Leu Gln
1               5                   10                  15

Lys Thr Ser Glu
            20

<210> 257
<211> 19
<212> PRT
<213> Homo sapiens

<220>
<221> MOD_RES

<222> (6)..(6)
<223> Phosphorylated Ser

<400> 257
Ser Arg Lys Asp Thr Ser Val Lys Ile Lys Ile Pro Pro Val Ala Glu
1               5                   10                  15

Ala Gly Trp

<210> 258
<211> 19
<212> PRT
<213> Homo sapiens

<220>
<221> MOD_RES
<222> (5)..(5)
<223> Phosphorylated Thr

<400> 258
Ser Arg Lys Asp Thr Ser Val Lys Ile Lys Ile Pro Pro Val Ala Glu
1               5                   10                  15

Ala Gly Trp

<210> 259
<211> 37
<212> PRT
<213> Homo sapiens

<220>
<221> MOD_RES
<222> (7)..(7)
<223> Phosphorylated Ser

<400> 259
Leu Gly Gln Arg Pro Leu Ser Gln Pro Ala Gly Ile Ser Thr Asn Pro
1               5                   10                  15

Phe Met Thr Gly Pro Ser Ser Ser Pro Phe Ala Ser Lys Pro Pro Thr
            20                  25                  30

Thr Asn Pro Phe Leu
        35

<210> 260
<211> 11
<212> PRT
<213> Homo sapiens

<220>
<221> MOD_RES
<222> (3)..(3)
<223> Phosphorylated Thr

<400> 260
Gln Thr Thr Gln Asp Ala Pro Glu Glu Val Arg
1               5                   10

<210> 261
<211> 16
<212> PRT
<213> Homo sapiens

<220>
<221> MOD_RES
<222> (7)..(7)
<223> Phosphorylated Thr

<400> 261
Ser Ile Thr Ile Leu Ser Thr Pro Glu Gly Thr Ser Ala Ala Cys Lys
1               5                   10                  15


<210> 262
<211> 16
<212> PRT
<213> Homo sapiens


<220>
<221> MOD_RES
<222> (5)..(5)
<223> Phosphorylated Thr

<400> 262
Trp Asp Phe Asp Thr Gln Glu Glu Tyr Ser Glu Tyr Met Asn Asn Lys
1               5                   10                  15


<210> 263
<211> 24
<212> PRT
<213> Homo sapiens


<220>
<221> MOD_RES
<222> (6)..(6)
<223> Phosphorylated Thr

<400> 263
Arg Asn Arg Gln Asp Thr Ser Asp Leu Thr Ser Glu Ala Val Glu Glu
1               5                   10                  15


Leu Ser Glu Ser Val His Gly Leu
            20


<210> 264
<211> 18
<212> PRT
<213> Homo sapiens


<220>
<221> MOD_RES
<222> (3)..(3)
<223> Phosphorylated Ser

<400> 264
Pro Ala Ser Pro Asp Arg Phe Ala Val Ser Ala Glu Ala Glu Asn Lys
1               5                   10                  15


Val Arg



<210> 265
<211> 19
<212> PRT
<213> Homo sapiens


<220>
<221> MOD_RES
<222> (7)..(7)
<223> Phosphorylated Ser

<400> 265
Gly Leu Ser Arg Arg Ser Ser Thr Ser Ser Glu Pro Thr Pro Thr Val
1               5                   10                  15

Lys Thr Leu

<210> 266
<211> 12
<212> PRT
<213> Homo sapiens

<220>
<221> MOD_RES
<222> (6)..(6)
<223> Phosphorylated Thr

<400> 266
Ser Gly Gly Leu Gln Thr Pro Glu Cys Leu Ser Arg
1               5                   10

<210> 267
<211> 17
<212> PRT
<213> Homo sapiens

<220>
<221> MOD_RES
<222> (8)..(8)
<223> Phosphorylated Ser

<400> 267
Pro Ser Pro Gly Thr Pro Thr Ser Pro Ser Asn Leu Thr Ser Gly Leu
1               5                   10                  15

Lys

<210> 268
<211> 20
<212> PRT
<213> Homo sapiens

<220>
<221> MOD_RES
<222> (12)..(12)
<223> Phosphorylated Ser

<400> 268
Ser Thr Ser Glu Ser Ser Ala Ser Phe Pro Arg Ser Pro Gly Gln Pro
1               5                   10                  15

Ser Ser Leu Arg
            20

<210> 269
<211> 10
<212> PRT
<213> Homo sapiens

<220>
<221> MOD_RES
<222> (3)..(3)
<223> Phosphorylated Thr

<400> 269
Gln Gln Thr Gln Ser Ala Leu Glu Gln Arg
1               5                   10


<210> 270
<211> 21
<212> PRT
<213> Homo sapiens


<220>
<221> MOD_RES
<222> (3)..(3)
<223> Phosphorylated Ser

<400> 270
Arg Pro Ser Gln Glu Gln Ser Ala Ser Ala Ser Ser Gly Gln Pro Gln
1               5                   10                  15


Ala Pro Leu Asn Arg
            20


<210> 271
<211> 22
<212> PRT
<213> Homo sapiens


<220>
<221> MOD_RES
<222> (9)..(9)
<223> Phosphorylated Thr

<400> 271
Leu Gly Lys Ser Glu Ala Pro Glu Thr Pro Met Glu Glu Glu Ala Glu
1               5                   10                  15


Leu Val Leu Thr Glu Lys
            20


<210> 272
<211> 15
<212> PRT
<213> Homo sapiens


<220>
<221> MOD_RES
<222> (7)..(7)
<223> Phosphorylated Ser

<400> 272
Ala Gly Ser Arg Pro Gln Ser Pro Ser Gly Asp Ala Asp Ala Arg
1               5                   10                  15


<210> 273
<211> 14
<212> PRT
<213> Homo sapiens


<220>
<221> MOD_RES
<222> (11)..(11)
<223> Phosphorylated Thr

<400> 273
Ala Ser Leu Gln Glu Thr His Phe Asp Ser Thr Gln Thr Lys
1               5                   10

<210> 274
<211> 35
<212> PRT
<213> Homo sapiens

<220>
<221> MOD_RES
<222> (29)..(29)
<223> Phosphorylated Thr

<400> 274
Val Leu Ala Ala Gly Gly Leu Gly Gln Gly Gly Gly Gly Gly Gln Ser
1               5                   10                  15

Ser Val Leu Ser Gly Ile Ser Leu Tyr Asp Pro Arg Thr Pro Asn Ala
            20                  25                  30

Gly Gly Lys
        35

<210> 275
<211> 15
<212> PRT
<213> Homo sapiens

<220>
<221> MOD_RES
<222> (4)..(4)
<223> Phosphorylated Thr

<400> 275
Arg Arg Ser Thr Val Leu Gly Leu Pro Gln His Val Gln Lys Glu
1               5                   10                  15

<210> 276
<211> 19
<212> PRT
<213> Homo sapiens

<220>
<221> MOD_RES
<222> (3)..(3)
<223> Phosphorylated Ser

<400> 276
Gly Leu Ser Gln Glu Gly Thr Gly Pro Pro Thr Ser Ala Gly Glu Gly
1               5                   10                  15

His Ser Arg

<210> 277
<211> 21
<212> PRT
<213> Homo sapiens

<220>
<221> MOD_RES
<222> (5)..(5)
<223> Phosphorylated Thr

<400> 277
Ser Arg Gln Pro Thr Glu Pro Lys Asp Thr Asp Thr Leu Val His Glu
1               5                   10                  15

Ala Gly Ser Gln Tyr

            20

<210> 278

<400> 278
000

<210> 279
<211> 18
<212> PRT
<213> Homo sapiens

<220>
<221> MOD_RES
<222> (16)..(16)
<223> Phosphorylated Ser

<400> 279
His Ser Phe Pro Ala Leu Tyr Tyr Glu Glu Gly Ala Asp Ser Leu Ser
1               5                   10                  15

Gln Arg

<210> 280
<211> 11
<212> PRT
<213> Homo sapiens

<220>
<221> MOD_RES
<222> (7)..(7)
<223> Phosphorylated Ser

<400> 280
Asn Leu Asn Arg Thr Pro Ser Thr Val Thr Leu
1               5                   10

<210> 281
<211> 16
<212> PRT
<213> Homo sapiens

<220>
<221> MOD_RES
<222> (4)..(4)
<223> Phosphorylated Ser

<400> 281
Glu Ala Leu Ser Gln Glu Ala Gly Gly Pro Glu Val Gln Gln Leu Arg
1               5                   10                  15

<210> 282
<211> 26
<212> PRT
<213> Homo sapiens

<220>
<221> MOD_RES
<222> (3)..(3)
<223> Phosphorylated Ser

<400> 282
Cys Pro Ser Gln Ser Ser Ser Arg Pro Ala Ala Met Ile Ser Gln Pro
1               5                   10                  15

Pro Thr Pro Pro Thr Gly Gln Pro Val Arg
            20                  25

<210> 283
<211> 12
<212> PRT
<213> Homo sapiens

<220>
<221> MOD_RES
<222> (10)..(10)
<223> Phosphorylated Thr

<400> 283
Lys Phe Asp Gly Val Glu Gly Pro Arg Thr Pro Lys
1               5                   10

<210> 284
<211> 26
<212> PRT
<213> Homo sapiens

<220>
<221> MOD_RES
<222> (14)..(14)
<223> Phosphorylated Ser

<400> 284
Cys Pro Ser Gln Ser Ser Ser Arg Pro Ala Ala Met Ile Ser Gln Pro
1               5                   10                  15

Pro Thr Pro Pro Thr Gly Gln Pro Val Arg
            20                  25

<210> 285
<211> 8
<212> PRT
<213> Homo sapiens

<220>
<221> MOD_RES
<222> (5)..(5)
<223> Phosphorylated Ser

<400> 285
Leu Arg Lys Pro Ser Tyr Ala Glu
1               5

<210> 286
<211> 11
<212> PRT
<213> Homo sapiens

<220>
<221> MOD_RES
<222> (4)..(4)
<223> Phosphorylated Thr

<400> 286
Arg Phe Ser Thr Tyr Thr Ser Asp Lys Asp Glu
1               5                   10

<210> 287
<211> 28
<212> PRT
<213> Homo sapiens

<220>
<221> MOD_RES
<222> (22)..(22)
<223> Phosphorylated Ser

<400> 287
Asp Ile Arg Asn Ser Thr Gly Ser Gln Asp Gly Pro Val Ser Asn Pro
1               5                   10                  15

Ser Ser Ser Asn Ser Ser Gln Asp Ser Leu His Lys
            20                  25

<210> 288
<211> 28
<212> PRT
<213> Homo sapiens

<220>
<221> MOD_RES
<222> (8)..(8)
<223> Phosphorylated Ser

<400> 288
Asp Ile Arg Asn Ser Thr Gly Ser Gln Asp Gly Pro Val Ser Asn Pro
1               5                   10                  15

Ser Ser Ser Asn Ser Ser Gln Asp Ser Leu His Lys
            20                  25

<210> 289
<211> 12
<212> PRT
<213> Homo sapiens

<220>
<221> MOD_RES
<222> (8)..(8)
<223> Phosphorylated Ser

<400> 289
Ser Leu Thr Thr Arg Glu Pro Ser Leu Ala Thr Trp
1               5                   10

<210> 290
<211> 10
<212> PRT
<213> Homo sapiens

<220>
<221> MOD_RES
<222> (5)..(5)
<223> Phosphorylated Thr

<400> 290
Pro Arg Ser Tyr Thr Met Asp Asp Ala Trp
1               5                   10

<210> 291
<211> 34
<212> PRT
<213> Homo sapiens

<220>
<221> MOD_RES
<222> (22)..(22)
<223> Phosphorylated Ser

<400> 291
Gly Pro Ser Gln Glu Glu Glu Asp Asn His Ser Asp Glu Glu Asp Arg
1               5                   10                  15

Ala Ser Glu Pro Lys Ser Gln Asp Gln Asp Ser Glu Val Asn Glu Leu
            20                  25                  30

Ser Arg

<210> 292
<211> 10
<212> PRT
<213> Homo sapiens

<220>
<221> MOD_RES
<222> (3)..(3)
<223> Phosphorylated Ser

<400> 292
Phe Ile Ser Gln Glu Leu Pro Ser Gln Arg
1               5                   10

<210> 293
<211> 24
<212> PRT
<213> Homo sapiens

<220>
<221> MOD_RES
<222> (8)..(8)
<223> Phosphorylated Ser

<400> 293
Gly Pro Val Pro Gly Asp Leu Ser Gln Thr Ser Glu Asp Gln Ser Leu
1               5                   10                  15

Ser Asp Phe Glu Ile Ser Asn Arg
            20

<210> 294
<211> 8
<212> PRT
<213> Homo sapiens

<220>
<221> MOD_RES
<222> (3)..(3)
<223> Phosphorylated Ser

<400> 294
Thr Phe Ser Gln Glu Val Ser Arg
1               5

<210> 295
<211> 19
<212> PRT
<213> Homo sapiens

<220>
<221> MOD_RES
<222> (6)..(6)
<223> Phosphorylated Thr

<400> 295
Thr Gln Arg Ser Ser Thr Asp Phe Ser Glu Leu Glu Gln Pro Arg Ser
1               5                   10                  15

Cys Leu Phe

<210> 296
<211> 19
<212> PRT
<213> Homo sapiens

<220>
<221> MOD_RES
<222> (12)..(12)
<223> Phosphorylated Thr

<400> 296
Thr Val Glu Val Ala Glu Gly Glu Ala Val Arg Thr Pro Gln Ser Val
1               5                   10                  15

Thr Ala Lys

<210> 297
<211> 28
<212> PRT
<213> Homo sapiens

<220>
<221> MOD_RES
<222> (16)..(16)
<223> Phosphorylated Ser

<400> 297
Ala Phe Pro Ser Gln Asn Gly Ser Ala Ala Val Gly Arg Trp Pro Ser
1               5                   10                  15

Leu Val Asp Arg Asn Thr Asp Asp Trp Glu Asn Phe
            20                  25

<210> 298
<211> 34
<212> PRT
<213> Homo sapiens

<220>
<221> MOD_RES
<222> (9)..(9)
<223> Phosphorylated Ser

<400> 298
Ala Gln Glu Thr Glu Ala Ala Pro Ser Gln Ala Pro Ala Asp Glu Pro
1               5                   10                  15

Glu Pro Glu Ser Ala Ala Ala Gln Ser Gln Glu Asn Gln Asp Thr Arg
            20                  25                  30

Pro Lys

<210> 299
<211> 20
<212> PRT
<213> Homo sapiens

<220>
<221> MOD_RES
<222> (15)..(15)
<223> Phosphorylated Ser

<400> 299
Met Leu Glu Asn Thr Asp Asn Ser Ser Pro Ser Thr Glu His Ser Gln
1               5                   10                  15

Gly Leu Glu Lys
            20

<210> 300
<211> 34
<212> PRT
<213> Homo sapiens

<220>
<221> MOD_RES
<222> (18)..(18)
<223> Phosphorylated Ser

<400> 300
Glu Gly Glu Ala Ala Ala Val Glu Gly Pro Cys Pro Ser Gln Glu Ser
1               5                   10                  15

Leu Ser Gln Glu Glu Asn Pro Glu Pro Thr Glu Asp Glu Arg Ser Glu
            20                  25                  30

Glu Lys

<210> 301
<211> 34
<212> PRT
<213> Homo sapiens

<220>
<221> MOD_RES
<222> (13)..(13)
<223> Phosphorylated Ser

<400> 301
Glu Gly Glu Ala Ala Ala Val Glu Gly Pro Cys Pro Ser Gln Glu Ser
1               5                   10                  15

Leu Ser Gln Glu Glu Asn Pro Glu Pro Thr Glu Asp Glu Arg Ser Glu
            20                  25                  30

Glu Lys

<210> 302
<211> 18
<212> PRT
<213> Homo sapiens

<220>
<221> MOD_RES
<222> (3)..(3)
<223> Phosphorylated Ser

<400> 302
Gln Ile Ser Gln Asp Phe Ser Glu Leu Ser Thr Gln Leu Thr Gly Val

1               5                   10                  15

Ala Arg

<210> 303
<211> 28
<212> PRT
<213> Homo sapiens

<220>
<221> MOD_RES
<222> (11)..(11)
<223> Phosphorylated Thr

<400> 303
Asn Lys Gln Ser Leu Lys Glu Arg Arg Asn Thr Arg Asp Glu Thr Asp
1               5                   10                  15

Thr Trp Ala Tyr Ile Ala Ala Glu Gly Asp Gln Glu
            20                  25

<210> 304
<211> 14
<212> PRT
<213> Homo sapiens

<220>
<221> MOD_RES
<222> (10)..(10)
<223> Phosphorylated Ser

<400> 304
Lys Glu Ile Leu Ile Glu Arg Ile Gln Ser Ile Lys Glu Glu
1               5                   10

<210> 305
<211> 22
<212> PRT
<213> Homo sapiens

<220>
<221> MOD_RES
<222> (10)..(10)
<223> Phosphorylated Thr

<400> 305
Ser Val Thr Ser Asn Gln Ser Asp Gly Thr Gln Glu Ser Cys Glu Ser
1               5                   10                  15

Pro Asp Val Leu Asp Arg
            20

<210> 306
<211> 25
<212> PRT
<213> Homo sapiens

<220>
<221> MOD_RES
<222> (4)..(4)
<223> Phosphorylated Ser

<400> 306
His Asn Tyr Ser Gln Asp Ala Ala Asp Ala Cys Asp Ile Asp Glu Ile
1               5                   10                  15

Glu Glu Val Pro Thr Thr Ser His Arg
            20                  25

<210> 307
<211> 24
<212> PRT
<213> Homo sapiens

<220>
<221> MOD_RES
<222> (6)..(6)
<223> Phosphorylated Ser

<400> 307
Glu Ile Glu Asp Leu Ser Gln Ser Gln Ser Pro Glu Ser Glu Thr Asp
1               5                   10                  15

Tyr Pro Val Ser Thr Asp Thr Arg
            20

<210> 308
<211> 28
<212> PRT
<213> Homo sapiens

<220>
<221> MOD_RES
<222> (7)..(7)
<223> Phosphorylated Thr

<400> 308
Val Ser Gly Thr Leu Asp Thr Pro Glu Lys Thr Val Asp Ser Gln Gly
1               5                   10                  15

Pro Thr Pro Val Cys Thr Pro Thr Phe Leu Glu Arg
            20                  25

<210> 309
<211> 22
<212> PRT
<213> Homo sapiens

<220>
<221> MOD_RES
<222> (10)..(10)
<223> Phosphorylated Thr

<400> 309
Ile Val Glu Asp Glu Glu Tyr Glu Thr Thr Gln Glu Tyr Glu Pro Ala
1               5                   10                  15

Gln Glu Pro Val Lys Lys
            20

<210> 310

<400> 310
000

<210> 311
<211> 26
<212> PRT
<213> Homo sapiens

<220>
<221> MOD_RES
<222> (19)..(19)
<223> Phosphorylated Thr

<400> 311
Asp Val Leu Gly Pro Ser Thr Val Val Ala Asn Ser Asp Glu Ser Gln
1               5                   10                  15

Leu Leu Thr Pro Gly Lys Met Ser Gln Arg
            20                  25

<210> 312
<211> 23
<212> PRT
<213> Homo sapiens

<220>
<221> MOD_RES
<222> (10)..(10)
<223> Phosphorylated Ser

<400> 312
Thr Gly Ile Arg Pro Ala Arg Val Val Ser Ser Thr Ser Glu Glu Glu
1               5                   10                  15

Glu Ala Phe Thr Glu Lys Phe
            20

<210> 313
<211> 20
<212> PRT
<213> Homo sapiens

<220>
<221> MOD_RES
<222> (8)..(8)
<223> Phosphorylated Ser

<400> 313
Ala Glu Glu Phe Val Val Leu Ser Gln Glu Pro Ser Val Thr Glu Thr
1               5                   10                  15

Ile Ala Pro Lys
            20

<210> 314
<211> 23
<212> PRT
<213> Homo sapiens

<220>
<221> MOD_RES
<222> (4)..(4)
<223> Phosphorylated Ser

<400> 314
Lys Arg Arg Ser Thr Gly Val Val Asn Ile Pro Ala Ala Glu Cys Leu
1               5                   10                  15

Asp Glu Tyr Glu Asp Asp Glu
            20

<210> 315
<211> 19

<212> PRT
<213> Homo sapiens

<220>
<221> MOD_RES
<222> (16)..(16)
<223> Phosphorylated Ser

<400> 315
Asn Ser Ala Asp Asp Glu Glu Leu Thr Asn Asp Ser Leu Thr Leu Ser
1               5                   10                  15

Gln Ser Lys

<210> 316
<211> 22
<212> PRT
<213> Homo sapiens

<220>
<221> MOD_RES
<222> (16)..(16)
<223> Phosphorylated Ser

<400> 316
Val Ala Ser Ile Glu Thr Gly Leu Ala Ala Ala Ala Ala Lys Leu Ser
1               5                   10                  15

Gln Gln Glu Glu Gln Lys
            20

<210> 317
<211> 18
<212> PRT
<213> Homo sapiens

<220>
<221> MOD_RES
<222> (11)..(11)
<223> Phosphorylated Thr

<400> 317
Gly Lys Val Glu Ile Asp Gln Gln Gln Leu Thr Gln Gln Gln Leu Asn
1               5                   10                  15

Gly Asn

<210> 318
<211> 18
<212> PRT
<213> Homo sapiens

<220>
<221> MOD_RES
<222> (5)..(5)
<223> Phosphorylated Ser

<400> 318
Gln Arg Leu Ser Ser Gly Phe Asp Asp Ile Asp Leu Pro Ser Ala Val
1               5                   10                  15

Lys Tyr

<210> 319
<211> 12
<212> PRT
<213> Homo sapiens


<220>
<221> MOD_RES
<222> (7)..(7)
<223> Phosphorylated Ser

<400> 319
Ala Gly Pro Glu Gly Thr Ser Gln Glu Ile Pro Lys
1               5                   10


<210> 320
<211> 19
<212> PRT
<213> Homo sapiens


<220>
<221> MOD_RES
<222> (7)..(7)
<223> Phosphorylated Thr

<400> 320
Gly Cys Gln Arg Ser Ser Thr Ser Asp Asp Asp Ser Gly Cys Ala Leu
1               5                   10                  15


Glu Glu Tyr



<210> 321
<211> 29
<212> PRT
<213> Homo sapiens


<220>
<221> MOD_RES
<222> (10)..(10)
<223> Phosphorylated Ser

<400> 321
Tyr Gln Ser Asp Met Tyr Trp Ala Gln Ser Gln Asp Gly Leu Gly Asp
1               5                   10                  15


Ser Ala Tyr Gly Ser His Pro Gly Pro Ala Ser Ser Arg
            20                  25


<210> 322
<211> 38
<212> PRT
<213> Homo sapiens


<220>
<221> MOD_RES
<222> (10)..(10)
<223> Phosphorylated Ser

<400> 322
Ala Ser Asn Ser Leu Ile Ser Gly Leu Ser Gln Asp Glu Glu Asp Pro
1               5                   10                  15


Pro Leu Pro Pro Thr Pro Met Asn Ser Leu Val Asp Glu Cys Pro Leu
            20                  25                  30

Asp Gln Gly Leu Pro Lys
        35

<210> 323
<211> 23
<212> PRT
<213> Homo sapiens

<220>
<221> MOD_RES
<222> (10)..(10)
<223> Phosphorylated Thr

<400> 323
Arg Ala Gly Ala Gly Gln Arg Pro Leu Thr Pro Asn Gln Gly Gln Gln
1               5                   10                  15

Gly Gln Gln Ala Glu Ser Leu
            20

<210> 324
<211> 20
<212> PRT
<213> Homo sapiens

<220>
<221> MOD_RES
<222> (18)..(18)
<223> Phosphorylated Thr

<400> 324
Ser Glu Tyr Ala Ala Asp Pro Trp Val Gln Asp Gln Met Gln Arg Lys
1               5                   10                  15

Leu Thr Leu Glu
            20

<210> 325
<211> 18
<212> PRT
<213> Homo sapiens

<220>
<221> MOD_RES
<222> (16)..(16)
<223> Phosphorylated Ser

<400> 325
Asp Leu Ser Asn Pro Phe Gly Gln Met Ser Leu Ser Arg Gln Gly Ser
1               5                   10                  15

Thr Glu

<210> 326
<211> 28
<212> PRT
<213> Homo sapiens

<220>
<221> MOD_RES
<222> (7)..(7)
<223> Phosphorylated Ser

<400> 326
Met Leu Ser Arg Asp Ser Ser Gln Glu Tyr Thr Asp Ser Thr Gly Ile

1               5                   10                  15

Asp Leu His Glu Phe Leu Val Asn Thr Leu Lys Lys
            20                  25

<210> 327
<211> 18
<212> PRT
<213> Homo sapiens

<220>
<221> MOD_RES
<222> (12)..(12)
<223> Phosphorylated Ser

<400> 327
Lys Asp Thr Ala Ala Val Val Ser Arg Gln Gly Ser Ser Leu Asn Leu
1               5                   10                  15

Phe Glu

<210> 328
<211> 26
<212> PRT
<213> Homo sapiens

<220>
<221> MOD_RES
<222> (23)..(23)
<223> Phosphorylated Ser

<400> 328
Ala Cys Asp Gly Ser Asp Asp Ile Phe Gly Leu Ser Thr Asp Ser Leu
1               5                   10                  15

Ser Arg Leu Arg Ser Pro Ser Val Leu Glu
            20                  25

<210> 329
<211> 17
<212> PRT
<213> Homo sapiens

<220>
<221> MOD_RES
<222> (6)..(6)
<223> Phosphorylated Ser

<400> 329
Ile Arg Arg Gln Ser Ser Ser Tyr Asp Asp Pro Trp Lys Ile Thr Asp
1               5                   10                  15

Glu

<210> 330
<211> 26
<212> PRT
<213> Homo sapiens

<220>
<221> MOD_RES
<222> (17)..(17)
<223> Phosphorylated Ser

<400> 330
Tyr Gln Asp Glu Tyr Gly Val Ser Ser Ile Ile Pro Asn Phe Gln Ile
1               5                   10                  15

Ser Gln Asp Ser Val Gly Asn Ser Asn Arg
            20                  25

<210> 331
<211> 14
<212> PRT
<213> Homo sapiens

<220>
<221> MOD_RES
<222> (8)..(8)
<223> Phosphorylated Thr

<400> 331
Ser Ala Ser Ala Ser Pro Leu Thr Pro Cys Ser Val Thr Arg
1               5                   10

<210> 332
<211> 14
<212> PRT
<213> Homo sapiens

<220>
<221> MOD_RES
<222> (4)..(4)
<223> Phosphorylated Ser

<400> 332
Arg Tyr Ala Ser Asn Val Cys Phe Thr Thr Leu Gly Thr Glu
1               5                   10

<210> 333
<211> 13
<212> PRT
<213> Homo sapiens

<220>
<221> MOD_RES
<222> (12)..(12)
<223> Phosphorylated Thr

<400> 333
His Ala Leu Gly Asp Gly Val Asp Arg Thr Ser Thr Ile
1               5                   10

<210> 334
<211> 20
<212> PRT
<213> Homo sapiens

<220>
<221> MOD_RES
<222> (7)..(7)
<223> Phosphorylated Ser

<400> 334
Thr Lys Glu Arg Gln Pro Ser Ile Asp Leu Thr Cys Val Ser Tyr Pro
1               5                   10                  15

Phe Asp Glu Phe
            20

<210> 335
<211> 15
<212> PRT
<213> Homo sapiens


<220>
<221> MOD_RES
<222> (5)..(5)
<223> Phosphorylated Ser

<400> 335
His Arg Ala Ala Ser Ser Ala Ala Gln Gly Ala Phe Gln Gly Asn
1               5                   10                  15


<210> 336
<211> 25
<212> PRT
<213> Homo sapiens


<220>
<221> MOD_RES
<222> (13)..(13)
<223> Phosphorylated Ser

<400> 336
Leu Asn Pro Ser Thr Gly Asn Arg Ser Arg Glu Gln Ser Ser Glu Ala
1               5                   10                  15


Ala Glu Thr Gly Val Ser Glu Asn Glu
            20                  25


<210> 337
<211> 17
<212> PRT
<213> Homo sapiens


<220>
<221> MOD_RES
<222> (12)..(12)
<223> Phosphorylated Ser

<400> 337
Ile Thr Asn Glu Gln Glu Glu Thr Tyr Ser Leu Ser Gln Ser Ser Gly
1               5                   10                  15


Lys



<210> 338
<211> 23
<212> PRT
<213> Homo sapiens


<220>
<221> MOD_RES
<222> (18)..(18)
<223> Phosphorylated Thr

<400> 338
Arg Pro Pro Thr Pro Ala Pro Trp Pro Ser Ser Gly Leu Gly Thr Pro
1               5                   10                  15


Leu Thr Pro Pro Lys Met Lys
            20

<210> 339
<211> 19
<212> PRT
<213> Homo sapiens

<220>
<221> MOD_RES
<222> (5)..(5)
<223> Phosphorylated Thr

<400> 339
Ser Arg His Ser Thr Ile Leu Asp Tyr Ile Asn Val Val Pro Thr Ala
1               5                   10                  15

Gly Pro Leu

<210> 340
<211> 19
<212> PRT
<213> Homo sapiens

<220>
<221> MOD_RES
<222> (11)..(11)
<223> Phosphorylated Thr

<400> 340
Gly Gln Thr Thr Leu Pro Phe Arg Pro Val Thr Gln Glu Glu Asp Asp
1               5                   10                  15

Asp Gln Arg

<210> 341
<211> 16
<212> PRT
<213> Homo sapiens

<220>
<221> MOD_RES
<222> (5)..(5)
<223> Phosphorylated Ser

<400> 341
Thr Arg Asn Val Ser Thr Gly Ser Ala Glu Asn Glu Glu Lys Ser Glu
1               5                   10                  15

<210> 342
<211> 10
<212> PRT
<213> Homo sapiens

<220>
<221> MOD_RES
<222> (6)..(6)
<223> Phosphorylated Ser

<400> 342
Gly Ser Leu Gly Ile Ser Gln Glu Glu Gln
1               5                   10

<210> 343
<211> 11
<212> PRT
<213> Homo sapiens

<220>
<221> MOD_RES
<222> (6)..(6)
<223> Phosphorylated Ser

<400> 343
Leu Gln Arg Ala Asn Ser His Phe Ile Glu Glu
1               5                   10


<210> 344
<211> 32
<212> PRT
<213> Homo sapiens


<220>
<221> MOD_RES
<222> (24)..(24)
<223> Phosphorylated Ser

<400> 344
Asn Ser Gln Val Gly Ser Asp Asn Ser Ser Ile Leu Leu Phe Asp Ser
1               5                   10                  15


Thr Gln Glu Ser Leu Pro Pro Ser Gln Asp Ile Pro Ala Ile Phe Arg
            20                  25                  30


<210> 345
<211> 31
<212> PRT
<213> Homo sapiens


<220>
<221> MOD_RES
<222> (21)..(21)
<223> Phosphorylated Ser

<400> 345
Ser Gln Ser Leu Thr Asn Ala Phe Asn Leu Pro Glu Pro Ala Pro Pro
1               5                   10                  15


Arg Pro Ser Leu Ser Gln Asp Glu Val Lys Ala Glu Thr Ile Arg
            20                  25                  30


<210> 346
<211> 27
<212> PRT
<213> Homo sapiens


<220>
<221> MOD_RES
<222> (5)..(5)
<223> Phosphorylated Ser

<400> 346
Gln Ser Ser Tyr Ser Gln Gln Pro Tyr Asn Asn Gln Gly Gln Gln Gln
1               5                   10                  15


Asn Met Glu Ser Ser Gly Ser Gln Gly Gly Arg
            20                  25


<210> 347
<211> 15
<212> PRT
<213> Homo sapiens

<220>
<221> MOD_RES
<222> (14)..(14)
<223> Phosphorylated Thr

<400> 347
Leu Asp Ala Gln Leu Asp Ala Tyr Asn Ala Arg Met Asp Thr Ser
1               5                   10                  15


<210> 348
<211> 22
<212> PRT
<213> Homo sapiens


<220>
<221> MOD_RES
<222> (11)..(11)
<223> Phosphorylated Thr

<400> 348
Asn Val Pro Pro Pro Ser Ile Glu Pro Leu Thr Pro Pro Pro Gln Tyr
1               5                   10                  15


Asp Glu Val Gln Glu Lys
            20


<210> 349
<211> 14
<212> PRT
<213> Homo sapiens


<220>
<221> MOD_RES
<222> (6)..(6)
<223> Phosphorylated Thr

<400> 349
His Leu Arg Asn Lys Thr Glu Asp Leu Glu Ala Thr Ser Glu
1               5                   10


<210> 350
<211> 11
<212> PRT
<213> Homo sapiens


<220>
<221> MOD_RES
<222> (8)..(8)
<223> Phosphorylated Ser

<400> 350
Ser Ile Leu Ser Arg Gln Glu Ser Ser Phe Phe
1               5                   10


<210> 351
<211> 22
<212> PRT
<213> Homo sapiens


<220>
<221> MOD_RES
<222> (14)..(14)
<223> Phosphorylated Ser

<400> 351
Val Lys Thr Glu Pro Glu Thr Pro Gly Pro Ser Cys Leu Ser Gln Glu
1               5                   10                  15

Gly Gln Thr Ala Val Lys
            20

<210> 352
<211> 20
<212> PRT
<213> Homo sapiens

<220>
<221> MOD_RES
<222> (6)..(6)
<223> Phosphorylated Ser

<400> 352
Leu Pro Gln Ser Leu Ser Gln Glu Gly Asp Phe Thr Pro Ala Ala Met
1               5                   10                  15

Gly Leu Leu Arg
            20

<210> 353
<211> 23
<212> PRT
<213> Homo sapiens

<220>
<221> MOD_RES
<222> (21)..(21)
<223> Phosphorylated Ser

<400> 353
Ser Ser Gln Gly Ser Ser Ser Val Ser Ser Asp Ala Pro Gly Asn Val
1               5                   10                  15

Leu Cys Ala Leu Ser Gln Lys
            20

<210> 354
<211> 9
<212> PRT
<213> Homo sapiens

<220>
<221> MOD_RES
<222> (7)..(7)
<223> Phosphorylated Thr

<400> 354
Ala Ala Phe Val Gln Leu Thr Gln Lys
1               5

<210> 355
<211> 17
<212> PRT
<213> Homo sapiens

<220>
<221> MOD_RES
<222> (11)..(11)
<223> Phosphorylated Thr

<400> 355
Glu Ser Ser Lys Glu Leu Glu Ser Pro Leu Thr Pro Gly Lys Val Cys
1               5                   10                  15

Arg

<210> 356
<211> 23
<212> PRT
<213> Homo sapiens

<220>
<221> MOD_RES
<222> (9)..(9)
<223> Phosphorylated Ser

<400> 356
Ser Lys Pro Asp Ala Ser Ser Ile Ser Gln Glu Glu Pro Gln Gly Glu
1               5                   10                  15

Ala Thr Pro Val Gly Asn Arg
            20